# EUROPEAN PATENT APPLICATION

(11) **EP 4 825 860 A1**
(43) Date of publication of application: **21.10.2026**
(21) Application number: 25793993.4
(22) Date of filing: 23.04.2025
(51) Int. Cl.: C07D 513/04, A61K 31/429, A61P 35/00

(54) **FUSED RING NITROGEN-CONTAINING COMPOUND, CRYSTAL FORM THEREOF, PREPARATION METHOD THEREFOR, AND USE THEREOF**

(30) Priority: 24.04.2024 CN 202410500685
(71) Applicant: Hangzhou Synrx Therapeutics Biomedical Technology Co., Ltd., Hangzhou, Zhejiang 311121 (CN)
(72) Inventor: HE, Hu, Hangzhou, Zhejiang 311121 (CN); KAN, Chao, Hangzhou, Zhejiang 311121 (CN); GE, Chongxun, Hangzhou, Zhejiang 311121 (CN); LIU, Song, Hangzhou, Zhejiang 311121 (CN)
(74) Representative: Barker Brettell LLP
(86) International application number: PCT/CN2025/090761
(87) International publication number: WO 2025/223483

(57) **Abstract**

The present invention provides a fused ring nitrogen-containing compound, a crystal form thereof, a preparation method therefor, and the use thereof. Specifically provided is a solvate of a compound represented by formula (I), a salt of the compound represented by formula (I), or a solvate of the salt of the compound represented by formula (I). The fused ring nitrogen-containing compound and the crystal form thereof provided by the present invention have good pharmaceutical activity andl wide medicinal prospects.

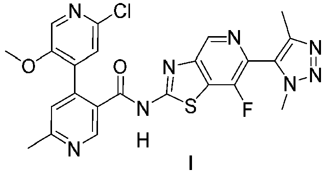

## Description

This application claims priority to Chinese Patent Application No. 202410500685X, filed on April 24, 2024. This application incorporates the entire content of the aforementioned Chinese patent application by reference.

### TECHNICAL FIELD

The present invention relates to a nitrogen-containing fused-ring compound, crystalline forms thereof, and preparation method therefor, and application thereof.

### BACKGROUND

DNA damage is an important mechanism by which many chemotherapeutic drugs exert their antitumor effects. DNA double-strand breaks (DSBs) are one of the most common types of damage in cells, which can be directly caused by ionizing radiation, or induced by ultraviolet light, reactive oxygen species (ROS), or other mutagens, such as common chemotherapeutic drugs like cisplatin, 5-FU, etoposide, etc. Unrepaired DNA damage can lead to functional blocks in cell transcription and replication, induce apoptosis or necrosis, and result in clearance by immune cells. Tumor cells possess efficient damage repair or alternative repair pathways. Targeting key proteins in DNA damage repair pathways, leading to the accumulation of unrepaired DNA damage and ultimately cell death, is one of the important strategies in cancer treatment.

DNA double-strand breaks are typically repaired by three pathways: non-homologous end joining (NHEJ), homologous recombination (HR), and alternative non-homologous end joining (Alt-NHEJ, also known as microhomology-mediated end joining, MMEJ, or TMEJ). Among these, the HR repair pathway only occurs during the G2 and S phases, achieving error-free repair under conditions where sister chromatids are present. In mammals, over 90% of DSB damage is repaired via the NHEJ pathway. It is an error-prone repair, resulting in deletion mutations of <30 bp, insertion mutations of <5 bp, or microhomologous sequences of <2 bp. Recent studies indicate that when HR and/or NHEJ are deficient, cells become highly dependent on the third pathway, MMEJ, to repair broken DNA. Inhibiting MMEJ leads to cell apoptosis. MMEJ is also an error-prone repair pathway; it relies on DNA polymerase theta to join microhomologous end sequences (theta-mediated end joining, TMEJ) to complete the repair of DNA double-strand breaks.

DNA polymerase theta (Pol theta or Pol θ) is a key protein in the MMEJ repair pathway. It is a unique multifunctional polymerase composed of an N-terminal helicase domain, a central domain, and a C-terminal polymerase domain. Basic research has found that the polymerase domain is essential for DNA extension at DSB damage repair sites, while the helicase domain and the central domain play key roles in the recognition and binding of Pol θ to substrates. Through Pol θ, the interaction between DNA and damage repair complexes (such as competitive binding to single-stranded DNA with RAD51) can be disrupted, thereby inhibiting the HR repair pathway. Furthermore, the helicase domain of Pol θ is also involved in DNA replication arrest; its functional loss will lead to increased replication stress in tumor cells, resulting in apoptosis.

Pol θ is not expressed or is expressed at low levels in normal tissue cells, but it is highly expressed in various tumors such as lung cancer, breast cancer, HR-deficient ovarian cancer, gastric cancer, and colon cancer, and is associated with poorer prognosis. Among these, Pol θ is overexpressed in over 70% of breast cancers. These phenomena indicate that Pol θ may play an important role in these cancers and is a potential tumor-specific target.

Studies involving knockdown or knockout of Pol θ in tumor cells have found that Pol θ deficiency can sensitize these cells to radiation, induce DSB generation, enhance replication fork instability, make tumor cells sensitive to genotoxic agents, potentially enhancing the effects of radiotherapy and chemotherapy, and is a potential drug target. Studies have also found that Pol θ exhibits synthetic lethality with HR deficiency, and its small molecule inhibitors can kill HR-deficient tumor cells both in vitro and in vivo. Particularly in PARP inhibitor (Olaparib, etc.) resistant tumors with HR deficiency reversion mutations, Pol θ inhibitors can resensitize cells to PARPi, providing valuable therapeutic opportunities. Additionally, given that Pol θ is a key protein in the MMEJ pathway, its functional impairment can also lead to increased genomic instability in cancer cells, increase somatic mutations, and facilitate the generation of tumor neoantigens. Furthermore, it has been reported that Pol θ is involved in cGAS-STING-mediated immune activation; therefore, targeting Pol θ also has the potential to enhance immunotherapy.

In summary, Pol θ is a highly promising target for cancer therapy. Designing ATPase activity inhibitors targeting the Pol θ protein to inhibit intracellular MMEJ, used alone or in combination with other chemotherapy, radiotherapy, antibody therapy, or immunotherapy, aims to kill tumor cells and holds great potential in the treatment of tumors such as lung cancer, breast cancer, HR-deficient ovarian cancer, gastric cancer, colon cancer, prostate cancer, and pancreatic cancer.

### SUMMARY OF THE INVENTION

The technical problem to be solved by the present invention is to overcome the single solid form of polymerase theta inhibitor compounds in the prior art. For this purpose, the present invention provides a nitrogen-containing fused-ring compound, crystalline forms thereof, and preparation method therefor, and application thereof. The nitrogen-containing fused-ring compound and crystalline forms thereof provided by the present invention show significant improvement in ATPase activity and protein inhibitory effects compared to the prior art.

The present invention solves the above technical problem through the following technical solutions.

The present invention provides a compound of formula II,
wherein the structure of the compound of formula II is: 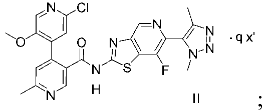
x' (solvent) is water or acetone;
q (molar equivalent of solvent) is 0.01-2.5.

Preferably, in the compound of formula II, q (molar equivalent of solvent) is 0.1, 0.5, 0.56, 0.6, 1, 1.5, 2, or 2.5.

In one embodiment, the compound of formula II is crystalline form A of the compound of formula II, and its X-ray powder diffraction pattern using Cu-Kα radiation, expressed in terms of 2θ angle, has diffraction peaks at 9.00±0.2°, 10.84±0.2°, 17.91±0.2°, and 22.05±0.2°.

In one embodiment, crystalline form A of the compound of formula II has its X-ray powder diffraction pattern using Cu-Kα radiation, expressed in terms of 2θ angle, further having diffraction peaks at one or more of the following positions: 5.98±0.2°, 11.94±0.2°, 18.36±0.2°, 21.30±0.2°, 23.29±0.2°, and 27.72±0.2°.

In one embodiment, crystalline form A of the compound of formula II has its X-ray powder diffraction pattern using Cu-Kα radiation, expressed in terms of 2θ angle, having diffraction peaks at 9.00±0.2°, 10.84±0.2°, 17.91±0.2°, 22.05±0.2°, 5.98±0.2°, 11.94±0.2°, 18.36±0.2°, 21.30±0.2°, and 23.29±0.2°.

In one embodiment, crystalline form A of the compound of formula II has its X-ray powder diffraction pattern using Cu-Kα radiation, expressed in terms of 2θ angle, further having diffraction peaks at one or more of the following positions: 13.46±0.2°, 14.16±0.2°, 14.72±0.2°, 19.99±0.2°, 20.65±0.2°, 24.6±0.2°, 25.62±0.2°, 28.96±0.2°, 29.68±0.2°, 30.29±0.2°, and 31.08±0.2°.

In one embodiment, crystalline form A of the compound of formula II has its X-ray powder diffraction pattern using Cu-Kα radiation, expressed in terms of 2θ angle, having diffraction peaks as shown in Table 1.

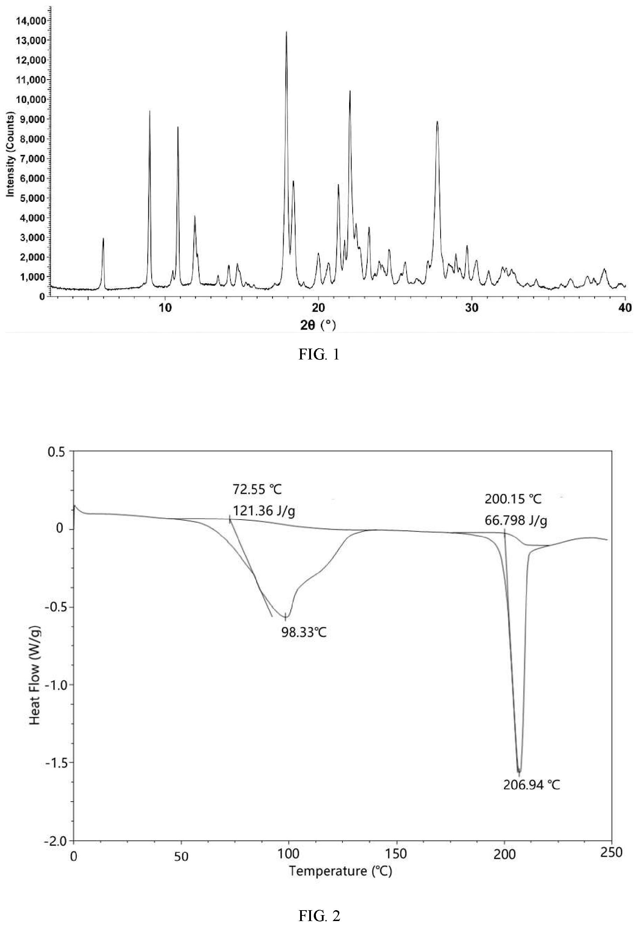

In one embodiment, crystalline form A of the compound of formula II has its X-ray powder diffraction pattern using Cu-Kα radiation substantially as shown in FIG. 1.

In one embodiment, crystalline form A of the compound of formula II has its differential scanning calorimetry (DSC) curve showing endothermic peaks at peak temperatures of 98.33±3°C and 206.94±3°C.

In one embodiment, the differential scanning calorimetry (DSC) curve of crystalline form A of the compound of formula II is substantially as shown in FIG. 2.

In one embodiment, crystalline form A of the compound of formula II has its thermogravimetric analysis (TGA) curve showing a weight loss of about 0.48% in the temperature range from 34.7±3°C to 70.0±3°C, a weight loss of about 2.11% in the temperature range from 70.0±3°C to 95.0±3C, a weight loss of about 1.38% in the temperature range from 95.0±3°C to 140.0±3°C, and a weight loss of about 0.34% in the temperature range from 140.0±3°C to 220.0±3°C.

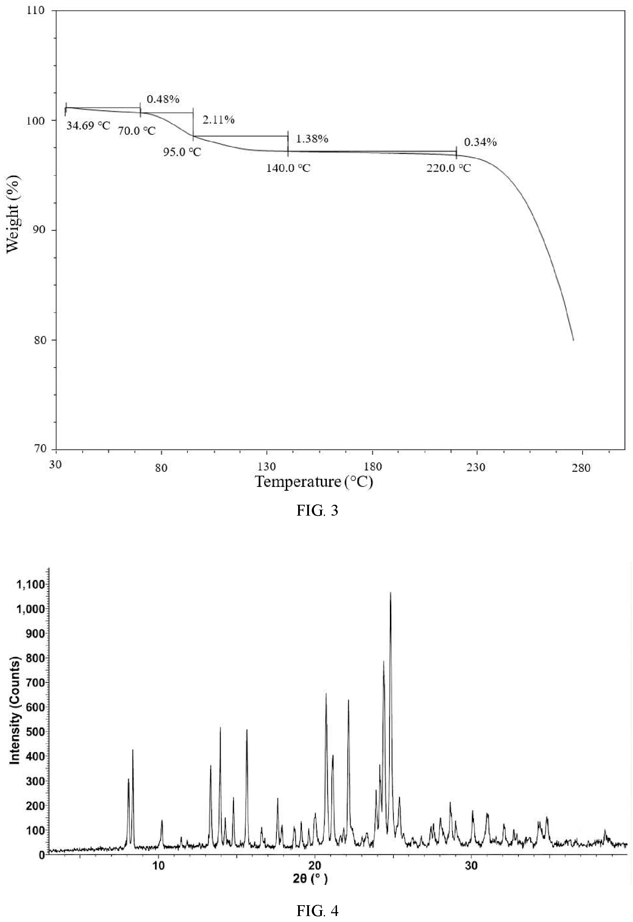

In one embodiment, the thermogravimetric analysis (TGA) curve of crystalline form A of the compound of formula II is substantially as shown in FIG. 3.

In one embodiment, in crystalline form A of the compound of formula II, x' is water, and q is 1. In one embodiment, the compound of formula II is crystalline form B of the compound of formula II, which has its X-ray powder diffraction pattern using Cu-Kα radiation, expressed in terms of 2θ angle, having diffraction peaks at 8.10±0.2°, 8.37±0.2°, 13.94±0.2°, 15.67±0.2°, 20.72±0.2°, 24.4±0.2°, and 24.83±0.2°.

In one embodiment, crystalline form B of the compound of formula II has its X-ray powder diffraction pattern using Cu-Kα radiation, expressed in terms of 2θ angle, further having diffraction peaks at one or more of the following positions: 13.33±0.2°, 14.79±0.2°, 17.63±0.2°, and 21.15±0.2°.

In one embodiment, crystalline form B of the compound of formula II has its X-ray powder diffraction pattern using Cu-Kα radiation, expressed in terms of 2θ angle, having diffraction peaks at 8.10±0.2°, 8.37±0.2°, 13.94±0.2°, 15.67±0.2°, 20.72±0.2°, 14.26±0.2°, 16.61±0.2°, 17.91±0.2°, and 18.70±0.2°.

In one embodiment, crystalline form B of the compound of formula II has its X-ray powder diffraction pattern using Cu-Kα radiation, expressed in terms of 2θ angle, further having diffraction peaks at one or more of the following positions: 14.26±0.2°, 16.61±0.2°, 17.91±0.2°, 18.70±0.2°, 19.13±0.2°, 19.62±0.2°, 20.03±0.2°, 23.92±0.2°, 24.20±0.2°, and 25.42±0.2°.

In one embodiment, crystalline form B of the compound of formula II has its X-ray powder diffraction pattern using Cu-Kα radiation, expressed in terms of 2θ angle, having diffraction peaks as shown in Table 2.

In one embodiment, crystalline form B of the compound of formula II has its X-ray powder diffraction pattern using Cu-Kα radiation substantially as shown in FIG. 4.

In one embodiment, in crystalline form B of the compound of formula II, x' is acetone, and q is 0.56, 0.5, or 0.6, for example, q is 0.56.

The present invention provides crystalline form C of a compound of formula I, which has its X-ray powder diffraction pattern using Cu-Kα radiation, expressed in terms of 2θ angle, having diffraction peaks at 11.51±0.2°, 18.01±0.2°, 22.56±0.2°, and 25.68±0.2°;

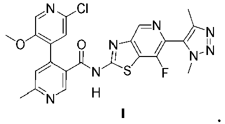

In one embodiment, crystalline form C of the compound of formula I has its X-ray powder diffraction pattern using Cu-Kα radiation, expressed in terms of 2θ angle, further having diffraction peaks at one or more of the following positions: 12.88±0.2°, 15.05±0.2°, 20.85±0.2°, 21.68±0.2°, 23.11±0.2°, and 24.79±0.2°.

Preferably, in crystalline form C of the compound of formula I, the diffraction peak at 11.51±0.2° in its X-ray powder diffraction pattern using Cu-Kα radiation, expressed in terms of 2θ angle, is the strongest peak.

In one embodiment, crystalline form C of the compound of formula I has its X-ray powder diffraction pattern using Cu-Kα radiation, expressed in terms of 2θ angle, having diffraction peaks at 11.51±0.2°, 18.01±0.2°, 22.56±0.2°, 25.68±0.2°, 12.88±0.2°, 15.05±0.2°, 20.85±0.2°, 21.68±0.2°, and 23.11±0.2°.

In one embodiment, crystalline form C of the compound of formula I has its X-ray powder diffraction pattern using Cu-Kα radiation, expressed in terms of 2θ angle, further having diffraction peaks at one or more of the following positions: 9.022±0.2°, 10.212±0.2°, 16.163±0.2°, 16.96±0.2°, 17.22±0.2°, 26.937±0.2°, 29.445±0.2°, 30.137±0.2°, and 30.354±0.2°.

In one embodiment, crystalline form C of the compound of formula I has its X-ray powder diffraction pattern using Cu-Kα radiation, expressed in terms of 2θ angle, having diffraction peaks as shown in Table 3.

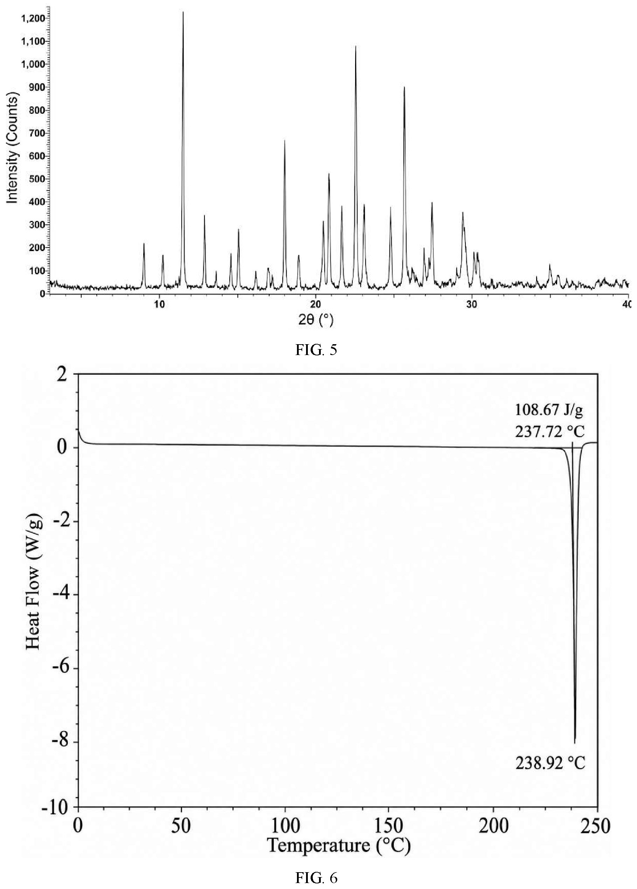

In one embodiment, crystalline form C of the compound of formula I has its X-ray powder diffraction pattern using Cu-Kα radiation substantially as shown in FIG. 5.

In one embodiment, crystalline form C of the compound of formula I has its differential scanning calorimetry (DSC) curve exhibiting an endothermic peak at a peak temperature of 238.92±3°C.

In one embodiment, the differential scanning calorimetry (DSC) curve of crystalline form C of the compound of formula I is substantially as shown in FIG. 6.

In one embodiment, crystalline form C of the compound of formula I has its thermogravimetric analysis (TGA) curve showing a weight loss of about 0.81% over the temperature range from 35.56±3°C to 230.00±3°C.

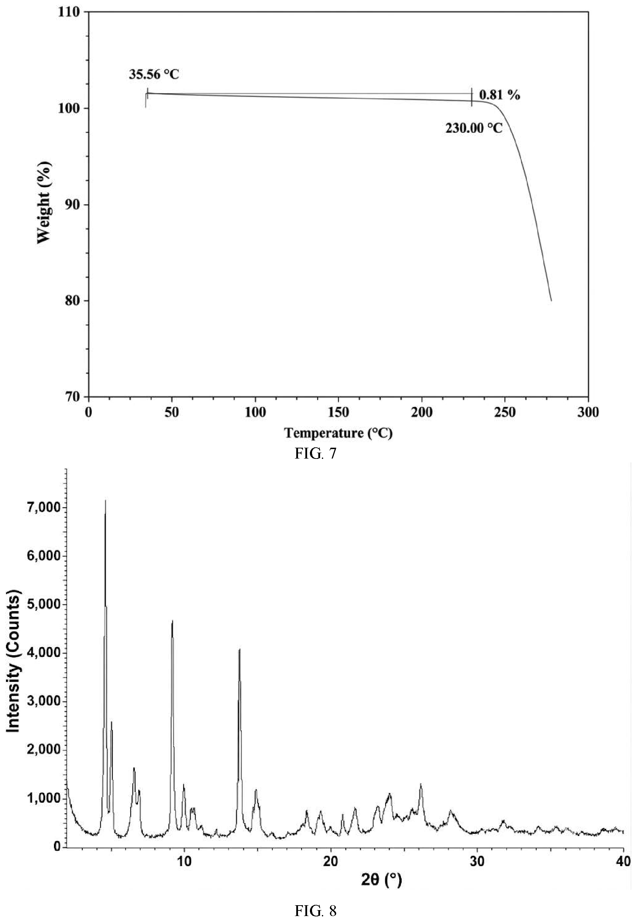

In one embodiment, the thermogravimetric analysis (TGA) curve of crystalline form C of the compound of formula I is substantially as shown in FIG. 7.

In one embodiment, crystalline form C of the compound of formula I is solvent-free.

The present invention provides a compound of formula III, wherein the structure of the compound of formula III is: 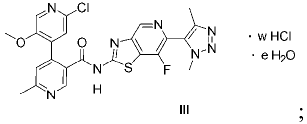 wherein w is 0.5-2.5; e is 0-2.

In one embodiment, in the compound of formula III, w (molar equivalents of hydrochloric acid) is 0.5, 1, 1.1, 1.6, 1.7, 1.8, 1.76, 1.06, 1.5, or 2.

In one embodiment, in the compound of formula III, e is 0 (solvent-free).

In one embodiment, in the compound of formula III, e (molar equivalents of water) is 1-2, for example, e is 1.5.

In one embodiment, the compound of formula III is crystalline form A of the compound of formula III, and its X-ray powder diffraction pattern obtained using Cu-Kα radiation, expressed in terms of 2θ angles, has diffraction peaks at 4.61±0.2°, 5.02±0.2°, 9.19±0.2°, and 13.76±0.2°. In one embodiment, crystalline form A of the compound of formula III has its X-ray powder diffraction pattern obtained using Cu-Kα radiation, expressed in terms of 2θ angles, further having diffraction peaks at one or more of the following positions: 6.57±0.2°, 6.89±0.2°, 9.96±0.2°, 10.48±0.2°, 10.64±0.2°, 14.69±0.2°, and 14.90±0.2°.

In one embodiment, crystalline form A of the compound of formula III has its X-ray powder diffraction pattern obtained using Cu-Kα radiation, expressed in terms of 2θ angles, having diffraction peaks at 4.61±0.2°, 5.02±0.2°, 9.19±0.2°, 13.76±0.2°, 6.57±0.2°, 6.89±0.2°, 9.96±0.2°, 10.48±0.2°, and 10.64±0.2°.

In one embodiment, crystalline form A of the compound of formula III has its X-ray powder diffraction pattern obtained using Cu-Kα radiation, expressed in terms of 2θ angles, further having diffraction peaks at one or more of the following positions: 18.34±0.2°, 19.13±0.2°, 19.30±0.2°, 20.81±0.2°, 21.68±0.2°, 23.09±0.2°, 24.03±0.2°, 26.13±0.2°, and 28.17±0.2°.

In one embodiment, crystalline form A of the compound of formula III has its X-ray powder diffraction pattern obtained using Cu-Kα radiation, expressed in terms of 2θ angles, having diffraction peaks as shown in Table 4.

In one embodiment, the X-ray powder diffraction pattern of crystalline form A of the compound of formula III, obtained using Cu-Kα radiation, is substantially as shown in FIG. 8. In one embodiment, in crystalline form A of the compound of formula III, w (molar equivalents of hydrochloric acid) is 1, 1.1, or 1.06, for example 1.06.

In one embodiment, in crystalline form A of the compound of formula III, e is 0 or 1.5. Preferably, e is 0. In one embodiment, the compound of formula III is crystalline form B of the compound of formula III, and its X-ray powder diffraction pattern obtained using Cu-Kα radiation, expressed in terms of 2θ angles, has diffraction peaks at 6.47±0.2°, 7.19±0.2°, 9.11±0.2°, 11.63±0.2°, and 14.45±0.2°.

In one embodiment, crystalline form B of the compound of formula III has its X-ray powder diffraction pattern obtained using Cu-Kα radiation, expressed in terms of 2θ angles, further having diffraction peaks at one or more of the following positions: 17.37±0.2°, 18.21±0.2°, 19.44±0.2°, 20.17±0.2°, 20.82±0.2°, 22.41±0.2°, and 24.58±0.2°.

In one embodiment, crystalline form B of the compound of formula III has its X-ray powder diffraction pattern obtained using Cu-Kα radiation, expressed in terms of 2θ angles, having diffraction peaks at 6.47±0.2°, 7.19±0.2°, 9.11±0.2°, 11.63±0.2°, 14.45±0.2°, 17.37±0.2°, 18.21±0.2°, 19.44±0.2°, 20.17±0.2°, and 20.82±0.2°.

In one embodiment, crystalline form B of the compound of formula III has its X-ray powder diffraction pattern obtained using Cu-Kα radiation, expressed in terms of 2θ angles, further having diffraction peaks at one or more of the following positions: 12.94±0.2°, 13.41±0.2°, 15.41±0.2°, 15.75±0.2°, 16.23±0.2°, 16.72±0.2°, 26.01±0.2°, 26.17±0.2°, 26.47±0.2°, 27.96±0.2°, 28.62±0.2°, 30.04±0.2°, and 30.37±0.2°.

In one embodiment, crystalline form B of the compound of formula III has its X-ray powder diffraction pattern obtained using Cu-Kα radiation, expressed in terms of 2θ angles, having diffraction peaks as shown in Table 5.

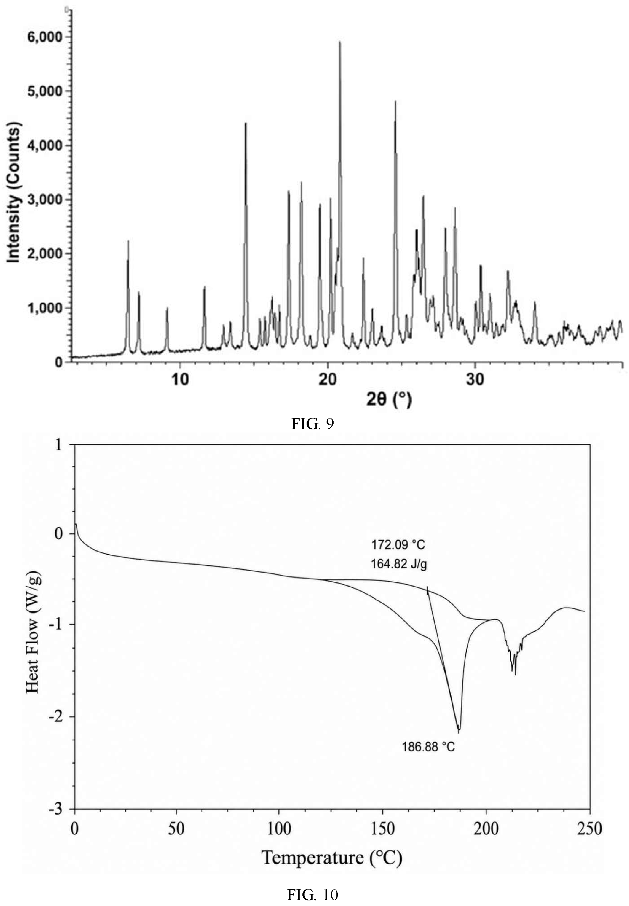

In one embodiment, the X-ray powder diffraction pattern of crystalline form B of the compound of formula III, obtained using Cu-Kα radiation, is substantially as shown in FIG. 9. In one embodiment, crystalline form B of the compound of formula III has its differential scanning calorimetry (DSC) curve showing an endothermic peak at a peak temperature of 186.88±3°C.

In one embodiment, the differential scanning calorimetry (DSC) curve of crystalline form B of the compound of formula III is substantially as shown in FIG. 10.

In one embodiment, crystalline form B of the compound of formula III has its thermogravimetric analysis (TGA) curve showing a weight loss of about 1.42% in the temperature range from 24.07±3°C to 120.0±3°C, and a weight loss of about 9.84% in the temperature range from 120.00±3°C to 205.00±3°C.

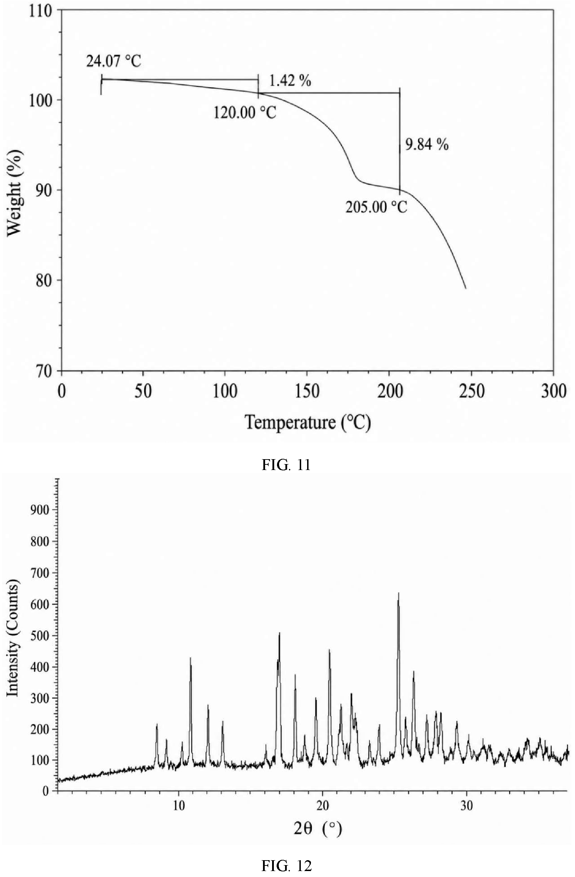

In one embodiment, the thermogravimetric analysis (TGA) curve of crystalline form B of the compound of formula III is substantially as shown in FIG. 11.

In one embodiment, in crystalline form B of the compound of formula III, w (molar equivalent of hydrochloric acid) is 1.76, 1.7, or 1.8, for example, 1.76.

In one embodiment, in crystalline form B of the compound of formula III, e is 0.

In one embodiment, the compound of formula III is crystalline form C of the compound of formula III, and its X-ray powder diffraction pattern obtained using Cu-Kα radiation, expressed in terms of 2θ angles, has diffraction peaks at 9.84±0.2°, 10.51±0.2°, 12.19±0.2°, 13.39±0.2°, and 14.40±0.2°.

In one embodiment, the X-ray powder diffraction pattern of crystalline form C of the compound of formula 111, obtained using Cu-Kα radiation and expressed in terms of 2θ angles, further has diffraction peaks at one or more of the following: 18.16±0.2°, 18.29±0.2°, 19.39±0.2°, 20.81±0.2°, 21.75±0.2°, 26.41±0.2°, and 27.48±0.2°.

In one embodiment, the X-ray powder diffraction pattern of crystalline form C of the compound of formula III, obtained using Cu-Kα radiation and expressed in terms of 2θ angles, has diffraction peaks at 9.84±0.2°, 10.51±0.2°, 12.19±0.2°, 13.39±0.2°, 14.40±0.2°, 18.16±0.2°, 18.29±0.2°, 19.39±0.2°, 20.81±0.2°, 21.75±0.2°, and 26.41±0.2°.

In one embodiment, the X-ray powder diffraction pattern of crystalline form C of the compound of formula III, obtained using Cu-Kα radiation and expressed in terms of 2θ angles, further additionally has diffraction peaks at one or more of the following: 11.61±0.2°, 20.02±0.2°, 22.51±0.2°, 22.90±0.2°, 23.25±0.2°, 23.49±0.2°, 24.46±0.2°, 25.11±0.2°, 26.94±0.2°, 28.37±0.2°, 28.97±0.2°, and 29.32±0.2°.

In one embodiment, crystalline form C of the compound of formula III has its X-ray powder diffraction pattern obtained using Cu-Kα radiation, expressed in terms of 2θ angles, having diffraction peaks as shown in Table 6.

In one embodiment, the X-ray powder diffraction pattern of crystalline form C of the compound of formula III, obtained using Cu-Kα radiation, is substantially as shown in FIG. 12. In one embodiment, in crystalline form C of the compound of formula III, w is 1.7, 1.8, or 1.6, for example, 1.7.

In one embodiment, in crystalline form C of the compound of formula III, e is 0.

The present invention provides a compound of formula IV, 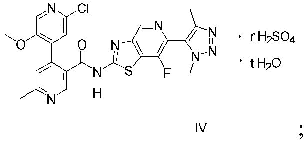 wherein r is 0.5-2; t is 0-2.

In one embodiment, in the compound of formula IV, r is 0.5, 0.9, 1, 0.97, 0.96, or 1.5.

In one embodiment, in the compound of formula IV, t is 0.

In one embodiment, in the compound of formula IV, t is 1-2, for example, t is 1.7.

In one embodiment, the compound of formula IV is crystalline form A of the compound of formula IV, and its X-ray powder diffraction pattern obtained using Cu-Kα radiation, expressed in terms of 2θ, has diffraction peaks at 5.14±0.2°, 9.01±0.2°, and 15.03±0.2°.

In one embodiment, crystalline form A of the compound of formula IV has an X-ray powder diffraction pattern obtained using Cu-Kα radiation, expressed in terms of 2θ, which further has diffraction peaks at one or more of the following positions: 11.45±0.2°, 17.44±0.2°, 21.45±0.2°, 21.78±0.2°, 24.82±0.2°, 25.95±0.2°, and 26.68±0.2°.

In one embodiment, crystalline form A of the compound of formula IV has an X-ray powder diffraction pattern obtained using Cu-Kα radiation, expressed in terms of 2θ, which has diffraction peaks at 5.14±0.2°, 9.01±0.2°, 15.03±0.2°, 11.45±0.2°, 17.44±0.2°, 21.45±0.2°, 21.78±0.2°, 24.82±0.2°, and 25.95±0.2°.

In one embodiment, crystalline form A of the compound of formula IV has an X-ray powder diffraction pattern obtained using Cu-Kα radiation, expressed in terms of 2θ, which further has diffraction peaks at one or more of the following positions: 6.11±0.2°, 6.60±0.2°, 7.35±0.2°, 8.63±0.2°, 14.62±0.2°, 16.45±0.2°, 20.40±0.2°, 23.81±0.2°, 25.47±0.2°, 27.96±0.2°, and 28.43±0.2°.

In one embodiment, crystalline form A of the compound of formula IV has an X-ray powder diffraction pattern obtained using Cu-Kα radiation, which has diffraction peaks as shown in Table 7.

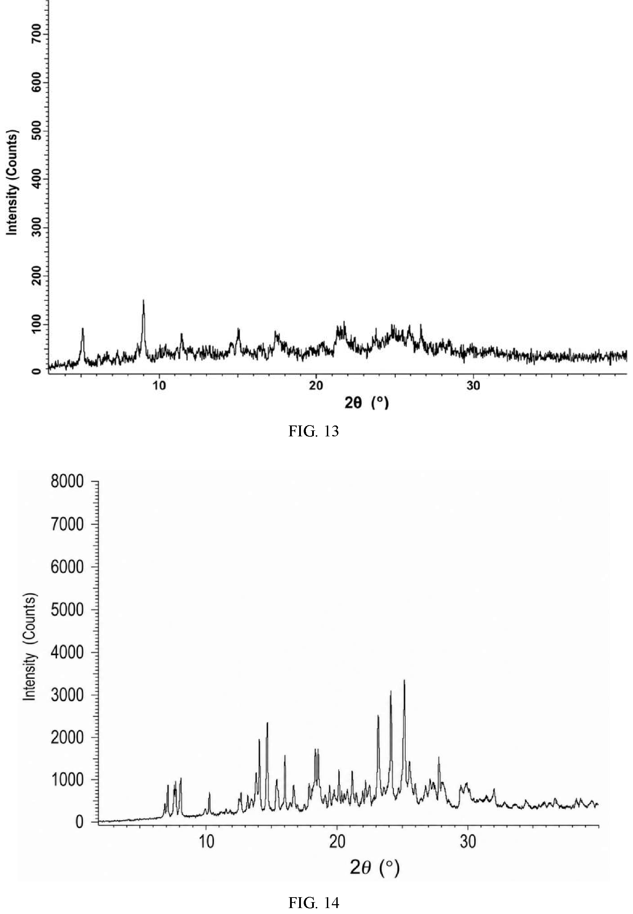

In one embodiment, crystalline form A of the compound of formula IV has an X-ray powder diffraction pattern obtained using Cu-Kα radiation, which is substantially as shown in FIG. 13. In one embodiment, in crystalline form A of the compound of formula IV, r is 1, 0.97, or 0.9, for example, 0.97.

In one embodiment, in crystalline form A of the compound of formula IV, t is 0.

In one embodiment, the compound of formula IV is crystalline form B of the compound of formula IV, and its X-ray powder diffraction pattern obtained using Cu-Kα radiation, expressed in terms of 2θ, has diffraction peaks at 7.11±0.2°, 7.57±0.2°, 7.72±0.2°, 8.10±0.2°, and 10.27±0.2°.

In one embodiment, crystalline form B of the compound of formula IV has an X-ray powder diffraction pattern obtained using Cu-Kα radiation, expressed in terms of 2θ, which further has diffraction peaks at one or more of the following positions: 13.86±0.2°, 14.11±0.2°, 14.71±0.2°, 15.44±0.2°, 16.07±0.2°, 23.18±0.2°, 24.18±0.2°, and 25.17±0.2°.

In one embodiment, crystalline form B of the compound of formula IV has an X-ray powder diffraction pattern obtained using Cu-Kα radiation, expressed in terms of 2θ, which has diffraction peaks at 7.11±0.2°, 7.57±0.2°, 7.72±0.2°, 8.10±0.2°, 13.86±0.2°, 14.11±0.2°, 14.71±0.2°, 23.18±0.2°, 24.18±0.2°, and 25.17±0.2°.

In one embodiment, crystalline form B of the compound of formula IV has an X-ray powder diffraction pattern obtained using Cu-Kα radiation, expressed in terms of 2θ, which further has diffraction peaks at one or more of the following positions: 10.27±0.2°, 12.55±0.2°, 12.67±0.2°, 13.19±0.2°, 16.73±0.2°, 17.92±0.2°, 18.38±0.2°, 18.58±0.2°, 20.18±0.2°, 21.2±0.2°, 22.2±0.2°, 25.58±0.2°, and 27.82±0.2°.

In one embodiment, crystalline form B of the compound of formula IV has an X-ray powder diffraction pattern obtained using Cu-Kα radiation, which has diffraction peaks as shown in Table 8.

In one embodiment, crystalline form B of the compound of formula IV has an X-ray powder diffraction pattern obtained using Cu-Kα radiation, which is substantially as shown in FIG. 14. In one embodiment, crystalline form B of the compound of formula IV has a differential scanning calorimetry (DSC) curve with endothermic peaks at peak temperatures of 34.85±3°C, 90.79±3°C, and 204.38±3°C.

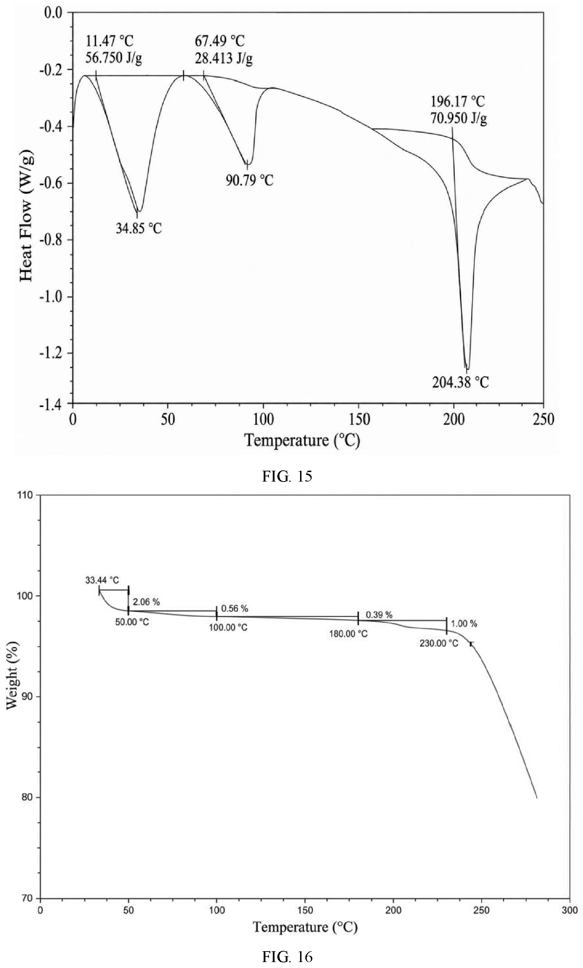

In one embodiment, the differential scanning calorimetry (DSC) curve of crystalline form B of the compound of formula IV is substantially as shown in FIG. 15.

In one embodiment, crystalline form B of the compound of formula IV has a thermogravimetric analysis (TGA) curve showing a weight loss of about 2.06% in the temperature range from 33.44±3°C to 50.0±3°C, a weight loss of about 0.56% in the temperature range from 50.0±3°C to 100.0±3°C, a weight loss of about 0.39% in the temperature range from 100.00±3°C to 180.00±3°C, and a weight loss of about 1.0% in the temperature range from 180.00±3°C to 230.00±3°C.

In one embodiment, the thermogravimetric analysis (TGA) curve of crystalline form B of the compound of formula IV is substantially as shown in FIG. 16.

In one embodiment, in crystalline form B of the compound of formula IV, r is 1, 0.9, or 0.96, for example, 0.96.

In one embodiment, in crystalline form B of the compound of formula IV, t is 0 or 1.7. Preferably, t is 0.

In one embodiment, in crystalline form B of the compound of formula IV, r is 1 and t is 0; or, r is 0.96 and t is 0.

The present invention provides a compound of formula V,
wherein the structure of the compound of formula V is: 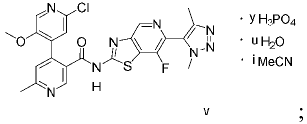 y is 0.5-1.5; u is 0-2; i is 0-2. Preferably, i and u are 0.

In one embodiment, in the compound of formula V, y is 1.1, 1.2, 1.14, 1, 1.09, or 1.05.

In one embodiment, in the compound of formula V, y and u are 0.

In one embodiment, in the compound of formula V, i is 0, and u is 0.1, 0.91, 0.9, or 1, for example, 0.91.

In one embodiment, in the compound of formula V, u is 0, and i is 0.1, 0.18, 0.2, or 0.5, for example, 0.18 or 0.1.

In one embodiment, the compound of formula V is crystalline form A of the compound of formula V, and its X-ray powder diffraction pattern obtained using Cu-Kα radiation, expressed in terms of 2θ angle, has diffraction peaks at 5.15±0.2°, 8.34±0.2°, 15.51±0.2°, and 15.88±0.2°. In one embodiment, crystalline form A of the compound of formula V has an X-ray powder diffraction pattern obtained using Cu-Kα radiation, expressed in terms of 2θ angle, which further has diffraction peaks at one or more of the following positions: 7.97±0.2°, 8.96±0.2°, 17.93±0.2°, 18.39±0.2°, 21.99±0.2°, and 27.73±0.2°.

In one embodiment, crystalline form A of the compound of formula V has an X-ray powder diffraction pattern obtained using Cu-Kα radiation, expressed in terms of 2θ angle, which has diffraction peaks at 5.15±0.2°, 8.34±0.2°, 15.51±0.2°, 15.88±0.2°, 8.96±0.2°, 17.93±0.2°, 18.39±0.2°, 21.99±0.2°, and 27.73±0.2°.

In one embodiment, crystalline form A of the compound of formula V has an X-ray powder diffraction pattern obtained using Cu-Kα radiation, expressed in terms of 2θ angle, which further has diffraction peaks at one or more of the following positions: 5.98±0.2°, 8.96±0.2°, 10.49±0.2°, 10.80±0.2°, 11.34±0.2°, 11.66±0.2°, 11.99±0.2°, 12.73±0.2°, 13.15±0.2°, 18.68±0.2°, 19.73±0.2°, 20.14±0.2°, 22.76±0.2°, and 23.24±0.2°.

In one embodiment, crystalline form A of the compound of formula V has an X-ray powder diffraction pattern obtained using Cu-Kα radiation, expressed in terms of 2θ angle, which has diffraction peaks as shown in Table 9.

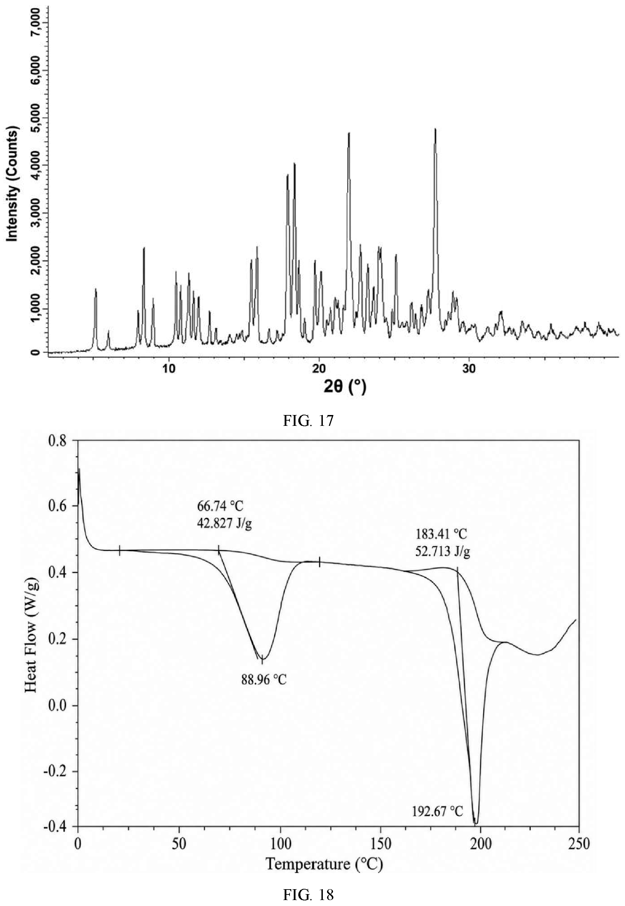

In one embodiment, crystalline form A of the compound of formula V has an X-ray powder diffraction pattern obtained using Cu-Kα radiation which is substantially as shown in FIG. 17. In one embodiment, crystalline form A of the compound of formula V has a differential scanning calorimetry (DSC) curve with endothermic peaks at peak temperatures of 88.96±3°C and 192.67±3°C.

In one embodiment, the differential scanning calorimetry (DSC) curve of crystalline form A of the compound of formula V is substantially as shown in FIG. 18.

In one embodiment, crystalline form A of the compound of formula V has a thermogravimetric analysis (TGA) curve showing a weight loss of about 0.92% in the temperature range from 32.34±3°C to 70.0±3°C, and a weight loss of about 2.1% in the temperature range from 70.0±3°C to 180.0±3°C.

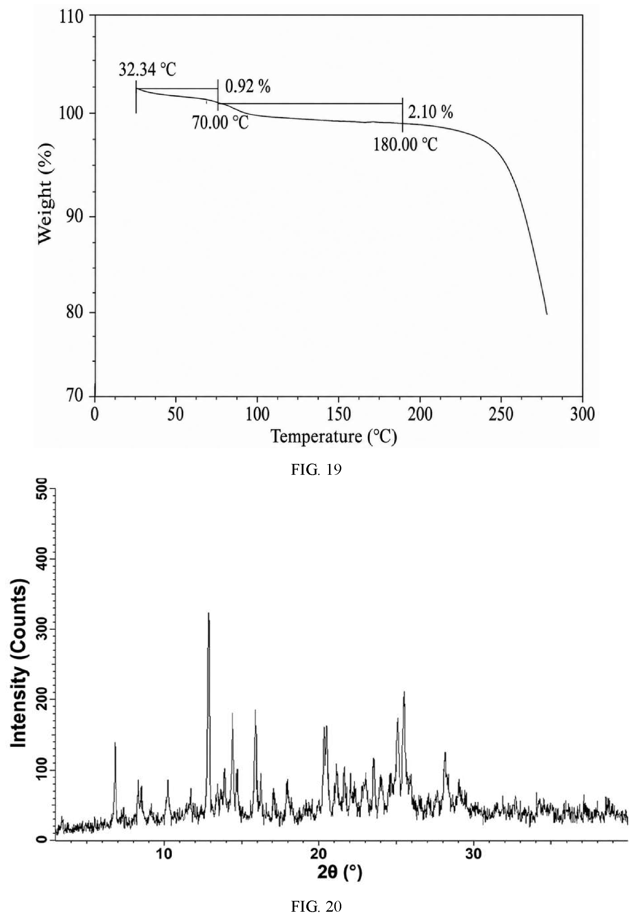

In one embodiment, the thermogravimetric analysis (TGA) curve of crystalline form A of the compound of formula V is substantially as shown in FIG. 19.

In one embodiment, in crystalline form A of the compound of formula V, y is 1.1, 1.2, or 1.14, for example, 1.14.

In one embodiment, in crystalline form A of the compound of formula V, i is 0, y is 1.1, and u is 0.9; or, i is 0, y is 1.14, and u is 0.91.

In one embodiment, the compound of formula V is crystalline form B of the compound of formula V, and its X-ray powder diffraction pattern obtained using Cu-Kα radiation, expressed in terms of 2θ angle, has diffraction peaks at 6.83±0.2°, 12.89±0.2°, 14.44±0.2°, 15.91±0.2°, 25.08±0.2°, and 25.50±0.2°.

In one embodiment, crystalline form B of the compound of formula V has an X-ray powder diffraction pattern obtained using Cu-Kα radiation, expressed in terms of 2θ angle, which further has diffraction peaks at one or more of the following positions: 8.32±0.2°, 10.26±0.2°, 13.93±0.2°, 14.67±0.2°, 20.35±0.2°, 20.49±0.2°, 21.15±0.2°, and 21.65±0.2°.

In one embodiment, crystalline form B of the compound of formula V has an X-ray powder diffraction pattern obtained using Cu-Kα radiation, expressed in terms of 2θ angle, which has diffraction peaks at 6.83±0.2°, 12.89±0.2°, 14.44±0.2°, 15.91±0.2°, 25.08±0.2°, 25.50±0.2°, 8.32±0.2°, 20.35±0.2°, and 20.49±0.2°.

In one embodiment, crystalline form B of the compound of formula V has an X-ray powder diffraction pattern obtained using Cu-Kα radiation, expressed in terms of 2θ angle, which further has diffraction peaks at one or more of the following positions: 8.52±0.2°, 9.17±0.2°, 13.46±0.2°, 16.25±0.2°, and 17.94±0.2°.

In one embodiment, crystalline form B of the compound of formula V has an X-ray powder diffraction pattern obtained using Cu-Kα radiation, expressed in terms of 2θ angle, which has diffraction peaks as shown in Table 10.

In one embodiment, crystalline form B of the compound of formula V has an X-ray powder diffraction pattern obtained using Cu-Kα radiation which is substantially as shown in FIG. 20. In one embodiment, in crystalline form B of the compound of formula V, y is 1.1, 1, or 1.09, for example, 1.09.

In one embodiment, in crystalline form B of the compound of formula V, u and i are 0.

In one embodiment, the compound of formula V is crystalline form C of the compound of formula V, and its X-ray powder diffraction pattern obtained using Cu-Kα radiation, expressed in terms of 2θ angle, has diffraction peaks at 5.19±0.2°, 8.39±0.2°, 10.47±0.2°, and 11.51±0.2°. In one embodiment, crystalline form C of the compound of formula V has an X-ray powder diffraction pattern obtained using Cu-Kα radiation, expressed in terms of 2θ angle, which further has diffraction peaks at one or more of the following positions: 15.68±0.2°, 15.87±0.2°, 18.01±0.2°, 22.55±0.2°, and 25.67±0.2°.

Preferably, in crystalline form C of the compound of formula V, the relative intensity of the diffraction peak at 11.51±0.2° in its X-ray powder diffraction pattern obtained using Cu-Kα radiation, expressed in terms of 2θ angle, is 80%-100%, and preferably the diffraction peak at 11.51±0.2° is the strongest peak.

In one embodiment, crystalline form C of the compound of formula V has an X-ray powder diffraction pattern obtained using Cu-Kα radiation, expressed in terms of 2θ angle, which further has diffraction peaks at one or more of the following positions: 11.67±0.2°, 12.73±0.2°, 12.89±0.2°, 14.59±0.2°, 15.05±0.2°, 18.76±0.2°, 19.81±0.2°, 20.48±0.2°, 20.84±0.2°, 21.68±0.2°, 22.10±0.2°, 24.80±0.2°, and 25.27±0.2°.

In one embodiment, crystalline form C of the compound of formula V has an X-ray powder diffraction pattern obtained using Cu-Kα radiation, expressed in terms of 2θ angle, which has diffraction peaks at 5.19±0.2°, 8.39±0.2°, 10.47±0.2°, 11.51±0.2°, 11.67±0.2°, 15.68±0.2°, 15.87±0.2°, 18.01±0.2°, and 22.55±0.2°.

In one embodiment, crystalline form C of the compound of formula V has an X-ray powder diffraction pattern obtained using Cu-Kα radiation, expressed in terms of 2θ angle, which has diffraction peaks as shown in Table 11.

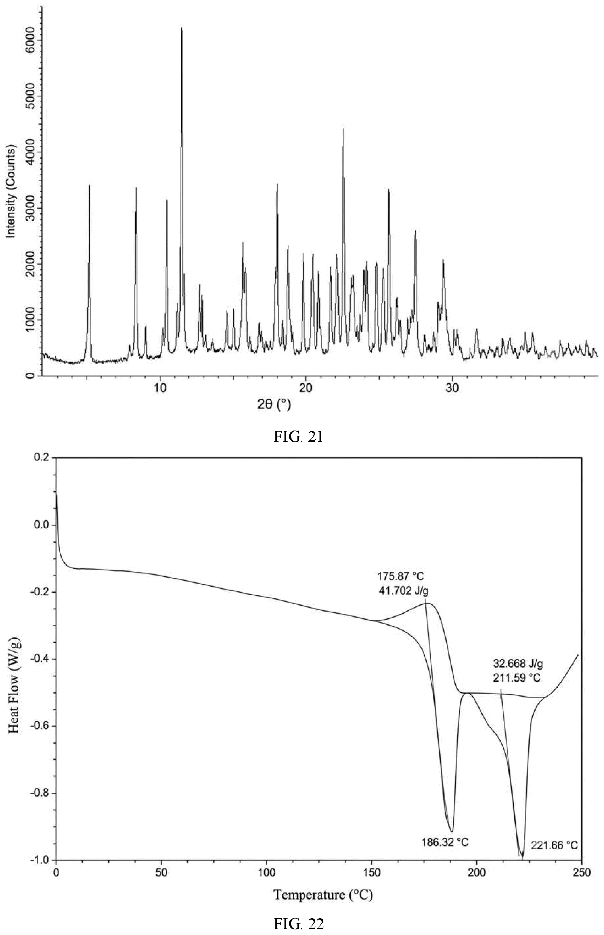

In one embodiment, crystalline form C of the compound of formula V has an X-ray powder diffraction pattern obtained using Cu-Kα radiation which is substantially as shown in FIG. 21. In one embodiment, crystalline form C of the compound of formula V has a differential scanning calorimetry (DSC) curve with endothermic peaks at peak temperatures of 188.32±3°C and 221.66±3°C.

In one embodiment, the differential scanning calorimetry (DSC) curve of crystalline form C of the compound of formula V is substantially as shown in FIG. 22.

In one embodiment, crystalline form C of the compound of formula V has a thermogravimetric analysis (TGA) curve showing a weight loss of about 0.71% in the temperature range from 29.55±3°C to 180.0±3°C and a weight loss of about 0.85% in the temperature range from 180.0±3°C to 220.0±3°C.

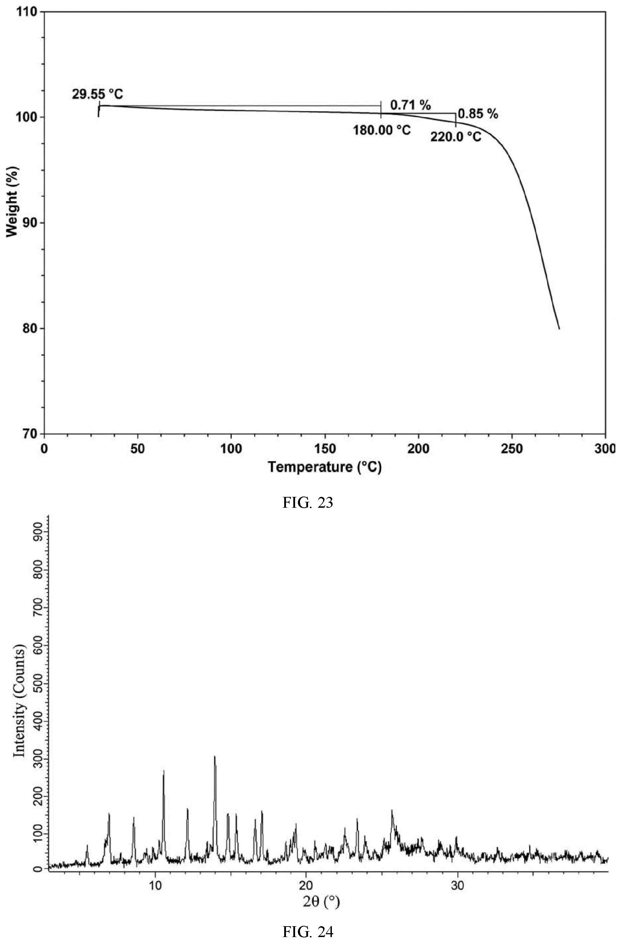

In one embodiment, the thermogravimetric analysis (TGA) curve of crystalline form C of the compound of formula V is substantially as shown in FIG. 23.

In one embodiment, in crystalline form C of the compound of formula V, y is 1.1, 1, or 1.05, for example, 1.05.

In one embodiment, in crystalline form C of the compound of formula V, u and i are 0, and y is 1; or, u is 0, i is 0.1, and y is 1.05.

The present invention provides a compound of formula VI, 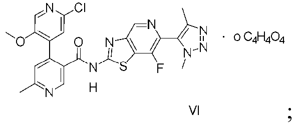
wherein, C₄H₄O₄ is fumaric acid;
o (molar equivalent of fumaric acid) is 1-1.1.

In one embodiment, in the compound of formula VI, o is 1, 1.01, 1.05, or 1.1.

In one embodiment, the compound of formula VI is crystalline form A of the compound of formula VI, which has an X-ray powder diffraction pattern obtained using Cu-Kα radiation, expressed in terms of 2θ angle, with diffraction peaks at 10.58+0.2°, 12.16±0.2°, and 13.98±0.2°.

In one embodiment, crystalline form A of the compound of formula VI has an X-ray powder diffraction pattern obtained using Cu-Kα radiation, expressed in terms of 2θ angle, which further has diffraction peaks at one or more of the following positions: 6.96±0.2°, 8.60±0.2°, 14.84±0.2°, 15.39±0.2°, 16.63±0.2°, and 17.08±0.2°.

In one embodiment, crystalline form A of the compound of formula VI has an X-ray powder diffraction pattern obtained using Cu-Kα radiation, expressed in terms of 2θ angle, with diffraction peaks at 10.58±0.2°, 12.16±0.2°, 13.98±0.2°, 6.96±0.2°, 8.60±0.2°, 14.84±0.2°, 15.39±0.2°, 16.63±0.2°, and 17.08±0.2°.

In one embodiment, crystalline form A of the compound of formula VI has an X-ray powder diffraction pattern obtained using Cu-Kα radiation, expressed in terms of 2θ angle, which further has diffraction peaks at one or more of the following positions: 6.73±0.2°, 9.38±0.2°, 10.28±0.2°, 13.45±0.2°, 19.28±0.2°, and 18.67±0.2°.

In one embodiment, crystalline form A of the compound of formula VI has an X-ray powder diffraction pattern obtained using Cu-Kα radiation, expressed in terms of 2θ angle, which has diffraction peaks as shown in Table 12.

In one embodiment, crystalline form A of the compound of formula VI has an X-ray powder diffraction pattern obtained using Cu-Kα radiation which is substantially as shown in FIG. 24. In one embodiment, in crystalline form A of the compound of formula VI, o is 1, 1.1, or 1.01, for example, 1.01.

The present invention provides a compound of formula VII, 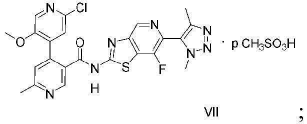
wherein CH₃SO₃H is methanesulfonic acid;
p (the molar equivalent of methanesulfonic acid) is 1-1.1.

In one embodiment, in the compound of formula VII, p is 1:1, 1:1.03, 1:1.05, or 1:1.1.

In one embodiment, in the compound of formula VII, p is 1, 1.03, 1.05, or 1.1.

In one embodiment, the compound of formula VII is crystalline form A of the compound of formula VII, which has an X-ray powder diffraction pattern obtained using Cu-Kα radiation, expressed in terms of 2θ angle, with diffraction peaks at 9.00±0.2°, 17.44±0.2°, and 21.74±0.2°.

In one embodiment, crystalline form A of the compound of formula VII has an X-ray powder diffraction pattern obtained using Cu-Kα radiation, expressed in terms of 2θ angle, which further has diffraction peaks at one or more of the following positions: 5.13±0.2°, 6.73±0.2°, 8.61±0.2°, 17.74±0.2°, 24.43±0.2°, and 25.32±0.2°.

In one embodiment, crystalline form A of the compound of formula VII has an X-ray powder diffraction pattern obtained using Cu-Kα radiation, expressed in terms of 2θ angle, with diffraction peaks at 9.00±0.2°, 17.44±0.2°, 21.74±0.2°, 5.13±0.2°, 6.73±0.2°, 8.61±0.2°, 17.74±0.2°, 24.43±0.2°, and 25.32±0.2°.

In one embodiment, crystalline form A of the compound of formula VII has an X-ray powder diffraction pattern obtained using Cu-Kα radiation, expressed in terms of 2θ angle, which further has diffraction peaks at one or more of the following positions: 15.55±0.2°, 10.05±0.2°, 11.57±0.2°, 22.37±0.2°, and 26.36±0.2°.

In one embodiment, crystalline form A of the compound of formula VII has an X-ray powder diffraction pattern obtained using Cu-Kα radiation, expressed in terms of 2θ angle, which has diffraction peaks as shown in Table 13.

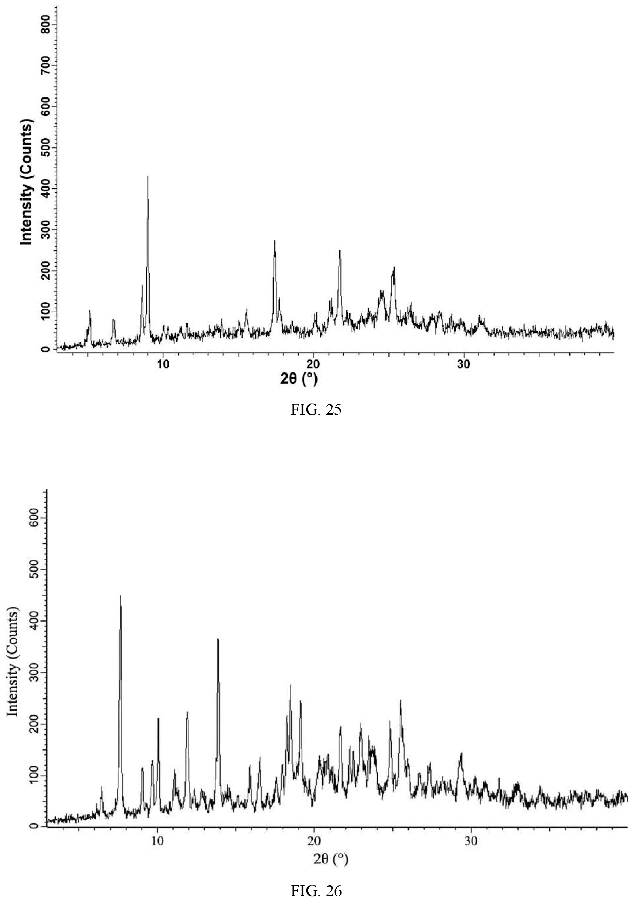

In one embodiment, crystalline form A of the compound of formula VII has an X-ray powder diffraction pattern obtained using Cu-Kα radiation which is substantially as shown in FIG. 25. In one embodiment, in crystalline form A of the compound of formula VII, p is 1, 1.1, or 1.03, for example, 1.03.

The present invention provides a compound of formula VIII, 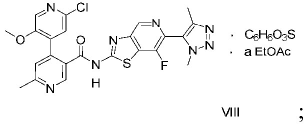
wherein C₆H₆O₃S is benzenesulfonic acid;
a (the molar equivalent of ethyl acetate) is 0-1.

In one embodiment, in the compound of formula VIII, a is 0 (solvent-free).

In one embodiment, in the compound of formula VIII, a is 0-0.1, for example, 0.07, 0.1, or 0. In one embodiment, the compound of formula VIII is crystalline form A of the compound of formula VIII, which has X-ray powder diffraction peaks expressed in 2θ angles using Cu-Kα radiation at 7.66±0.2°, 10.09±0.2°, 11.92±0.2°, and 13.91±0.2°.

In one embodiment, crystalline form A of the compound of formula VIII has X-ray powder diffraction peaks expressed in 2θ angles using Cu-Kα radiation further at one or more of the following: 9.06±0.2°, 9.72±0.2°, 11.10±0.2°, 15.91±0.2°, 16.55±0.2°, 18.00±0.2°, 18.27±0.2°, and 19.16±0.2°.

In one embodiment, crystalline form A of the compound of formula VIII has X-ray powder diffraction peaks expressed in 2θ angles using Cu-Kα radiation at 7.66±0.2°, 10.09±0.2°, 11.92±0.2°, 13.91±0.2°, 9.06±0.2°, 9.72±0.2°, 11.10±0.2°, 15.91±0.2°, and 16.55±0.2°.

In one embodiment, crystalline form A of the compound of formula VIII has X-ray powder diffraction peaks expressed in 2θ angles using Cu-Kα radiation further at one or more of the following: 20.35±0.2°, 20.89±0.2°, 21.10±0.2°, 21.68±0.2°, 22.27±0.2°, 22.49±0.2°, 22.97±0.2°, 23.50±0.2°, 24.84±0.2°, 25.52±0.2°, and 29.36±0.2°.

In one embodiment, crystalline form A of the compound of formula VIII has X-ray powder diffraction peaks using Cu-Kα radiation as shown in Table 14.

In one embodiment, crystalline form A of the compound of formula VIII has an X-ray powder diffraction pattern obtained using Cu-Kα radiation which is substantially as shown in FIG. 26. In one embodiment, in crystalline form A of the compound of formula VIII, a is 0, 0.1, or 0.07, for example, 0.07.

The present invention provides a compound of formula IX, 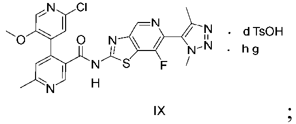
wherein d (the molar equivalent of p-toluenesulfonic acid) is 1-2;
g (solvent) is water, tetrahydrofuran, isopropanol, 1,4-dioxane, or methyl tert-butyl ether;
h (the molar equivalent of solvent) is 0-3.

In one embodiment, in the compound of formula IX, d is 1, 1.1, 1.2, 1.02, or 1.5.

In one embodiment, in the compound of formula IX, h is 0 (solvent-free).

In one embodiment, in the compound of formula IX, h is 0.1-3. For example, g is water and h is 3; or g is tetrahydrofuran and h is 0.2, 0.1, or 0.16; or g is isopropanol and h is 0.9, 0.98, or 1; or g is 1,4-dioxane and h is 3, 2.96, or 2.9; or g is methyl tert-butyl ether and h is 0.4, 0.45, or 0.5.

In one embodiment,the compound of formula IX is crystalline form A of the compound of formula IX, and its X-ray powder diffraction pattern using Cu-Kα radiation, expressed in terms of 2θ angles, has diffraction peaks at 5.96±0.2°, 16.71±0.2°, 17.79±0.2°, 20.33±0.2°, and 24.66±0.2°.

In one embodiment, crystalline form A of the compound of formula IX has an X-ray powder diffraction pattern using Cu-Kα radiation, expressed in terms of 2θ angles, further having diffraction peaks at one or more of the following positions: 10.57±0.2°, 12.34±0.2°, 12.77±0.2°, 14.06±0.2°, 14.40±0.2°, 14.97±0.2°, 19.88±0.2°, 22.64±0.2°, 23.18±0.2°, and 24.22±0.2°.

In one embodiment, crystalline form A of the compound of formula IX has an X-ray powder diffraction pattern using Cu-Kα radiation, expressed in terms of 2θ angles, having diffraction peaks at 5.96±0.2°, 16.71±0.2°, 17.79±0.2°, 20.33±0.2°, 24.66±0.2°, 10.57±0.2°, 12.34±0.2°, 12.77±0.2°, 14.06±0.2°, 14.40±0.2°, and 14.97±0.2°.

In one embodiment, crystalline form A of the compound of formula IX has an X-ray powder diffraction pattern using Cu-Kα radiation, expressed in terms of 2θ angles, further having diffraction peaks at one or more of the following positions: 9.93±0.2°, 10.13±0.2°, 11.03±0.2°, 11.88±0.2°, 17.56±0.2°, 18.41±0.2°, 18.70±0.2°, 20.68±0.2°, 22.11±0.2°, 25.43±0.2°, 25.98±0.2°, 27.35±0.2°, 27.52±0.2°, 28.08±0.2°, and 29.17±0.2°.

In one embodiment, crystalline form A of the compound of formula IX has an X-ray powder diffraction pattern using Cu-Kα radiation with diffraction peaks as shown in Table 15.

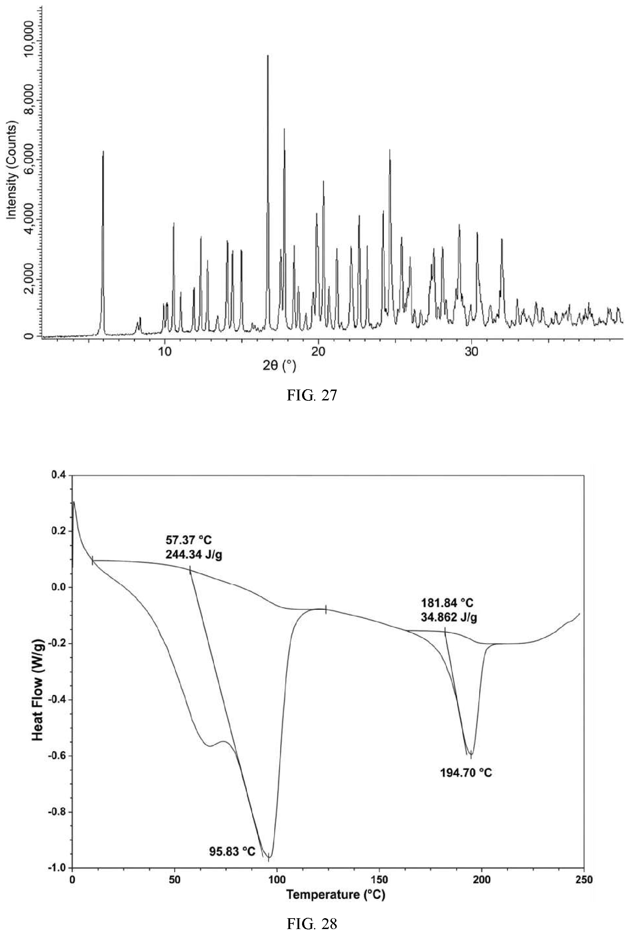

In one embodiment, crystalline form A of the compound of formula IX has an X-ray powder diffraction pattern using Cu-Kα radiation substantially as shown in FIG. 27.

In one embodiment, crystalline form A of the compound of formula IX has a differential scanning calorimetry (DSC) curve with endothermic peaks at peak temperatures of 95.83±3°C and 194.7±3°C.

In one embodiment, the differential scanning calorimetry (DSC) curve of crystalline form A of the compound of formula IX is substantially as shown in FIG. 28.

In one embodiment, crystalline form A of the compound of formula IX has a thermogravimetric analysis (TGA) curve showing a weight loss of about 2.03% in the temperature range from 30.52±3°C to 60.0±3°C, a weight loss of about 5.13% in the temperature range from 60.0±3°C to 100.0±3°C, and a weight loss of about 0.5% in the temperature range from 100.0±3°C to 190.0±3°C.

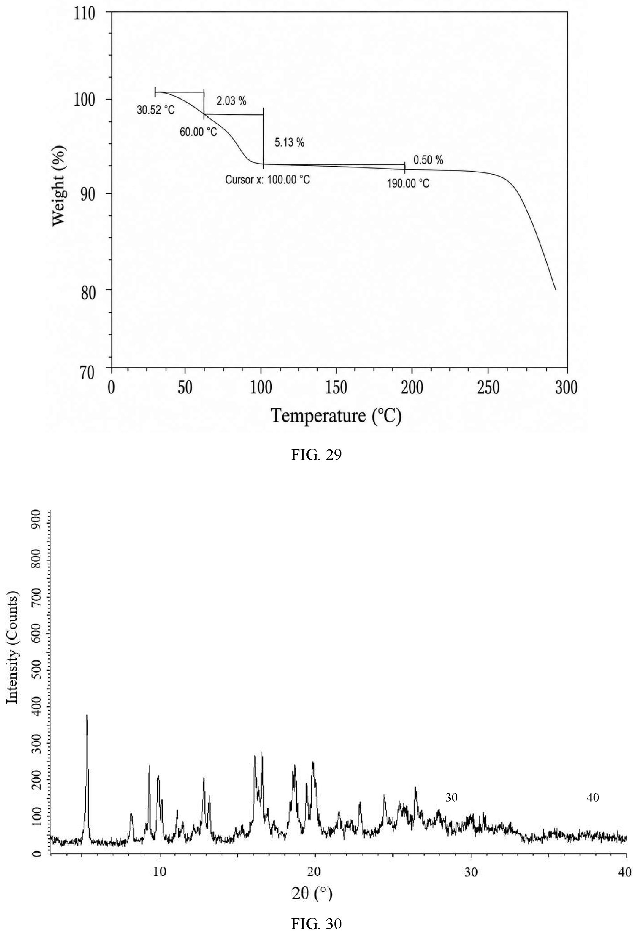

In one embodiment, the thermogravimetric analysis (TGA) curve of crystalline form A of the compound of formula IX is substantially as shown in FIG. 29.

In one embodiment, in crystalline form A of the compound of formula IX, g is water, d is 1, and h is 3.

In one embodiment, the compound of formula IX is a single crystal of the compound of formula IX, wherein
g is water, h is 3, and d is 1;
it has the following unit cell parameters: triclinic crystal system (Triclinic), space group p-1; a = 10.6990(12) Å, α = 89.299(7)°, b = 10.7640(13) Å, β = 86.519(6)°, c = 14.9358(17) Å, γ = 84.364(6)°, unit cell volume = 1708.6(3) A³, number of asymmetric units per unit cell Z = 2, crystal density is 1.46 mg/m³.

In one embodiment,the compound of formula IX is crystalline form B of the compound of formula IX, and its X-ray powder diffraction pattern using Cu-Kα radiation, expressed in terms of 2θ angles, has diffraction peaks at 5.34±0.2°, 9.34±0.2°, 9.92±0.2°, 12.84±0.2°, and 16.61±0.2°.

In one embodiment, crystalline form B of the compound of formula IX has an X-ray powder diffraction pattern using Cu-Kα radiation, expressed in terms of 2θ angles, further having diffraction peaks at one or more of the following positions: 8.20±0.2°, 10.13±0.2°, 11.12±0.2°, 13.18±0.2°, 16.13±0.2°, 18.67±0.2°, 19.47±0.2°, and 19.85±0.2°.

In one embodiment, crystalline form B of the compound of formula IX has an X-ray powder diffraction pattern using Cu-Kα radiation, expressed in terms of 2θ angles, having diffraction peaks at 5.34±0.2°, 9.34±0.2°, 9.92±0.2°, 12.84±0.2°, 16.61±0.2°, 8.20±0.2°, 10.13±0.2°, 13.18±0.2°, and 16.13±0.2°.

In one embodiment, crystalline form B of the compound of formula IX has an X-ray powder diffraction pattern using Cu-Kα radiation, expressed in terms of 2θ angles, further having diffraction peaks at one or more of the following positions: 11.47±0.2°, 16.36±0.2°, 18.60±0.2°, and 21.53±0.2°.

In one embodiment, crystalline form B of the compound of formula IX has an X-ray powder diffraction pattern using Cu-Kα radiation, expressed in terms of 2θ angles, having diffraction peaks as shown in Table 16.

In one embodiment, crystalline form B of the compound of formula IX has an X-ray powder diffraction pattern using Cu-Kα radiation substantially as shown in FIG. 30.

In one embodiment, in crystalline form B of the compound of formula IX, d is 1.

In one embodiment, in crystalline form B of the compound of formula IX, h is 0.

The present invention provides a compound of formula X, 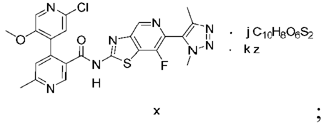
wherein C₁₉H₈O₆S₂ is 1,5-naphthalenedisulfonic acid;
j (molar equivalent of 1,5-naphthalenedisulfonic acid) is 0.5-1.5;
z (solvent) is water or acetonitrile;
k is 0-5.

In one embodiment, in the compound of formula X, j is 1, 0.9, 1.1, 1.02, or 0.93.

In one embodiment, in the compound of formula X, k is 0 (solvent-free).

In one embodiment, in the compound of formula X, k is 0.01-5, for example, z is water and k is 5 or 4.8; or, for example, z is acetonitrile and k is 0.07.

In one embodiment, the compound of formula X is crystalline form A of the compound of formula X, and its X-ray powder diffraction pattern using Cu-Kα radiation, expressed in terms of 2θ angles, has diffraction peaks at 7.46±0.2°, 12.01±0.2°, and 21.70±0.2°.

In one embodiment, crystalline form A of the compound of formula X has an X-ray powder diffraction pattern using Cu-Kα radiation, expressed in terms of 2θ angles, further having diffraction peaks at one or more of the following positions: 11.43±0.2°, 13.53±0.2°, 17.24±0.2°, 18.49±0.2°, 18.77±0.2°, 21.08±0.2°, 24.57±0.2°, and 28.44±0.2°.

In one embodiment, crystalline form A of the compound of formula X has an X-ray powder diffraction pattern using Cu-Kα radiation, expressed in terms of 2θ angles, having diffraction peaks at 7.46±0.2°, 12.01±0.2°, 21.70±0.2°, 11.43±0.2°, 13.53±0.2°, 17.24±0.2°, 18.49±0.2°, 18.77±0.2°, and 21.08±0.2°.

In one embodiment, crystalline form A of the compound of formula X has an X-ray powder diffraction pattern using Cu-Kα radiation, expressed in terms of 2θ angles, further having diffraction peaks at one or more of the following positions: 5.71±0.2°, 13.93±0.2°, 14.91±0.2°, 15.34±0.2°, 22.12±0.2°, 22.80±0.2°, 22.95±0.2°, 24.99±0.2°, and 26.08±0.2°.

In one embodiment, crystalline form A of the compound of formula X has an X-ray powder diffraction pattern using Cu-Kα radiation, expressed in terms of 2θ angles, having diffraction peaks as shown in Table 17.

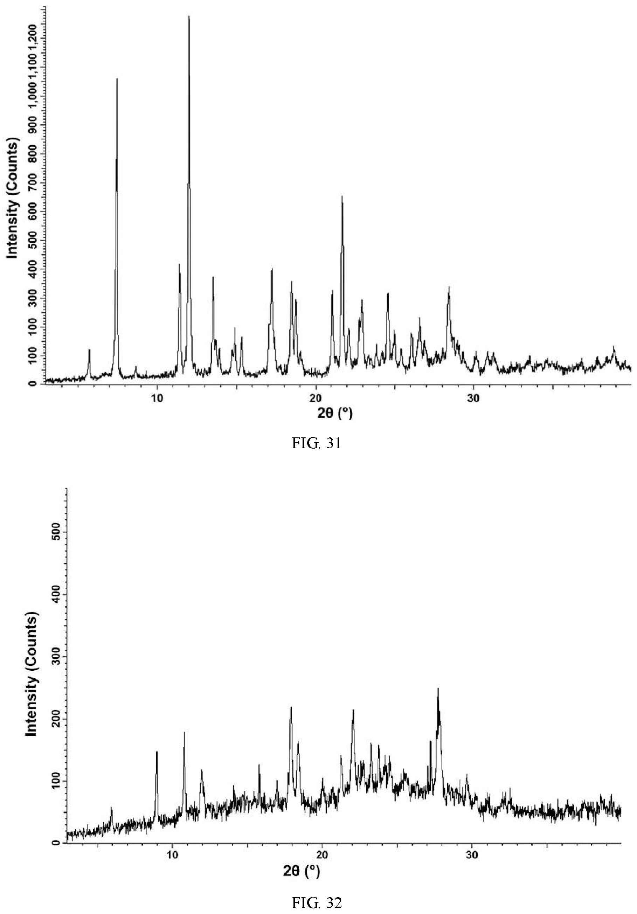

In one embodiment, crystalline form A of the compound of formula X has an X-ray powder diffraction pattern using Cu-Kα radiation substantially as shown in FIG. 31.

In one embodiment, in crystalline form A of the compound of formula X, k is 0, and j is 1, 1.1, or 1.02, for example, 1.02.

In one embodiment, the compound of formula X is crystalline form B of the compound of formula X, and its X-ray powder diffraction pattern using Cu-Kα radiation, expressed in terms of 2θ angles, has diffraction peaks at 8.96±0.2°, 10.81±0.2°, 17.92±0.2°, and 22.07±0.2°.

In one embodiment, crystalline form B of the compound of formula X has an X-ray powder diffraction pattern using Cu-Kα radiation, expressed in terms of 2θ angles, further having diffraction peaks at one or more of the following positions: 11.98±0.2°, 18.4±0.2°, 21.27±0.2°, 23.27±0.2°, 27.26±0.2°, and 27.77±0.2°.

In one embodiment, crystalline form B of the compound of formula X has an X-ray powder diffraction pattern using Cu-Kα radiation, expressed in terms of 2θ angles, further having diffraction peaks at one or more of the following positions: 5.95±0.2°, 14.09±0.2°, 15.80±0.2°, 16.97±0.2°, 20.03±0.2°, 22.42±0.2°, and 23.79±0.2°.

In one embodiment, crystalline form B of the compound of formula X has an X-ray powder diffraction pattern using Cu-Kα radiation, expressed in terms of 2θ angles, having diffraction peaks at 8.96±0.2°, 10.81±0.2°, 17.92±0.2°, 22.07±0.2°, 11.98±0.2°, 18.4±0.2°, 21.27±0.2°, and 23.27±0.2°.

In one embodiment, crystalline form B of the compound of formula X has an X-ray powder diffraction pattern using Cu-Kα radiation, expressed in terms of 2θ angles, having diffraction peaks as shown in Table 18.

In one embodiment, crystalline form B of the compound of formula X has an X-ray powder diffraction pattern using Cu-Kα radiation substantially as shown in FIG. 32.

In one embodiment, in crystalline form B of the compound of formula X, k is 0, and j is 0.9, 1, or 0.93, for example, 0.93.

In one embodiment, the compound of formula X is crystalline form C of the compound of formula X, and its X-ray powder diffraction pattern using Cu-Kα radiation, expressed in terms of 2θ angles, has diffraction peaks at 11.30±0.2°, 22.61±0.2°, 24.69±0.2°, 26.16±0.2°, and 28.57±0.2°.

In one embodiment, crystalline form C of the compound of formula X has an X-ray powder diffraction pattern using Cu-Kα radiation, expressed in terms of 2θ angles, further having diffraction peaks at one or more of the following positions: 6.36±0.2°, 12.82±0.2°, 17.07±0.2°, 17.48±0.2°, 18.61±0.2°, 20.24±0.2°, and 20.94±0.2°.

Preferably, crystalline form C of the compound of formula X has an X-ray powder diffraction pattern using Cu-Kα radiation, expressed in terms of 2θ angles, wherein the relative intensity of the diffraction peak at 22.61±0.2° is 50%-100%, and preferably the diffraction peak at 22.61±0.2° is the strongest peak.

In one embodiment, crystalline form C of the compound of formula X has an X-ray powder diffraction pattern using Cu-Kα radiation, expressed in terms of 2θ angles, having diffraction peaks at 11.30±0.2°, 22.61±0.2°, 24.69±0.2°, 26.16±0.2°, 28.57±0.2°, 6.36±0.2°, 18.61±0.2°, 12.82±0.2°, and 17.07±0.2°.

In one embodiment, crystalline form C of the compound of formula X has an X-ray powder diffraction pattern using Cu-Kα radiation, expressed in terms of 2θ angles, further having diffraction peaks at one or more of the following positions: 12.11±0.2° and 15.12±0.2°.

In one embodiment, crystalline form C of the compound of formula X has an X-ray powder diffraction pattern using Cu-Kα radiation, expressed in terms of 2θ angles, having diffraction peaks as shown in Table 19.

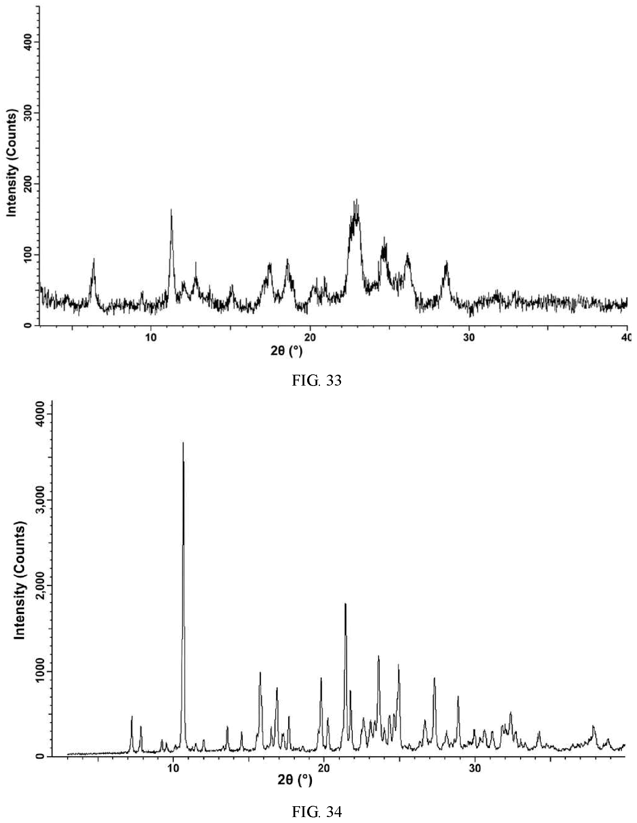

In one embodiment, crystalline form C of the compound of formula X has an X-ray powder diffraction pattern using Cu-Kα radiation substantially as shown in FIG. 33.

In one embodiment, in crystalline form C of the compound of formula X, j is 1.

In one embodiment, in crystalline form C of the compound of formula X, k is 0 and j is 1, or, z is acetonitrile and k is 0.07.

In one embodiment, the compound of formula X is crystalline form D of the compound of formula X, and its X-ray powder diffraction pattern using Cu-Kα radiation, expressed in terms of 2θ angles, has diffraction peaks at 10.68±0.2° and 21.44±0.2°;
wherein z is water.

In one embodiment, crystalline form D of the compound of formula X has an X-ray powder diffraction pattern using Cu-Kα radiation, expressed in terms of 2θ angles, further having diffraction peaks at one or more of the following positions: 15.77±0.2°, 16.88±0.2°, 19.82±0.2°, 21.76±0.2°, 23.62±0.2°, 24.95±0.2°, and 27.32±0.2°.

In one embodiment, crystalline form D of the compound of formula X has an X-ray powder diffraction pattern using Cu-Kα radiation, expressed in terms of 2θ angles, having diffraction peaks at 10.68±0.2°, 21.44±0.2°, 15.77±0.2°, 16.88±0.2°, 19.82±0.2°, 21.76±0.2°, 23.62±0.2°, and 24.95±0.2°.

In one embodiment, crystalline form D of the compound of formula X has an X-ray powder diffraction pattern using Cu-Kα radiation, expressed in terms of 2θ angles, further having diffraction peaks at one or more of the following positions: 7.27±0.2°, 7.87±0.2°, 13.59±0.2°, 14.54±0.2°, 16.25±0.2°, 17.29±0.2°, 17.66±0.2°, 19.63±0.2°, 20.27±0.2°, 22.62±0.2°, 23.09±0.2°, 24.33±0.2°, 24.64±0.2°, 26.37±0.2°, and 28.88±0.2°.

In one embodiment, crystalline form D of the compound of formula X has an X-ray powder diffraction pattern using Cu-Kα radiation, expressed in terms of 2θ angles, having diffraction peaks as shown in Table 20.

In one embodiment, crystalline form D of the compound of formula X has an X-ray powder diffraction pattern using Cu-Kα radiation substantially as shown in FIG. 34.

In one embodiment, crystalline form D of the compound of formula X has a differential scanning calorimetry (DSC) curve with endothermic peaks at peak temperatures of 62.55±3°C, 86.97±3°C, and 267.15±3°C.

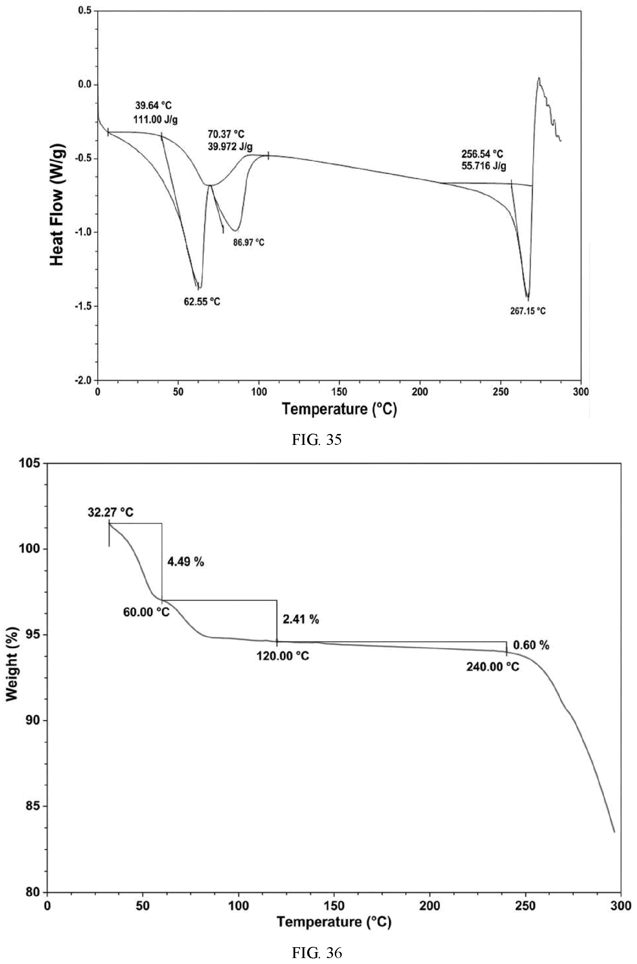

In one embodiment, the differential scanning calorimetry (DSC) curve of crystalline form D of the compound of formula X is substantially as shown in FIG. 35.

In one embodiment, crystalline form D of the compound of formula X has a thermogravimetric analysis (TGA) curve showing a weight loss of about 4.49% in the temperature range from 32.27:1:3°C to 60.0±3°C, a weight loss of about 2.41% in the temperature range from 60.0±3°C to 120.0±3°C, and a weight loss of about 0.6% in the temperature range from 120.0±3°C to 240.0±3°C.

In one embodiment, the thermogravimetric analysis (TGA) curve of crystalline form D of the compound of formula X is substantially as shown in FIG. 36.

In one embodiment, in crystalline form D of the compound of formula X, j is 1 or 1.02, for example 1.02.

In one embodiment, in crystalline form D of the compound of formula X, k is 5, 4.8, or 4, for example 4.8.

In one embodiment, in crystalline form D of the compound of formula X, z is water, j is 1, and k is 5; or, j is 1.02, and k is 4.8.

The present invention provides a compound of formula XI, 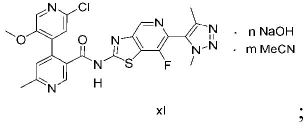 wherein n (the molar equivalent of sodium ion) is 0-1.5.

In one embodiment, in the compound of formula XI, n is 0.6, 1.1, 1.13, or 1.2.

In one embodiment, in the compound of formula XI, m is 0 (solvent-free).

In one embodiment, in the compound of formula XI, m is 0.5-1.5, for example 1, 1.07, or 1.1. In one embodiment, the compound of formula XI is crystalline form A of the compound of formula XI, and its X-ray powder diffraction pattern using Cu-Kα radiation, expressed in terms of 2θ angle, has diffraction peaks at 12.78±0.2°, 14.09±0.2°, and 14.98±0.2°.

In one embodiment, crystalline form A of the compound of formula XI has an X-ray powder diffraction pattern using Cu-Kα radiation, expressed in terms of 2θ angle, further having diffraction peaks at one or more of the following: 10.67±0.2°, 18.82±0.2°, 19.49±0.2°, and 23.58±0.2°.

In one embodiment, crystalline form A of the compound of formula XI has an X-ray powder diffraction pattern using Cu-Kα radiation, expressed in terms of 2θ angle, further having diffraction peaks at one or more of the following: 6.9±0.2°, 9.39±0.2°, 17.22±0.2°, 19.35±0.2°, 21.80±0.2°, 22.45±0.2°, 24.15±0.2°, and 27.926±0.2°.

In one embodiment, crystalline form A of the compound of formula XI has an X-ray powder diffraction pattern using Cu-Kα radiation, expressed in terms of 2θ angle, having diffraction peaks at 12.78±0.2°, 14.09±0.2°, 14.98±0.2°, 10.67±0.2°, 18.82±0.2°, 19.49±0.2°, 23.58±0.2°, and 21.80±0.2°.

In one embodiment, crystalline form A of the compound of formula XI has an X-ray powder diffraction pattern using Cu-Kα radiation, expressed in terms of 2θ angle, having diffraction peaks as shown in Table 21.

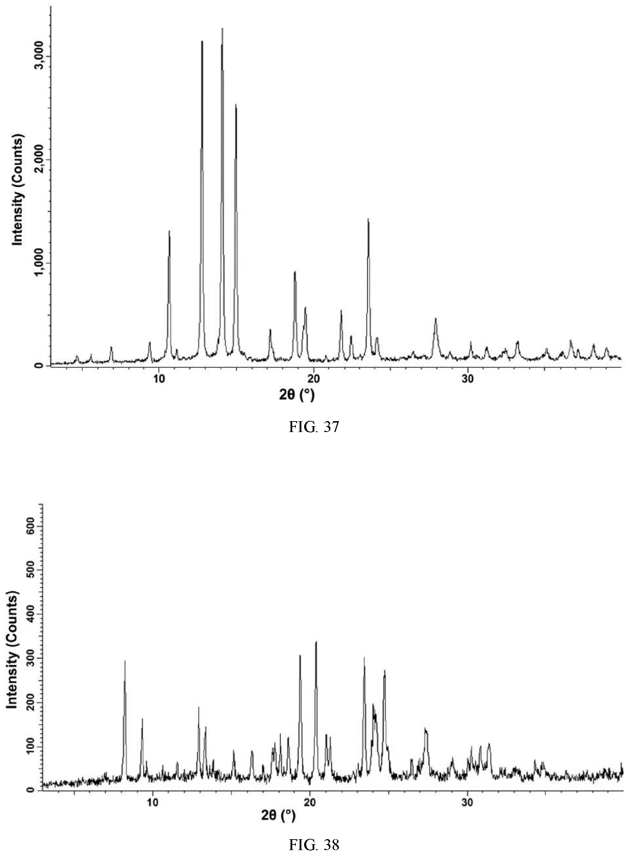

In one embodiment, crystalline form A of the compound of formula XI has an X-ray powder diffraction pattern using Cu-Kα radiation substantially as shown in FIG. 37.

In one embodiment, in crystalline form A of the compound of formula XI, m is 0, and n is 0.6, 0.7, or 0.62, for example 0.62.

In one embodiment, the compound of formula XI is crystalline form B of the compound of formula XI, and its X-ray powder diffraction pattern using Cu-Kα radiation, expressed in terms of 2θ angle, has diffraction peaks at 8.22±0.2°, 19.38±0.2°, 20.38±0.2°, 23.43±0.2°, and 24.74±0.2°.

In one embodiment, crystalline form B of the compound of formula XI has an X-ray powder diffraction pattern using Cu-Kα radiation, expressed in terms of 2θ angle, further having diffraction peaks at one or more of the following: 9.31±0.2°, 12.92±0.2°, 13.62±0.2°, 24.17±0.2°, and 27.37±0.2°.

In one embodiment, crystalline form B of the compound of formula XI has an X-ray powder diffraction pattern using Cu-Kα radiation, expressed in terms of 2θ angle, having diffraction peaks at 8.22±0.2°, 19.38±0.2°, 20.38±0.2°, 23.43±0.2°, 24.74±0.2°, 9.31±0.2°, 12.92±0.2°, and 24.17±0.2°.

In one embodiment, crystalline form B of the compound of formula XI has an X-ray powder diffraction pattern using Cu-Kα radiation, expressed in terms of 2θ angle, further having diffraction peaks at one or more of the following: 15.13±0.2°, 16.29±0.2°, 17.63±0.2°, 17.77±0.2°, 18.12±0.2°, 18.63±0.2°, 21.04±0.2°, 21.28±0.2°, and 26.46±0.2°.

In one embodiment, crystalline form B of the compound of formula XI has an X-ray powder diffraction pattern using Cu-Kα radiation, expressed in terms of 2θ angle, having diffraction peaks as shown in Table 22.

In one embodiment, crystalline form B of the compound of formula XI has an X-ray powder diffraction pattern using Cu-Kα radiation substantially as shown in FIG. 38.

In one embodiment, in crystalline form B of the compound of formula XI, n is 1.1, 1.2, or 1.13, for example 1.13.

In one embodiment, in crystalline form B of the compound of formula XI, n is 1.1 and m is 1.1; or, n is 1.13 and m is 1.07.

In one embodiment, the compound of formula IX is crystalline form C of the compound of formula IX, and its X-ray powder diffraction pattern using Cu-Kα radiation, expressed in terms of 2θ angle, has diffraction peaks at 5.23±0.2° and 19.82±0.2°.

In one embodiment, crystalline form C of the compound of formula IX has an X-ray powder diffraction pattern using Cu-Kα radiation, expressed in terms of 2θ angle, further having diffraction peaks at one or more of the following: 9.21±0.2°, 16.11±0.2°, 16.57±0.2°, 19.39±0.2°, and 20.01±0.2°.

Preferably, crystalline form C of the compound of formula IX has an X-ray powder diffraction pattern using Cu-Kα radiation, expressed in terms of 2θ angle, wherein the relative intensity of the diffraction peak at 19.82±0.2° is 60%-100%, preferably the relative intensity is 70%-80%, for example 71.2%.

In one embodiment, crystalline form C of the compound of formula IX has an X-ray powder diffraction pattern using Cu-Kα radiation, expressed in terms of 2θ angle, having diffraction peaks at 5.23±0.2°, 19.82±0.2°, 9.21±0.2°, 16.11±0.2°, 16.57±0.2°, 19.39±0.2°, 9.80±0.2°, 11.12±0.2°, and 20.01±0.2°.

In one embodiment, crystalline form C of the compound of formula IX has an X-ray powder diffraction pattern using Cu-Kα radiation, expressed in terms of 2θ angle, further having diffraction peaks at one or more of the following: 8.07±0.2°, 8.26±0.2°, 9.80±0.2°, 11.12±0.2°, 12.72±0.2°, 15.24±0.2°, 15.48±0.2°, 18.20±0.2°, 18.71±0.2°, 21.52±0.2°, 23.51±0.2°, and 23.83±0.2°.

In one embodiment, crystalline form C of the compound of formula IX has an X-ray powder diffraction pattern using Cu-Kα radiation, expressed in terms of 2θ angle, having diffraction peaks as shown in Table 23.

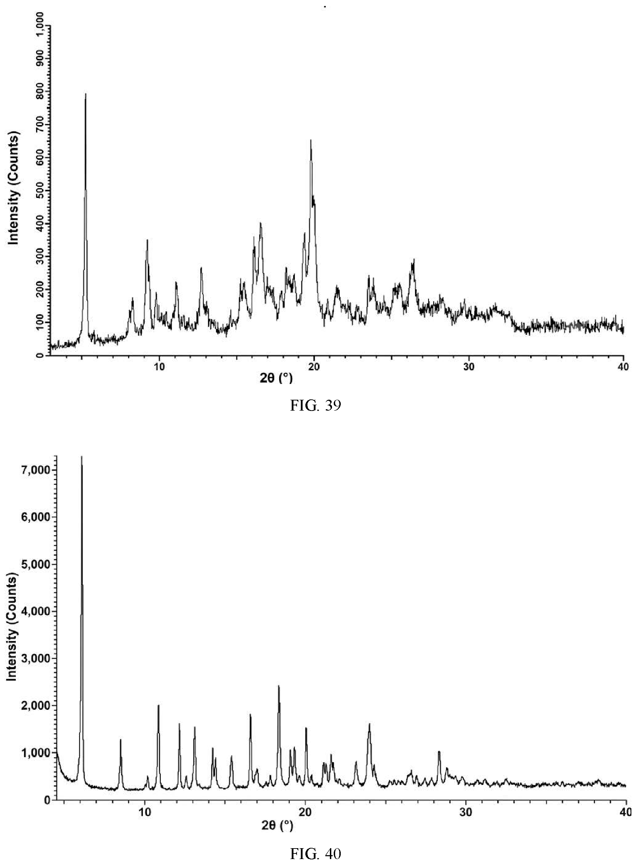

In one embodiment, crystalline form C of the compound of formula IX has an X-ray powder diffraction pattern using Cu-Kα radiation substantially as shown in FIG. 39.

In one embodiment, in crystalline form C of the compound of formula IX, d is 1, 1.1, or 1.02, for example 1.02.

In one embodiment, in crystalline form C of the compound of formula IX, g is tetrahydrofuran, d is 1, and h is 0.2; or, g is tetrahydrofuran, d is 1.02, and h is 0.16; or, d is 1 and h is 0.

In one embodiment, the compound of formula IX is crystalline form D of the compound of formula IX, and its X-ray powder diffraction pattern using Cu-Kα radiation, expressed in terms of 2θ angle, has diffraction peaks at 6.13±0.2°, 10.87±0.2°, and 18.38±0.2°.

In one embodiment, crystalline form D of the compound of formula IX has an X-ray powder diffraction pattern using Cu-Kα radiation, expressed in terms of 2θ angle, further having diffraction peaks at one or more of the following: 8.52±0.2°, 12.18±0.2°, 13.12±0.2°, 16.60±0.2°, 20.07±0.2°, and 24.00±0.2°.

Preferably, crystalline form D of the compound of formula IX has an X-ray powder diffraction pattern using Cu-Kα radiation, expressed in terms of 2θ angle, wherein the relative intensity of the diffraction peak at 6.13±0.2° is 80%-100%, and preferably the diffraction peak at 6.13±0.2° is the strongest peak.

In one embodiment, the compound of formula IX is crystalline form D of the compound of formula IX, and its X-ray powder diffraction pattern using Cu-Kα radiation, expressed in terms of 2θ angle, has diffraction peaks at 6.13±0.2°, 10.87±0.2°, 18.38±0.2°, 8.52±0.2°, 12.18±0.2°, 13.12±0.2°, 16.60±0.2°, 20.07±0.2°, and 24.00±0.2°.

In one embodiment, crystalline form D of the compound of formula IX has an X-ray powder diffraction pattern using Cu-Kα radiation, expressed in terms of 2θ angle, further having diffraction peaks at one or more of the following: 14.25±0.2°, 14.42±0.2°, 17.00±0.2°, 19.09±0.2°, 19.34±0.2°, 21.12±0.2°, 21.28±0.2°, 21.59±0.2°, 21.72±0.2°, 23.14±0.2°, and 24.27±0.2°.

In one embodiment, crystalline form D of the compound of formula IX has an X-ray powder diffraction pattern using Cu-Kα radiation, expressed in terms of 2θ angle, having diffraction peaks as shown in Table 24.

In one embodiment, crystalline form D of the compound of formula IX has an X-ray powder diffraction pattern using Cu-Kα radiation substantially as shown in FIG. 40.

In one embodiment, in crystalline form D of the compound of formula IX, d is 1/3.

In one embodiment, in crystalline form D of the compound of formula IX, g is water, d is 1, and h is 0, 1, or 3.

In one embodiment, the compound of formula IX is crystalline form E of the compound of formula IX, and its X-ray powder diffraction pattern using Cu-Kα radiation, expressed in terms of 2θ angle, has diffraction peaks at 5.66±0.2°, 11.50±0.2°, 17.97±0.2°, 19.83±0.2°, 23.95±0.2°, and 26.58±0.2°.

In one embodiment, crystalline form E of the compound of formula IX has an X-ray powder diffraction pattern using Cu-Kα radiation, expressed in terms of 2θ angle, further having diffraction peaks at one or more of the following: 13.85±0.2°, 14.30±0.2°, 17.74±0.2°, 19.04±0.2°, 21.93±0.2°, 22.25±0.2°, 23.08±0.2°, and 23.43±0.2°.

In one embodiment, crystalline form E of the compound of formula IX has an X-ray powder diffraction pattern using Cu-Kα radiation, expressed in terms of 2θ angle, having diffraction peaks at 5.66±0.2°, 11.50±0.2°, 17.97±0.2°, 19.83±0.2°, 23.95±0.2°, 13.85±0.2°, 14.30±0.2°, 17.74±0.2°, 19.04±0.2°, and 21.93±0.2°.

In one embodiment, crystalline form E of the compound of formula IX has an X-ray powder diffraction pattern using Cu-Kα radiation, expressed in terms of 2θ angle, further having diffraction peaks at one or more of the following: 8.45±0.2°, 9.02±0.2°, 9.84±0.2°, 11.19±0.2°, 12.70±0.2°, 15.59±0.2°, 16.56±0.2°, 16.93±0.2°, 18.75±0.2°, 20.55±0.2°, 25.05±0.2°, and 26.98±0.2°.

In one embodiment, crystalline form E of the compound of formula IX has an X-ray powder diffraction pattern using Cu-Kα radiation, expressed in terms of 2θ angle, having diffraction peaks as shown in Table 25.

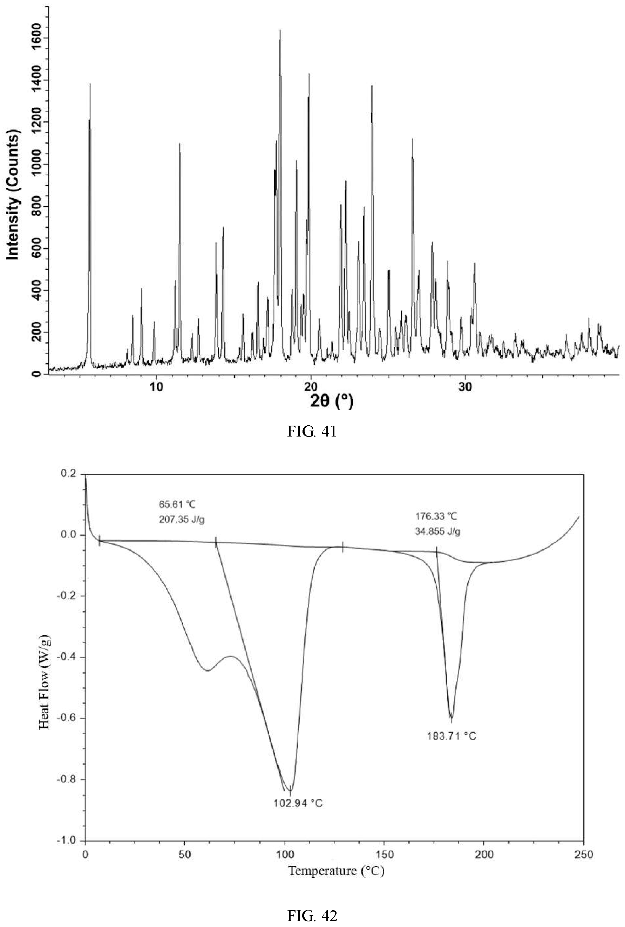

In one embodiment, crystalline form E of the compound of formula IX has an X-ray powder diffraction pattern using Cu-Kα radiation substantially as shown in FIG. 41.

In one embodiment, crystalline form E of the compound of formula IX has a differential scanning calorimetry (DSC) curve with endothermic peaks at peak temperatures of 102.94±3°C and 183.71±3°C.

In one embodiment, the differential scanning calorimetry (DSC) curve of crystalline form E of the compound of formula IX is substantially as shown in FIG. 42.

In one embodiment, crystalline form E of the compound of formula IX has a thermogravimetric analysis (TGA) curve showing a weight loss of about 3.11% in the temperature range from 33.06±3°C to 55.0±3°C, a weight loss of about 3.82% in the temperature range from 55.0±3°C to 120.0±3°C, and a weight loss of about 1.21% in the temperature range from 120.0±3°C to 190.0±3°C.

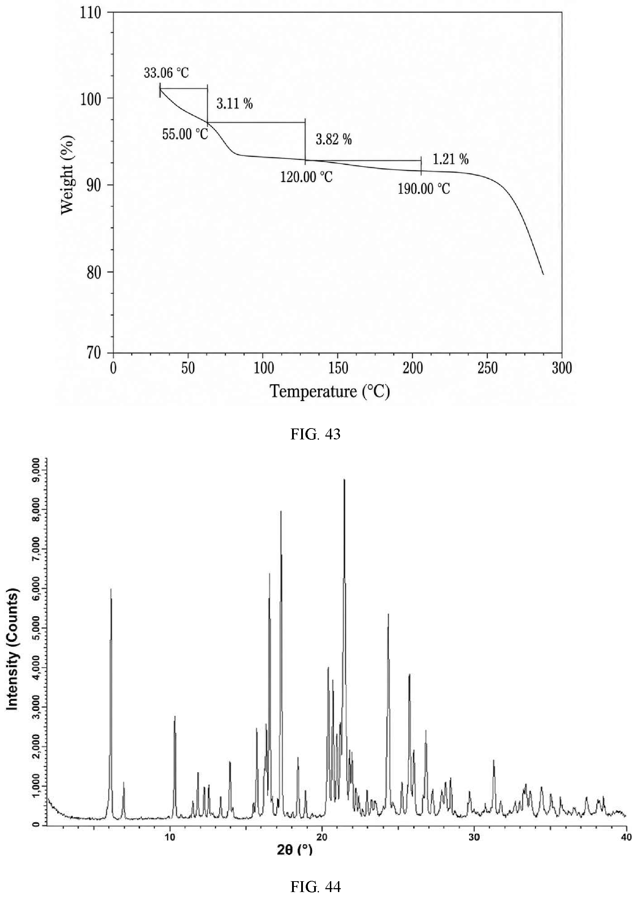

In one embodiment, the thermogravimetric analysis (TGA) curve of crystalline form E of the compound of formula IX is substantially as shown in FIG. 43.

In one embodiment, in crystalline form E of the compound of formula IX, d is 1, 1.1, or 1.02, for example, 1.02.

In one embodiment, in crystalline form E of the compound of formula IX, d is 1, g is isopropanol, and h is 1; or, d is 1.02, g is isopropanol, and h is 0.98.

In one embodiment, the compound of formula IX is crystalline form F of the compound of formula IX, and its X-ray powder diffraction pattern using Cu-Kα radiation, expressed in terms of 2θ angle, has diffraction peaks at 6.15±0.2°, 16.55±0.2°, 17.30±0.2°, and 21.48±0.2°.

In one embodiment, crystalline form F of the compound of formula IX has an X-ray powder diffraction pattern using Cu-Kα radiation, expressed in terms of 2θ angle, further having diffraction peaks at one or more of the following: 10.34±0.2°, 15.73±0.2°, 16.34±0.2°, 20.42±0.2°, 20.72±0.2°, 24.34±0.2°, and 25.74±0.2°.

In one embodiment, crystalline form F of the compound of formula IX has an X-ray powder diffraction pattern using Cu-Kα radiation, expressed in terms of 2θ angle, with diffraction peaks at 6.15±0.2°, 16.55±0.2°, 17.30±0.2°, 21.48±0.2°, 10.34±0.2°, 15.73±0.2°, 16.34±0.2°, 20.42±0.2°, and 20.72±0.2°.

In one embodiment, crystalline form F of the compound of formula IX has an X-ray powder diffraction pattern using Cu-Kα radiation, expressed in terms of 2θ angle, further having diffraction peaks at one or more of the following: 6.99±0.2°, 11.85±0.2°, 12.27±0.2°, 12.57±0.2°, 13.97±0.2°, 18.42±0.2°, 20.98±0.2°, 21.22±0.2°, 21.83±0.2°, 21.97±0.2°, 26.02±0.2°, and 26.83±0.2°.

In one embodiment, crystalline form F of the compound of formula IX has an X-ray powder diffraction pattern using Cu-Kα radiation, expressed in terms of 2θ angle, having diffraction peaks as shown in Table 26.

In one embodiment, the X-ray powder diffraction pattern of crystalline form F of the compound of formula IX, using Cu-Kα radiation, is substantially as shown in FIG. 44.

In one embodiment, crystalline form F of the compound of formula IX has a differential scanning calorimetry (DSC) curve with an endothermic peak at a peak temperature of 266.84±3°C.

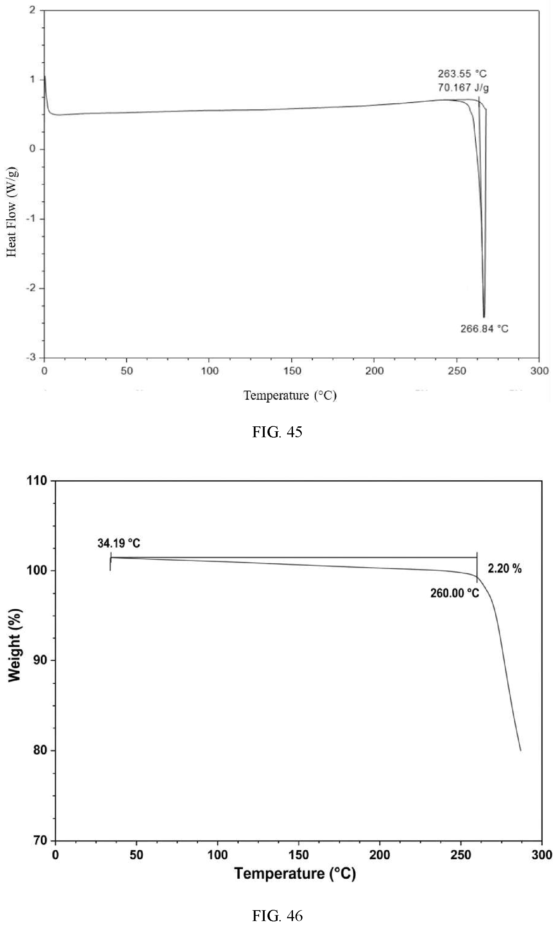

In one embodiment, the differential scanning calorimetry (DSC) curve of crystalline form F of the compound of formula IX is substantially as shown in FIG. 45.

In one embodiment, crystalline form F of the compound of formula IX has a thermogravimetric analysis (TGA) curve showing a weight loss of about 2.2% in the temperature range from 34.19±3°C to 260.0±3°C.

In one embodiment, the thermogravimetric analysis (TGA) curve of crystalline form F of the compound of formula IX is substantially as shown in FIG. 46.

In one embodiment, in crystalline form F of the compound of formula IX, d is 1, 1.1, or 1.02, for example, 1.02.

In one embodiment, in crystalline form F of the compound of formula IX, d is 1, and h is 0.

In one embodiment, the compound of formula IX is crystalline form G of the compound of formula IX, and its X-ray powder diffraction pattern using Cu-Kα radiation, expressed in terms of 2θ angle, has diffraction peaks at 7.65±0.2°, 13.76±0.2°, 20.35±0.2°, 21.01±0.2°, and 21.57±0.2°;
g is 1,4-dioxane.

In one embodiment, crystalline form G of the compound of formula IX has an X-ray powder diffraction pattern using Cu-Kα radiation, expressed in terms of 2θ angle, further having diffraction peaks at one or more of the following: 23.83±0.2°, 25.37±0.2°, 25.63±0.2°, and 27.60±0.2°.

In one embodiment, crystalline form G of the compound of formula IX has an X-ray powder diffraction pattern using Cu-Kα radiation, expressed in terms of 2θ angle, with diffraction peaks at 7.65±0.2°, 13.76±0.2°, 20.35±0.2°, 21.01±0.2°, 21.57±0.2°, 23.83±0.2°, 25.37±0.2°, 25.63±0.2°, and 27.60±0.2°.

In one embodiment, crystalline form G of the compound of formula IX has an X-ray powder diffraction pattern using Cu-Kα radiation, expressed in terms of 2θ angle, further having diffraction peaks at one or more of the following: 11.87±0.2°, 12.60±0.2°, 12.76±0.2°, 16.07±0.2°, 20.05±0.2°, 22.75±0.2°, and 23.83±0.2°.

In one embodiment, crystalline form G of the compound of formula IX has an X-ray powder diffraction pattern using Cu-Kα radiation, expressed in terms of 2θ angle, with diffraction peaks as shown in Table 27.

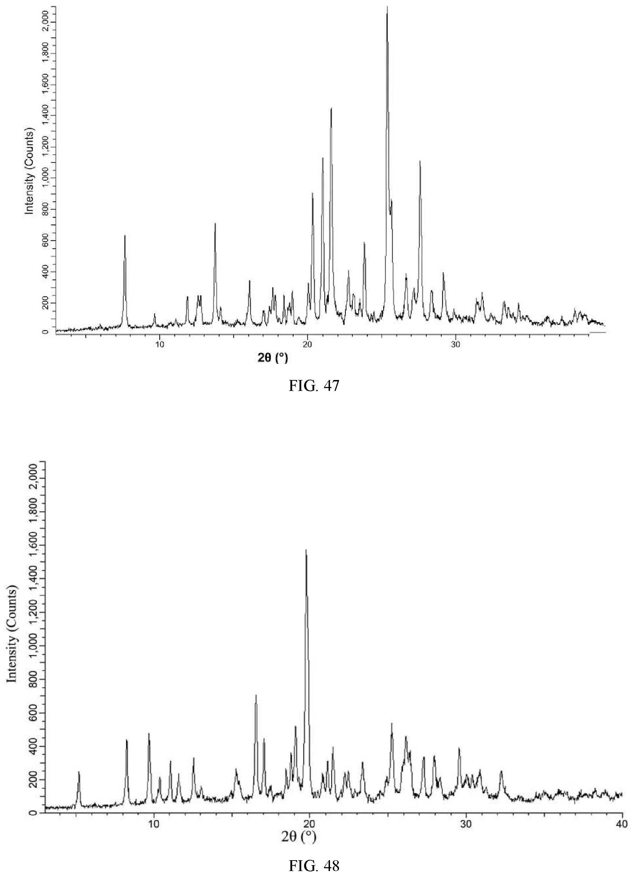

In one embodiment, the X-ray powder diffraction pattern of crystalline form G of the compound of formula IX using Cu-Kα radiation is substantially as shown in FIG. 47.

In one embodiment, in crystalline form G of the compound of formula IX, d is 1, 1.1, or 1.2, for example, 1.1.

In one embodiment, in crystalline form G of the compound of formula IX, d is 1, g is 1,4-dioxane, and h is 3; or, d is 1.1, g is 1,4-dioxane, and h is 2.96.

In one embodiment, the compound of formula IX is crystalline form H of the compound of formula IX, and its X-ray powder diffraction pattern using Cu-Kα radiation, expressed in terms of 2θ angle, has diffraction peaks at 8.27±0.2°, 9.70±0.2°, 16.55±0.2°, and 19.78±0.2°;
g is methyl tert-butyl ether.

In one embodiment, crystalline form H of the compound of formula IX has an X-ray powder diffraction pattern using Cu-Kα radiation, expressed in terms of 2θ angle, further having diffraction peaks at one or more of the following: 10.39±0.2°, 11.07±0.2°, 11.60±0.2°, 12.56±0.2°, 17.07±0.2°, 19.09±0.2°, 21.12±0.2°, 21.46±0.2°, 25.23±0.2°, and 27.27±0.2°.

In one embodiment, crystalline form H of the compound of formula IX has an X-ray powder diffraction pattern using Cu-Kα radiation, expressed in terms of 2θ angle, with diffraction peaks at 8.27±0.2°, 9.70±0.2°, 16.55±0.2°, 19.78±0.2°, 11.07±0.2°, 12.56±0.2°, 17.07±0.2°, 19.09±0.2°, and 21.46±0.2°.

In one embodiment, crystalline form H of the compound of formula IX has an X-ray powder diffraction pattern using Cu-Kα radiation, expressed in terms of 2θ angle, further having diffraction peaks at one or more of the following: 5.21±0.2°, 11.60±0.2°, 15.29±0.2°, 18.79±0.2°, and 23.36±0.2°.

In one embodiment, crystalline form H of the compound of formula IX has an X-ray powder diffraction pattern using Cu-Kα radiation, expressed in terms of 2θ angle, with diffraction peaks as shown in Table 28.

In one embodiment, crystalline form H of the compound of formula IX has an X-ray powder diffraction pattern using Cu-Kα radiation substantially as shown in FIG. 48.

In one embodiment, crystalline form H of the compound of formula IX has a differential scanning calorimetry (DSC) curve with endothermic peaks at peak temperatures of 45.53±3°C, 155.23±3°C, and 191.45±3°C.

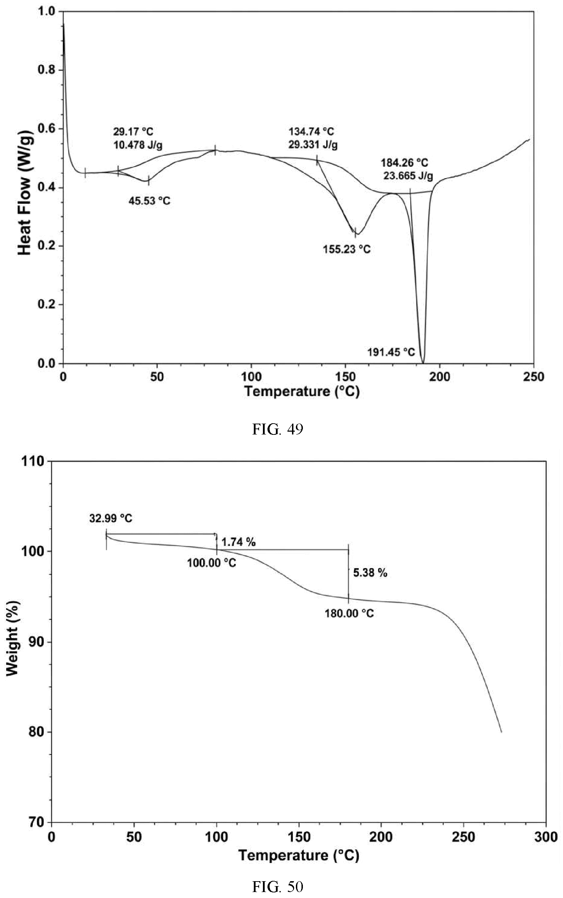

In one embodiment, the differential scanning calorimetry (DSC) curve of crystalline form H of the compound of formula IX is substantially as shown in FIG. 49.

In one embodiment, crystalline form H of the compound of formula IX has a thermogravimetric analysis (TGA) curve showing a weight loss of about 1.74% in the temperature range from 32.99±3°C to 100.0±3°C, and a weight loss of about 5.38% in the temperature range from 100.0±3°C to 180.0±3°C.

In one embodiment, the thermogravimetric analysis (TGA) curve of crystalline form H of the compound of formula IX is substantially as shown in FIG. 50.

In one embodiment, in crystalline form H of the compound of formula IX, g is methyl tert-butyl ether, and h is 0.5, 0.4, or 0.45, for example, 0.45.

In one embodiment, in crystalline form H of the compound of formula IX, d is 1:1 (i.e., 1).

In one embodiment, in crystalline form H of the compound of formula IX, g is methyl tert-butyl ether, h is 0.5, and d is 1.

In one embodiment, the compound of formula IX is crystalline form I of the compound of formula IX, and its X-ray powder diffraction pattern using Cu-Kα radiation, expressed in terms of 2θ angle, has diffraction peaks at 6.01±0.2°, 10.63±0.2°, and 12.79±0.2°.

In one embodiment, crystalline form I of the compound of formula IX has an X-ray powder diffraction pattern using Cu-Kα radiation, expressed in terms of 2θ angle, further having diffraction peaks at one or more of the following: 14.20±0.2°, 16.67±0.2°, 18.00±0.2°, 18.74±0.2°, 20.07±0.2°, and 24.06±0.2°.

Preferably, crystalline form I of the compound of formula IX has an X-ray powder diffraction pattern using Cu-Kα radiation, expressed in terms of 2θ angle, wherein the relative intensity of the diffraction peak at 6.01±0.2° is 80%-100%, and preferably the diffraction peak at 6.01±0.2° is the strongest peak.

In one embodiment, crystalline form I of the compound of formula IX has an X-ray powder diffraction pattern using Cu-Kα radiation, expressed in terms of 2θ angle, having diffraction peaks at 6.01±0.2°, 10.63±0.2°, 12.79±0.2°, 14.20±0.2°, 16.67±0.2°, 18.00±0.2°, 20.07±0.2°, and 24.06±0.2°.

In one embodiment, crystalline form I of the compound of formula IX has an X-ray powder diffraction pattern using Cu-Kα radiation, expressed in terms of 2θ angle, further having diffraction peaks at one or more of the following: 11.99±0.2°, 17.84±0.2°, and 23.22±0.2°.

In one embodiment, crystalline form I of the compound of formula IX has an X-ray powder diffraction pattern using Cu-Kα radiation, expressed in terms of 2θ angle, having diffraction peaks as shown in Table 29.

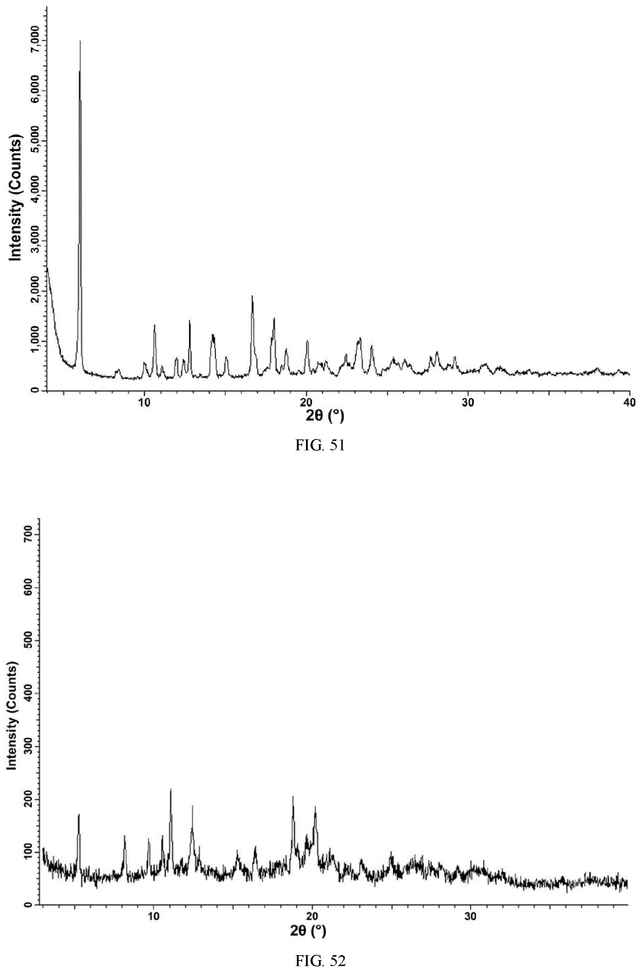

In one embodiment, crystalline form I of the compound of formula IX has an X-ray powder diffraction pattern using Cu-Kα radiation substantially as shown in FIG. 51.

In one embodiment, in crystalline form I of the compound of formula IX, d is 1.

In one embodiment, in crystalline form I of the compound of formula IX, h is 0.

In one embodiment, in crystalline form I of the compound of formula IX, d is 1 and h is 0.

In one embodiment, the compound of formula IX is crystalline form J of the compound of formula IX, and its X-ray powder diffraction pattern using Cu-Kα radiation, expressed in terms of 2θ angle, has diffraction peaks at 5.27±0.2°, 8.18±0.2°, 9.72±0.2°, 10.54±0.2°, 11.06±0.2°, 12.44±0.2°, 18.79±0.2°, and 20.21±0.2°.

In one embodiment, crystalline form J of the compound of formula IX has an X-ray powder diffraction pattern using Cu-Kα radiation, expressed in terms of 2θ angle, further having diffraction peaks at one or more of the following: 15.30±0.2°, 16.41±0.2°, 18.79±0.2°, 19.07±0.2°, 19.66±0.2°, and 21.25±0.2°.

In one embodiment, crystalline form J of the compound of formula IX has an X-ray powder diffraction pattern using Cu-Kα radiation, expressed in terms of 2θ angle, having diffraction peaks as shown in Table 30.

In one embodiment, crystalline form J of the compound of formula IX has an X-ray powder diffraction pattern using Cu-Kα radiation substantially as shown in FIG. 52.

In one embodiment, in crystalline form J of the compound of formula IX, d is 1.

In one embodiment, in crystalline form J of the compound of formula IX, d is 1 and h is 0.

The present invention provides a method for preparing the compound of formula II described above, comprising the step of: reacting the compound of formula II (e.g., crystalline form A of the compound of formula II) with a mixed solvent of a ketone solvent (e.g., acetone) and water to obtain the compound of formula II; preferably, the mixed solvent of the ketone solvent (e.g., acetone) and water is a mixed solution of acetone and water in a volume ratio of 19:1 (acetone/water).

The present invention provides a method for preparing the compound of formula III described above, comprising the step of: performing a salt formation reaction of the compound of formula I with hydrochloric acid in an ester solvent (e.g., ethyl acetate) or a nitrile solvent (e.g., acetonitrile) to obtain the compound of formula III.

The present invention provides a method for preparing the compound of formula IV described above, comprising the step of: performing a salt formation reaction of the compound of formula I with sulfuric acid in an ester solvent (e.g., ethyl acetate) or a nitrile solvent (e.g., acetonitrile) to obtain the compound of formula IV.

The present invention provides a method for preparing the compound of formula V described above, comprising the step of: performing a salt formation reaction of the compound of formula I with phosphoric acid in an ester solvent (e.g., ethyl acetate) or a nitrile solvent (e.g., acetonitrile) to obtain the compound of formula V.

The present invention provides a method for preparing the compound of formula VI or formula VII described above, comprising the step of: performing a salt formation reaction of the compound of formula I with methanesulfonic acid or fumaric acid in a nitrile solvent (e.g., acetonitrile) to obtain the compound of formula VI or formula VII.

The present invention provides a method for preparing the compound of formula VIII described above, comprising the step of: performing a salt formation reaction of the compound of formula I with benzenesulfonic acid in an ester solvent (e.g., ethyl acetate) to obtain the compound of formula VIII.

The present invention provides a method for preparing the compound of formula IX described above, comprising the step of: performing a salt formation reaction of the compound of formula I with p-toluenesulfonic acid in a mixed solvent of a ketone solvent (e.g., acetone) and water, or in a nitrile solvent (e.g., acetonitrile), to obtain the compound of formula IX; preferably, the mixed solvent of the ketone solvent (e.g., acetone) and water is a mixed solution of acetone and water in a volume ratio of 19:1 (acetone/water);
or, mixing the compound of formula IX with a solvent and drying to obtain the compound of formula IX; wherein the solvent is one or more selected from an ether solvent (e.g., tetrahydrofuran, 1,4-dioxane, or methyl tert-butyl ether), an alcohol solvent (e.g., isopropanol), and a nitrile solvent (e.g., acetonitrile).

The present invention provides a method for preparing the compound of formula X described above, comprising the step of: performing a salt formation reaction of the compound of formula I with 1,5-naphthalenedisulfonic acid in a mixed solvent of a ketone solvent (e.g., acetone) and water, an ester solvent (e.g., ethyl acetate), or a nitrile solvent (e.g., acetonitrile), to obtain the compound of formula X; preferably, the mixed solvent of the ketone solvent and water is a mixed solution of acetone and water in a volume ratio of 19:1 (acetone/water).

The present invention provides a method for preparing the compound of formula XI described above, comprising the step of: performing a salt formation reaction of the compound of formula I with NaOH in an ester solvent (e.g., ethyl acetate) to obtain the compound of formula XI.

The present invention provides a method for preparing crystalline form B of the compound of formula II, crystalline form C of the compound of formula I, crystalline form A of the compound of formula III described above, crystalline form B of the compound of formula III, crystalline form C of the compound of formula III, crystalline form A of the compound of formula IV, crystalline form B of the compound of formula IV, crystalline form A of the compound of formula V, crystalline form B of the compound of formula V, crystalline form C of the compound of formula V, crystalline form A of the compound of formula VI, crystalline form A of the compound of formula VII, crystalline form A of the compound of formula VIII, crystalline form A of the compound of formula IX, crystalline form B of the compound of formula IX, crystalline form A of the compound of formula X, crystalline form B of the compound of formula X, crystalline form C of the compound of formula X, crystalline form D of the compound of formula X, crystalline form A of the compound of formula XI, or crystalline form B of the compound of formula XI, comprising the steps of: cooling a mixture of the compound and a solvent to induce crystallization, and drying to obtain the crystalline form;
wherein, when the compound is the compound of formula II and the solvent is a mixed solvent of acetone and water, crystalline form B of the compound of formula II is obtained;
when the compound is the compound of formula II and the solvent is ethyl acetate or acetonitrile, crystalline form C of the compound of formula I is obtained;
when the compound is the compound of formula III and the solvent is ethyl acetate, crystalline form A or crystalline form B of the compound of formula III is obtained;
when the compound is the compound of formula III and the solvent is acetonitrile, crystalline form C of the compound of formula III is obtained;
when the compound is the compound of formula IV and the solvent is ethyl acetate, crystalline form A of the compound of formula IV is obtained;
when the compound is the compound of formula IV and the solvent is acetonitrile, crystalline form B of the compound of formula IV is obtained;
when the compound is the compound of formula V and the solvent is ethyl acetate, crystalline form A of the compound of formula V is obtained;
when the compound is the compound of formula V and the solvent is acetonitrile, crystalline form B of the compound of formula V is obtained;
when the compound is the compound of formula V and the solvent is acetonitrile, and crystalline form B of the compound of formula V is added to the mixture, crystalline form C of the compound of formula V is obtained;
when the compound is the compound of formula VI and the solvent is acetonitrile, crystalline form A of the compound of formula VI is obtained;
when the compound is the compound of formula VII and the solvent is acetonitrile, crystalline form A of the compound of formula VII is obtained;
when the compound is the compound of formula VIII and the solvent is ethyl acetate, crystalline form A of the compound of formula VIII is obtained;
when the compound is the compound of formula IX and the solvent is a mixed solution of acetone and water, crystalline form A of the compound of formula IX is obtained;
when the compound is the compound of formula IX and the solvent is acetonitrile, crystalline form B of the compound of formula IX is obtained;
when the compound is the compound of formula X and the solvent is a mixed solution of acetone and water, crystalline form A of the compound of formula X is obtained;
when the compound is the compound of formula X and the solvent is ethyl acetate, crystalline form B of the compound of formula X is obtained;
when the compound is the compound of formula X and the solvent is acetonitrile, crystalline form C of the compound of formula X is obtained;
when the compound is the compound of formula X and the solvent is a mixed solution of acetone and water, crystalline form D of the compound of formula X is obtained;
when the compound is the compound of formula XI and the solvent is ethyl acetate, crystalline form A of the compound of formula XI is obtained;
when the compound is the compound of formula XI and the solvent is acetonitrile, crystalline form B of the compound of formula XI is obtained.

The present invention provides a method for preparing crystalline form C, crystalline form E, crystalline form F, crystalline form G, and crystalline form H of the compound of formula IX, comprising the following steps: subjecting a mixture of the compound and a solvent to drying and crystallization to obtain the crystalline form;
when the compound is the compound of formula IX (preferably crystalline form A of the compound of formula IX) and the solvent is tetrahydrofuran, crystalline form C of the compound of formula IX is obtained;
when the compound is the compound of formula IX (preferably crystalline form A of the compound of formula IX) and the solvent is isopropanol, crystalline form E of the compound of formula IX is obtained;
when the compound is the compound of formula IX (preferably crystalline form A of the compound of formula IX) and the solvent is acetonitrile and n-heptane, crystalline form F of the compound of formula IX is obtained;
when the compound is the compound of formula IX (preferably crystalline form A of the compound of formula IX) and the solvent is 1,4-dioxane, crystalline form G of the compound of formula IX is obtained;
when the compound is the compound of formula IX (preferably crystalline form A of the compound of formula IX) and the solvent is a mixed solution of acetonitrile and methyl tert-butyl ether, crystalline form H of the compound of formula IX is obtained.

In the preparation method, the cooling crystallization is, for example, cooling from 50°C to 25°C.

In the preparation method, the mixed solution of acetone and water may be a mixed solution of acetone and water with a volume ratio of 19:1 (acetone/water).

In the preparation method, the drying is, for example, centrifugation and/or vacuum drying.

Preferably, the method for preparing crystalline form B of the compound of formula II comprises the following steps: subjecting crystalline form A of the compound of formula II to crystallization with a mixture of acetone and water (e.g., a suspension) to obtain crystalline form B of the compound of formula II.

In the method for preparing crystalline form B of the compound of formula II, the crystallization is, for example, cooling crystallization (e.g., cooling from 50°C to 25°C) and drying (e.g., centrifugation and/or vacuum drying) to obtain crystalline form B of the compound of formula II.

In the method for preparing crystalline form B of the compound of formula II, the mixed solution of acetone and water may be a mixed solution of acetone and water with a volume ratio of 19:1 (acetone/water).

Preferably, the method for preparing crystalline form C of the compound of formula I comprises the following steps: subjecting crystalline form A of the compound of formula II to crystallization with a mixture of ethyl acetate or acetonitrile (e.g., a suspension) to obtain crystalline form C of the compound of formula I.

In one embodiment, in the method for preparing crystalline form C of the compound of formula I, the crystallization is cooling crystallization (e.g., cooling from 50°C to 25°C) and drying (e.g., centrifugation and/or vacuum drying).

Preferably, the method for preparing crystalline form A of the compound of formula III, crystalline form B of the compound of formula III, crystalline form C of the compound of formula III, crystalline form A of the compound of formula IV, crystalline form B of the compound of formula IV, crystalline form A of the compound of formula V, crystalline form B of the compound of formula V, crystalline form A of the compound of formula VI, crystalline form A of the compound of formula VII, crystalline form A of the compound of formula VIII, crystalline form A of the compound of formula IX, crystalline form B of the compound of formula IX, crystalline form A of the compound of formula X, crystalline form B of the compound of formula X, or crystalline form C of the compound of formula X comprises the following steps: subjecting a mixture of the compound of formula I, a solvent, and an acid to crystallization to obtain a crystalline form;
wherein the solvent is ethyl acetate and the acid is hydrochloric acid to obtain crystalline form A of the compound of formula III;
the solvent is ethyl acetate and the acid is hydrochloric acid to obtain crystalline form B of the compound of formula III;
the solvent is acetonitrile and the acid is hydrochloric acid to obtain crystalline form C of the compound of formula III;
the solvent is ethyl acetate and the acid is sulfuric acid to obtain crystalline form A of the compound of formula IV;
the solvent is acetonitrile and the acid is sulfuric acid to obtain crystalline form B of the compound of formula IV;
the solvent is ethyl acetate and the acid is phosphoric acid to obtain crystalline form A of the compound of formula V;
the solvent is acetonitrile and the acid is phosphoric acid to obtain crystalline form B of the compound of formula V;
the solvent is acetonitrile and the acid is fumaric acid to obtain crystalline form A of the compound of formula VI;
the solvent is acetonitrile and the acid is methanesulfonic acid to obtain crystalline form A of the compound of formula VII;
the solvent is ethyl acetate and the acid is benzenesulfonic acid to obtain crystalline form A of the compound of formula VIII;
the solvent is a mixed solution of acetone and water and the acid is p-toluenesulfonic acid to obtain crystalline form A of the compound of formula IX;
the solvent is acetonitrile and the acid is p-toluenesulfonic acid to obtain crystalline form B of the compound of formula IX;
the solvent is a mixed solution of acetone and water and the acid is 1,5-naphthalenedisulfonic acid to obtain crystalline form A of the compound of formula X;
the solvent is ethyl acetate and the acid is 1,5-naphthalenedisulfonic acid to obtain crystalline form B of the compound of formula X;
the solvent is acetonitrile and the acid is 1,5-naphthalenedisulfonic acid to obtain crystalline form C of the compound of formula X.

Preferably, in the preparation method, the crystallization is cooling crystallization (e.g., cooling from 50°C to 25°C) and drying (e.g., vacuum drying).

Preferably, in the preparation method, the molar ratio of the hydrochloric acid to the compound of formula I may be (0.8-1.2):1 (preferably 1:1) to obtain crystalline form A of the compound of formula III.

Preferably, in the preparation method, the molar ratio of the hydrochloric acid to the compound of formula I may be (1.6-2.2):1 (preferably 2:1) to obtain crystalline form B of the compound of formula III.

Preferably, in the preparation method, the mixed solution of acetone and water is a mixed solution of acetone and water with a volume ratio of 19:1 (acetone/water).

Preferably, in the preparation method, the compound of formula I is in a conventional form in the art (e.g., amorphous form of the compound of formula I or crystalline form A of the compound of formula II). Preferably, when obtaining crystalline form A of the compound of formula III, crystalline form B of the compound of formula III, crystalline form C of the compound of formula III, or crystalline form A of the compound of formula IV, the compound of formula I is crystalline form A of the compound of formula II.

Preferably, in the preparation method, when the acid is hydrochloric acid, sulfuric acid, or phosphoric acid, the acid is an ethyl acetate solution of the acid, for example, a mixed solution obtained by mixing a concentrated aqueous acid solution with ethyl acetate in a volume ratio of 1:9 (acid/ethyl acetate).

Preferably, the preparation method for crystalline form C of the compound of formula V comprises the following steps: mixing a mixture of the compound of formula I, acetonitrile, and phosphoric acid with crystalline form B of the compound of formula V, followed by crystallization to obtain crystalline form C of the compound of formula V;
wherein the compound of formula I and the acid are as described in any embodiment of the present invention.

Preferably, in the preparation method for crystalline form C of the compound of formula V, the crystallization is cooling crystallization (e.g., cooling from 50°C to 25°C) and drying (e.g., vacuum drying).

Preferably, the preparation methods for crystalline form C, crystalline form E, crystalline form F, crystalline form G, and crystalline form H of the compound of formula IX comprise the following step: crystallizing a mixture of crystalline form A of the compound of formula IX and a solvent;
wherein the solvent is tetrahydrofuran to obtain crystalline form C of the compound of formula IX;
the solvent is isopropanol to obtain crystalline form E of the compound of formula IX;
the solvent is acetonitrile and n-heptane to obtain crystalline form F of the compound of formula IX;
the solvent is 1,4-dioxane to obtain crystalline form G of the compound of formula IX;
the solvent is a mixed solution of acetonitrile and methyl tert-butyl ether (e.g., a mixed solution with a volume ratio of 1:4 (acetonitrile/methyl tert-butyl ether)) to obtain crystalline form H of the compound of formula IX.

In one embodiment, in the preparation method, the crystallization is drying crystallization (e.g., vacuum drying at 50°C).

In one embodiment, in the preparation method, when obtaining crystalline form G of the compound of formula IX, the crystallization is filtration.

In one embodiment, in the preparation method, when obtaining crystalline form F of the compound of formula IX, the preparation method comprises the following step: adding n-heptane to a mixed solvent of crystalline form A of the compound of formula IX and acetonitrile, followed by crystallization.

In one embodiment, in the preparation method, when obtaining crystalline form H of the compound of formula IX, the preparation method comprises the following step: adding methyl tert-butyl ether to a mixed solvent of crystalline form A of the compound of formula IX and acetonitrile, followed by crystallization.

The present invention provides a preparation method for crystalline form I of the compound of formula IX, comprising the following step: heating crystalline form A of the compound of formula IX at 25°C (preferably under nitrogen protection) to obtain crystalline form I of the compound of formula IX.

Preferably, the preparation method for crystalline form D of the compound of formula IX comprises the following step: heating crystalline form A of the compound of formula IX at 55°C (preferably under nitrogen protection) to obtain crystalline form D of the compound of formula IX.

Preferably, the preparation method for crystalline form J of the compound of formula IX comprises the following step: heating crystalline form H of the compound of formula IX at 170°C (preferably under nitrogen protection) to obtain crystalline form J of the compound of formula IX.

The present invention provides a preparation method for a single crystal of the compound of formula IX, comprising the following steps:
Step (1): mixing a mixture of the compound of formula I, acetone, and water with crystalline form A of the compound of formula IX;
Step (2): adding a mixture of toluene-4-sulfonic acid, acetone and water, and crystallizing to obtain a single crystal of the compound of formula IX.

In the preparation method for the single crystal of the compound of formula IX, in step (1), the mass ratio of the compound of formula I to acetone may be (0.5-2):(8-12), for example, 1.63:10.3.

In the preparation method for the single crystal of the compound of formula IX, in step (1), the mass ratio of acetone to water may be (9-11):(0.5-1.5), for example, 10.3:1.3.

In the preparation method for the single crystal of the compound of formula IX, in step (1), the mixing may be performed at 45-55°C.

In the preparation method for the single crystal of the compound of formula IX, in step (1), the mass ratio of crystalline form A of the compound of formula IX to the compound of formula I may be 1:100 to 1:10, for example, 8:163.

In the preparation method for the single crystal of the compound of formula IX, in step (2), the mass ratio of toluene-4-sulfonic acid, acetone, and water may be (1-2.5):(5-10):(0.5-1.5), for example, 0.58:2.57:0.32.

In the preparation method for the single crystal of the compound of formula IX, in step (2), the toluene-4-sulfonic acid may be toluene-4-sulfonic acid monohydrate.

In the preparation method for the single crystal of the compound of formula IX, in step (2), the mixture may be added in two portions, preferably the first addition is performed at 45-55°C, with a mass ratio of toluene-4-sulfonic acid to the compound of formula I of 1:(2-4), for example, 0.58:1.63, and the second addition is performed at 20-30°C, with a mass ratio of toluene-4-sulfonic acid to the compound of formula I of 1:(4-8), for example, 0.29:1.63.

In one embodiment, in the preparation method for the single crystal of the compound of formula IX, after the crystallization, filtration is performed to obtain the single crystal of the compound of formula IX.

Preferably, the preparation method for crystalline form D of the compound of formula X comprises the following step: crystallizing a mixture of crystalline form A of the compound of formula X, a mixed solution of acetone and water, and 1,5-naphthalenedisulfonic acid to obtain crystalline form D of the compound of formula X.

In one embodiment, in the preparation method for crystalline form D of the compound of formula X, the crystallization is cooling crystallization (e.g., suspension cooling from 50°C to 25°C) and drying (e.g., vacuum drying).

In one embodiment, in the preparation method for crystalline form D of the compound of formula X, the mixed solution of acetone and water is a mixed solution of acetone and water with a volume ratio of 19:1 (acetone/water).

Preferably, the preparation method for crystalline form A or crystalline form B of the compound of formula XI comprises the following step: crystallizing a mixture of the compound of formula I, a solvent, and a sodium salt (e.g., NaOH) to obtain a crystalline form of the sodium salt of the compound of formula I or a solvate thereof;
wherein, when the solvent is ethyl acetate, crystalline form A of the compound of formula XI is obtained;
when the solvent is acetonitrile, crystalline form B of the compound of formula XI is obtained. In one embodiment, in the preparation method for crystalline form A or crystalline form B of the compound of formula XI, the crystallization is cooling crystallization (e.g., suspension cooling from 50°C to 25°C) and drying (e.g., vacuum drying).

The present invention provides a pharmaceutical composition comprising substance X and a pharmaceutically acceptable excipient, wherein said substance X is the compound of formula II, III, IV, V, VI, VII, VIII, IX, X, or XI as described above, or crystalline form C of the compound of formula I as described above.

The present invention provides a use of substance X or the pharmaceutical composition as described above in the manufacture of a medicament, wherein in said use, said medicament is for treating lung cancer, breast cancer, HR-deficient ovarian cancer, gastric cancer, prostate cancer, pancreatic cancer, or colon cancer; said substance X is the compound of formula II, III, IV, V, VI, VII, VIII, IX, X, or XI as described above, or crystalline form C of the compound of formula I as described above.

Preferably, said substance X is crystalline form A of the compound of formula II, crystalline form B of the compound of formula II, crystalline form A of the compound of formula III, crystalline form B of the compound of formula III, crystalline form C of the compound of formula III, crystalline form A of the compound of formula IV, crystalline form B of the compound of formula IV, crystalline form A of the compound of formula V, crystalline form B of the compound of formula V, crystalline form C of the compound of formula V, crystalline form A of the compound of formula VI, crystalline form A of the compound of formula VII, crystalline form A of the compound of formula VIII, crystalline form A of the compound of formula X, crystalline form B of the compound of formula X, crystalline form C of the compound of formula X, crystalline form D of the compound of formula X, crystalline form A of the compound of formula XI, crystalline form B of the compound of formula XI, crystalline form A of the compound of formula IX, crystalline form B of the compound of formula IX, crystalline form C of the compound of formula IX, crystalline form D of the compound of formula IX, crystalline form E of the compound of formula IX, crystalline form F of the compound of formula IX, crystalline form G of the compound of formula IX, crystalline form H of the compound of formula IX, crystalline form I of the compound of formula IX, or crystalline form J of the compound of formula IX.

### Definition of Terms:

As used herein and unless otherwise specified, the term "solvate" refers to a crystalline form of a substance containing solvent in its crystal structure. The term "hydrate" refers to a solvate in which the solvent in the crystal structure is water.

As used herein and unless otherwise specified, the terms "about" and "approximately," when used in connection with providing a numerical value or range of values to characterize a specific solid form, indicate that the value or range of values may deviate to an extent considered reasonable by a person skilled in the art (e.g., taking error into account) while still describing said specific solid form: for example, a specific temperature or temperature range describing a melting, dehydration, desolvation, or glass transition temperature; a mass change, such as a mass change with temperature or humidity; a solvent content or water content in units such as mass or percentage; or a peak position in an analysis such as by IR or Raman spectroscopy or XRPD, or a peak position (temperature of thermal event) in an analysis such as DSC or TGA. For example, in specific embodiments, the terms "about" and "approximately," when used in this context, indicate that the numerical value or range of values may vary within ±5%, ±4%, ±3%, ±2%, ±1.5%, ±1%, ±0.5%, or ±0.25% of said value or range of values. The tilde (i.e., "~") preceding a numerical value or range of values as used herein indicates "about" or "approximately."

As used herein and unless otherwise specified, in an X-ray powder diffraction pattern, the position of a diffraction peak or the relative intensity of diffraction peaks may vary due to factors such as the measurement instrument, measurement method/conditions, etc. For any specific crystalline form, there may be an error in the peak positions, for example, the measurement error for the 2θ value is ±0.2°. Therefore, when identifying each crystalline form, this error should be taken into account, and variations within this error also fall within the scope of the present application.

As used herein, when referring to a specific salt, composition, excipient, etc., as "pharmaceutically acceptable," it means that the salt, composition, excipient, etc., is generally non-toxic, safe, and suitable for use in a subject, preferably a mammalian subject, more preferably a human subject.

As used herein, the structure of the compound of formula I is:

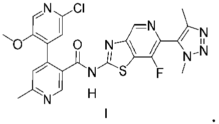

As used herein and unless otherwise specified, the term "excipient" refers to those excipients widely adopted in the field of pharmaceutical production. Excipients are primarily used to provide a safe, stable, and functional pharmaceutical composition, and may also provide means to achieve a desired dissolution rate of the active ingredient after administration to a subject, or to facilitate effective absorption of the active ingredient after administration of the composition to a subject. An excipient may be an inert filler, or it may provide a certain function, such as stabilizing the overall pH of the composition or preventing degradation of the active ingredient in the composition.

Without departing from common knowledge in the art, the aforementioned preferred conditions can be combined in any manner to obtain various preferred examples of the present invention. The reagents and raw materials used in the present invention are all commercially available.

The positive and progressive effects of the present invention are as follows: the salt forms and crystalline forms of the nitrogen-containing fused-ring compound provided by the present invention exhibit good drug activity, possess favorable stability and solubility, and have promising pharmaceutical prospects.

### BRIEF DESCRIPTION OF THE DRAWINGS

FIG. 1 is an X-ray powder diffraction pattern of crystalline form A of the compound of formula II.
FIG. 2 is a differential scanning calorimetry thermogram of crystalline form A of the compound of formula II.
FIG. 3 is a thermogravimetric analysis thermogram of crystalline form A of the compound of formula II.
FIG. 4 is an X-ray powder diffraction pattern of crystalline form B of the compound of formula II.
FIG. 5 is an X-ray powder diffraction pattern of crystalline form C of the compound of formula I.
FIG. 6 is a differential scanning calorimetry thermogram of crystalline form C of the compound of formula I.
FIG. 7 is a thermogravimetric analysis thermogram of crystalline form C of the compound of formula I.
FIG. 8 is an X-ray powder diffraction pattern of crystalline form A of the compound of formula III.
FIG. 9 is an X-ray powder diffraction pattern of crystalline form B of the compound of formula III.
FIG. 10 is a differential scanning calorimetry thermogram of crystalline form B of the compound of formula III.
FIG. 11 is a thermogravimetric analysis thermogram of crystalline form B of the compound of formula III.
FIG. 12 is an X-ray powder diffraction pattern of crystalline form C of the compound of formula III.
FIG. 13 is an X-ray powder diffraction pattern of crystalline form A of the compound of formula IV.
FIG. 14 is an X-ray powder diffraction pattern of crystalline form B of the compound of formula IV.
FIG. 15 is a differential scanning calorimetry thermogram of crystalline form B of the compound of formula IV.
FIG. 16 is a thermogravimetric analysis thermogram of crystalline form B of the compound of formula IV.
FIG. 17 is an X-ray powder diffraction pattern of crystalline form A of the compound of formula V.
FIG. 18 is a differential scanning calorimetry thermogram of crystalline form A of the compound of formula V.
FIG. 19 is a thermogravimetric analysis thermogram of crystalline form A of the compound of formula V
FIG. 20 is an X-ray powder diffraction pattern of crystalline form B of the compound of formula V
FIG. 21 is an X-ray powder diffraction pattern of crystalline form C of the compound of formula V
FIG. 22 is a differential scanning calorimetry thermogram of crystalline form C of the compound of formula V.
FIG. 23 is a thermogravimetric analysis thermogram of crystalline form C of the compound of formula V
FIG. 24 is an X-ray powder diffraction pattern of crystalline form A of the compound of formula VI.
FIG. 25 is an X-ray powder diffraction pattern of crystalline form A of the compound of formula VII.
FIG. 26 is an X-ray powder diffraction pattern of crystalline form A of the compound of formula VIII.
FIG. 27 is an X-ray powder diffraction pattern of crystalline form A of the compound of formula IX.
FIG. 28 is a differential scanning calorimetry thermogram of crystalline form A of the compound of formula IX.
FIG. 29 is a thermogravimetric analysis thermogram of crystalline form A of the compound of formula IX.
FIG. 30 is an X-ray powder diffraction pattern of crystalline form B of the compound of formula IX.
FIG. 31 is an X-ray powder diffraction pattern of crystalline form A of the compound of formula X.
FIG. 32 is an X-ray powder diffraction pattern of crystalline form B of the compound of formula X.
FIG. 33 is an X-ray powder diffraction pattern of crystalline form C of the compound of formula X.
FIG. 34 is an X-ray powder diffraction pattern of crystalline form D of the compound of formula X.
FIG. 35 is a differential scanning calorimetry thermogram of crystalline form D of the compound of formula X.
FIG. 36 is a thermogravimetric analysis thermogram of crystalline form D of the compound of formula X.
FIG. 37 is an X-ray powder diffraction pattern of crystalline form A of the compound of formula XI.
FIG. 38 is an X-ray powder diffraction pattern of crystalline form B of the compound of formula XI.
FIG. 39 is an X-ray powder diffraction pattern of crystalline form C of the compound of formula IX.
FIG. 40 is an X-ray powder diffraction pattern of crystalline form D of the compound of formula IX.
FIG. 41 is an X-ray powder diffraction pattern of crystalline form E of the compound of formula IX.
FIG. 42 is a differential scanning calorimetry thermogram of crystalline form E of the compound of formula IX.
FIG. 43 is a thermogravimetric analysis thermogram of crystalline form E of the compound of formula IX.
FIG. 44 is an X-ray powder diffraction pattern of crystalline form F of the compound of formula IX.
FIG. 45 is a differential scanning calorimetry thermogram of crystalline form F of the compound of formula IX.
FIG. 46 is a thermogravimetric analysis thermogram of crystalline form F of the compound of formula IX.
FIG. 47 is an X-ray powder diffraction pattern of crystalline form G of the compound of formula IX.
FIG. 48 is an X-ray powder diffraction pattern of crystalline form H of the compound of formula IX.
FIG. 49 is a differential scanning calorimetry thermogram of crystalline form H of the compound of formula IX.
FIG. 50 is a thermogravimetric analysis thermogram of crystalline form H of the compound of formula IX.
FIG. 51 is an X-ray powder diffraction pattern of crystalline form I of the compound of formula IX.
FIG. 52 is an X-ray powder diffraction pattern of crystalline form J of the compound of formula IX.
FIG. 53 shows XRPD patterns of crystalline form C of the compound of formula I after storage under open conditions at 25°C/60% RH and under sealed conditions at 60°C.
FIG. 54 shows XRPD patterns of crystalline form C of the compound of formula V after storage under open conditions at 25°C/60% RH and under sealed conditions at 60°C.
FIG. 55 shows XRPD patterns of crystalline form A of the compound of formula IX after storage under open conditions at 25°C/60% RH and under sealed conditions at 60°C.
FIG. 56 shows XRPD patterns of crystalline form F of the compound of formula IX after storage under open conditions at 25°C/60% RH and under sealed conditions at 60°C.
FIG. 57 is a molecular structure diagram of a single crystal of the compound of formula IX.

### DETAILED DESCRIPTION

The present invention is further illustrated by the following examples, which are not intended to limit the scope of the present invention. For experimental methods without specified conditions in the following examples, conventional methods and conditions were followed, or instructions of commercial products were selected.

The test method for X-ray powder diffraction (XRPD) involved in the present invention is shown in the following table:

| **Parameter** | |
|---|---|
| Model | Bruker A8 advance X-ray diffractometer |
| X-ray tube setting | Cu/K-Alpha1 (λ = 1.5406Å) |
| Tube voltage | 40 kV |
| Tube current | 40 mA |
| Scan range | 2°~40° |
| Step size | 0.02° |
| Step time | 0.4 or 0.3 or 0.12 or 0.06 seconds |
| Sample stage rotation speed | 15 rpm |

The single crystal test method involved in the present invention is as follows:
Model: Bruker A8 advance X-ray diffractometer
X-ray source: High-intensity micro-focus Cu/Mo automatic switching dual-source system, using diamond heat conduction technology, with Mo source power of 70 W and Cu source power of 60 W.
Micro-focus source, spot size not larger than 100 mm.
X-ray optics system, multilayer film micro-focus optics system.
Goniometer: Kappa (Kappa, ω, 2θ, φ) four-circle goniometer, equipped with an automatic goniometer head.
Detector: New semiconductor two-dimensional imaging technology detector, with both photon counting and integration functions; detection area 208×128 mm²; pixel size: ≤135 µm×135 µm, 1:1 coupling with the chip, no beam cone ratio.
Low-temperature cooling system: Supports testing in the temperature range of 80 K to 500 K.

The test methods for thermogravimetric analysis (TGA) curves and differential scanning calorimetry (DSC) curves involved in the present invention are shown in the following table:

| Parameter | DSC | TGA |
|---|---|---|
| Model | TA Instruments Discovery 2500 | TA Instruments Discovery 5500 |
| Sample pan | Tzero pan and sealed lid with a manually punched pinhole of about 0.7 mm in diameter | Aluminum pan |
| Temperature range | 0~250°C | Room temperature to 300°C |
| Heating rate (°C/min) | 10°C/min | 10°C/min |
| Purge gas | N₂, 50 mL/min | N₂, balance 10 mL/min, Sample chamber 25 mL/min |

The hygroscopicity characterization and the definition of hygroscopic weight gain involved in the present invention (refer to the Hygroscopicity Test Guideline for Drugs, Part IV, Chinese Pharmacopoeia 2020 Edition) are as follows:
Deliquescent: Absorbs sufficient moisture to form a liquid;
Extremely hygroscopic: Hygroscopic weight gain not less than 15%;
Hygroscopic: Hygroscopic weight gain less than 15% but not less than 2%;
Slightly hygroscopic: Hygroscopic weight gain less than 2% but not less than 0.2%;
Non-hygroscopic or almost non-hygroscopic: Hygroscopic weight gain less than 0.2%.

The water content test method is as follows: Karl Fischer titration (KF)

| | |
|---|---|
| Equipment model | Metrohm 851/885 system |
| Method | Coulometric titration |
| Oven temperature (Oven temperature) | 150°C |
| Extraction time | 120 s |
| Nitrogen flow rate | 50 mL/min |
| Sample amount | ~5-30 mg |

The ion chromatography test conditions involved in this application are as follows:

| | |
|---|---|
| Instrument model | Metrohm 940 professional IC |
| Liquid collector | 889 IC |
| Detector | Conductivity detector |
| Eluent (anion) | 3.2 mmol/L aqueous sodium carbonate solution + 1.0 mmol/L aqueous sodium bicarbonate solution |
| Eluent (cation) | 1.7 mmol/L HNO₃ + 0.7 mmol/L pyridinecarboxylic acid |
| Suppressor solutions | 2% H₃PO₄ |
| Column | Anion A SUPP 5-150 or Cation Column C4-150 |
| Column temperature | 30°C |
| Flow rate | 0.7 mL/min (anion) or 0.9 mL/min (cation) |
| Diluent | ACN/water (1 : 1; v : v) |
| Injection volume | 20 µL |

The nuclear magnetic resonance (NMR) test conditions involved in this application are as follows:

| | |
|---|---|
| Equipment model | Bruker Avance-AV 400M (for ¹H NMR) |
| Frequency | 400 MHz |
| Probe | 5 mm PABBO BB/19F-1H/D Z-GRD Z108618/0406 (for ¹H NMR) |
| Number of scans | 8 |
| Temperature | 297.6K |
| Relaxation delay | 1 second |

The polarized light microscopy (PLM) test conditions involved in this application are as follows:

| | |
|---|---|
| Equipment model | Leica DM4 P |
| Method | Crossed polarizers, with silicone oil (Crossed polarizer, silicone oil added) |

The high-performance liquid chromatography (HPLC) test conditions involved in this application are as follows:

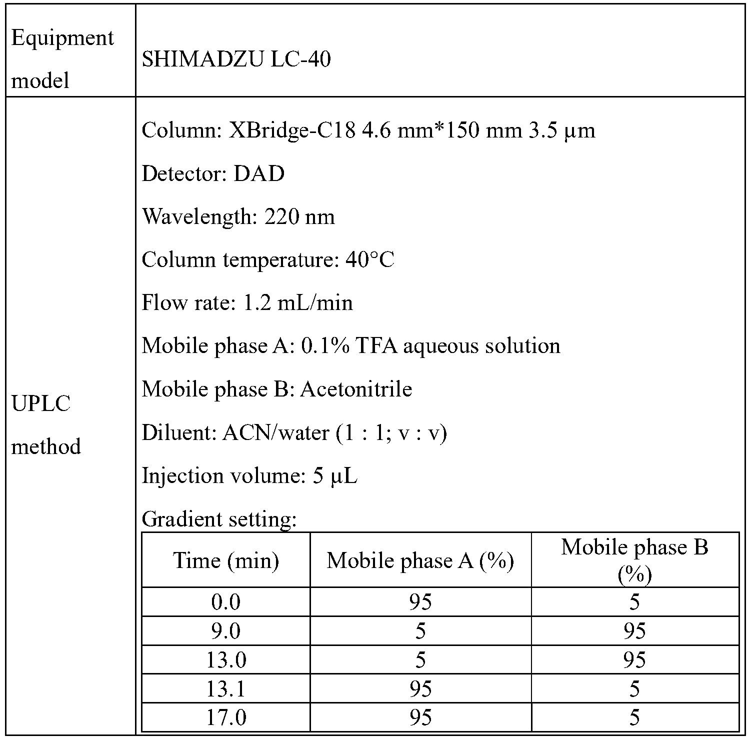

### Example 1. Preparation method of the compound of Formula II

### Intermediate 1: 2'-chloro-5'-methoxy-6-methyl-[4,4'-bipyridine]-3-carboxylic acid

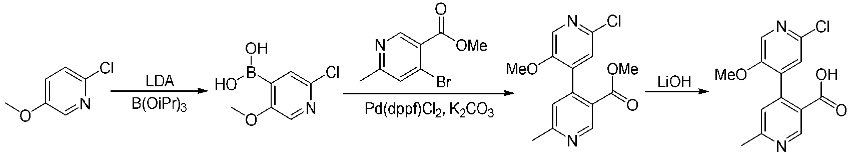

### Step 1: (2-chloro-5-methoxypyridin-4-yl)boronic acid

2-Chloro-5-methoxypyridine (10.0 g, 69.5 mmol) was dissolved in 250 mL of tetrahydrofuran, and under nitrogen protection, the solution was cooled to -65°C. A 2 mol/L solution of lithium diisopropylamide (70 mL) was added dropwise slowly while maintaining the temperature below -60°C. After the addition was complete, the reaction was maintained at the low temperature for 2 hours. Then, triisopropyl borate (26.2 g, 139 mmol) was added dropwise slowly at -65°C. The mixture was stirred at -65°C for an additional hour and then allowed to warm to room temperature overnight. The reaction mixture was quenched by adding 100 mL of water under an ice-water bath. The resulting aqueous solution was extracted twice with ethyl acetate, and the organic phases were discarded. The aqueous phase was adjusted to pH 5-6 with a 2M hydrochloric acid aqueous solution, resulting in the precipitation of a large amount of solid. The solid was collected by filtration under reduced pressure and dried to afford the title compound (11.4 g, white solid, yield 88.1%). LC/MS (ESI) m/z: 188.0 [M+H]⁺.

### Step 2: Methyl 2'-chloro-5'-methoxy-6-methyl-[4,4'-bipyridine]-3-carboxylate

Methyl 4-bromo-6-methylnicotinate (5 g, 21.7 mmol), (2-chloro-5-methoxypyridin-4-yl)boronic acid (4.07 g, 21.7 mmol), and potassium carbonate (9 g, 65.2 mmol) were dissolved in a mixture of 180 mL of 1,4-dioxane and 36 mL of water. Under nitrogen protection, bis(triphenylphosphine)palladium(II) chloride (1.59 g, 2.17 mmol) was added, and the mixture was heated to 80°C and reacted for 2 hours. The reaction mixture was cooled to room temperature, filtered, and then partitioned with water and ethyl acetate for extraction. The organic phase was concentrated and purified by silica gel column chromatography (petroleum ether/ethyl acetate system: 0-50% ethyl acetate) to afford the title compound (5.20 g, white solid, yield 81.7%). LC/MS (ESI) m/z: 293.0 [M+H] ⁺.

### Step 3: 2'-chloro-5'-methoxy-6-methyl-[4,4'-bipyridine]-3-carboxylic acid

Methyl 2'-chloro-5'-methoxy-6-methyl-[4,4'-bipyridine]-3-carboxylate (5.2 g, 17.7 mmol) was dissolved in a mixture of 50 mL of tetrahydrofuran and 50 mL of water. Lithium hydroxide (0.64 g, 26.6 mmol) was added, and the mixture was stirred at room temperature overnight. After concentrating under reduced pressure to remove tetrahydrofuran, the pH was adjusted to 5-6 with hydrochloric acid, resulting in the precipitation of a large amount of white solid. The solid was collected by filtration under reduced pressure, and the filter cake was dried to afford the title compound (4.82 g, white solid, yield 96.7%). LC/MS (ESI) m/z: 279.1 [M+H] ⁺.

### 2'-chloro-N-(6-(1,4-dimethyl-1H-1,2,3-triazol-5-yl)-7-fluorothiazolo[4,5-c]pyridin-2-yl)-5'-methoxy-6-methyl-[4,4'-bipyridine]-3-carboxamide

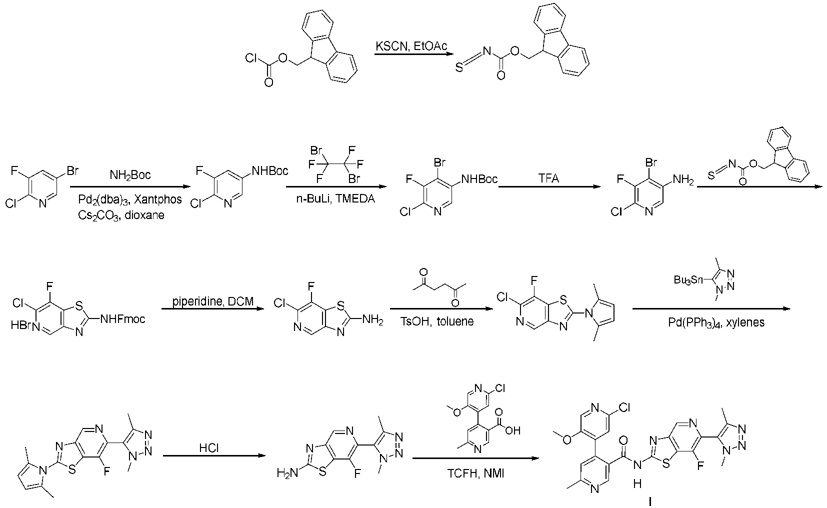

### Step 1: O-((9H-fluoren-9-yl)methyl)carbonisothiocyanatidate

To a solution of potassium thiocyanate (20.7 g, 213 mmol) in ethyl acetate (160 mL) at 0°C was added dropwise a solution of 9-fluorenylmethyl chloroformate (50 g, 193 mmol) in ethyl acetate (160 mL). The mixture was stirred at room temperature for 16 hours. The mixture was filtered, and the filtrate was concentrated under reduced pressure. The residue was purified by silica gel column chromatography (eluent: petroleum ether/ethyl acetate = 20, *V*/*V*) to afford the title compound (yellow oil, 16.0 g, yield 29.4%). ¹H NMR (400 MHz, DMSO-*d₆*) δ 7.89 (d, *J =* 7.6 Hz, 2H), 7.69 (d, *J =* 7.6 Hz, 2H), 7.45-7.39 (m, 2H), 7.36-7.31 (m, 2H), 4.30-4.23 (m, 2H), 4.23-4.18 (m, 1H).

### Step 2: Tert-butyl (6-chloro-5-fluoropyridin-3-yl)carbamate

To a solution of 2-chloro-3-fluoro-5-bromopyridine (8.00 g, 38.0 mmol), tert-butyl carbamate (4.90 g, 41.8 mmol), cesium carbonate (24.8 g, 76.0 mmol), and 4,5-bis(diphenylphosphino)-9,9-dimethylxanthene (880 mg, 1.52 mmol) in 1,4-dioxane (160 mL) was added bis(dibenzylideneacetone)palladium(0) (1.04 g, 1.14 mmol). The mixture was stirred at 85°C under nitrogen atmosphere for 20 hours. The mixture was concentrated under reduced pressure. The residue was purified by silica gel column chromatography (eluent: petroleum ether/ethyl acetate = 4, *V*/*V*) to afford the title compound (yellow solid, 6.10 g, yield 65.0%). LC/MS (ESI) m/z: 246.9 [M+H]⁺.

### Step 3: Tert-butyl (4-bromo-6-chloro-5-fluoropyridin-3-yl)carbamate

Tert-butyl (6-chloro-5-fluoropyridin-3-yl)carbamate (6.10 g, 24.7 mmol) and N,N,N',N'-tetramethylethylenediamine (8.62 g, 74.2 mmol) were dissolved in diethyl ether (130 mL). Under nitrogen atmosphere, the reaction mixture was cooled to -60°C, and 1.6 M n-butyllithium (46.4 mL, 74.2 mmol) was added dropwise. After the addition was complete, the mixture was warmed to -20°C and stirred for 1.5 hours. The reaction mixture was cooled to -60°C, and 1,2-dibromotetrafluoroethane (19.9 g, 76.7 mmol) was added dropwise. After the addition was complete, the mixture was slowly warmed to room temperature, quenched with 1 N HCl (92 mL), and extracted with ethyl acetate. The organic phase was washed with water and brine, dried over anhydrous sodium sulfate, and filtered. The mixture was concentrated under reduced pressure to afford the title compound (yellow solid, 8.0 g, yield 99.4%). LC/MS (ESI) m/z: 326.9 [M+H]⁺.

### Step 4: 4-bromo-6-chloro-5-fluoropyridin-3-amine

Tert-butyl (4-bromo-6-chloro-5-fluoropyridin-3-yl)carbamate (8.0 g, 24.6 mmol) was dissolved in dichloromethane (100 mL) and trifluoroacetic acid (50 mL). The reaction mixture was stirred at room temperature for one hour. The mixture was concentrated under reduced pressure to give a residue, which was redissolved in ethyl acetate and neutralized with saturated aqueous sodium bicarbonate solution. The mixture was extracted with ethyl acetate. The organic phase was washed with water and brine, dried over anhydrous sodium sulfate, and filtered. The mixture was concentrated under reduced pressure to afford the title compound (yellow solid, 5.7 g, yield 103%). LC/MS (ESI) m/z: 224.8 [M+H]⁺.

### Step 5: (9H-fluoren-9-yl)methyl (6-chloro-7-fluorothiazolo[4,5-c]pyridin-2-yl)carbamate hydrobromide

4-bromo-6-chloro-5-fluoropyridin-3-amine (5.7 g, 25.3 mmol) was dissolved in acetone (100 mL), and O-((9H-fluoren-9-yl)methyl)carbonisothiocyanatidate (8.54 g, 30.3 mmol) was added. The mixture was stirred at 50°C overnight. The reaction mixture was cooled to room temperature, filtered, and the filter cake was rinsed with acetone. The solid was dried under reduced pressure to afford the title compound (7.7 g, white solid, yield 60.1%). LC/MS (ESI) m/z: 426.0 [M+H]⁺.

### Step 6: 6-chloro-7-fluorothiazolo[4,5-c]pyridin-2-amine

To a solution of (9H-fluoren-9-yl)methyl (6-chloro-7-fluorothiazolo[4,5-c]pyridin-2-yl)carbamate hydrobromide (7.7 g, 18.1 mmol) in dichloromethane (80 mL) was added piperidine (17.9 mL, 181 mmol), and the mixture was stirred at room temperature for one hour. Water was added to the reaction mixture, and the mixture was back-extracted with ethyl acetate. The aqueous phase was concentrated under reduced pressure. The residue was first purified by silica gel column chromatography (eluent: dichloromethane/methanol = 10, *V*/*V*), and then further purified by slurrying in dichloromethane to afford the title compound (white solid, 1.45 g, yield 39.4%). LC/MS (ESI) m/z: 204.1 [M+H]⁺.

### Step 7: 6-chloro-2-(2,5-dimethyl-1H-pyrrol-1-yl)-7-fluorothiazolo[4,5-c]pyridine

To a solution of 6-chloro-7-fluorothiazolo[4,5-c]pyridin-2-amine (1.45 g, 7.12 mmol) and acetylacetone (1.65 g, 14.5 mmol) in toluene (30 mL) was added p-toluenesulfonic acid monohydrate (0.25 g, 1.42 mmol), and the mixture was refluxed at 140°C with water separation for two hours. The reaction mixture was cooled to room temperature, ethyl acetate was added, and the mixture was washed with an aqueous sodium bicarbonate solution and then with saturated brine. The mixture was concentrated under reduced pressure, and the residue was purified by silica gel column chromatography (eluent: petroleum ether/ethyl acetate = 10, *V*/*V*) to afford the title compound (white solid, 1.5 g, yield 74.8%). LC/MS (ESI) m/z: 282.1 [M+H]⁺.

### Step 8: 6-(1,4-dimethyl-1H-1,2,3-triazol-5-yl)-2-(2,5-dimethyl-1H-pyrrol-1-yl)-7-fluorothiazolo[4,5-c]pyridine

To a solution of 6-chloro-2-(2,5-dimethyl-1H-pyrrol-1-yl)-7-fluorothiazolo[4,5-c]pyridine (1.5 g, 5.32 mmol) and 1,4-dimethyl-5-(tributylstannyl)-1H-1,2,3-triazole (3.08 g, 7.99 mmol) in xylene (30 mL) was added tetrakis(triphenylphosphine)palladium (1.85 g, 1.60 mmol). The mixture was stirred at 150°C under nitrogen protection for 5 hours. The mixture was concentrated under reduced pressure, and the residue was purified by silica gel column chromatography (eluent: petroleum ether/ethyl acetate = 3, *V*/*V*) to afford the title compound (pale yellow solid, 1.25 g, yield 68.7%). LC/MS (ESI) m/z: 343.1 [M+H]⁺.

### Step 9: 6-(1,4-dimethyl-1H-1,2,3-triazol-5-yl)-7-fluorothiazolo[4,5-c]pyridin-2-amine hydrochloride

6-(1,4-dimethyl-1H-1,2,3-triazol-5-yl)-2-(2,5-dimethyl-1H-pyrrol-1-yl)-7-fluorothiazolo[4,5-c] pyridine (1.25 g, 3.65 mmol) was dissolved in 2 N HCl (15 mL). The reaction mixture was stirred at 80°C for two hours. The mixture was concentrated under reduced pressure, and the residue was slurried with acetonitrile to afford the title compound (pale yellow solid, 1.0 g, yield 91.1%). LC/MS (ESI) m/z: 265.1 [M+H]⁺.

### Step 10: 2'-chloro-N-(6-(1,4-dimethyl-1H-1,2,3-triazol-5-yl)-7-fluorothiazolo[4,5-c]pyridin-2-yl)-5'-methoxy-6-methyl-[4,4'-bipyridine]-3-carboxamide

6-(1,4-dimethyl-1H-1,2,3-triazol-5-yl)-7-fluorothiazolo[4,5-c]pyridin-2-amine hydrochloride (1.0 g, 3.78 mmol), 2'-chloro-5'-methoxy-6-methyl-[4,4'-bipyridine]-3-carboxylic acid (1.16 g, 4.16 mmol), and N-methylimidazole (2.17 g, 26.5 mmol) were dissolved in acetonitrile (20 mL). The mixture was stirred at 70°C for 5 minutes, then N,N,N',N'-tetramethylchloroformamidinium hexafluorophosphate (5.31 g, 18.9 mmol) was added to the reaction mixture, and stirring was continued at 70°C for 2 hours. As the starting material remained unreacted, additional N-methylimidazole (0.62 g, 7.56 mmol) and N,N,N',N'-tetramethylchloroformamidinium hexafluorophosphate (1.06 g, 3.78 mmol) were added to the reaction mixture. Water was added to the reaction mixture, and the mixture was extracted with ethyl acetate. The organic phase was washed with water and brine, then concentrated under reduced pressure. The residue was first purified by silica gel column chromatography (eluent: dichloromethane/methanol = 20, *V*/*V*), then slurried with ethyl acetate to afford the title compound (834.7 mg, white solid, yield 42.0%). ¹H NMR (400 MHz, DMSO-*d₆*) δ 13.63 (s, 1H), 9.14 (d, *J =* 2.0 Hz, 1H), 8.90 (s, 1H), 8.18 (s, 1H), 7.62 (s, 1H), 7.51 (s, 1H), 4.03 (s, 3H), 3.61 (s, 3H), 2.62 (s, 3H), 2.26 (d, *J* = 1.6 Hz, 3H). LC/MS (ESI) m/z: 525.2 [M+H]⁺.

The obtained white solid is crystalline form A of the compound of formula I, its XRPD pattern is shown in FIG. 1, and it has the diffraction peaks shown in Table 1 below.

**Table 1**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 5.984 | 14.75874 | 18.40% | 27 | 24.6 | 3.6159 | 13.90% |
| 2 | 9.003 | 9.81492 | 68.00% | 28 | 25.347 | 3.51101 | 3.60% |
| 3 | 10.5 | 8.41809 | 6.10% | 29 | 25.62 | 3.47424 | 8.20% |
| 4 | 10.837 | 8.15715 | 62.30% | 30 | 26.413 | 3.3717 | 1.60% |
| 5 | 11.941 | 7.40557 | 28.40% | 31 | 27.165 | 3.28003 | 6.30% |
| 6 | 13.462 | 6.57211 | 4.10% | 32 | 27.717 | 3.216 | 63.30% |
| 7 | 14.156 | 6.25128 | 8.50% | 33 | 28.498 | 3.12958 | 7.20% |
| 8 | 14.72 | 6.013 | 8.70% | 34 | 28.956 | 3.08106 | 12.50% |
| 9 | 15.31 | 5.78276 | 1.40% | 35 | 29.184 | 3.0575 | 6.80% |
| 10 | 15.455 | 5.72891 | 1.60% | 36 | 29.679 | 3.00767 | 15.80% |
| 11 | 15.79 | 5.6079 | 1.20% | 37 | 30.294 | 2.94797 | 9.40% |
| 12 | 17.153 | 5.16531 | 2.20% | 38 | 31.083 | 2.87498 | 5.90% |
| 13 | 17.91 | 4.94869 | 100.00% | 39 | 31.992 | 2.7953 | 7.20% |
| 14 | 18.357 | 4.82916 | 40.90% | 40 | 32.218 | 2.77621 | 7.00% |
| 15 | 19.02 | 4.66238 | 2.50% | 41 | 32.567 | 2.74722 | 6.60% |
| 16 | 19.994 | 4.43722 | 12.90% | 42 | 32.731 | 2.73381 | 5.30% |
| 17 | 20.649 | 4.29795 | 8.90% | 43 | 33.609 | 2.66443 | 1.40% |
| 18 | 21.304 | 4.16734 | 40.50% | 44 | 34.17 | 2.62191 | 3.90% |
| 19 | 21.71 | 4.0903 | 18.00% | 45 | 34.631 | 2.58808 | 0.70% |
| 20 | 22.052 | 4.02768 | 77.00% | 46 | 35.461 | 2.52937 | 0.50% |
| 21 | 22.448 | 3.9574 | 23.80% | 47 | 35.815 | 2.50521 | 1.70% |
| 22 | 22.631 | 3.92595 | 14.80% | 48 | 36.41 | 2.46559 | 3.40% |
| 23 | 23.291 | 3.81612 | 23.90% | 49 | 37.525 | 2.39489 | 4.00% |
| 24 | 23.679 | 3.75448 | 4.60% | 50 | 37.961 | 2.36834 | 3.90% |
| 25 | 23.96 | 3.71101 | 8.40% | 51 | 38.629 | 2.32896 | 7.00% |
| 26 | 24.139 | 3.68389 | 7.70% | 52 | 39.669 | 2.27022 | 1.50% |

The DSC pattern of crystalline form A of the compound of formula II is shown in FIG. 2. The DSC curve shows two endothermic peaks at peak temperatures of 98.33°C (enthalpy value 121.36 J/g) and 206.94°C (enthalpy value 66.798 J/g), with onset points (Onset x) at 72.55°C and 200.15°C, respectively. The TGA pattern is shown in FIG. 3. The sample lost 0.48% of its weight when heated from 34.69°C to 70.0°C, 2.11% from 70.0°C to 95.0°C, 1.38% from 95.0°C to 140.0°C, and approximately 0.34% in the temperature range from 140.0°C to 220.0°C. Based on the DSC and TGA analysis, crystalline form A is a monohydrate.

### Example 1-2 Preparation of Crystalline Form C of the Compound of Formula I

Approximately 30 mg of crystalline form A of the compound of formula II was weighed and placed in a 2 mL glass vial, and 0.5 mL of ethyl acetate or acetonitrile was added to form a suspension. The resulting sample was suspended at 50°C for 2 hours, then allowed to cool naturally to 25°C and suspended at 25°C for 3 days. The resulting suspension was centrifuged through a 0.45 µm nylon filter membrane at 14,000 rpm. The obtained solid was vacuum dried at 50°C for 2 hours to yield crystalline form C.

The obtained white solid is crystalline form C of the compound of formula I, its XRPD pattern is shown in FIG. 5, and it has the diffraction peaks shown in Table 3 below. The DSC pattern of crystalline form C of the compound of formula I is shown in FIG. 6. The DSC curve shows an endothermic peak at a peak temperature of 238.92°C (enthalpy value 106.67 J/g). The TGA pattern is shown in FIG. 7. The sample lost 0.81% of its weight when heated from 35.56°C to 230.0°C. Based on the DSC and TGA analysis, crystalline form C of the free base of the compound of formula I is an anhydrate.

**Table 3**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 9.022 | 9.79429 | 16.10% | 26 | 28.103 | 3.17262 | 2.40% |
| 2 | 10.212 | 8.65524 | 11.40% | 27 | 28.604 | 3.11826 | 2.30% |
| 3 | 11.25 | 7.85911 | 3.90% | 28 | 29.038 | 3.07259 | 4.90% |
| 4 | 11.506 | 7.68478 | 100.00% | 29 | 29.445 | 3.03098 | 24.40% |
| 5 | 12.878 | 6.86853 | 28.70% | 30 | 29.491 | 3.02643 | 14.70% |
| 6 | 13.61 | 6.50087 | 6.60% | 31 | 30.137 | 2.963 | 13.50% |
| 7 | 14.575 | 6.07273 | 14.20% | 32 | 30.354 | 2.94229 | 12.90% |
| 8 | 15.052 | 5.88114 | 22.80% | 33 | 30.683 | 2.91145 | 2.00% |
| 9 | 16.163 | 5.47954 | 5.70% | 34 | 31.261 | 2.85896 | 1.80% |
| 10 | 16.96 | 5.22352 | 7.10% | 35 | 31.719 | 2.81869 | 1.70% |
| 11 | 17.22 | 5.14526 | 5.40% | 36 | 32.564 | 2.74745 | 1.90% |
| 12 | 18.011 | 4.92107 | 59.10% | 37 | 33.128 | 2.70203 | 1.90% |
| 13 | 18.91 | 4.68917 | 12.10% | 38 | 34.117 | 2.62587 | 4.40% |
| 14 | 19.944 | 4.44835 | 1.60% | 39 | 34.866 | 2.57115 | 2.40% |
| 15 | 20.479 | 4.33321 | 26.60% | 40 | 34.973 | 2.56353 | 8.80% |
| 16 | 20.853 | 4.25635 | 42.00% | 41 | 35.477 | 2.5283 | 5.60% |
| 17 | 21.676 | 4.09669 | 32.30% | 42 | 36.053 | 2.48919 | 1.90% |
| 18 | 22.56 | 3.93806 | 98.50% | 43 | 36.415 | 2.46528 | 2.30% |
| 19 | 23.106 | 3.84624 | 29.20% | 44 | 36.885 | 2.43497 | 1.80% |
| 20 | 24.789 | 3.58872 | 31.90% | 45 | 37.062 | 2.42369 | 1.90% |
| 21 | 25.679 | 3.46636 | 84.80% | 46 | 38.06 | 2.36241 | 2.70% |
| 22 | 26.207 | 3.39769 | 5.00% | 47 | 38.395 | 2.34256 | 3.10% |
| 23 | 26.937 | 3.30724 | 15.00% | 48 | 39.217 | 2.29537 | 2.80% |
| 24 | 27.259 | 3.26894 | 11.20% | 49 | 39.732 | 2.26679 | 2.00% |
| 25 | 27.439 | 3.24784 | 35.40% | | | | |

### Example 2 Crystalline Form A of the Compound of Formula III

30 mg of crystalline form A of the compound of formula II was added to 0.5 mL of ethyl acetate, and the temperature was raised to 50°C. 0.05 mL of a dilute hydrochloric acid solution was added to the system (preparation method for dilute hydrochloric acid: 0.1 mL of concentrated hydrochloric acid (37% wt) dissolved in 0.9 mL of ethyl acetate). The entire system was stirred at 50°C for 2 hours, cooled to 25°C with stirring, filtered, and the obtained wet product was vacuum dried at 50°C for 2 hours to yield crystalline form A of the compound of formula III. Ion chromatography showed that the molar ratio of the compound of formula I to hydrochloric acid was 1:1.06. The XRPD pattern of crystalline form A of the compound of formula III is shown in FIG. 8, and it has the diffraction peaks shown in Table 4 below.

**Table 4**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 4.61 | 19.15403 | 100.00% | 18 | 19.13 | 4.63574 | 6.10% |
| 2 | 5.023 | 17.58006 | 34.20% | 19 | 19.304 | 4.59425 | 8.20% |
| 3 | 6.572 | 13.43895 | 21.10% | 20 | 19.964 | 4.44391 | 2.40% |
| 4 | 6.892 | 12.81446 | 14.10% | 21 | 20.808 | 4.26544 | 6.40% |
| 5 | 9.191 | 9.61374 | 70.70% | 22 | 21.684 | 4.09519 | 8.00% |
| 6 | 9.966 | 8.86786 | 16.20% | 23 | 23.087 | 3.84938 | 5.90% |
| 7 | 10.48 | 8.43464 | 8.90% | 24 | 24.031 | 3.70025 | 11.90% |
| 8 | 10.641 | 8.30701 | 8.30% | 25 | 24.497 | 3.6309 | 5.70% |
| 9 | 11.12 | 7.95062 | 2.40% | 26 | 25.141 | 3.53926 | 4.20% |
| 10 | 12.193 | 7.25335 | 2.00% | 27 | 25.56 | 3.48225 | 7.00% |
| 11 | 13.76 | 6.4306 | 62.30% | 28 | 26.127 | 3.4079 | 14.40% |
| 12 | 14.686 | 6.02699 | 9.60% | 29 | 27.539 | 3.23628 | 1.40% |
| 13 | 14.902 | 5.94022 | 14.80% | 30 | 28.173 | 3.16496 | 7.20% |
| 14 | 15.952 | 5.55141 | 1.80% | 31 | 31.795 | 2.81213 | 3.10% |
| 15 | 17.087 | 5.18506 | 1.20% | 32 | 34.164 | 2.62238 | 1.90% |
| 16 | 18.101 | 4.89688 | 4.20% | 33 | 35.404 | 2.53331 | 1.80% |
| 17 | 18.344 | 4.83252 | 9.10% | 34 | 36.041 | 2.48998 | 2.20% |

### Example 3 Crystalline Form B of the Compound of Formula III

30 mg of crystalline form A of the compound of formula II was added to 0.5 mL of ethyl acetate, and the temperature was raised to 50°C. 0.096 mL of a dilute hydrochloric acid solution was added to the system (preparation method for dilute hydrochloric acid: 0.1 mL of concentrated hydrochloric acid dissolved in 0.9 mL of ethyl acetate). The entire system was stirred at 50°C for 2 hours, then cooled to 25°C with stirring, filtered, and the obtained wet product was vacuum dried at 50°C for 2 hours to yield crystalline form B of the compound of formula III. Ion chromatography showed that the molar ratio of the compound of formula I to hydrochloric acid was 1:1.76. The XRPD pattern of crystalline form B of the compound of formula III is shown in FIG. 9, and it has the diffraction peaks shown in Table 5 below.

**Table 5**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 6.469 | 13.65234 | 32.50% | 34 | 26.962 | 3.30424 | 13.30% |
| 2 | 7.191 | 12.28265 | 18.80% | 35 | 27.149 | 3.28196 | 14.10% |
| 3 | 9.111 | 9.69816 | 13.90% | 36 | 27.471 | 3.24414 | 4.90% |
| 4 | 11.634 | 7.60053 | 19.40% | 37 | 27.956 | 3.18897 | 40.00% |
| 5 | 12.94 | 6.83578 | 7.20% | 38 | 28.616 | 3.11698 | 44.60% |
| 6 | 13.406 | 6.59963 | 8.00% | 39 | 29.004 | 3.07608 | 7.10% |
| 7 | 14.45 | 6.125 | 71.00% | 40 | 29.161 | 3.0599 | 8.10% |
| 8 | 15.407 | 5.74645 | 9.70% | 41 | 29.389 | 3.03669 | 4.40% |
| 9 | 15.75 | 5.62221 | 10.40% | 42 | 30.04 | 2.97233 | 15.60% |
| 10 | 16.234 | 5.45541 | 15.50% | 43 | 30.365 | 2.94122 | 29.10% |
| 11 | 16.429 | 5.3914 | 10.70% | 44 | 30.626 | 2.9168 | 6.10% |
| 12 | 16.722 | 5.2973 | 13.20% | 45 | 30.999 | 2.88256 | 16.20% |
| 13 | 17.367 | 5.10202 | 49.40% | 46 | 31.421 | 2.84476 | 5.90% |
| 14 | 18.209 | 4.86807 | 51.20% | 47 | 31.869 | 2.80577 | 4.20% |
| 15 | 18.817 | 4.71221 | 3.70% | 48 | 32.198 | 2.77791 | 25.10% |
| 16 | 19.443 | 4.56187 | 47.20% | 49 | 32.644 | 2.74097 | 11.10% |
| 17 | 20.166 | 4.39987 | 51.30% | 50 | 32.917 | 2.71881 | 10.10% |
| 18 | 20.496 | 4.32982 | 23.70% | 51 | 33.638 | 2.66217 | 1.30% |
| 19 | 20.628 | 4.30239 | 32.20% | 52 | 34.045 | 2.63129 | 13.20% |
| 20 | 20.823 | 4.26252 | 100.00% | 53 | 34.445 | 2.60161 | 2.00% |
| 21 | 21.657 | 4.10023 | 4.60% | 54 | 35.025 | 2.55988 | 2.90% |
| 22 | 22.215 | 3.99843 | 2.30% | 55 | 35.664 | 2.51546 | 5.10% |
| 23 | 22.406 | 3.96476 | 31.50% | 56 | 36.067 | 2.48825 | 4.60% |
| 24 | 23.012 | 3.86166 | 12.10% | 57 | 36.293 | 2.47331 | 6.50% |
| 25 | 23.445 | 3.79133 | 2.80% | 58 | 36.489 | 2.46042 | 5.50% |
| 26 | 23.628 | 3.76249 | 6.90% | 59 | 37.026 | 2.42601 | 6.20% |
| 27 | 23.81 | 3.73402 | 2.50% | 60 | 37.339 | 2.40634 | 2.70% |
| 28 | 24.578 | 3.61916 | 83.80% | 61 | 38.15 | 2.35709 | 3.90% |
| 29 | 25.33 | 3.5134 | 9.00% | 62 | 38.451 | 2.33931 | 5.20% |
| 30 | 25.839 | 3.44522 | 20.50% | 63 | 39.006 | 2.30729 | 2.50% |
| 31 | 26.006 | 3.42347 | 39.10% | 64 | 39.263 | 2.29276 | 5.20% |
| 32 | 26.166 | 3.40301 | 27.40% | 65 | 39.814 | 2.26231 | 5.90% |
| 33 | 26.468 | 3.36485 | 47.20% | | | | |

The DSC pattern of crystalline form B of the compound of formula III is shown in FIG. 10. The DSC curve shows an endothermic peak at a peak temperature of 186.88°C (enthalpy value 164.82 J/g), with an onset point (Onset x) of 172.09°C. The TGA pattern is shown in FIG. 11. The sample lost 1.42% of its weight when heated from 24.07°C to 120.0°C, and 9.84% from 120.0°C to 205.0°C. Based on the DSC and TGA analysis, crystalline form B of the compound of formula III is an anhydrate.

### Example 4 Crystalline Form C of the Compound of Formula III

30 mg of crystalline form A of the compound of formula II was added to 0.5 mL of acetonitrile, and the temperature was raised to 50°C. 0.096 mL of a dilute hydrochloric acid solution was added to the system (preparation method for dilute hydrochloric acid: 0.1 mL of concentrated hydrochloric acid dissolved in 0.9 mL of ethyl acetate). The entire system was stirred at 50°C for 2 hours, cooled to 25°C with stirring, filtered, and the obtained wet product was vacuum dried at 50°C for 2 hours to obtain crystalline form C of the compound of formula III. The molar ratio of the compound of formula I to hydrochloric acid was 1:1.7. The XRPD pattern of crystalline form C of the compound of formula III is shown in FIG. 12, and it has the diffraction peaks shown in Table 6 below.

**Table 6**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 9.84 | 8.9813 | 22.30% | 21 | 26.935 | 3.30754 | 17.80% |
| 2 | 10.511 | 8.4094 | 14.40% | 22 | 27.482 | 3.24292 | 51.00% |
| 3 | 11.606 | 7.61836 | 12.00% | 23 | 28.37 | 3.1434 | 25.40% |
| 4 | 12.187 | 7.25646 | 61.90% | 24 | 28.965 | 3.08014 | 25.90% |
| 5 | 13.393 | 6.60555 | 33.30% | 25 | 29.322 | 3.04348 | 26.90% |
| 6 | 14.401 | 6.14577 | 24.90% | 26 | 29.949 | 2.98119 | 6.50% |
| 7 | 17.352 | 5.10637 | 10.30% | 27 | 30.421 | 2.93597 | 15.70% |
| 8 | 18.158 | 4.8815 | 86.70% | 28 | 31.245 | 2.86039 | 11.50% |
| 9 | 18.293 | 4.84598 | 78.90% | 29 | 31.602 | 2.82887 | 5.70% |
| 10 | 19.386 | 4.57505 | 53.40% | 30 | 32.238 | 2.7745 | 11.00% |
| 11 | 20.016 | 4.43254 | 15.90% | 31 | 32.62 | 2.74286 | 9.60% |
| 12 | 20.805 | 4.2661 | 37.40% | 32 | 33.448 | 2.67686 | 3.30% |
| 13 | 21.747 | 4.08336 | 64.40% | 33 | 34.041 | 2.6316 | 6.10% |
| 14 | 22.514 | 3.94607 | 33.80% | 34 | 34.684 | 2.58422 | 7.70% |
| 15 | 22.896 | 3.88101 | 13.30% | 35 | 35.296 | 2.54081 | 10.10% |
| 16 | 23.245 | 3.82352 | 34.10% | 36 | 36.157 | 2.48228 | 13.90% |
| 17 | 23.488 | 3.78457 | 29.40% | 37 | 36.574 | 2.45491 | 4.00% |
| 18 | 24.462 | 3.63601 | 12.20% | 38 | 37.903 | 2.37183 | 6.30% |
| 19 | 25.113 | 3.54325 | 16.90% | 39 | 38.471 | 2.33815 | 7.40% |
| 20 | 26.409 | 3.37219 | 100.00% | | | | |

### Example 5 Crystalline Form A of the Compound of Formula IV

30 mg of crystalline form A of the compound of formula II was added to 0.5 mL of ethyl acetate, and the temperature was raised to 50°C. 0.033 mL of a dilute sulfuric acid solution was added to the system (preparation method for dilute sulfuric acid: 0.1 mL of sulfuric acid dissolved in 0.9 mL of ethyl acetate). The entire system was stirred at 50°C for 2 hours, cooled to 25°C with stirring, filtered, and the obtained wet product was vacuum dried at 50°C for 2 hours to obtain crystalline form A of the compound of formula IV. The molar ratio of the compound of formula I to sulfuric acid was 1:0.97. The XRPD pattern of crystalline form A of the compound of formula IV is shown in FIG. 13, and it has the diffraction peaks shown in Table 7 below.

**Table 7**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 5.143 | 17.16934 | 60.20% | 12 | 20.403 | 4.34933 | 33.50% |
| 2 | 6.108 | 14.45907 | 16.10% | 13 | 21.449 | 4.13949 | 33.20% |
| 3 | 6.598 | 13.38491 | 14.80% | 14 | 21.781 | 4.07705 | 58.50% |
| 4 | 7.345 | 12.02656 | 20.40% | 15 | 23.807 | 3.73458 | 40.20% |
| 5 | 8.628 | 10.24052 | 23.70% | 16 | 24.819 | 3.58454 | 67.70% |
| 6 | 9.008 | 9.80933 | 100.00% | 17 | 25.473 | 3.49394 | 51.50% |
| 7 | 11.45 | 7.72193 | 39.40% | 18 | 25.946 | 3.4313 | 49.20% |
| 8 | 14.624 | 6.05231 | 18.30% | 19 | 26.679 | 3.33862 | 54.90% |
| 9 | 15.033 | 5.8885 | 35.20% | 20 | 27.959 | 3.18865 | 6.00% |
| 10 | 16.445 | 5.38619 | 17.10% | 21 | 28.427 | 3.13718 | 29.90% |
| 11 | 17.437 | 5.08168 | 33.50% | | | | |

### Example 6 Crystalline Form B of the Compound of Formula IV

30 mg of the compound of formula I was added to 0.5 mL of acetonitrile, and the temperature was raised to 50°C. 0.033 mL of a dilute sulfuric acid solution was added to the system (preparation method for dilute sulfuric acid: 0.1 mL of sulfuric acid dissolved in 0.9 mL of ethyl acetate). The entire system was stirred at 50°C for 2 hours, cooled to 25°C with stirring, filtered, and the obtained wet product was vacuum dried at 50°C for 2 hours to obtain crystalline form B of the compound of formula IV. The molar ratio of the compound of formula I to sulfuric acid was 1:0.96. The XRPD pattern of crystalline form B of the compound of formula IV is shown in FIG. 14, and it has the diffraction peaks shown in Table 8 below.

**Table 8**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 6.868 | 12.86005 | 9.90% | 35 | 21.518 | 4.12631 | 8.90% |
| 2 | 7.106 | 12.42925 | 22.80% | 36 | 21.975 | 4.04158 | 11.20% |
| 3 | 7.566 | 11.67521 | 24.00% | 37 | 22.201 | 4.00088 | 20.90% |
| 4 | 7.716 | 11.44879 | 24.70% | 38 | 22.509 | 3.94688 | 14.50% |
| 5 | 8.1 | 10.90706 | 26.40% | 39 | 22.856 | 3.88771 | 8.00% |
| 6 | 9.963 | 8.87076 | 5.10% | 40 | 23.179 | 3.83422 | 67.20% |
| 7 | 10.269 | 8.60717 | 16.20% | 41 | 23.617 | 3.76419 | 11.60% |
| 8 | 11.2 | 7.89415 | 2.00% | 42 | 24.18 | 3.67773 | 94.90% |
| 9 | 11.554 | 7.65303 | 2.30% | 43 | 24.741 | 3.59564 | 12.60% |
| 10 | 11.859 | 7.45666 | 2.80% | 44 | 25.171 | 3.53519 | 100.00% |
| 11 | 12.546 | 7.04959 | 13.30% | 45 | 25.575 | 3.48024 | 31.60% |
| 12 | 12.674 | 6.97885 | 14.40% | 46 | 25.996 | 3.42481 | 14.30% |
| 13 | 13.192 | 6.70605 | 12.00% | 47 | 26.391 | 3.37441 | 4.40% |
| 14 | 13.486 | 6.56063 | 8.90% | 48 | 26.802 | 3.32368 | 13.60% |
| 15 | 13.859 | 6.38461 | 27.20% | 49 | 27.162 | 3.28036 | 18.40% |
| 16 | 14.114 | 6.26974 | 55.00% | 50 | 27.403 | 3.25211 | 14.20% |
| 17 | 14.71 | 6.01728 | 63.20% | 51 | 27.814 | 3.20496 | 41.70% |
| 18 | 15.439 | 5.73483 | 21.70% | 52 | 28.093 | 3.1738 | 14.80% |
| 19 | 16.074 | 5.50951 | 43.00% | 53 | 28.575 | 3.1213 | 5.40% |
| 20 | 16.445 | 5.38608 | 5.20% | 54 | 29.494 | 3.02609 | 14.30% |
| 21 | 16.729 | 5.2952 | 18.90% | 55 | 29.893 | 2.98659 | 16.50% |
| 22 | 17.005 | 5.20983 | 4.60% | 56 | 30.118 | 2.9648 | 13.30% |
| 23 | 17.552 | 5.04867 | 4.20% | 57 | 31.454 | 2.84184 | 8.00% |
| 24 | 17.918 | 4.94655 | 19.00% | 58 | 32.036 | 2.79159 | 14.30% |
| 25 | 18.378 | 4.82369 | 46.30% | 59 | 32.834 | 2.72552 | 4.20% |
| 26 | 18.576 | 4.77263 | 44.20% | 60 | 33.662 | 2.66033 | 2.50% |
| 27 | 19.097 | 4.64357 | 8.20% | 61 | 34.515 | 2.59649 | 4.60% |
| 28 | 19.463 | 4.55724 | 15.30% | 62 | 35.429 | 2.53161 | 2.70% |
| 29 | 19.796 | 4.4813 | 12.50% | 63 | 35.851 | 2.50278 | 2.10% |
| 30 | 20.182 | 4.39642 | 28.90% | 64 | 36.348 | 2.46966 | 3.90% |
| 31 | 20.394 | 4.35117 | 11.90% | 65 | 36.7 | 2.4468 | 7.70% |
| 32 | 20.588 | 4.31055 | 15.90% | 66 | 38.306 | 2.34784 | 6.40% |
| 33 | 20.818 | 4.26344 | 11.70% | 67 | 38.66 | 2.32714 | 3.80% |
| 34 | 21.206 | 4.18636 | 27.10% | 68 | 39.469 | 2.28128 | 4.30% |

The DSC pattern of crystalline form B of the compound of formula IV is shown in FIG. 15. The DSC curve shows endothermic peaks at peak temperatures of 34.85°C (enthalpy value 56.75 J/g), 90.79°C (enthalpy value 28.413 J/g), and 204.38°C (enthalpy value 70.95 J/g), with onset points (Onset x) of 11.47°C, 67.49°C, and 196.17°C, respectively. The TGA pattern is shown in FIG. 16. The sample lost 2.06% of its weight when heated from 33.44°C to 50.0°C, 0.56% from 50.0°C to 100.0°C, 0.39% from 100.0°C to 180.0°C, and 1% from 180.0°C to 230.0°C.

### Example 7 Crystalline Form A of the Compound of Formula V

30 mg of the compound of formula I was added to 0.5 mL of ethyl acetate, and the temperature was raised to 50°C. 0.041 mL of a dilute phosphoric acid solution was added to the system (preparation method for dilute phosphoric acid: 0.1 mL of phosphoric acid dissolved in 0.9 mL of ethyl acetate). The entire system was stirred at 50°C for 2 hours, cooled to 25°C with stirring, filtered, and the resulting wet product was vacuum dried at 50°C for 2 hours to obtain crystalline form A of the compound of formula V. The molar ratio of the compound of formula I to phosphoric acid was 1:1.14, and crystalline form A of the compound of formula V is a hydrate. The XRPD pattern of crystalline form A of the phosphate salt of the compound of formula I is shown in FIG. 17, and it has the diffraction peaks shown in Table 9 below.

**Table 9**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 5.153 | 17.13442 | 24.70% | 35 | 23.241 | 3.82427 | 33.70% |
| 2 | 5.982 | 14.76142 | 8.10% | 36 | 23.632 | 3.76186 | 23.60% |
| 3 | 7.969 | 11.08548 | 16.00% | 37 | 23.98 | 3.70798 | 58.60% |
| 4 | 8.343 | 10.58926 | 44.50% | 38 | 24.1 | 3.68976 | 43.00% |
| 5 | 8.96 | 9.86201 | 21.40% | 39 | 24.465 | 3.6355 | 7.40% |
| 6 | 10.489 | 8.42719 | 33.70% | 40 | 24.875 | 3.57651 | 14.30% |
| 7 | 10.799 | 8.18598 | 26.70% | 41 | 25.132 | 3.5405 | 39.10% |
| 8 | 11.337 | 7.79853 | 32.40% | 42 | 25.542 | 3.48466 | 5.60% |
| 9 | 11.657 | 7.58527 | 24.10% | 43 | 25.831 | 3.44632 | 6.90% |
| 10 | 11.989 | 7.37587 | 21.90% | 44 | 26.177 | 3.40159 | 13.30% |
| 11 | 12.726 | 6.95054 | 15.30% | 45 | 26.438 | 3.3685 | 10.80% |
| 12 | 13.146 | 6.72941 | 7.00% | 46 | 26.83 | 3.3202 | 11.90% |
| 13 | 13.457 | 6.57471 | -0.10% | 47 | 27.281 | 3.26636 | 20.30% |
| 14 | 14.069 | 6.28966 | 2.80% | 48 | 27.734 | 3.21404 | 100.00% |
| 15 | 14.607 | 6.05938 | 1.40% | 49 | 28.441 | 3.13574 | 9.60% |
| 16 | 14.842 | 5.96398 | 5.00% | 50 | 28.63 | 3.11548 | 11.10% |
| 17 | 15.505 | 5.71038 | 38.40% | 51 | 28.936 | 3.08321 | 22.30% |
| 18 | 15.884 | 5.57482 | 44.10% | 52 | 29.165 | 3.0595 | 18.40% |
| 19 | 16.687 | 5.3085 | 6.50% | 53 | 29.592 | 3.0163 | 5.80% |
| 20 | 17.26 | 5.13366 | 4.00% | 54 | 30.38 | 2.93983 | 7.70% |
| 21 | 17.929 | 4.94353 | 75.20% | 55 | 31.268 | 2.85833 | 5.70% |
| 22 | 18.387 | 4.82144 | 84.80% | 56 | 31.763 | 2.81491 | 8.30% |
| 23 | 18.679 | 4.7465 | 37.50% | 57 | 32.113 | 2.78507 | 11.50% |
| 24 | 19.062 | 4.65205 | 11.40% | 58 | 32.719 | 2.73479 | 5.20% |
| 25 | 19.731 | 4.49575 | 38.50% | 59 | 32.966 | 2.71493 | 4.70% |
| 26 | 20.14 | 4.4055 | 31.90% | 60 | 33.517 | 2.67154 | 8.90% |
| 27 | 20.516 | 4.32562 | 12.40% | 61 | 33.934 | 2.63964 | 6.50% |
| 28 | 20.76 | 4.27521 | 14.20% | 62 | 34.599 | 2.5904 | 3.10% |
| 29 | 21.054 | 4.2162 | 29.80% | 63 | 35.435 | 2.53122 | 7.30% |
| 30 | 21.278 | 4.17241 | 26.00% | 64 | 36.132 | 2.48393 | 2.10% |
| 31 | 21.643 | 4.10273 | 22.90% | 65 | 37.141 | 2.41876 | 4.10% |
| 32 | 21.992 | 4.03848 | 88.50% | 66 | 37.699 | 2.38421 | 6.90% |
| 33 | 22.507 | 3.94723 | 13.30% | 67 | 38.63 | 2.32888 | 6.10% |
| 34 | 22.762 | 3.90364 | 42.90% | 68 | 39.602 | 2.2739 | 2.50% |

The DSC pattern of crystalline form A of the compound of formula V is shown in FIG. 18. The DSC curve shows two endothermic peaks at peak temperatures of 88.96°C (enthalpy value 42.827 J/g) and 192.67°C (enthalpy value 52.713 J/g), with onset points (Onset x) of 66.74°C and 183.41°C, respectively. The TGA pattern is shown in FIG. 19. The sample lost 0.92% of its weight when heated from 32.34°C to 70.0°C, and 2.10% from 70.0°C to 180.0°C. Based on the DSC and TGA analyses, crystalline form A of the phosphate salt of the compound of formula I (crystalline form A of the compound of formula V) is a hydrate, and the water content calculated from TGA is 0.91 equivalents.

### Example 8 Crystalline Form B of the Compound of Formula V

30 mg of the compound of formula I was added to 0.5 mL of acetonitrile, and the temperature was raised to 50°C. 0.041 mL of a dilute phosphoric acid solution was added to the system (preparation method for dilute phosphoric acid: 0.1 mL of phosphoric acid dissolved in 0.9 mL of ethyl acetate). The entire system was stirred at 50°C for 2 hours, cooled to 25°C with stirring, filtered, and the resulting wet product was vacuum dried at 50°C for 2 hours to obtain crystalline form B of the phosphate salt of the compound of formula I (crystalline form B of the compound of formula V). The molar ratio of the compound of formula I to phosphoric acid was 1: 1.09. The XRPD pattern of crystalline form B of the phosphate salt of the compound of formula I (crystalline form B of the compound of formula V) is shown in FIG. 20, and it has the diffraction peaks shown in Table 10 below.

**Table 10**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 3.412 | 25.87783 | 5.60% | 25 | 21.646 | 4.10215 | 22.50% |
| 2 | 6.833 | 12.92626 | 38.40% | 26 | 22.054 | 4.02735 | 20.10% |
| 3 | 7.273 | 12.14545 | 6.10% | 27 | 22.311 | 3.98146 | 15.20% |
| 4 | 8.32 | 10.61883 | 22.40% | 28 | 22.826 | 3.89276 | 13.30% |
| 5 | 8.523 | 10.36627 | 17.60% | 29 | 23.005 | 3.86291 | 16.40% |
| 6 | 9.17 | 9.6366 | 12.30% | 30 | 23.559 | 3.77327 | 24.00% |
| 7 | 10.256 | 8.61794 | 21.70% | 31 | 23.983 | 3.70754 | 25.70% |
| 8 | 11.718 | 7.5458 | 8.60% | 32 | 24.675 | 3.60507 | 19.70% |
| 9 | 12.885 | 6.86488 | 100.00% | 33 | 25.081 | 3.54768 | 49.20% |
| 10 | 13.462 | 6.57226 | 16.40% | 34 | 25.496 | 3.49088 | 59.20% |
| 11 | 13.761 | 6.43017 | 7.80% | 35 | 25.917 | 3.43502 | 9.70% |
| 12 | 13.928 | 6.35326 | 20.10% | 36 | 26.578 | 3.35112 | 2.90% |
| 13 | 14.438 | 6.1301 | 51.20% | 37 | 27.083 | 3.28974 | 9.50% |
| 14 | 14.668 | 6.03436 | 21.10% | 38 | 27.63 | 3.22589 | 8.30% |
| 15 | 15.905 | 5.56779 | 47.80% | 39 | 28.154 | 3.16703 | 24.60% |
| 16 | 16.247 | 5.45135 | 23.60% | 40 | 29.058 | 3.07055 | 17.10% |
| 17 | 16.609 | 5.33328 | 10.10% | 41 | 31.515 | 2.83649 | 8.10% |
| 18 | 17.103 | 5.18037 | 7.60% | 42 | 31.958 | 2.79817 | -3.30% |
| 19 | 17.943 | 4.93961 | 14.80% | 43 | 32.727 | 2.7342 | 11.80% |
| 20 | 19.204 | 4.61801 | 3.90% | 44 | 34.248 | 2.61615 | 9.60% |
| 21 | 20 | 4.436 | 8.00% | 45 | 35.914 | 2.4985 | 7.00% |
| 22 | 20.352 | 4.36009 | 50.60% | 46 | 37.149 | 2.41827 | 2.40% |
| 23 | 20.488 | 4.33131 | 48.30% | 47 | 38.731 | 2.32303 | 12.90% |
| 24 | 21.145 | 4.1982 | 19.40% | | | | |

### Example 9 Crystalline Form C of the Compound of Formula V

800.6 mg of the compound of formula I was added to 12 mL of acetonitrile, and the temperature was raised to 50°C. 1.09 mL of a dilute phosphoric acid solution was added to the system (preparation method for dilute phosphoric acid: 0.1 mL of phosphoric acid dissolved in 0.9 mL of ethyl acetate). 3 mg of seeds of crystalline form B of the compound of formula V were added. The entire system was stirred at 50°C for 2 hours, cooled to 25°C with stirring, filtered, and the resulting wet product was vacuum dried at 50°C for 2 hours to obtain crystalline form C of the compound of formula V. The molar ratio of the compound of formula I to phosphoric acid was 1:1.05. Crystalline form C of the compound of formula V is an anhydrate, and NMR shows that crystalline form C of the phosphate salt of the compound of formula I (crystalline form C of the compound of formula V) contains 0.1 equivalent of acetonitrile. The XRPD pattern of crystalline form C of the compound of formula I is shown in FIG. 21, and it has the diffraction peaks shown in Table 11 below.

**Table 11**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 5.194 | 17.00128 | 49.10% | 41 | 24.561 | 3.62162 | 2.70% |
| 2 | 7.95 | 11.11257 | 4.30% | 42 | 24.804 | 3.58669 | 30.20% |
| 3 | 8.388 | 10.53338 | 51.00% | 43 | 25.27 | 3.52153 | 28.10% |
| 4 | 9.027 | 9.78834 | 9.70% | 44 | 25.665 | 3.46825 | 60.90% |
| 5 | 9.443 | 9.35808 | 0.90% | 45 | 26.002 | 3.42398 | 4.60% |
| 6 | 10.215 | 8.65246 | 8.60% | 46 | 26.193 | 3.39955 | 18.70% |
| 7 | 10.468 | 8.44417 | 48.10% | 47 | 26.423 | 3.37046 | 11.90% |
| 8 | 11.192 | 7.89926 | 16.20% | 48 | 26.94 | 3.30687 | 12.40% |
| 9 | 11.506 | 7.68472 | 100.00% | 49 | 27.185 | 3.27765 | 13.70% |
| 10 | 11.672 | 7.57563 | 23.90% | 50 | 27.467 | 3.24469 | 39.90% |
| 11 | 12.726 | 6.95069 | 21.10% | 51 | 28.108 | 3.17204 | 7.00% |
| 12 | 12.885 | 6.86505 | 17.90% | 52 | 28.431 | 3.13677 | 2.50% |
| 13 | 13.137 | 6.73409 | 5.10% | 53 | 28.725 | 3.10532 | 8.90% |
| 14 | 13.623 | 6.49459 | 3.90% | 54 | 29.083 | 3.06794 | 13.50% |
| 15 | 14.586 | 6.06817 | 12.80% | 55 | 29.412 | 3.03439 | 33.20% |
| 16 | 15.052 | 5.88112 | 13.40% | 56 | 30.123 | 2.96435 | 10.90% |
| 17 | 15.682 | 5.64618 | 33.50% | 57 | 30.326 | 2.94497 | 9.90% |
| 18 | 15.865 | 5.58146 | 26.00% | 58 | 30.528 | 2.92594 | 3.90% |
| 19 | 16.158 | 5.48123 | 5.20% | 59 | 31.259 | 2.85912 | 2.00% |
| 20 | 16.787 | 5.27694 | 9.60% | 60 | 31.674 | 2.82261 | 9.10% |
| 21 | 16.935 | 5.23145 | 7.20% | 61 | 32.155 | 2.78146 | 2.40% |
| 22 | 17.236 | 5.14075 | 3.60% | 62 | 32.544 | 2.74915 | 3.70% |
| 23 | 17.57 | 5.04367 | 3.80% | 63 | 32.701 | 2.73627 | 2.80% |
| 24 | 17.898 | 4.95191 | 25.20% | 64 | 33.023 | 2.71038 | 3.50% |
| 25 | 18.012 | 4.92095 | 56.50% | 65 | 33.438 | 2.67767 | 7.20% |
| 26 | 18.393 | 4.81986 | 10.90% | 66 | 33.929 | 2.64 | 6.00% |
| 27 | 18.756 | 4.72738 | 34.70% | 67 | 34.274 | 2.61419 | 3.70% |
| 28 | 19.092 | 4.64491 | 7.00% | 68 | 34.754 | 2.57922 | 3.40% |
| 29 | 19.809 | 4.47827 | 32.70% | 69 | 34.975 | 2.56342 | 9.70% |
| 30 | 20.481 | 4.33291 | 32.90% | 70 | 35.465 | 2.52912 | 8.40% |
| 31 | 20.843 | 4.25843 | 28.10% | 71 | 35.731 | 2.51089 | 3.10% |
| 32 | 21.676 | 4.09663 | 29.00% | 72 | 36.389 | 2.46699 | 4.20% |
| 33 | 22.103 | 4.01842 | 32.30% | 73 | 36.885 | 2.43497 | 1.20% |
| 34 | 22.551 | 3.93962 | 79.40% | 74 | 37.388 | 2.40336 | 6.80% |
| 35 | 23.098 | 3.84751 | 34.60% | 75 | 37.963 | 2.36822 | 4.20% |
| 36 | 23.226 | 3.82662 | 25.20% | 76 | 38.424 | 2.34089 | 3.70% |
| 37 | 23.486 | 3.78492 | 9.60% | 77 | 38.711 | 2.32418 | 4.50% |
| 38 | 23.704 | 3.75051 | 12.70% | 78 | 39.164 | 2.29832 | 6.40% |
| 39 | 23.943 | 3.71361 | 27.10% | 79 | 39.661 | 2.27068 | 3.30% |
| 40 | 24.147 | 3.68273 | 30.10% | | | | |

The DSC curve of the crystalline form C of the compound of formula V is shown in FIG. 22. The DSC curve shows endothermic peaks at peak temperatures of 188.32°C (enthalpy value 41.702 J/g) and 221.66°C (enthalpy value 32.668 J/g), with onset points (Onset x) at 175.87°C and 32.668°C, respectively. The TGA curve is shown in FIG. 23. The sample shows a weight loss of 0.71% when heated from 29.55°C to 180.0°C, and a weight loss of 0.85% when heated from 180.0°C to 220.0°C.

### Example 10 Crystalline Form A of the Compound of Formula VI

30 mg of the compound of formula I and 7.04 mg of fumaric acid were added to 0.5 mL of acetonitrile, and the temperature was raised to 50°C. The entire system was stirred at 50°C for 2 hours, then cooled to 25°C with stirring, filtered, and the resulting wet product was vacuum dried at 50°C for 2 hours to obtain crystalline form A of the compound of formula VI. The molar ratio of the compound of formula I to fumaric acid was 1:1.01. The XRPD pattern of crystalline form A of the fumarate salt of the compound of formula I (crystalline form A of the compound of formula VI) is shown in FIG. 24, and it has diffraction peaks as shown in Table 12 below.

NMR: ¹H NMR (400 MHz, DMSO-*d*₆) δ 13.65 (s, 1H), 13.11 (s, 2H), 9.16 (d, *J =* 2.0 Hz, 1H), 8.92 (s, 1H), 8.19 (s, 1H), 7.63 (s, 1H), 7.53 (s, 1H), 6.63 (s, 2H), 4.03 (s, 3H), 3.61 (s, 3H), 2.26 (d, *J =* 1.6 Hz, 3H).

**Table 12**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 5.527 | 15.97563 | 16.80% | 24 | 19.864 | 4.466 | 7.80% |
| 2 | 6.733 | 13.11834 | 20.90% | 25 | 20.599 | 4.30833 | 15.60% |
| 3 | 6.959 | 12.69269 | 47.50% | 26 | 21.267 | 4.1745 | 14.80% |
| 4 | 7.737 | 11.41816 | 9.70% | 27 | 21.759 | 4.08112 | 11.20% |
| 5 | 8.599 | 10.2745 | 42.10% | 28 | 22.345 | 3.97554 | 13.80% |
| 6 | 9.379 | 9.42209 | 14.10% | 29 | 22.552 | 3.93942 | 32.30% |
| 7 | 9.91 | 8.91803 | 9.40% | 30 | 22.758 | 3.90426 | 21.80% |
| 8 | 10.278 | 8.59978 | 21.00% | 31 | 23.378 | 3.80204 | 39.60% |
| 9 | 10.577 | 8.35741 | 90.90% | 32 | 23.946 | 3.71313 | 11.20% |
| 10 | 12.156 | 7.2748 | 53.50% | 33 | 24.521 | 3.6274 | 9.20% |
| 11 | 13.45 | 6.57797 | 21.50% | 34 | 25.146 | 3.53865 | 25.40% |
| 12 | 13.735 | 6.44195 | 12.60% | 35 | 25.709 | 3.4624 | 40.00% |
| 13 | 13.982 | 6.3287 | 100.00% | 36 | 27.37 | 3.2559 | 13.20% |
| 14 | 14.844 | 5.96313 | 42.10% | 37 | 27.682 | 3.21995 | 12.40% |
| 15 | 15.391 | 5.75233 | 43.10% | 38 | 28.842 | 3.09299 | 16.60% |
| 16 | 15.733 | 5.62829 | 7.20% | 39 | 29.908 | 2.98517 | 23.30% |
| 17 | 16.631 | 5.32636 | 35.60% | 40 | 31.774 | 2.81395 | 11.50% |
| 18 | 17.077 | 5.18824 | 52.80% | 41 | 32.663 | 2.73942 | 10.20% |
| 19 | 17.37 | 5.10132 | 5.40% | 42 | 34.767 | 2.57831 | 16.80% |
| 20 | 18.669 | 4.74913 | 21.60% | 43 | 35.226 | 2.54572 | 6.90% |
| 21 | 18.97 | 4.67444 | 20.90% | 44 | 37.118 | 2.42017 | 3.80% |
| 22 | 19.16 | 4.62861 | 26.60% | 45 | 38.115 | 2.35915 | 8.40% |
| 23 | 19.276 | 4.60083 | 34.30% | | | | |

### Example 11 Crystalline Form A of the Compound of Formula VII

30 mg of the compound of formula I was added to 0.5 mL of acetonitrile, and the temperature was raised to 50°C. 0.039 mL of a methanesulfonic acid solution (0.1 mL of methanesulfonic acid dissolved in 0.9 mL of ethyl acetate) was added. The entire system was stirred at 50°C for 2 hours, then cooled to 25°C with stirring, filtered, and the resulting wet product was vacuum dried at 50°C for 2 hours to obtain crystalline form A of the compound of formula VII. The molar ratio of the compound of formula I to methanesulfonic acid was 1:1.03. The XRPD pattern of crystalline form A of the compound of formula VII is shown in FIG. 25, and it has diffraction peaks as shown in Table 13 below.

NMR:¹H NMR (400 MHz, DMSO-*d₆*) δ 13.64 (s, 1H), 9.15 (d, *J =* 2.0 Hz, 1H), 8.92 (s, 1H), 8.19 (s, 1H), 7.63 (s, 1H), 7.54 (s, 1H), 4.03 (s, 3H), 3.62 (s, 3H), 2.63 (s, 3H), 2.29 (s, 3H), 2.26 (d, *J =* 1.6 Hz, 3H).

**Table 13**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 5.132 | 17.20602 | 16.30% | 16 | 21.155 | 4.19632 | 9.00% |
| 2 | 6.727 | 13.12872 | 13.80% | 17 | 21.744 | 4.08391 | 49.70% |
| 3 | 8.614 | 10.2569 | 32.10% | 18 | 22.365 | 3.97189 | 12.10% |
| 4 | 8.997 | 9.82161 | 100.00% | 19 | 23.183 | 3.8336 | 6.10% |
| 5 | 10.049 | 8.79483 | 9.80% | 20 | 23.707 | 3.75013 | 7.80% |
| 6 | 10.341 | 8.5475 | 6.50% | 21 | 24.426 | 3.64125 | 18.70% |
| 7 | 11.167 | 7.91683 | 4.40% | 22 | 25.322 | 3.51441 | 31.50% |
| 8 | 11.566 | 7.64514 | 9.50% | 23 | 26.356 | 3.37883 | 12.50% |
| 9 | 13.927 | 6.35347 | 5.60% | 24 | 27.293 | 3.26497 | 7.90% |
| 10 | 15.062 | 5.87733 | 7.50% | 25 | 27.926 | 3.19232 | 11.90% |
| 11 | 15.551 | 5.69372 | 13.90% | 26 | 28.326 | 3.14817 | 13.10% |
| 12 | 17.441 | 5.08075 | 58.10% | 27 | 29.137 | 3.0624 | 10.00% |
| 13 | 17.739 | 4.99592 | 18.10% | 28 | 29.856 | 2.99021 | 8.70% |
| 14 | 18.62 | 4.76144 | 8.90% | 29 | 31.067 | 2.87641 | 1.70% |
| 15 | 20.076 | 4.41946 | 3.30% | | | | |

### Example 12 Crystalline Form A of the Compound of Formula VIII

30 mg of the compound of formula I and 9.68 mg of benzenesulfonic acid were added to 0.5 mL of ethyl acetate, and the temperature was raised to 50°C. The entire system was stirred at 50°C for 2 hours, then cooled to 25°C with stirring, filtered, and the resulting wet product was vacuum dried at 50°C for 2 hours to obtain crystalline form A of the compound of formula VIII. The molar ratio of the compound of formula I to benzenesulfonic acid was 1: 1.00, and NMR showed the molar ratio of the compound of formula I to ethyl acetate was 1:0.07. The XRPD pattern of crystalline form A of the compound of formula VIII is shown in FIG. 26, and it has diffraction peaks as shown in Table 14 below.

NMR: ¹H NMR (400 MHz, DMSO-*d₆*) δ 13.64 (s, 1H), 9.15 (d, *J* = 2.0 Hz, 1H), 8.92 (s, 1H), 8.19 (s, 1H), 7.63 (s, 1H), 7.60-7.58 (m, 2H), 7.54 (s, 1H), 7.34-7.29 (m, 3H), 4.03 (s, 3H), 3.61 (s, 3H), 2.63 (s, 3H), 2.26 (d, *J =* 1.6 Hz, 3H).

**Table 14**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 6.475 | 13.6404 | 14.30% | 22 | 20.349 | 4.3607 | 19.20% |
| 2 | 7.661 | 11.53042 | 100.00% | 23 | 20.89 | 4.24904 | 20.10% |
| 3 | 9.059 | 9.75377 | 21.40% | 24 | 21.099 | 4.20735 | 9.60% |
| 4 | 9.719 | 9.09297 | 20.80% | 25 | 21.675 | 4.09672 | 31.10% |
| 5 | 10.087 | 8.76259 | 45.10% | 26 | 22.266 | 3.98938 | 24.30% |
| 6 | 11.104 | 7.96192 | 18.60% | 27 | 22.494 | 3.94942 | 19.60% |
| 7 | 11.921 | 7.41792 | 44.30% | 28 | 22.968 | 3.86906 | 28.30% |
| 8 | 12.359 | 7.15589 | 10.80% | 29 | 23.497 | 3.78306 | 22.00% |
| 9 | 12.984 | 6.81282 | 9.10% | 30 | 24.844 | 3.58097 | 32.30% |
| 10 | 13.906 | 6.36334 | 79.00% | 31 | 25.515 | 3.48834 | 42.40% |
| 11 | 14.416 | 6.13904 | 10.20% | 32 | 25.974 | 3.42768 | 18.20% |
| 12 | 15.142 | 5.84662 | 6.30% | 33 | 26.695 | 3.33665 | 10.20% |
| 13 | 15.912 | 5.56516 | 19.50% | 34 | 27.335 | 3.25998 | 8.50% |
| 14 | 16.551 | 5.35171 | 20.20% | 35 | 28.189 | 3.16317 | 8.50% |
| 15 | 17.038 | 5.19977 | 9.40% | 36 | 29.356 | 3.04006 | 20.20% |
| 16 | 17.601 | 5.03481 | 12.70% | 37 | 30.275 | 2.94982 | 10.90% |
| 17 | 17.995 | 4.92535 | 18.50% | 38 | 30.805 | 2.90025 | 10.10% |
| 18 | 18.269 | 4.85222 | 41.70% | 39 | 31.765 | 2.81476 | 5.80% |
| 19 | 18.507 | 4.79024 | 58.50% | 40 | 32.965 | 2.715 | 9.50% |
| 20 | 19.159 | 4.62871 | 50.10% | 41 | 34.371 | 2.6071 | 11.60% |
| 21 | 19.716 | 4.49913 | 11.20% | | | | |

### Example 13 Crystalline Form A of the Compound of Formula IX

30 mg of the compound of Formula I and 10.44 mg of p-toluenesulfonic acid were added to 0.5 mL ofacetone/water (95 : 5, v : v), and the temperature was raised to 50°C. The entire system was stirred at 50°C for 2 hours, cooled to 25°C with stirring, filtered, and the resulting wet product was vacuum dried at 50°C for 2 hours to obtain Crystalline Form A of the compound of Formula IX. The XRPD pattern of Crystalline Form A of the compound of Formula IX is shown in FIG. 27, and it has diffraction peaks as shown in Table 15 below.

NMR: ¹H NMR (400 MHz, DMSO-*d*₆) δ 13.64 (s, 1H), 9.15 (d, *J* = 2.0 Hz, 1H), 8.91 (s, 1H), 8.18 (s, 1H), 7.63 (s, 1H), 7.53 (s, 1H), 7.47 (d, *J =* 8.4 Hz, 2H), 7.11 (d, *J =* 7.6 Hz, 2H), 4.03 (s, 3H), 3.61 (s, 3H), 2.63 (s, 3H), 2.29 (s, 3H), 2.26 (d, *J =* 1.6 Hz, 3H).

**Table 15**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 5.962 | 14.81202 | 57.90% | 39 | 25.978 | 3.42709 | 27.10% |
| 2 | 8.204 | 10.76821 | 4.10% | 40 | 26.258 | 3.39119 | 6.70% |
| 3 | 8.371 | 10.55406 | 5.70% | 41 | 26.662 | 3.3408 | 6.70% |
| 4 | 9.927 | 8.90302 | 10.20% | 42 | 27.349 | 3.25839 | 21.60% |
| 5 | 10.133 | 8.72234 | 8.40% | 43 | 27.516 | 3.23902 | 30.80% |
| 6 | 10.573 | 8.36071 | 36.50% | 44 | 27.793 | 3.20733 | 7.70% |
| 7 | 11.032 | 8.01334 | 14.20% | 45 | 28.075 | 3.1757 | 31.60% |
| 8 | 11.882 | 7.44222 | 14.90% | 46 | 28.299 | 3.15109 | 10.80% |
| 9 | 12.339 | 7.16786 | 33.30% | 47 | 28.965 | 3.08021 | 15.00% |
| 10 | 12.767 | 6.9283 | 24.90% | 48 | 29.166 | 3.05937 | 39.40% |
| 11 | 13.419 | 6.59304 | 5.20% | 49 | 29.35 | 3.04064 | 12.90% |
| 12 | 14.06 | 6.29386 | 32.00% | 50 | 29.915 | 2.9845 | 7.40% |
| 13 | 14.396 | 6.14788 | 28.30% | 51 | 30.364 | 2.94138 | 40.50% |
| 14 | 14.974 | 5.91163 | 29.20% | 52 | 31.21 | 2.86355 | 6.60% |
| 15 | 15.682 | 5.64651 | 2.80% | 53 | 31.645 | 2.82513 | 5.50% |
| 16 | 15.845 | 5.58857 | 1.80% | 54 | 31.834 | 2.80877 | 16.00% |
| 17 | 16.714 | 5.30007 | 100.00% | 55 | 31.958 | 2.7982 | 37.80% |
| 18 | 17.555 | 5.04799 | 29.70% | 56 | 32.619 | 2.74298 | 2.80% |
| 19 | 17.785 | 4.98319 | 73.50% | 57 | 32.954 | 2.71585 | 11.80% |
| 20 | 18.413 | 4.81446 | 31.00% | 58 | 33.386 | 2.68169 | 6.40% |
| 21 | 18.696 | 4.74244 | 15.60% | 59 | 33.718 | 2.65606 | 3.40% |
| 22 | 19.188 | 4.62193 | 5.90% | 60 | 34.173 | 2.62168 | 10.50% |
| 23 | 19.668 | 4.51003 | 13.00% | 61 | 34.59 | 2.59107 | 8.40% |
| 24 | 19.877 | 4.4632 | 43.60% | 62 | 35.191 | 2.54821 | 3.00% |
| 25 | 20.33 | 4.36466 | 57.50% | 63 | 35.191 | 2.54821 | 3.00% |
| 26 | 20.684 | 4.29085 | 15.20% | 64 | 35.477 | 2.52831 | 3.80% |
| 27 | 21.198 | 4.18782 | 29.10% | 65 | 35.913 | 2.49857 | 5.40% |
| 28 | 21.486 | 4.13245 | 2.70% | 66 | 36.124 | 2.48447 | 4.50% |
| 29 | 22.112 | 4.01691 | 32.50% | 67 | 36.345 | 2.4699 | 9.70% |
| 30 | 22.637 | 3.92478 | 45.20% | 68 | 36.981 | 2.42881 | 2.20% |
| 31 | 23.176 | 3.83469 | 32.40% | 69 | 37.406 | 2.40222 | 6.20% |
| 32 | 23.856 | 3.72701 | 2.20% | 70 | 37.623 | 2.38887 | 10.10% |
| 33 | 24.224 | 3.67124 | 46.00% | 71 | 37.83 | 2.37624 | 4.30% |
| 34 | 24.66 | 3.6073 | 72.50% | 72 | 38.317 | 2.34719 | 3.70% |
| 35 | 25.148 | 3.53832 | 6.60% | 73 | 38.881 | 2.31439 | 7.90% |
| 36 | 25.427 | 3.5001 | 32.70% | 74 | 39.015 | 2.30674 | 5.20% |
| 37 | 25.651 | 3.47007 | 8.80% | 75 | 39.544 | 2.27711 | 5.00% |
| 38 | 25.836 | 3.4456 | 13.40% | | | | |

The DSC pattern of Crystalline Form A of the compound of Formula IX is shown in FIG. 28. The DSC curve shows two endothermic peaks at peak temperatures of 95.83°C (enthalpy value 244.34 J/g) and 194.7°C (enthalpy value 34.862 J/g), with onset points (Onset x) at 57.37°C and 181.84°C, respectively. The TGA pattern is shown in FIG. 29. The sample showed a weight loss of 2.03% when heated from 30.52°C to 60.0°C, a weight loss of 5.13% when heated from 60.0°C to 100.0°C, and a weight loss of 0.50% when heated from 100.0°C to 190.0°C. NMR data indicate that in Crystalline Form A of the compound of Formula IX, the molar ratio of water to the compound of Formula I to p-toluenesulfonic acid is 3:1:1.

### Example 13-1 Single Crystal of the Compound of Formula IX

The compound of Formula I (1.63 kg, 3.11 mol) was added to a mixed solution of 10.3 kg of acetone and 1.30 kg of water, the temperature was controlled at 45-55°C, and 80 g of Crystalline Form A of the compound of Formula IX was added to obtain a mixture. 0.58 kg of p-toluenesulfonic acid monohydrate was dissolved in 2.57 kg of acetone and 0.32 kg of water, and the resulting solution was added dropwise to the above mixture. The mixture was stirred at a controlled temperature of 45-55°C for 2 hours. The temperature was lowered to 20-30°C, and the mixture was stirred for 2 hours. 0.29 kg of p-toluenesulfonic acid monohydrate was dissolved in 1.28 kg of acetone and 0.16 kg of water, and the resulting solution was added dropwise to the above mixture. The mixture was stirred at a controlled temperature of 20-30°C for 2 hours. The mixture was filtered to obtain a single crystal of the compound of Formula IX, wherein the molar ratio of water to the compound of Formula I to p-toluenesulfonic acid is 3:1:1.

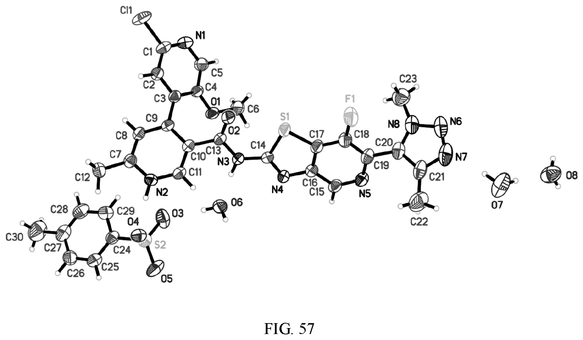

The molecular structure of the single crystal of the compound of Formula IX is shown in FIG. 57, and the unit cell parameters are shown in the table below:

| | |
|---|---|
| Unit cell dimensions/mm³ | 0.200 × 0.180 × 0.150 mm³ |
| Wavelength | 1.54178 Å |
| Crystal system | Triclinic |
| Space group | P-1 |
| a/Å | 10.6990(12) Å |
| b/Å | 10.7640(13) Å |
| c/Å | 14.9358(17) Å |
| α/° | 89.299(7)° |
| β/° | 86.519(6)° |
| γ/° | 84.364(6)° |
| Unit cell volume/Å³ | 1708.6(3) Å³ |
| Z | 2 |
| Unit cell density mg/m³ | 1.460 mg/m³ |
| F (000) | 780 |
| Absorption coefficient mm ⁻¹ | 2.717 mm⁻¹ |
| Diffraction index range (h/k/l) | -12<=h<=12, -12<=k<=12, -17<=l<=17 |
| Temperature/K | 173(2) K |
| Theta range for data collection/° | 2.964 to 66.596° |
| Data/restraints/parameters | 5746 / 1 / 477 |
| Absorption correction | Semi-empirical from equivalents |
| Max. and min. transmission | 0.7531 and 0.6736 |
| Refinement method | Full-matrix least-squares on F² |
| Goodness-of-fit on F² | 0.894 |
| Final R indices [I>=2σ(I)] | R1 = 0.0359, wR2 = 0.1027 |
| R factor (Final R indices) [all data] | R1 = 0.0435, wR2 = 0.1109 |
| Largest diff. peak and hole / e.Å⁻³ | 0.300 and -0.432 e.Å⁻³ |
| Reflections collected | 21870 |
| Independent reflections | 5746 [R(int) = 0.0379] |
| Absolute structure parameter | N/A |

### Example 14 Crystalline Form B of the Compound of Formula IX

30 mg of the compound of Formula I and 10.44 mg of p-toluenesulfonic acid were added to 0.5 mL of acetonitrile, and the temperature was raised to 50°C. The entire system was stirred at 50°C for 2 hours, then cooled to 25°C with stirring, filtered, and the obtained wet product was vacuum dried at 50°C for 2 hours to obtain Crystalline Form B of the compound of Formula IX. The molar ratio of the compound of Formula I to p-toluenesulfonic acid is 1:1.00. The XRPD pattern of Crystalline Form B of the p-toluenesulfonate salt of the compound of Formula I (Crystalline Form B of the compound of Formula IX) is shown in FIG. 30, and it has diffraction peaks as shown in Table 16 below.

NMR: ¹H NMR (400 MHz, DMSO-*d*₆) δ 13.65 (s, 1H), 9.15 (d, *J =* 2.0 Hz, 1H), 8.92 (s, 1H), 8.19 (s, 1H), 7.63 (s, 1H), 7.54 (s, 1H), 7.47 (d, *J =* 8.4 Hz, 2H), 7.11 (d, *J =* 7.6 Hz, 2H), 4.03 (s, 3H), 3.61 (s, 3H), 2.63 (s, 3H), 2.29 (s, 3H), 2.26 (d, *J =* 1.6 Hz, 3H).

**Table 16**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 5.339 | 16.53947 | 100.00% | 21 | 19.465 | 4.55658 | 31.00% |
| 2 | 8.195 | 10.78059 | 23.30% | 22 | 19.846 | 4.46999 | 52.90% |
| 3 | 9.083 | 9.72794 | 13.30% | 23 | 21.533 | 4.12355 | 18.40% |
| 4 | 9.335 | 9.46653 | 63.50% | 24 | 22.137 | 4.0123 | 7.00% |
| 5 | 9.921 | 8.90842 | 50.50% | 25 | 22.875 | 3.88447 | 22.70% |
| 6 | 10.125 | 8.72971 | 32.20% | 26 | 24.412 | 3.6434 | 31.40% |
| 7 | 11.117 | 7.95254 | 16.10% | 27 | 25.425 | 3.50047 | 23.50% |
| 8 | 11.469 | 7.70919 | 17.90% | 28 | 25.749 | 3.45708 | 23.10% |
| 9 | 12.189 | 7.25545 | 13.10% | 29 | 26.479 | 3.36337 | 23.50% |
| 10 | 12.839 | 6.88971 | 54.40% | 30 | 26.833 | 3.31984 | 12.40% |
| 11 | 13.182 | 6.71091 | 34.90% | 31 | 27.344 | 3.25898 | 8.80% |
| 12 | 14.888 | 5.94554 | 8.90% | 32 | 27.945 | 3.19024 | 19.80% |
| 13 | 15.262 | 5.80086 | 13.80% | 33 | 28.263 | 3.15503 | 9.20% |
| 14 | 16.132 | 5.48968 | 63.90% | 34 | 28.665 | 3.11168 | 10.00% |
| 15 | 16.362 | 5.41311 | 46.10% | 35 | 29.119 | 3.06425 | 9.00% |
| 16 | 16.606 | 5.3341 | 73.50% | 36 | 29.963 | 2.97978 | 22.10% |
| 17 | 16.891 | 5.24471 | 13.20% | 37 | 30.835 | 2.89751 | 8.80% |
| 18 | 17.307 | 5.11958 | 9.50% | 38 | 32.466 | 2.75553 | 14.10% |
| 19 | 18.597 | 4.76732 | 37.50% | 39 | 35.254 | 2.54373 | 5.30% |
| 20 | 18.67 | 4.74878 | 60.70% | | | | |

### Example 15 Crystalline Form A of the compound of Formula X

30 mg of the compound of Formula I and 22.06 mg of 1,5-naphthalenedisulfonic acid were added to 0.5 mL of acetone/water (95:5, v:v), and the temperature was raised to 50°C. The entire system was stirred at 50°C for 2 hours, then cooled to 25°C with stirring, filtered, and the obtained wet product was vacuum dried at 50°C for 2 hours to obtain Crystalline Form A of the compound of Formula X. The molar ratio of the compound of Formula I to 1,5-naphthalenedisulfonic acid is 1:1.02. The XRPD pattern of Crystalline Form A of the compound of Formula X is shown in FIG. 31, and it has diffraction peaks as shown in Table 17 below.

NMR: ¹H NMR (400 MHz, DMSO-*d*₆) δ 13.64 (s, 1H), 9.15 (d, *J* = 2.0 Hz, 1H), 8.91 (s, 1H), 8.85 (d, *J =* 8.4 Hz, 2H), 8.18 (s, 1H), 7.92 (d, *J =* 7.2 Hz, 2H), 7.63 (s, 1H), 7.53 (s, 1H), 7.40 (dd, *J* = 8.4, 7.6 Hz, 2H), 4.03 (s, 3H), 3.61 (s, 3H), 2.63 (s, 3H), 2.26 (d, *J =* 1.6 Hz, 3H).

**Table 17**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 5.713 | 15.4567 | 7.20% | 25 | 23.845 | 3.7287 | 3.90% |
| 2 | 7.456 | 11.84734 | 81.90% | 26 | 24.23 | 3.67024 | 3.00% |
| 3 | 8.673 | 10.18701 | 2.90% | 27 | 24.571 | 3.62016 | 20.40% |
| 4 | 11.43 | 7.73558 | 25.50% | 28 | 24.994 | 3.55975 | 9.80% |
| 5 | 12.012 | 7.36179 | 100.00% | 29 | 25.441 | 3.49833 | 3.30% |
| 6 | 12.345 | 7.16417 | 2.10% | 30 | 26.083 | 3.41361 | 8.90% |
| 7 | 13.532 | 6.53813 | 27.00% | 31 | 26.593 | 3.34923 | 10.50% |
| 8 | 13.703 | 6.45705 | 9.00% | 32 | 26.898 | 3.31198 | 6.70% |
| 9 | 13.928 | 6.35325 | 7.70% | 33 | 28.049 | 3.17861 | 5.80% |
| 10 | 14.751 | 6.00068 | 5.80% | 34 | 28.435 | 3.13633 | 20.50% |
| 11 | 14.914 | 5.93552 | 13.20% | 35 | 28.687 | 3.10937 | 8.10% |
| 12 | 15.324 | 5.77744 | 9.10% | 36 | 28.976 | 3.07906 | 6.30% |
| 13 | 17.235 | 5.14087 | 28.30% | 37 | 29.329 | 3.04281 | 4.20% |
| 14 | 17.708 | 5.00472 | 1.60% | 38 | 30.168 | 2.95998 | 3.30% |
| 15 | 18.485 | 4.79589 | 25.50% | 39 | 30.869 | 2.89436 | 6.10% |
| 16 | 18.767 | 4.72462 | 21.20% | 40 | 31.214 | 2.86319 | 5.40% |
| 17 | 19.02 | 4.66231 | 4.70% | 41 | 32.378 | 2.76287 | 2.70% |
| 18 | 21.077 | 4.21171 | 24.30% | 42 | 33.52 | 2.6713 | 2.80% |
| 19 | 21.695 | 4.093 | 49.30% | 43 | 34.636 | 2.58771 | 1.80% |
| 20 | 22.12 | 4.01545 | 11.90% | 44 | 36.84 | 2.43779 | 2.90% |
| 21 | 22.48 | 3.95186 | 2.60% | 45 | 37.805 | 2.37776 | 2.60% |
| 22 | 22.796 | 3.89782 | 10.50% | 46 | 38.41 | 2.34167 | 1.90% |
| 23 | 22.948 | 3.87227 | 17.80% | 47 | 38.842 | 2.31667 | 5.70% |
| 24 | 23.354 | 3.80594 | 2.00% | 48 | 39.543 | 2.27716 | 2.50% |

### Example 16 Crystalline Form B of the compound of Formula X

30 mg of the compound of Formula I and 22.06 mg of 1,5-naphthalenedisulfonic acid were added to 0.5 mL of ethyl acetate, and the temperature was raised to 50°C. The entire system was stirred at 50°C for 2 hours, then cooled to 25°C with stirring, filtered, and the obtained wet product was vacuum dried at 50°C for 2 hours to obtain Crystalline Form B of the compound of Formula X. The molar ratio of the compound of Formula I to 1,5-naphthalenedisulfonic acid is 1:0.93. The XRPD pattern of Crystalline Form B of the compound of Formula X is shown in FIG. 32, and it has diffraction peaks as shown in Table 18 below.

NMR: ¹H NMR (400 MHz, DMSO-*d*₆) δ 13.64 (s, 1H), 9.15 (d, *J* = 2.0 Hz, 1H), 8.91 (s, 1H), 8.86 (d, *J =* 8.4 Hz, 2H), 8.18 (s, 1H), 7.92 (d, *J =* 7.2 Hz, 2H), 7.63 (s, 1H), 7.53 (s, 1H), 7.40 (dd, *J* = 8.4, 7.6 Hz, 2H), 4.03 (s, 3H), 3.61 (s, 3H), 2.63 (s, 3H), 2.26 (d, *J* = 1.6 Hz, 3H).

**Table 18**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 5.946 | 14.85159 | 20.80% | 18 | 23.793 | 3.73676 | 58.00% |
| 2 | 8.964 | 9.85725 | 81.20% | 19 | 24.141 | 3.6836 | 23.80% |
| 3 | 10.807 | 8.18027 | 91.00% | 20 | 24.553 | 3.62279 | 42.60% |
| 4 | 11.977 | 7.38357 | 46.80% | 21 | 25.565 | 3.48153 | 28.00% |
| 5 | 14.091 | 6.28001 | 23.00% | 22 | 26.293 | 3.38677 | 12.50% |
| 6 | 14.696 | 6.02294 | 14.40% | 23 | 26.989 | 3.30096 | 3.00% |
| 7 | 15.797 | 5.6055 | 48.20% | 24 | 27.256 | 3.26925 | 74.60% |
| 8 | 16.97 | 5.22056 | 29.20% | 25 | 27.772 | 3.2097 | 70.10% |
| 9 | 17.916 | 4.94708 | 100.00% | 26 | 29.656 | 3.00995 | 34.10% |
| 10 | 18.4 | 4.81795 | 64.10% | 27 | 30.281 | 2.94924 | 15.00% |
| 11 | 20.028 | 4.42978 | 30.00% | 28 | 31.009 | 2.88165 | 15.20% |
| 12 | 20.734 | 4.28054 | 23.10% | 29 | 32.208 | 2.77701 | 15.40% |
| 13 | 21.272 | 4.17353 | 45.00% | 30 | 32.528 | 2.75045 | 26.80% |
| 14 | 22.072 | 4.02397 | 86.10% | 31 | 36.294 | 2.47322 | 25.50% |
| 15 | 22.422 | 3.96191 | 36.20% | 32 | 37.499 | 2.39649 | 16.40% |
| 16 | 22.76 | 3.90387 | 32.30% | 33 | 38.636 | 2.32852 | 20.10% |
| 17 | 23.273 | 3.81897 | 51.70% | 34 | 39.292 | 2.29116 | 20.30% |

### Example 17 Crystalline Form C of the compound of Formula X

30 mg of the compound of Formula X and 22.06 mg of 1,5-naphthalenedisulfonic acid were added to 0.5 mL of acetonitrile, and the temperature was raised to 50°C. The entire system was stirred at 50°C for 2 hours, then cooled to 25°C with stirring, filtered, and the obtained wet product was vacuum dried at 50°C for 2 hours to obtain Crystalline Form C of the compound of Formula X. The molar ratio of the compound of Formula I to 1,5-naphthalenedisulfonic acid is 1:1.00. The XRPD pattern of Crystalline Form C of the compound of Formula X is shown in FIG. 33, and it has diffraction peaks as shown in Table 19 below.

NMR: ¹H NMR (400 MHz, DMSO-*d*₆) δ 13.65 (s, 1H), 9.16 (d, *J =* 2.0 Hz, 1H), 8.92 (s, 1H), 8.86 (d, *J =* 8.4 Hz, 2H), 8.19 (s, 1H), 7.92 (d, *J =* 7.2 Hz, 2H), 7.63 (s, 1H), 7.53 (s, 1H), 7.40 (dd, *J* = 8.4, 7.6 Hz, 2H), 4.03 (s, 3H), 3.61 (s, 3H), 2.63 (s, 3H), 2.26 (d, *J* = 1.6 Hz, 3H).

**Table 19**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 4.648 | 18.99788 | -0.90% | 11 | 18.606 | 4.76513 | 39.10% |
| 2 | 6.359 | 13.88777 | 51.00% | 12 | 20.236 | 4.38474 | 26.60% |
| 3 | 9.423 | 9.37769 | 16.00% | 13 | 20.944 | 4.23819 | 35.50% |
| 4 | 11.302 | 7.82283 | 98.80% | 14 | 22.606 | 3.93014 | 100.00% |
| 5 | 12.105 | 7.30558 | 26.70% | 15 | 24.693 | 3.60257 | 71.00% |
| 6 | 12.82 | 6.89996 | 55.10% | 16 | 26.159 | 3.40389 | 52.30% |
| 7 | 13.646 | 6.48392 | 15.50% | 17 | 28.569 | 3.12198 | 38.80% |
| 8 | 15.115 | 5.85673 | 30.30% | 18 | 31.642 | 2.82537 | 9.30% |
| 9 | 17.068 | 5.1909 | 40.10% | 19 | 32.751 | 2.73219 | 4.40% |
| 10 | 17.476 | 5.07047 | 37.10% | | | | |

### Example 18 Crystalline Form D of the compound of Formula X

400.23 mg of the compound of Formula I, 294.79 mg of 1,5-naphthalenedisulfonic acid, and 3 mg of Crystalline Form A of the 1,5-naphthalenedisulfonate salt of the compound of Formula I were added to 6.6 mL of acetone/water (95:5, v:v), and the temperature was raised to 50°C. The entire system was stirred at 50°C for 2 hours, then cooled to 25°C with stirring, filtered, and the obtained wet product was vacuum dried at 50°C for 2 hours to obtain Crystalline Form D of the compound of Formula X. The molar ratio of the compound of Formula I to 1,5-naphthalenedisulfonic acid is 1:1.02. The XRPD pattern of Crystalline Form D of the compound of Formula X is shown in FIG. 34, and it has diffraction peaks as shown in Table 20 below.

NMR: ¹H NMR (400 MHz, DMSO-*d*₆) δ 13.65 (s, 1H), 9.16 (d, *J =* 2.0 Hz, 1H), 8.92 (s, 1H), 8.86 (d, *J =* 8.4 Hz, 2H), 8.19 (s, 1H), 7.92 (d, *J =* 7.2 Hz, 2H), 7.63 (s, 1H), 7.53 (s, 1H), 7.40 (dd, *J* = 8.4, 7.6 Hz, 2H), 4.03 (s, 3H), 3.61 (s, 3H), 2.63 (s, 3H), 2.26 (d, *J* = 1.6 Hz, 3H).

**Table 20**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 7.269 | 12.15102 | 11.50% | 32 | 24.953 | 3.56557 | 30.20% |
| 2 | 7.872 | 11.22197 | 7.90% | 33 | 25.376 | 3.50703 | 1.20% |
| 3 | 9.251 | 9.55183 | 4.50% | 34 | 25.665 | 3.46825 | 1.60% |
| 4 | 9.566 | 9.23802 | 2.80% | 35 | 26.37 | 3.37708 | 2.40% |
| 5 | 10.138 | 8.71834 | 1.70% | 36 | 26.691 | 3.33723 | 9.50% |
| 6 | 10.682 | 8.27576 | 100.00% | 37 | 27.319 | 3.26192 | 24.90% |
| 7 | 11.491 | 7.69425 | 3.20% | 38 | 28.123 | 3.17044 | 6.50% |
| 8 | 12.029 | 7.35148 | 3.10% | 39 | 28.418 | 3.13815 | 2.00% |
| 9 | 13.333 | 6.63559 | 1.60% | 40 | 28.881 | 3.08892 | 20.30% |
| 10 | 13.593 | 6.50901 | 8.50% | 41 | 29.287 | 3.047 | 1.90% |
| 11 | 14.537 | 6.08826 | 6.30% | 42 | 29.544 | 3.02108 | 2.90% |
| 12 | 15.551 | 5.69372 | 8.20% | 43 | 29.954 | 2.98063 | 7.20% |
| 13 | 15.77 | 5.61503 | 24.60% | 44 | 30.343 | 2.94335 | 3.30% |
| 14 | 16.245 | 5.45179 | 1.30% | 45 | 30.626 | 2.9168 | 7.00% |
| 15 | 16.5 | 5.36836 | 7.40% | 46 | 31.161 | 2.86788 | 5.70% |
| 16 | 16.875 | 5.24981 | 20.10% | 47 | 31.818 | 2.81021 | 8.00% |
| 17 | 17.287 | 5.12542 | 4.30% | 48 | 31.993 | 2.79519 | 9.20% |
| 18 | 17.66 | 5.01827 | 10.70% | 49 | 32.37 | 2.76349 | 12.40% |
| 19 | 18.586 | 4.77007 | 1.60% | 50 | 32.703 | 2.73616 | 5.40% |
| 20 | 19.627 | 4.51945 | 7.10% | 51 | 33.037 | 2.70921 | 3.30% |
| 21 | 19.815 | 4.47691 | 26.00% | 52 | 33.33 | 2.68606 | 2.40% |
| 22 | 20.266 | 4.37825 | 11.50% | 53 | 34.29 | 2.61305 | 4.70% |
| 23 | 21.438 | 4.1416 | 73.20% | 54 | 34.759 | 2.57886 | 2.60% |
| 24 | 21.762 | 4.08063 | 21.90% | 55 | 35.072 | 2.55657 | 1.60% |
| 25 | 22.623 | 3.92722 | 10.80% | 56 | 36.503 | 2.45954 | 2.00% |
| 26 | 23.09 | 3.84893 | 9.80% | 57 | 37.339 | 2.40638 | 2.30% |
| 27 | 23.366 | 3.80395 | 8.90% | 58 | 37.585 | 2.39118 | 2.80% |
| 28 | 23.618 | 3.76395 | 32.00% | 59 | 37.892 | 2.37252 | 8.20% |
| 29 | 24.004 | 3.70429 | 6.80% | 60 | 38.562 | 2.33283 | 1.20% |
| 30 | 24.333 | 3.65496 | 11.80% | 61 | 38.815 | 2.3182 | 4.10% |
| 31 | 24.642 | 3.60992 | 11.70% | | | | |

The DSC profile of Crystalline Form D of the compound of Formula X is shown in FIG. 35. The DSC curve shows three endothermic peaks at peak temperatures of 62.55°C (enthalpy value 111.00 J/g), 86.97°C (enthalpy value 39.972 J/g), and 267.15°C (enthalpy value 55.716 J/g), with onset points (Onset x) at 39.64°C, 70.37°C, and 256.54°C, respectively. The TGA profile is shown in FIG. 36. The sample lost 4.49% of its weight when heated from 32.27°C to 60.0°C, lost 2.41% when heated from 60.0°C to 120.0°C, and lost 0.60% when heated from 120.0°C to 240.0°C. NMR data show that Crystalline Form D of the compound of Formula X contains only 0.1% acetone. The KF moisture test result shows a water content of 9.1%, indicating that Crystalline Form D contains water equivalent to 4.8 equivalents.

### Example 19 Crystalline Form A of the compound of Formula XI

30 mg of the compound of Formula I and 2.50 mg of sodium hydroxide were added to 0.5 mL of ethyl acetate, and the temperature was raised to 50°C. The entire system was stirred at 50°C for 2 hours, then cooled to 25°C with stirring, filtered, and the obtained wet product was vacuum dried at 50°C for 2 hours to obtain Crystalline Form A of the compound of Formula XI. The molar ratio of the compound of Formula I to sodium is 1:0.62. The XRPD pattern of Crystalline Form A of the compound of Formula XI is shown in FIG. 37, and it has diffraction peaks as shown in Table 21 below.

**Table 21**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 4.687 | 18.83695 | 1.80% | 19 | 23.031 | 3.85854 | 1.40% |
| 2 | 5.583 | 15.81629 | 2.20% | 20 | 23.577 | 3.77046 | 45.80% |
| 3 | 6.9 | 12.80028 | 4.30% | 21 | 24.148 | 3.68254 | 6.00% |
| 4 | 9.385 | 9.41639 | 5.20% | 22 | 26.476 | 3.36378 | 1.90% |
| 5 | 10.665 | 8.28879 | 37.80% | 23 | 27.172 | 3.27925 | 1.00% |
| 6 | 11.146 | 7.93194 | 3.10% | 24 | 27.926 | 3.19239 | 13.80% |
| 7 | 12.782 | 6.91996 | 95.20% | 25 | 28.848 | 3.09237 | 2.40% |
| 8 | 14.093 | 6.27919 | 100.00% | 26 | 30.214 | 2.95564 | 5.60% |
| 9 | 14.975 | 5.9112 | 76.20% | 27 | 31.248 | 2.86014 | 2.40% |
| 10 | 15.521 | 5.70471 | 2.40% | 28 | 32.47 | 2.75519 | 3.00% |
| 11 | 15.881 | 5.57597 | 0.90% | 29 | 33.25 | 2.69236 | 4.70% |
| 12 | 17.222 | 5.14467 | 9.30% | 30 | 35.119 | 2.55326 | 4.00% |
| 13 | 18.821 | 4.71122 | 27.70% | 31 | 36.156 | 2.48235 | 2.70% |
| 14 | 19.351 | 4.58332 | 9.80% | 32 | 36.698 | 2.44694 | 5.60% |
| 15 | 19.489 | 4.55115 | 16.80% | 33 | 37.176 | 2.41653 | 2.20% |
| 16 | 20.821 | 4.26281 | 1.70% | 34 | 38.159 | 2.35655 | 4.50% |
| 17 | 21.804 | 4.07295 | 15.90% | 35 | 39.026 | 2.30615 | 3.10% |
| 18 | 22.448 | 3.9575 | 7.40% | | | | |

### Example 20 Crystalline Form B of the compound of Formula XI

30 mg of the compound of Formula I and 2.50 mg of sodium hydroxide were added to 0.5 mL of acetonitrile, and the temperature was raised to 50°C. The entire system was stirred at 50°C for 2 hours, cooled to 25°C and stirred, filtered, and the resulting wet product was vacuum dried at 50°C for 2 hours to obtain Crystalline Form B of the compound of Formula XI. The molar ratio of the compound of Formula I to sodium was 1:1.13, and the molar ratio of the compound of Formula I to the solvent acetonitrile was 1:1.07. The XRPD pattern of Crystalline Form B of the compound of Formula XI is shown in FIG. 38, and it has the diffraction peaks shown in Table 22 below.

**Table 22**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 5.541 | 15.93667 | 3.50% | 23 | 22.743 | 3.90677 | 6.00% |
| 2 | 6.923 | 12.7585 | 5.20% | 24 | 23.048 | 3.85572 | 11.60% |
| 3 | 8.22 | 10.74816 | 77.00% | 25 | 23.433 | 3.79331 | 82.60% |
| 4 | 9.311 | 9.49097 | 44.20% | 26 | 24.167 | 3.67965 | 43.60% |
| 5 | 9.596 | 9.20947 | 13.00% | 27 | 24.736 | 3.59641 | 68.40% |
| 6 | 10.632 | 8.31437 | 8.80% | 28 | 25.913 | 3.43554 | 2.60% |
| 7 | 11.563 | 7.64664 | 11.30% | 29 | 26.456 | 3.36626 | 14.90% |
| 8 | 12.922 | 6.84547 | 46.00% | 30 | 26.863 | 3.31623 | 11.40% |
| 9 | 13.323 | 6.64052 | 31.20% | 31 | 27.373 | 3.25556 | 27.40% |
| 10 | 13.622 | 6.49544 | 7.90% | 32 | 29.056 | 3.07075 | 11.70% |
| 11 | 13.827 | 6.39957 | 15.60% | 33 | 30.037 | 2.97261 | 13.70% |
| 12 | 15.128 | 5.85168 | 18.50% | 34 | 30.266 | 2.95061 | 20.30% |
| 13 | 16.288 | 5.43764 | 18.70% | 35 | 30.853 | 2.89585 | 20.40% |
| 14 | 17.021 | 5.20501 | 8.70% | 36 | 31.404 | 2.84628 | 18.40% |
| 15 | 17.629 | 5.027 | 16.60% | 37 | 32.211 | 2.77675 | 12.00% |
| 16 | 17.773 | 4.98662 | 23.40% | 38 | 33.098 | 2.7044 | 10.30% |
| 17 | 18.124 | 4.89075 | 33.20% | 39 | 34.323 | 2.61062 | 15.80% |
| 18 | 18.632 | 4.7585 | 31.00% | 40 | 34.876 | 2.57048 | 11.10% |
| 19 | 19.378 | 4.57704 | 90.40% | 41 | 36.294 | 2.47322 | 2.20% |
| 20 | 20.377 | 4.35482 | 100.00% | 42 | 37.623 | 2.38884 | 5.40% |
| 21 | 21.035 | 4.22005 | 32.30% | 43 | 38.731 | 2.32303 | 9.20% |
| 22 | 21.278 | 4.17238 | 30.90% | 44 | 39.873 | 2.25907 | 5.30% |

### Example 21 Crystalline Form C of the compound of Formula IX

199.4 mg of Crystalline Form A of the p-toluenesulfonate salt of the compound of Formula I (Crystalline Form A of the compound of Formula IX) was added to 5.2 mL of tetrahydrofuran, stirred at 25°C, filtered, and vacuum dried at 50°C for 2 hours to obtain Crystalline Form C of the compound of Formula IX, which is a solvate of tetrahydrofuran. The molar ratio of the compound of Formula I to p-toluenesulfonic acid and tetrahydrofuran was 1:1.02:0.16. The XRPD pattern of Crystalline Form C of the compound of Formula IX is shown in FIG. 39, and it has the diffraction peaks shown in Table 23 below.

**Table 23**

| **Peak** | **Diffraction Angle (°, 2θ)** | **d-Spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 5.23 | 16.88487 | 100.00% | 24 | 19.385 | 4.57526 | 32.40% |
| 2 | 5.781 | 15.27577 | 5.00% | 25 | 19.82 | 4.47596 | 71.20% |
| 3 | 8.067 | 10.9514 | 10.80% | 26 | 20.007 | 4.43447 | 44.60% |
| 4 | 8.259 | 10.69689 | 15.40% | 27 | 20.87 | 4.25293 | 9.40% |
| 5 | 9.205 | 9.59966 | 38.30% | 28 | 21.518 | 4.12633 | 12.70% |
| 6 | 9.797 | 9.02074 | 15.20% | 29 | 21.885 | 4.05803 | 7.90% |
| 7 | 10.137 | 8.71938 | 8.20% | 30 | 22.265 | 3.98958 | 15.40% |
| 8 | 10.442 | 8.46535 | 6.60% | 31 | 22.76 | 3.90396 | 6.20% |
| 9 | 10.917 | 8.09813 | 8.20% | 32 | 23.512 | 3.78074 | 21.30% |
| 10 | 11.117 | 7.95271 | 14.80% | 33 | 23.829 | 3.73113 | 12.00% |
| 11 | 11.559 | 7.64976 | 4.40% | 34 | 24.282 | 3.66256 | 6.90% |
| 12 | 12.719 | 6.95419 | 24.10% | 35 | 24.532 | 3.6258 | 8.90% |
| 13 | 13.084 | 6.76127 | 6.90% | 36 | 25.261 | 3.52275 | 12.70% |
| 14 | 14.601 | 6.06167 | 4.30% | 37 | 25.498 | 3.49051 | 12.60% |
| 15 | 15.243 | 5.8078 | 17.70% | 38 | 26.44 | 3.36825 | 22.20% |
| 16 | 15.482 | 5.71887 | 14.20% | 39 | 26.718 | 3.33385 | 8.70% |
| 17 | 16.113 | 5.49622 | 35.50% | 40 | 27.335 | 3.26006 | 10.20% |
| 18 | 16.569 | 5.34603 | 40.00% | 41 | 28.317 | 3.14917 | 11.00% |
| 19 | 16.976 | 5.21884 | 17.70% | 42 | 29.731 | 3.00254 | 9.60% |
| 20 | 17.355 | 5.10576 | 6.40% | 43 | 30.06 | 2.97044 | 5.40% |
| 21 | 17.894 | 4.95308 | 11.00% | 44 | 31.485 | 2.8391 | 5.00% |
| 22 | 18.198 | 4.87091 | 19.60% | 45 | 32.604 | 2.74421 | 5.40% |
| 23 | 18.712 | 4.73836 | 15.10% | | | | |

### Example 22 Crystalline Form D of the compound of Formula IX

Crystalline Form A of the compound of Formula IX was heated under a nitrogen atmosphere at 55°C to obtain Crystalline Form D of the compound of Formula IX. The molar ratio of the compound of Formula I to p-toluenesulfonic acid was 1:1. The XRPD pattern of Crystalline Form D of the p-toluenesulfonate salt of the compound of Formula I (Crystalline Form D of the compound of Formula IX) is shown in FIG. 40, and it has the diffraction peaks shown in Table 24 below.

**Table 24**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 6.127 | 14.41406 | 100.00% | 27 | 23.136 | 3.84136 | 7.90% |
| 2 | 8.518 | 10.37289 | 16.50% | 28 | 23.997 | 3.70544 | 19.80% |
| 3 | 10.193 | 8.67124 | 4.60% | 29 | 24.266 | 3.66497 | 8.20% |
| 4 | 10.87 | 8.13289 | 27.30% | 30 | 25.276 | 3.52065 | 0.90% |
| 5 | 12.179 | 7.26166 | 22.40% | 31 | 25.551 | 3.4835 | 1.10% |
| 6 | 12.59 | 7.02527 | 3.10% | 32 | 25.788 | 3.45202 | 2.10% |
| 7 | 13.119 | 6.74327 | 19.70% | 33 | 26.012 | 3.42274 | 1.20% |
| 8 | 13.401 | 6.60209 | 1.40% | 34 | 26.47 | 3.36459 | 3.20% |
| 9 | 14.247 | 6.21148 | 14.00% | 35 | 26.93 | 3.30808 | 3.20% |
| 10 | 14.418 | 6.13826 | 10.90% | 36 | 27.442 | 3.24752 | 2.30% |
| 11 | 15.416 | 5.74307 | 10.40% | 37 | 27.867 | 3.19895 | 2.30% |
| 12 | 16.596 | 5.33741 | 24.70% | 38 | 28.331 | 3.14765 | 12.30% |
| 13 | 17.002 | 5.21068 | 5.80% | 39 | 28.816 | 3.09575 | 5.10% |
| 14 | 17.574 | 5.04238 | 1.60% | 40 | 29.353 | 3.04029 | 3.00% |
| 15 | 17.839 | 4.96809 | 3.60% | 41 | 29.783 | 2.99737 | 2.70% |
| 16 | 18.376 | 4.82432 | 33.50% | 42 | 30.728 | 2.90733 | 1.80% |
| 17 | 19.089 | 4.64558 | 11.90% | 43 | 31.199 | 2.86452 | 2.10% |
| 18 | 19.342 | 4.58548 | 13.10% | 44 | 31.956 | 2.79836 | 1.30% |
| 19 | 19.638 | 4.51695 | 3.30% | 45 | 32.518 | 2.75125 | 2.10% |
| 20 | 20.069 | 4.42091 | 20.90% | 46 | 34.492 | 2.59823 | 0.80% |
| 21 | 20.391 | 4.35189 | 3.10% | 47 | 35.57 | 2.52192 | 0.20% |
| 22 | 21.123 | 4.20265 | 8.40% | 48 | 36 | 2.49272 | 1.70% |
| 23 | 21.28 | 4.17204 | 8.20% | 49 | 37.011 | 2.42697 | 0.90% |
| 24 | 21.589 | 4.11296 | 10.60% | 50 | 37.645 | 2.38754 | 1.40% |
| 25 | 21.72 | 4.08848 | 8.50% | 51 | 38.269 | 2.35 | 2.00% |
| 26 | 22.095 | 4.01988 | 2.30% | 52 | 39.074 | 2.30341 | 1.30% |

### Example 23 Crystalline Form E of the compound of Formula IX

40 mg of Crystalline Form A of the compound of Formula IX was added to 0.2 mL of isopropanol, heated to 50°C with shaking, filtered, and vacuum dried at 50°C for 2 hours to obtain Crystalline Form E of the isopropanol solvate of the p-toluenesulfonate salt of the compound of Formula I (Crystalline Form E of the compound of Formula IX). Its 1H NMR spectrum showed that this crystalline form contained 0.98 equivalents of isopropanol. In Crystalline Form E of the compound of Formula IX, the molar ratio of the compound of Formula I, p-toluenesulfonic acid, and isopropanol was 1:1.02:0.98. The XRPD pattern of Crystalline Form E of the compound of Formula IX is shown in FIG. 41, and it has the diffraction peaks shown in Table 25 below.

NMR: ¹H NMR (400 MHz, DMSO-*d*₆) δ 13.65 (s, 1H), 9.15 (d, *J* = 2.0 Hz, 1H), 8.92 (s, 1H), 8.19 (s, 1H), 7.63 (s, 1H), 7.54 (s, 1H), 7.47 (d, *J* = 8.4 Hz, 2H), 7.11 (d, *J =* 7.6 Hz, 2H), 4.03 (s, 3H), 3.78 (sept, *J* = 7.0 Hz, 1H), 3.61 (s, 3H), 2.63 (s, 3H), 2.29 (s, 3H), 2.26 (d, *J* = 1.6 Hz, 3H), 1.09 (d, *J* = 7.0 Hz, 6H).

**Table 25**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 5.105 | 17.29659 | 1.20% | 34 | 23.946 | 3.7132 | 89.20% |
| 2 | 5.656 | 15.61287 | 78.90% | 35 | 24.461 | 3.63618 | 8.70% |
| 3 | 8.105 | 10.89934 | 4.90% | 36 | 25.046 | 3.55248 | 20.10% |
| 4 | 8.451 | 10.45407 | 14.30% | 37 | 25.494 | 3.4911 | 9.20% |
| 5 | 9.022 | 9.79358 | 22.30% | 38 | 25.85 | 3.44377 | 14.10% |
| 6 | 9.839 | 8.98291 | 12.30% | 39 | 26.135 | 3.40691 | 11.30% |
| 7 | 11.192 | 7.89963 | 25.00% | 40 | 26.582 | 3.35059 | 76.50% |
| 8 | 11.501 | 7.68798 | 65.80% | 41 | 26.981 | 3.30196 | 25.20% |
| 9 | 12.286 | 7.19858 | 7.80% | 42 | 27.858 | 3.19999 | 35.60% |
| 10 | 12.704 | 6.96221 | 13.30% | 43 | 28.054 | 3.17809 | 27.00% |
| 11 | 13.853 | 6.38731 | 36.70% | 44 | 28.344 | 3.14618 | 4.30% |
| 12 | 14.296 | 6.1907 | 41.20% | 45 | 28.859 | 3.09124 | 29.50% |
| 13 | 15.359 | 5.76451 | 5.00% | 46 | 29.053 | 3.07103 | 5.60% |
| 14 | 15.586 | 5.68079 | 14.80% | 47 | 29.717 | 3.00391 | 13.70% |
| 15 | 16.204 | 5.46575 | 8.60% | 48 | 30.36 | 2.94177 | 17.10% |
| 16 | 16.56 | 5.349 | 23.50% | 49 | 30.578 | 2.92124 | 32.90% |
| 17 | 16.925 | 5.23427 | 6.90% | 50 | 30.951 | 2.88692 | 6.40% |
| 18 | 17.18 | 5.15736 | 18.90% | 51 | 31.536 | 2.83466 | 6.50% |
| 19 | 17.741 | 4.99534 | 68.20% | 52 | 31.738 | 2.81713 | 5.10% |
| 20 | 17.974 | 4.93107 | 92.90% | 53 | 32.454 | 2.75658 | 6.10% |
| 21 | 18.754 | 4.72791 | 22.60% | 54 | 32.801 | 2.72817 | 2.10% |
| 22 | 19.043 | 4.65667 | 64.50% | 55 | 33.226 | 2.69424 | 5.10% |
| 23 | 19.341 | 4.58571 | 17.70% | 56 | 33.693 | 2.65797 | 4.10% |
| 24 | 19.516 | 4.54484 | 18.90% | 57 | 34.651 | 2.58661 | 1.80% |
| 25 | 19.834 | 4.47269 | 100.00% | 58 | 35.358 | 2.53655 | 3.40% |
| 26 | 20.549 | 4.31873 | 12.80% | 59 | 36.121 | 2.48464 | 3.30% |
| 27 | 21.069 | 4.21326 | 3.60% | 60 | 36.545 | 2.45681 | 8.60% |
| 28 | 21.373 | 4.1541 | 5.60% | 61 | 37.162 | 2.41742 | 3.80% |
| 29 | 21.934 | 4.049 | 47.00% | 62 | 37.521 | 2.39514 | 7.80% |
| 30 | 22.246 | 3.99292 | 58.60% | 63 | 38.009 | 2.36545 | 11.40% |
| 31 | 22.467 | 3.95407 | 15.70% | 64 | 38.724 | 2.32343 | 6.20% |
| 32 | 23.082 | 3.8502 | 40.90% | 65 | 39.556 | 2.27643 | 2.40% |
| 33 | 23.431 | 3.79359 | 53.10% | | | | |

The DSC profile of Crystalline Form E of the compound of Formula IX is shown in FIG. 42. The DSC curve shows two endothermic peaks at peak temperatures of 102.94°C (enthalpy value 207.35 J/g) and 183.71°C (enthalpy value 34.855 J/g), with onset points (Onset x) at 65.61°C and 176.33°C, respectively. The TGA profile is shown in FIG. 43. The sample showed a weight loss of 3.11% upon heating from 33.06°C to 55.0°C, a weight loss of 3.82% upon heating from 55.0°C to 120.00°C, and a weight loss of 1.21% upon heating from 120.0°C to 190.0°C.

### Example 24 Crystalline Form F of the compound of Formula IX

40 mg of Crystalline Form A of the compound of Formula IX was added to 1.6 mL of acetonitrile and dissolved with filtration. The filtrate was maintained at 25°C, and 3.2 mL of n-heptane was added to the filtrate. Solids precipitated from the system, which were filtered and vacuum dried at 50°C for 2 hours to obtain Crystalline Form F of the compound of Formula IX. The molar ratio of the compound of Formula I to p-toluenesulfonic acid was 1:1.02. The XRPD pattern of Crystalline Form F of the compound of Formula IX is shown in FIG. 44, and it has the diffraction peaks shown in Table 26 below.

¹H NMR (400 MHz, DMSO-*d*₆) δ 13.64 (s, 1H), 9.15 (d, *J =* 2.0 Hz, 1H), 8.91 (s, 1H), 8.19 (s, 1H), 7.63 (s, 1H), 7.53 (s, 1H), 7.47 (d, *J* = 8.4 Hz, 2H), 7.12 (d, *J* = 7.6 Hz, 2H), 4.02 (s, 3H), 3.61 (s, 3H), 2.63 (s, 3H), 2.29 (s, 3H), 2.26 (d, *J =* 1.6 Hz, 3H).

**Table 26**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 6.146 | 14.36817 | 55.40% | 39 | 23.461 | 3.7888 | 3.70% |
| 2 | 6.985 | 12.6444 | 9.10% | 40 | 24.029 | 3.70048 | 2.60% |
| 3 | 9.906 | 8.92193 | 0.40% | 41 | 24.342 | 3.65369 | 60.90% |
| 4 | 10.338 | 8.55009 | 26.50% | 42 | 24.635 | 3.6108 | 3.60% |
| 5 | 10.747 | 8.22588 | 0.80% | 43 | 25.251 | 3.52415 | 10.30% |
| 6 | 11.517 | 7.67738 | 4.60% | 44 | 25.738 | 3.45859 | 45.90% |
| 7 | 11.849 | 7.46291 | 12.00% | 45 | 26.024 | 3.42116 | 20.90% |
| 8 | 12.265 | 7.21058 | 8.20% | 46 | 26.651 | 3.34211 | 6.20% |
| 9 | 12.57 | 7.03612 | 8.70% | 47 | 26.827 | 3.32061 | 26.20% |
| 10 | 12.727 | 6.94986 | 1.30% | 48 | 27.268 | 3.26786 | 6.30% |
| 11 | 13.351 | 6.62658 | 5.50% | 49 | 27.871 | 3.19849 | 7.20% |
| 12 | 13.965 | 6.33642 | 15.30% | 50 | 28.126 | 3.17008 | 9.90% |
| 13 | 14.137 | 6.25979 | 2.80% | 51 | 28.463 | 3.13334 | 13.30% |
| 14 | 15.519 | 5.70521 | 4.30% | 52 | 28.743 | 3.10342 | 1.30% |
| 15 | 15.732 | 5.62843 | 24.80% | 53 | 29.705 | 3.00513 | 7.40% |
| 16 | 16.335 | 5.42215 | 26.30% | 54 | 29.989 | 2.97725 | 2.10% |
| 17 | 16.549 | 5.35248 | 67.90% | 55 | 30.185 | 2.95844 | 1.50% |
| 18 | 16.727 | 5.29583 | 5.50% | 56 | 30.524 | 2.9263 | 1.60% |
| 19 | 17.083 | 5.1862 | 5.00% | 57 | 30.716 | 2.90842 | 4.30% |
| 20 | 17.303 | 5.12084 | 86.30% | 58 | 31.277 | 2.85758 | 18.20% |
| 21 | 17.726 | 4.99973 | 0.90% | 59 | 31.732 | 2.81761 | 4.60% |
| 22 | 18.067 | 4.90588 | 1.30% | 60 | 32.331 | 2.76673 | 1.90% |
| 23 | 18.423 | 4.81198 | 17.40% | 61 | 32.696 | 2.73667 | 4.00% |
| 24 | 18.917 | 4.68734 | 7.80% | 62 | 32.983 | 2.71352 | 4.30% |
| 25 | 19.365 | 4.58003 | 0.70% | 63 | 33.237 | 2.69335 | 8.80% |
| 26 | 19.824 | 4.47498 | 0.50% | 64 | 33.376 | 2.6825 | 9.50% |
| 27 | 20.419 | 4.34589 | 41.00% | 65 | 33.68 | 2.65897 | 7.50% |
| 28 | 20.72 | 4.28351 | 39.90% | 66 | 34.419 | 2.60351 | 8.60% |
| 29 | 20.98 | 4.23092 | 23.30% | 67 | 35.046 | 2.55837 | 7.20% |
| 30 | 21.217 | 4.18425 | 26.20% | 68 | 35.674 | 2.51474 | 6.40% |
| 31 | 21.479 | 4.13372 | 100.00% | 69 | 36.241 | 2.47671 | 1.10% |
| 32 | 21.825 | 4.06909 | 19.90% | 70 | 36.574 | 2.45491 | 1.80% |
| 33 | 21.972 | 4.04201 | 18.40% | 71 | 36.884 | 2.43503 | 1.10% |
| 34 | 22.242 | 3.99369 | 8.20% | 72 | 37.361 | 2.40501 | 6.30% |
| 35 | 22.433 | 3.96011 | 6.30% | 73 | 38.144 | 2.3574 | 3.10% |
| 36 | 22.689 | 3.91601 | 1.80% | 74 | 38.48 | 2.33761 | 6.40% |
| 37 | 22.973 | 3.86827 | 8.10% | 75 | 39.118 | 2.30093 | 1.70% |
| 38 | 23.269 | 3.81961 | 4.20% | | | | |

The DSC profile of Crystalline Form F of the compound of Formula IX is shown in FIG. 45. The DSC curve shows an endothermic peak at a peak temperature of 266.84°C (enthalpy value 70.167 J/g), with an onset point (Onset x) of 263.55°C. The TGA profile is shown in FIG. 46. The sample showed a weight loss of 2.2% upon heating from 34.19°C to 260.0°C.

### Example 25 Crystalline Form G of the compound of Formula IX

40 mg of Crystalline Form A of the compound of Formula IX was added to 0.2 mL of 1,4-dioxane, heated to 50°C with shaking, filtered, and vacuum dried at 50°C for 2 hours to obtain Crystalline Form G of the compound of Formula IX. NMR analysis indicated that this crystalline form contained 3 equivalents of dioxane. The molar ratio of the compound of Formula I, p-toluenesulfonic acid, and 1,4-dioxane was 1:1.10:2.96. The XRPD pattern of Crystalline Form G of the compound of Formula IX is shown in FIG. 47, and it has the diffraction peaks shown in Table 27 below.

NMR: ¹H NMR (400 MHz, DMSO-*d*₆) δ 13.65 (s, 1H), 9.15 (d, *J* = 2.0 Hz, 1H), 8.92 (s, 1H), 8.19 (s, 1H), 7.63 (s, 1H), 7.54 (s, 1H), 7.47 (d, *J =* 8.4 Hz, 2H), 7.11 (d, *J =* 7.6 Hz, 2H), 4.03 (s, 3H), 3.57 (s, 24H), 3.61 (s, 3H), 2.63 (s, 3H), 2.29 (s, 3H), 2.26 (d, *J =* 1.6 Hz, 3H).

**Table 27**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 7.646 | 11.55367 | 25.60% | 26 | 23.096 | 3.8478 | 7.00% |
| 2 | 9.655 | 9.15311 | 3.90% | 27 | 23.513 | 3.78055 | 6.80% |
| 3 | 10.734 | 8.23557 | 1.20% | 28 | 23.827 | 3.73146 | 23.40% |
| 4 | 11.096 | 7.96733 | 1.80% | 29 | 24.468 | 3.63515 | 3.10% |
| 5 | 11.868 | 7.45081 | 7.90% | 30 | 25.372 | 3.50758 | 100.00% |
| 6 | 12.603 | 7.01819 | 8.50% | 31 | 25.632 | 3.47261 | 35.80% |
| 7 | 12.759 | 6.93236 | 8.00% | 32 | 26.652 | 3.34203 | 12.20% |
| 8 | 13.756 | 6.43239 | 28.70% | 33 | 27.17 | 3.2794 | 8.70% |
| 9 | 14.113 | 6.27053 | 5.20% | 34 | 27.595 | 3.22992 | 51.60% |
| 10 | 16.072 | 5.5101 | 13.10% | 35 | 28.363 | 3.14416 | 8.50% |
| 11 | 17.036 | 5.2006 | 3.70% | 36 | 29.174 | 3.05861 | 15.80% |
| 12 | 17.432 | 5.08315 | 5.10% | 37 | 29.887 | 2.98725 | 2.90% |
| 13 | 17.641 | 5.02357 | 11.10% | 38 | 31.461 | 2.84122 | 4.60% |
| 14 | 17.82 | 4.97352 | 8.60% | 39 | 31.788 | 2.8128 | 9.50% |
| 15 | 18.409 | 4.8157 | 8.90% | 40 | 32.382 | 2.76247 | 2.80% |
| 16 | 18.753 | 4.72799 | 6.10% | 41 | 33.264 | 2.69128 | 6.70% |
| 17 | 18.969 | 4.67472 | 9.60% | 42 | 33.57 | 2.6674 | 4.70% |
| 18 | 19.41 | 4.56954 | 2.20% | 43 | 33.857 | 2.64545 | 3.80% |
| 19 | 20.052 | 4.42453 | 12.10% | 44 | 34.249 | 2.61606 | 6.50% |
| 20 | 20.348 | 4.36084 | 40.00% | 45 | 34.781 | 2.57725 | 2.50% |
| 21 | 21.009 | 4.22519 | 49.00% | 46 | 36.21 | 2.47876 | 2.60% |
| 22 | 21.306 | 4.16701 | 10.50% | 47 | 37.185 | 2.41595 | 1.70% |
| 23 | 21.568 | 4.11689 | 63.70% | 48 | 38.068 | 2.36195 | 4.70% |
| 24 | 22.232 | 3.99544 | 2.10% | 49 | 38.407 | 2.34188 | 3.40% |
| 25 | 22.748 | 3.90587 | 15.20% | 50 | 38.739 | 2.32259 | 1.90% |

### Example 26 Crystalline Form H of the compound of Formula IX

40 mg of Crystalline Form A of the compound of Formula IX was added to 3.0 mL of acetonitrile and dissolved with filtration. 12 mL of methyl tert-butyl ether was added to the filtrate, resulting in precipitation of a solid, which was filtered to obtain Crystalline Form H of the compound of Formula IX. NMR analysis indicated it contained 0.45 equivalents of methyl tert-butyl ether. The molar ratio of the compound of Formula I, p-toluenesulfonic acid, and the solvent methyl tert-butyl ether was 1:1.00:0.5. The XRPD pattern of Crystalline Form H of the compound of Formula IX is shown in FIG. 48, and it has the diffraction peaks shown in Table 28 below.

NMR: ¹H NMR (400 MHz, DMSO-*d*₆) δ 13.65 (s, 1H), 9.15 (d, *J* = 2.0 Hz, 1H), 8.92 (s, 1H), 8.19 (s, 1H), 7.63 (s, 1H), 7.54 (s, 1H), 7.47 (d, *J* = 8.4 Hz, 2H), 7.11 (d, *J* = 7.6 Hz, 2H), 4.03 (s, 3H), 3.61 (s, 3H), 3.08 (s, 1.5H) 2.63 (s, 3H), 2.29 (s, 3H), 2.26 (d, *J* = 1.6 Hz, 3H), 1.11 (s, 4.5H).

**Table 28**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 5.207 | 16.95771 | 14.10% | 24 | 22.437 | 3.95941 | 11.80% |
| 2 | 8.269 | 10.6847 | 27.00% | 25 | 23.355 | 3.80574 | 16.20% |
| 3 | 9.701 | 9.11034 | 29.10% | 26 | 24.458 | 3.63664 | 4.60% |
| 4 | 10.392 | 8.5059 | 9.80% | 27 | 24.922 | 3.56991 | 4.80% |
| 5 | 11.07 | 7.98592 | 17.20% | 28 | 25.232 | 3.52673 | 30.70% |
| 6 | 11.597 | 7.62467 | 11.30% | 29 | 26.139 | 3.40641 | 26.00% |
| 7 | 12.557 | 7.04336 | 18.00% | 30 | 26.334 | 3.38168 | 19.10% |
| 8 | 13.05 | 6.77887 | 4.30% | 31 | 27.268 | 3.26793 | 16.00% |
| 9 | 14.964 | 5.91575 | 4.00% | 32 | 27.951 | 3.18958 | 16.80% |
| 10 | 15.286 | 5.79185 | 10.80% | 33 | 28.311 | 3.14978 | 7.50% |
| 11 | 16.554 | 5.35087 | 43.40% | 34 | 29.557 | 3.01977 | 23.20% |
| 12 | 17.072 | 5.18967 | 23.20% | 35 | 30.05 | 2.97142 | 10.70% |
| 13 | 17.463 | 5.07429 | 2.80% | 36 | 30.383 | 2.93961 | 10.10% |
| 14 | 18.079 | 4.90283 | 2.20% | 37 | 30.874 | 2.89397 | 9.50% |
| 15 | 18.485 | 4.79597 | 11.50% | 38 | 31.272 | 2.85799 | 4.00% |
| 16 | 18.793 | 4.71809 | 19.70% | 39 | 32.212 | 2.77672 | 12.70% |
| 17 | 19.087 | 4.64603 | 29.50% | 40 | 33.403 | 2.68034 | 1.80% |
| 18 | 19.784 | 4.48383 | 100.00% | 41 | 35.024 | 2.55994 | 2.10% |
| 19 | 20.812 | 4.26473 | 9.10% | 42 | 36.282 | 2.474 | 3.00% |
| 20 | 21.118 | 4.20364 | 15.80% | 43 | 37.312 | 2.40804 | 2.10% |
| 21 | 21.463 | 4.13686 | 23.00% | 44 | 38.273 | 2.3498 | 3.70% |
| 22 | 21.926 | 4.05052 | 3.50% | 45 | 38.916 | 2.31242 | 2.80% |
| 23 | 22.237 | 3.99455 | 7.90% | | | | |

The DSC pattern of Crystalline Form H of the compound of Formula IX is shown in FIG. 49. The DSC curve shows endothermic peaks at peak temperatures of 45.53°C (enthalpy value 10.478 J/g), 155.23°C (enthalpy value 29.331 J/g), and 191.45°C (enthalpy value 23.665 J/g), with onset points (Onset x) at 29.17°C, 134.74°C, and 184.26°C, respectively. The TGA pattern is shown in FIG. 50. The sample showed a weight loss of 1.74% when heated from 32.99°C to 100.0°C, and a weight loss of 5.38% when heated from 100.0°C to 180.0°C.

### Example 27 Crystalline Form I of the compound of Formula IX

Crystalline Form A of the compound of Formula IX was heated under a nitrogen atmosphere at 25°C to obtain Crystalline Form I of the compound of Formula IX. The molar ratio of the compound of Formula I to p-toluenesulfonic acid was 1:1. The XRPD pattern of Crystalline Form I of the compound of Formula I is shown in FIG. 51, and it has the diffraction peaks shown in Table 29 below.

**Table 29**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 6.01 | 14.69474 | 100.00% | 22 | 22.213 | 3.99885 | 2.80% |
| 2 | 8.343 | 10.5898 | 2.70% | 23 | 22.458 | 3.95579 | 7.20% |
| 3 | 10.023 | 8.81831 | 4.60% | 24 | 22.676 | 3.91814 | 3.60% |
| 4 | 10.63 | 8.31585 | 16.60% | 25 | 23.219 | 3.82777 | 14.60% |
| 5 | 11.082 | 7.97786 | 3.70% | 26 | 24.06 | 3.69589 | 10.00% |
| 6 | 11.991 | 7.37471 | 7.00% | 27 | 24.725 | 3.59791 | 2.20% |
| 7 | 12.4 | 7.13247 | 5.80% | 28 | 25.351 | 3.51041 | 5.30% |
| 8 | 12.792 | 6.91459 | 18.40% | 29 | 25.658 | 3.46913 | 3.90% |
| 9 | 14.204 | 6.23046 | 14.50% | 30 | 26.084 | 3.41342 | 4.80% |
| 10 | 15.035 | 5.88764 | 6.30% | 31 | 26.374 | 3.37659 | 3.40% |
| 11 | 16.674 | 5.31265 | 26.20% | 32 | 27.684 | 3.2197 | 5.90% |
| 12 | 17.842 | 4.96727 | 17.20% | 33 | 28.076 | 3.17566 | 6.60% |
| 13 | 17.999 | 4.92439 | 19.30% | 34 | 28.81 | 3.09642 | 2.00% |
| 14 | 18.468 | 4.80044 | 3.40% | 35 | 29.18 | 3.05794 | 4.90% |
| 15 | 18.743 | 4.73046 | 8.60% | 36 | 30.919 | 2.88983 | 2.90% |
| 16 | 19.542 | 4.53886 | 1.30% | 37 | 31.837 | 2.80853 | 1.40% |
| 17 | 20.069 | 4.42085 | 11.90% | 38 | 32.985 | 2.71338 | 1.00% |
| 18 | 20.069 | 4.42085 | 11.90% | 39 | 33.718 | 2.65607 | 1.50% |
| 19 | 20.418 | 4.34616 | 1.30% | 40 | 37.941 | 2.36955 | 2.80% |
| 20 | 20.9 | 4.2469 | 3.50% | 41 | 39.251 | 2.29347 | 1.80% |
| 21 | 21.201 | 4.18728 | 4.40% | | | | |

### Example 28 Crystalline Form J of the compound of Formula IX

Crystalline Form H of the compound of Formula IX was heated under a nitrogen atmosphere at 170°C. After solvent removal, Crystalline Form J of the compound of Formula IX was obtained. The molar ratio of the compound of Formula I to p-toluenesulfonic acid was 1:1.01. The XRPD pattern of Crystalline Form J of the compound of Formula IX is shown in FIG. 52, and it has the diffraction peaks shown in Table 30 below.

**Table 30**

| **Peak** | **Diffraction Angle (°, 2θ)** | **d-spacing (Å)** | **Relative Intensity** | **Peak** | **Diffraction Angle (°, 2θ)** | **d-spacing (Å)** | **Relative Intensity** |
|---|---|---|---|---|---|---|---|
| 1 | 5.272 | 16.74922 | 57.30% | 13 | 19.073 | 4.64948 | 35.50% |
| 2 | 8.181 | 10.79936 | 50.00% | 14 | 19.655 | 4.51309 | 35.70% |
| 3 | 9.719 | 9.09269 | 40.00% | 15 | 20.205 | 4.3915 | 62.00% |
| 4 | 10.542 | 8.38506 | 39.80% | 16 | 21.25 | 4.17776 | 27.60% |
| 5 | 11.063 | 7.99099 | 100.00% | 17 | 22.327 | 3.97857 | 2.00% |
| 6 | 11.681 | 7.56951 | 12.90% | 18 | 23.074 | 3.85153 | 20.40% |
| 7 | 12.442 | 7.10847 | 84.30% | 19 | 25.084 | 3.54725 | 0.50% |
| 8 | 12.876 | 6.8697 | 36.10% | 20 | 26.478 | 3.36356 | 35.00% |
| 9 | 15.295 | 5.7885 | 32.70% | 21 | 27.524 | 3.23811 | 7.20% |
| 10 | 16.408 | 5.39808 | 32.30% | 22 | 28.157 | 3.16666 | 20.90% |
| 11 | 17.765 | 4.98876 | 8.00% | 23 | 29.266 | 3.04914 | 20.40% |
| 12 | 18.786 | 4.71982 | 95.30% | | | | |

### Test Example 1

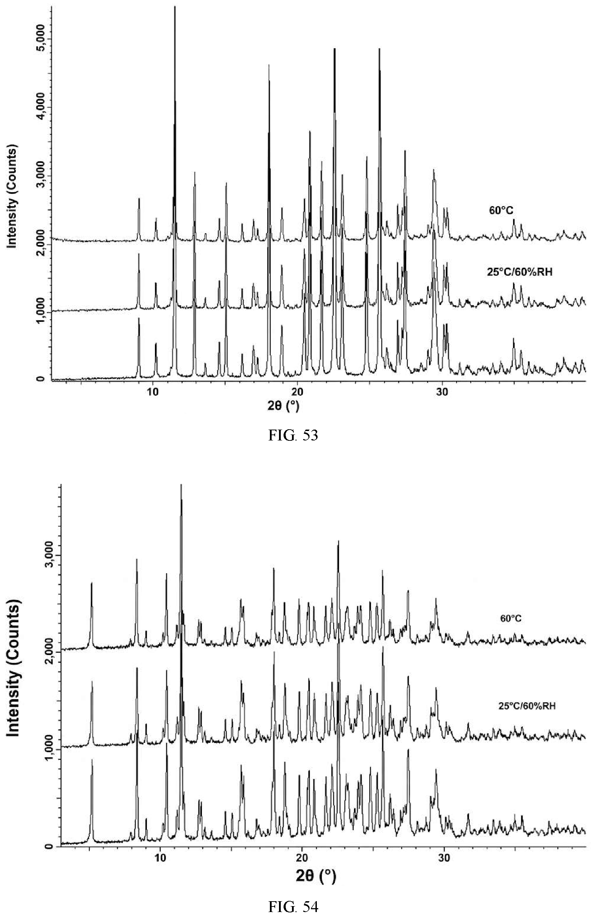

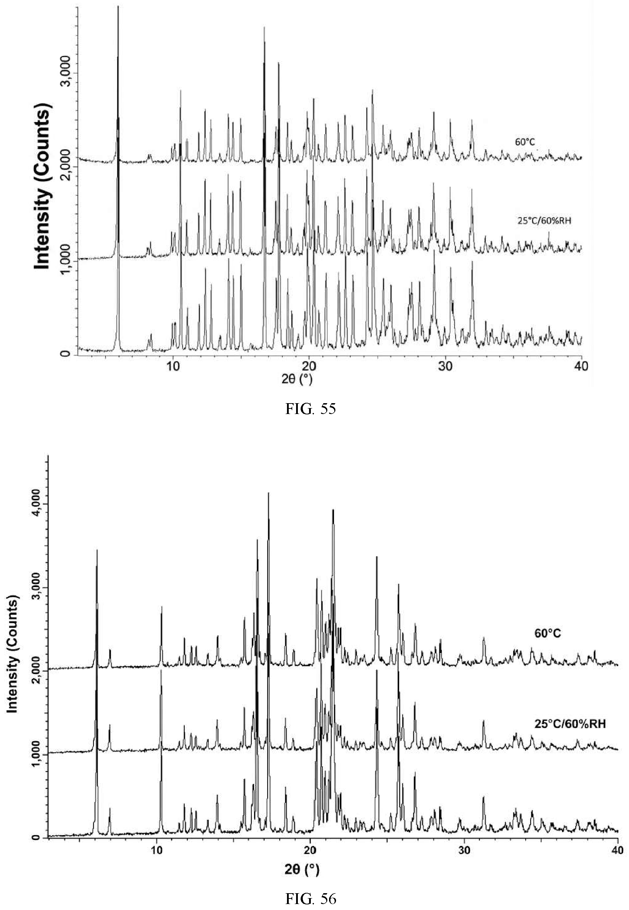

The crystalline form C of the compound of formula I obtained in Example 1-2, the crystalline form C of the compound of formula V obtained in Example 9, the crystalline form A of the compound of formula IX obtained in Example 13, and the crystalline form F of the compound of formula IX obtained in Example 24 were placed under open conditions at 25°C/60% RH and sealed conditions at 60°C for two weeks. As shown in FIG. 53, FIG. 54, FIG. 55, and FIG. 56, the crystalline forms exhibited good stability.

### Test Example 2

Test method: A gradient of 40-0-95-0-40% RH was set, with dm/dt 0.002/s. The system was equilibrated for 60~360 min for every 10% RH change. The test temperature was 25°C.

Test results showed that the crystalline form C of the compound of formula I was non-hygroscopic, and the crystalline form remained unchanged before and after the test. The crystalline form A of the compound of formula III exhibited moderate hygroscopicity, absorbing 4.8% water. The crystalline form A of the compound of formula IX absorbed 0.2% water, and the crystalline form C of the compound of formula V absorbed 1.7% water.

### Test Example 3

The crystalline form C of the compound of formula I, the crystalline form A of the compound of formula III, the crystalline form C of the compound of formula V, and the crystalline form A of the compound of formula IX obtained in the above examples were subjected to solubility testing. The test method was as follows:
The crystalline form C of the compound of formula I (8 mg), the crystalline form A of the compound of formula III (9 mg), the crystalline form A of the compound of formula IX (11.7 mg), and the crystalline form C of the compound of formula V (10 mg) were weighed into 20 mL glass vials, respectively. 4 mL of medium was added to each. The resulting suspensions or clear solutions were stirred at 37°C at 400 rpm. Samples (0.5 mL each) were taken at 0.5 hours and 2 hours, and centrifuged at 37°C at 14,000 rpm for 5 min. The resulting filtrates were finally subjected to content testing.

The results showed that the crystalline forms obtained in the present application had good solubility, as shown in the table below:
A indicates solubility of 0.01-0.1 mg/mL, B indicates solubility of 0.1-1 mg/mL.

| | Physical Form | | | | | | | |
|---|---|---|---|---|---|---|---|---|
| | Crystalline form C of the compound of formula I | | Crystalline form A of the compound of formula III | | Crystalline form C of the compound of formula V | | Crystalline form A of the compound of formula IX | |
| | Solubility (mg/mL) | | | | | | | |
| Dissolution Time | 0.5h | 2h | 0.5h | 2h | 0.5h | 2h | 0.5h | 2h |
| Water | A | A | A | A | A | A | A | A |
| Fasted State Simulated Gastric Fluid | A | A | B | B | B | B | B | B |
| Fasted State Simulated Intestinal Fluid | A | A | B | A | B | B | B | B |
| Fed State Simulated Intestinal Fluid | A | A | B | B | B | B | B | B |

### Rat PK Assay

The purpose of this experiment was to study the pharmacokinetics of the test compounds administered orally to SD rats (SPF grade, Beijing Vital River Laboratory Animal Technology Co., Ltd.). The experimental procedure was as follows:
1) The above compound crystalline forms were uniformly dispersed in a mixture of 99.9% (0.5% MC (450 cp)/water) + 0.1% Tween 80 at a concentration of 5.0 mg/mL, and then administered orally (10 mL/kg);
2) Blood samples were collected at 0.083, 0.25, 0.5, 1, 2, 4, 6, 8, and 24 hours, with 0.25 mL collected each time, and EDTA-K2 was added. After collection, the blood samples were placed on ice and centrifuged within 1 hour to separate plasma (centrifugation conditions: 6000 g, 3 minutes, 2-8°C). Plasma samples were stored in a -80°C freezer prior to analysis;
3) For sample analysis, the concentration of the compound in the plasma samples was analyzed using an LC-MS/MS method. For the plasma assay results, pharmacokinetic parameters were calculated using WinNonlin (Phoenix, version 8.2.0) or other similar software.

The results showed that the crystalline forms of the present application exhibited high exposure and favorable metabolic performance, as shown in the table below:

| Physical Form | Dose | Tₘₐₓ (h) | Cₘₐₓ (ng/mL) | AUC₍₀₋ₜ₎ (h*ng/mL) |
|---|---|---|---|---|
| Crystalline Form A of the compound of Formula II | 50mg/kg | 10.7 | 543 | 5997 |
| Crystalline Form A of the compound of Formula IX | 50mg/kg | 0.833 | 97800 | 743182 |

### Test Example 4

### 1. Biochemical Activity Assay for Pol θ Inhibition (ADP Glo Assay)

The N-terminus of the Pol θ protein contains a helicase domain with ATPase activity, capable of hydrolyzing ATP to ADP. The ATPase activity of the Pol θ protein and the inhibitory effect of small molecule compounds on the protein can be analyzed using the ADP-Glo detection kit (ADP-Glo^{™} Kinase Assay, Promega, V9102). The specific assay method is as follows:
1) The helicase domain of the Pol θ protein was purified using an insect system to obtain protein with purity >90%. ssDNA (SEQ ID NO.1: 5'-CCAGTGAATTGTTGCTCGGTACCTGCTAAC-3', Hangzhou Yocon Biotechnology Co., Ltd.) was ordered as the substrate for the Pol θ protein; a reaction buffer containing 10 mM DTT (Dithiothreitol), 20 mM MgCl₂, and Tris-HCl pH 7.5 was prepared;
2) A 2x ssDNA-Pol θ premix was prepared, and the reaction was carried out in a 384-well plate. A control group containing only buffer was set up, and the premix was added to all other groups. Compounds were added using an automated pipettor (Thermo, Multidrop 8), starting at a concentration of 10 µM and diluted at a 1/3 ratio, with a total of 9 detection points set, each with 2 replicates;
3) A 2x ATP solution was prepared, and an equal volume of the 2x ATP solution was added to all wells, followed by incubation at room temperature for 60 min;
4) According to the Promega reagent instructions, the ADP-Glo Detection Reagent was added to the reaction system, followed by incubation at room temperature for 60 min;
5) According to the Promega reagent instructions, the Kinase Detection Reagent was added to the reaction system, followed by incubation at room temperature for 60 min. The chemiluminescence signal was detected using a microplate reader (Thermo, VarioskanLUX), with the reading interval per well set to 1000 ms;

Data analysis was performed on the measurement results: the CV% of the control group detection results should be less than 10%, and the z' value should be greater than 0.5. For data meeting the above quality control results, the compound inhibition rate was calculated (Inhibition rate (%) = 100 * (control group average - experimental group) / (control group average - buffer control group average)). GraphPad Prism8 was used to perform nonlinear regression fitting of the compound inhibition rate curve and derive the IC50 value.

### 2. Pol θ Inhibitor Cell Viability Assay

The in vitro efficacy of the inhibitors was evaluated using a cell viability assay with CellTiter-Glo reagent (Promega, G7573), i.e., the commonly used CTG assay. The specific assay method is as follows:
1) DLD1 and DLD1-/- cells were cultured. One day before the assay, cells were digested with trypsin (0.025% Trypsin-EDTA, Hyclone), centrifuged at 1000 rpm for 3 min, and collected. Cells were counted using a cell counter (Shanghai Monwei Biomedical Technology Co.,Ltd., SmartCell600A.SC1006) and seeded into a 96-well white culture plate at an appropriate seeding ratio.
2) Drug treatment was performed 24 hours after cell plating, recorded as Day 0. Compound addition was performed using an automated pipettor (Thermo, Multidrop 8), with the plate set as a 96-well plate, starting concentration set at 30 µM, diluted at a 1/3 ratio, with a total of 9 detection points set, each with 2 replicates. Cells were cultured in a 37°C, 5% CO₂ incubator.
3) Medium changes were performed on Day 3 and Day 7, and compounds were added according to step 2).
4) On Day 10, the cell culture plate was removed, and an equal volume of CTG reagent (which needed to be equilibrated to temperature and premixed according to the reagent instructions beforehand) was added. The plate was gently mixed for 10 min (speed 300) on a constant temperature mixer (Hangzhou Allsheng Instruments Co.,Ltd., MSC-100), and chemiluminescence detection was performed using a microplate reader (Thermo, VarioskanLUX).
5) Data analysis was performed on the measurement results: the CV% of the control group detection results should be less than 20%, and the z' value should be greater than 0.5. For data meeting the above quality control results, cell viability was calculated (Inhibition rate (%) = 100 * (control group average - experimental group) / (control group average - medium control group average)). GraphPad Prism8 was used to perform nonlinear regression fitting of the compound inhibition rate curve and derive the IC50 value.

### Data List:

Specific data for the biochemical assay and cell viability assay are shown in the table below:

| Example | Biochemical Assay IC₅₀ (nM) | Cell Viability Assay IC₅₀ (nM) |
|---|---|---|
| 1 | 3.31 | <1.52 |

### 3. Solubility Assay

1) Preparation of 0.1 M Na₂PO₄ buffer (pH 7.4): Add 11 g of Na₂HPO₄ (FW: 141.96) and 3.5 g of NaH₂PO₄·2H₂O (FW: 156.03) to 1 L of Milli-Q water, and adjust the pH to 7.4 using phosphoric acid or sodium hydroxide;
2) Add 10 µL of the test compound (concentration: 10 mM in DMSO) to 990 µL of the Na₂PO₄ buffer prepared in step 1 (final DMSO concentration: 1%);
3) Shake the sample tube at room temperature for 2 hours (1000 rpm/min);
4) Calibration curve preparation:
a) Prepare a 300 µM spiking solution (SS):
Add 6 µL (10 mM in DMSO) of the test compound stock solution to 194 µL of MeOH/ACN (4:1);
b) Draw the standard curve:

| | MeOH:ACN (4:1) mixture (µL) | STD ID | STD Conc. (µM) |
|---|---|---|---|
| 100 µL SS (300 µM) | 400 | STD 8 | 60 |
| 100 µL STD 8 | 200 | STD 7 | 20 |
| 100 µL STD 7 | 100 | STD 6 | 10 |
| 100 µL STD 6 | 150 | STD 5 | 4 |
| 100 µL STD 5 | 400 | STD 4 | 0.8 |
| 100 µL STD 4 | 300 | STD 3 | 0.2 |
| 100 µL STD 3 | 100 | STD 2 | 0.1 |
| 100 µL STD 2 | 400 | STD 1 | 0.02 |

5) Centrifuge the sample (10 minutes, 12000 rpm) to precipitate insoluble particles. Filter the supernatant through a 0.22 µm filter, then transfer the supernatant to a new centrifuge tube.
6) Dilute the supernatant 10-fold with 100 mM buffer
Add 10 µL of the supernatant to 90 µL of buffer (100 mM) to achieve a 10-fold dilution;
7) LC-MS/MS (API 4000) sample preparation
Add 10 µL of the sample (10-fold dilution) and standard curve samples to 400 µL of solution (MeOH : ACN = 1 : 1), then perform the detection. Calculate the solubility value based on the standard curve.

The experimental results are shown in the table below:

| Example | Solubility (µM) |
|---|---|
| 1 | 61.8 |

Although specific embodiments of the present invention have been described above, those skilled in the art should understand that these are merely illustrative examples. Various changes or modifications can be made to these embodiments without departing from the principle and essence of the present invention. Therefore, the scope of protection of the present invention is defined by the appended claims.

## Claims

1. A nitrogen-containing fused-ring compound, **characterized in that** it is a compound of formula II, a compound of formula III, a compound of formula IV, a compound of formula V, a compound of formula VI, a compound of formula VII, a compound of formula VIII, a compound of formula IX, a compound of formula X, or a compound of formula XI; 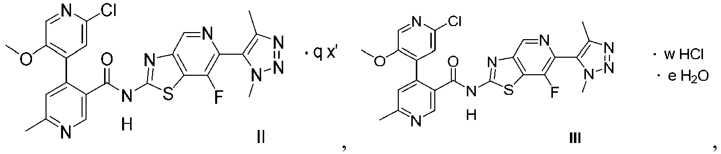 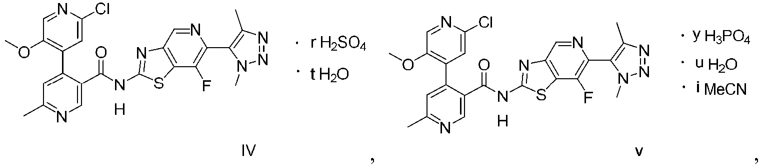 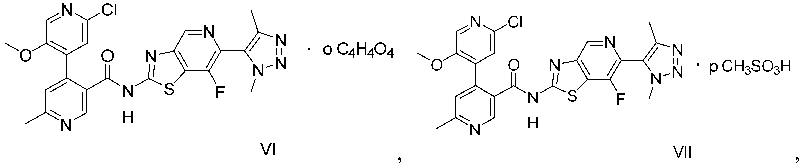 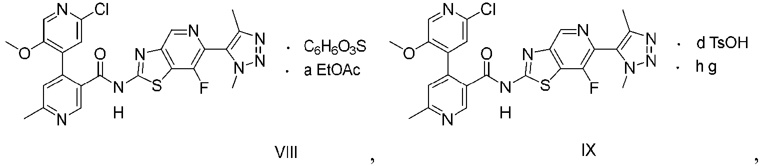 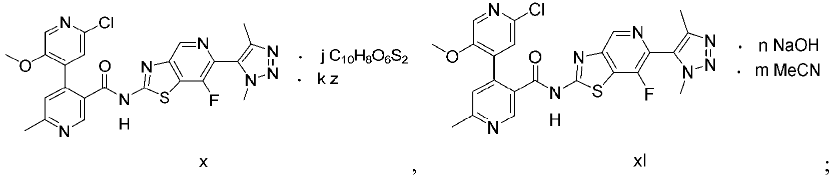 wherein,
in the compound of formula II, x' is water or acetone, and q is 0.01-2.5;
in the compound of formula III, w is 0.5-2.5, and e is 0-2;
in the compound of formula IV, r is 0.5-2, and t is 0-2;
in the compound of formula V, y is 0.5-1.5, u is 0-2, and i is 0-2;
in the compound of formula VI, C₄H₄O₄ is fumaric acid, and o is 1-1.1;
in the compound of formula VII, CH₃SO₃H is methanesulfonic acid, and p is 1-1.1;
in the compound of formula VIII, C₆H₆O₃S is benzenesulfonic acid, and a is 0-1;
in the compound of formula IX, d is 1-2, g is water, tetrahydrofuran, isopropanol, 1,4-dioxane, or methyl tert-butyl ether, and h is 0-3;
in the compound of formula X, C₁₀H₈O₆S₂ is 1,5-naphthalenedisulfonic acid, j is 0.5-1.5, z is water or acetonitrile, and k is 0-5;
in the compound of formula XI, n is 0-1.5, and m is 0 or 0.1-1.5.

2. The nitrogen-containing fused-ring compound according to claim 1, **characterized in that** it satisfies one or more of the following conditions:
(1) in said compound of formula II, q is 0.1, 0.5, 0.56, 0.6, 1, 1.5, 2, or 2.5;
(2) in said compound of formula III, w is 0.5, 1, 1.1, 1.6, 1.7, 1.8, 1.76, 1.06, 1.5, or 2;
(3) in said compound of formula III, e is 0 or 1-2, for example, e is 1.5;
(4) in said compound of formula IV, r is 0.5, 0.9, 1, 0.97, 0.96, or 1.5;
(5) in said compound of formula IV, t is 0 or 1-2, for example, t is 1.7;
(6) in said compound of formula V, y is 1.1, 1.2, 1.14, 1, 1.09, or 1.05;
preferably, y and u are 0; or, i is 0, and u is 0.1, 0.91, 0.9, or 1, for example, i is 0 and u is 0.91; or, u is 0, and i is 0.1, 0.18, 0.2, or 0.5, for example, u is 0 and i is 0.18 or 0.1;
(7) in said compound of formula VI, o is 1, 1.01, 1.05, or 1.1;
(8) in said compound of formula VII, p is 1, 1.03, 1.05, or 1.1;
(9) in said compound of formula VIII, a is 0, or, a is 0-0.1, for example, 0.07, 0.1, or 0;
(10) in said compound of formula IX, d is 1, 1.1, 1.2, 1.02, or 1.5;
(11) in said compound of formula IX, h is 0; or, h is 0.1-3, for example, g is water and h is 3; or, g is tetrahydrofuran and h is 0.2, 0.1, or 0.16; or, g is isopropanol and h is 0.9, 0.98, or 1; or, g is 1,4-dioxane and h is 3, 2.96, or 2.9; or, g is methyl tert-butyl ether and h is 0.4, 0.45, or 0.5;
(12) in said compound of formula X, j is 1, 0.9, 1.1, 1.02, or 0.93;
(13) in said compound of formula X, k is 0, or, k is 0.01-5, for example, z is water and k is 5 or 4.8; or, z is acetonitrile and k is 0.07;
(14) in said compound of formula XI, n is 0.6, 1.1, 1.13, or 1.2;
(15) in said compound of formula XI, m is 0, or, m is 0.5-1.5, for example, 1, 1.07, or 1.1.

3. A crystalline form C of a compound of formula I or the nitrogen-containing fused-ring compound according to claim 1 or 2, **characterized in that** it satisfies one or more of the following conditions:
(1) said compound of formula II is crystalline form A of the compound of formula II, and its X-ray powder diffraction pattern using Cu-Kα radiation, expressed in terms of 2θ angle, has diffraction peaks at 9.00±0.2°, 10.84±0.2°, 17.91±0.2°, and 22.05±0.2°;
(2) said compound of formula II is crystalline form B of the compound of formula II, and its X-ray powder diffraction pattern using Cu-Kα radiation, expressed in terms of 20 angle, has diffraction peaks at 8.10±0.2°, 8.37±0.2°, 13.94±0.2°, 15.67±0.2°, 20.72±0.2°, 24.4±0.2°, and 24.83±0.2°;
(3) said crystalline form C of the compound of formula I has an X-ray powder diffraction pattern using Cu-Kα radiation, expressed in terms of 2θ angle, with diffraction peaks at 11.51±0.2°, 18.01±0.2°, 22.56±0.2°, and 25.68±0.2°;
wherein the structure of the compound of formula I is: 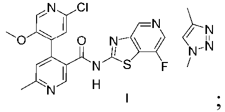
(4) said compound of formula III is crystalline form A of the compound of formula III, and its X-ray powder diffraction pattern using Cu-Kα radiation, expressed in terms of 2θ angle, has diffraction peaks at 4.61±0.2', 5.02±0.2°, 9.19±0.2°, and 13.76±0.2°;
(5) said compound of formula III is crystalline form B of the compound of formula III, and its X-ray powder diffraction pattern using Cu-Kα radiation, expressed in terms of 2θ angle, has diffraction peaks at 6.47±0.2°, 7.19±0.2°, 9.11±0.2°, 11.63±0.2°, and 14.45±0.2°;
(6) said compound of formula III is crystalline form C of the compound of formula III, and its X-ray powder diffraction pattern using Cu-Kα radiation, expressed in terms of 2θ angle, has diffraction peaks at 9.84±0.2°, 10.51±0.2°, 12.19±0.2°, 13.39±0.2°, and 14.40±0.2°;
(7) said compound of formula IV is crystalline form A of the compound of formula IV, and its X-ray powder diffraction pattern using Cu-Kα radiation, expressed in terms of 2θ angle, has diffraction peaks at 5.14±0.2°, 9.01±0.2°, and 15.03±0.2°;
(8) said compound of formula IV is crystalline form B of the compound of formula IV, and its X-ray powder diffraction pattern using Cu-Kα radiation, expressed in terms of 2θ angle, has diffraction peaks at 7.11±0.2°, 7.57±0.2°, 7.72±0.2°, 8.10±0.2°, and 10.27±0.2°;
(9) said compound of formula V is crystalline form A of the compound of formula V, and its X-ray powder diffraction pattern using Cu-Kα radiation, expressed in terms of 2θ angle, has diffraction peaks at 5.15±0.2°, 8.34±0.2°, 15.51±0.2°, and 15.88±0.2°;
(10) said compound of formula V is crystalline form B of the compound of formula V, and its X-ray powder diffraction pattern using Cu-Kα radiation, expressed in terms of 2θ angle, has diffraction peaks at 6.83±0.2°, 12.89±0.2°, 14.44±0.2°, 15.91±0.2°, 25.08±0.2°, and 25.50±0.2°;
(11) said compound of formula V is crystalline form C of the compound of formula V, and its X-ray powder diffraction pattern using Cu-Kα radiation, expressed in terms of 2θ angle, has diffraction peaks at 5.19±0.2°, 8.39±0.2°, 10.47±0.2°, and 11.51±0.2°;
(12) said compound of formula VI is crystalline form A of the compound of formula VI, and its X-ray powder diffraction pattern using Cu-Kα radiation, expressed in terms of 2θ angle, has diffraction peaks at 10.58±0.2°, 12.16±0.2°, and 13.98±0.2°;
(13) said compound of formula VII is crystalline form A of the compound of formula VII, and its X-ray powder diffraction pattern using Cu-Kα radiation, expressed in terms of 2θ angle, has diffraction peaks at 9.00±0.2°, 17.44±0.2°, and 21.74±0.2°;
(14) said compound of formula VIII is crystalline form A of the compound of formula VIII, and its X-ray powder diffraction pattern using Cu-Kα radiation, expressed in terms of 2θ angle, has diffraction peaks at 7.66±0.2°, 10.09±0.2°, 11.92±0.2°, and 13.91±0.2°;
(15) said compound of formula IX is crystalline form A of the compound of formula IX, and its X-ray powder diffraction pattern using Cu-Kα radiation, expressed in terms of 2θ angle, has diffraction peaks at 5.96±0.2°, 16.71±0.2°, 17.79±0.2°, 20.33±0.2°, and 24.66±0.2°;
(16) said compound of formula IX is crystalline form B of the compound of formula IX, and its X-ray powder diffraction pattern using Cu-Kα radiation, expressed in terms of 2θ angle, has diffraction peaks at 5.34±0.2°, 9.34±0.2°, 9.92±0.2°, 12.84±0.2°, and 16.61±0.2°;
(17) said compound of formula X is crystalline form A of the compound of formula X, and its X-ray powder diffraction pattern using Cu-Kα radiation, expressed in terms of 2θ angle, has diffraction peaks at 7.46±0.2°, 12.01±0.2°, and 21.70±0.2°;
(18) said compound of formula X is crystalline form B of the compound of formula X, and its X-ray powder diffraction pattern using Cu-Kα radiation, expressed in terms of 2θ angle, has diffraction peaks at 8.96±0.2°, 10.81±0.2°, 17.92±0.2°, and 22.07±0.2°;
(19) said compound of formula X is crystalline form C of the compound of formula X, and its X-ray powder diffraction pattern using Cu-Kα radiation, expressed in terms of 2θ angle, has diffraction peaks at 11.30±0.2°, 22.61±0.2°, 24.69±0.2°, 26.16±0.2°, and 28.57±0.2°;
(20) said compound of formula X is crystalline form D of the compound of formula X, and its X-ray powder diffraction pattern using Cu-Kα radiation, expressed in terms of 20 angle, has diffraction peaks at 10.68±0.2° and 21.44±0.2°, wherein z is water;
(21) said compound of formula XI is crystalline form A of the compound of formula XI, and its X-ray powder diffraction pattern using Cu-Kα radiation, expressed in terms of 20 angle, has diffraction peaks at 12.78±0.2°, 14.09±0.2°, and 14.98±0.2°;
(22) said compound of formula XI is crystalline form B of the compound of formula XI, and its X-ray powder diffraction pattern using Cu-Kα radiation, expressed in terms of 2θ angle, has diffraction peaks at 8.22±0.2°, 19.38±0.2°, 20.38±0.2°, 23.43±0.2°, and 24.74±0.2°;
(23) said compound of formula IX is crystalline form C of the compound of formula IX, and its X-ray powder diffraction pattern using Cu-Kα radiation, expressed in terms of 2θ angle, has diffraction peaks at 5.23±0.2° and 19.82±0.2°;
(24) said compound of formula IX is crystalline form D of the compound of formula IX, and its X-ray powder diffraction pattern using Cu-Kα radiation, expressed in terms of 2θ angle, has diffraction peaks at 6.13±0.2°, 10.87±0.2°, and 18.38±0.2°;
(25) said compound of formula IX is crystalline form E of the compound of formula IX, and its X-ray powder diffraction pattern using Cu-Kα radiation, expressed in terms of 2θ angle, has diffraction peaks at 5.66±0.2°, 11.50±0.2°, 17.97±0.2°, 19.83±0.2°, 23.95±0.2°, and 26.58±0.2°;
(26) said compound of formula IX is crystalline form F of the compound of formula IX, and its X-ray powder diffraction pattern using Cu-Kα radiation, expressed in terms of 2θ angle, has diffraction peaks at 6.15±0.2°, 16.55±0.2°, 17.30±0.2°, and 21.48±0.2°;
(27) said compound of formula IX is crystalline form G of the compound of formula IX, and its X-ray powder diffraction pattern using Cu-Kα radiation, expressed in terms of 2θ angle, has diffraction peaks at 7.65±0.2°, 13.76±0.2°, 20.35±0.2°, 21.01±0.2°, and 21.57±0.2°, wherein g is 1,4-dioxane;
(28) said compound of formula IX is crystalline form H of the compound of formula IX, and its X-ray powder diffraction pattern using Cu-Kα radiation, expressed in terms of 2θ angle, has diffraction peaks at 8.27±0.2°, 9.70±0.2°, 16.55±0.2°, and 19.78±0.2°, wherein g is methyl tert-butyl ether;
(29) said compound of formula IX is crystalline form I of the compound of formula IX, and its X-ray powder diffraction pattern using Cu-Kα radiation, expressed in terms of 2θ angle, has diffraction peaks at 6.01±0.2°, 10.63±0.2°, and 12.79±0.2°;
(30) said compound of formula IX is crystalline form J of the compound of formula IX, and its X-ray powder diffraction pattern using Cu-Kα radiation, expressed in terms of 2θ angle, has diffraction peaks at 5.27±0.2°, 8.18±0.2°, 9.72±0.2°, 10.54±0.2°, 11.06±0.2°, 12.44±0.2°, 18.79±0.2°, and 20.21±0.2°.

4. The crystalline form C of the compound of formula I or said nitrogen-containing fused-ring compound according to claim 3, **characterized in that** it satisfies one or more of the following conditions:
(1) said crystalline form A of the compound of formula II, its X-ray powder diffraction pattern using Cu-Kα radiation, expressed in terms of 20 angle, further has diffraction peaks at one or more of the following: 5.98±0.2°, 11.94±0.2°, 18.36±0.2°, 21.30±0.2°, 23.29±0.2°, and 27.72±0.2°;
for example, said crystalline form A of the compound of formula II, its X-ray powder diffraction pattern using Cu-Kα radiation, expressed in terms of 20 angle, has diffraction peaks at 9.00±0.2°, 10.84±0.2°, 17.91±0.2°, 22.05±0.2°, 5.98±0.2°, 11.94±0.2°, 18.36±0.2°, 21.30±0.2°, and 23.29±0.2°;
preferably, said crystalline form A of the compound of formula II, its X-ray powder diffraction pattern using Cu-Kα radiation, expressed in terms of 2θ angle, further has diffraction peaks at one or more of the following: 13.46±0.2°, 14.16±0.2°, 14.72±0.2°, 19.99±0.2°, 20.65±0.2°, 24.6±0.2°, 25.62±0.2°, 28.96±0.2°, 29.68±0.2°, 30.29±0.2°, and 31.08±0.2°;
(2) said crystalline form B of the compound of formula II, its X-ray powder diffraction pattern using Cu-Kα radiation, expressed in terms of 2θ angle, further has diffraction peaks at one or more of the following: 13.33±0.2°, 14.79±0.2°, 17.63±0.2°, and 21.15±0.2°;
for example, said crystalline form B of the compound of formula II, its X-ray powder diffraction pattern using Cu-Kα radiation, expressed in terms of 2θ angle, has diffraction peaks at 8.10±0.2°, 8.37±0.2°, 13.94±0.2°, 15.67±0.2°, 20.72±0.2°, 14.26±0.2°, 16.61±0.2°, 17.91±0.2°, and 18.70±0.2°;
preferably, said crystalline form B of the compound of formula II, its X-ray powder diffraction pattern using Cu-Kα radiation, expressed in terms of 2θ angle, further has diffraction peaks at one or more of the following: 14.26±0.2°, 16.61±0.2°, 17.91±0.2°, 18.70±0.2°, 19.13±0.2°, 19.62±0.2°, 20.03±0.2°, 23.92±0.2°, 24.20±0.2°, and 25.42±0.2°;
(3) said crystalline form C of the compound of formula I, its X-ray powder diffraction pattern using Cu-Kα radiation, expressed in terms of 2θ angle, further has diffraction peaks at one or more of the following: 12.88±0.2°, 15.05±0.2°, 20.85±0.2°, 21.68±0.2°, 23.11±0.2°, and 24.79±0.2°;
for example, in said crystalline form C of the compound of formula I, its X-ray powder diffraction pattern using Cu-Kα radiation, expressed in terms of 2θ angle, has the diffraction peak at 11.51±0.2° as the strongest peak;
for example, said crystalline form C of the compound of formula I, its X-ray powder diffraction pattern using Cu-Kα radiation, expressed in terms of 2θ angle, has diffraction peaks at 11.51±0.2°, 18.01±0.2°, 22.56±0.2°, 25.68±0.2°, 12.88±0.2°, 15.05±0.2°, 20.85±0.2°, 21.68±0.2°, and 23.11±0.2°;
preferably, said crystalline form C of the compound of formula I, its X-ray powder diffraction pattern using Cu-Kα radiation, expressed in terms of 2θ angle, further has diffraction peaks at one or more of the following: 9.022±0.2°, 10.212±0.2°, 16.163±0.2°, 16.96±0.2°, 17.22±0.2°, 26.937±0.2°, 29.445±0.2°, 30.137±0.2°, and 30.354±0.2°;
(4) said crystalline form A of the compound of formula III, its X-ray powder diffraction pattern using Cu-Kα radiation, expressed in terms of 2θ angle, further has diffraction peaks at one or more of the following: 6.57±0.2°, 6.89±0.2°, 9.96±0.2°, 10.48±0.2°, 10.64±0.2°, 14.69±0.2°, and 14.90±0.2°;
for example, said crystalline form A of the compound of formula III, its X-ray powder diffraction pattern using Cu-Kα radiation, expressed in terms of 2θ angle, has diffraction peaks at 4.61±0.2°, 5.02±0.2°, 9.19±0.2°, 13.76±0.2°, 6.57±0.2°, 6.89±0.2°, 9.96±0.2°, 10.48±0.2°, and 10.64±0.2°;
preferably, said crystalline form A of the compound of formula III, its X-ray powder diffraction pattern using Cu-Kα radiation, expressed in terms of 20 angle, further has diffraction peaks at one or more of the following: 18.34±0.2°, 19.13±0.2°, 19.30±0.2°, 20.81±0.2°, 21.68±0.2°, 23.09±0.2°, 24.03±0.2°, 26.13±0.2°, and 28.17±0.2°;
(5) said crystalline form B of the compound of formula III, its X-ray powder diffraction pattern using Cu-Kα radiation, expressed in terms of 2θ angle, further has diffraction peaks at one or more of the following: 17.37±0.2°, 18.21±0.2°, 19.44±0.2°, 20.17±0.2°, 20.82±0.2°, 22.41±0.2°, and 24.58±0.2°;
for example, said crystalline form B of the compound of formula III, its X-ray powder diffraction pattern using Cu-Kα radiation, expressed in terms of 2θ angle, has diffraction peaks at 6.47±0.2°, 7.19±0.2°, 9.11±0.2°, 11.63±0.2°, 14.45±0.2°, 17.37±0.2°, 18.21±0.2°, 19.44±0.2°, 20.17±0.2°, and 20.82±0.2°;
preferably, said crystalline form B of the compound of formula III, its X-ray powder diffraction pattern using Cu-Kα radiation, expressed in terms of 2θ angle, further has diffraction peaks at one or more of the following: 12.94±0.2°, 13.41±0.2°, 15.41±0.2°, 15.75±0.2°, 16.23±0.2°, 16.72±0.2°, 26.01±0.2°, 26.17±0.2°, 26.47±0.2°, 27.96±0.2°, 28.62±0.2°, 30.04±0.2°, and 30.37±0.2°;
(6) said crystalline form C of the compound of formula III, its X-ray powder diffraction pattern using Cu-Kα radiation, expressed in terms of 20 angle, further has diffraction peaks at one or more of the following: 18.16±0.2°, 18.29±0.2°, 19.39±0.2°, 20.81±0.2°, 21.75±0.2°, 26.41±0.2°, and 27.48±0.2°;
for example, said crystalline form C of the compound of formula III, its X-ray powder diffraction pattern using Cu-Kα radiation, expressed in terms of 2θ angle, has diffraction peaks at 9.84±0.2°, 10.51±0.2°, 12.19±0.2°, 13.39±0.2°, 14.40±0.2°, 18.16±0.2°, 18.29±0.2°, 19.39±0.2°, 20.81±0.2°, 21.75±0.2°, and 26.41±0.2°;
preferably, said crystalline form C of the compound of formula III, its X-ray powder diffraction pattern using Cu-Kα radiation, expressed in terms of 2θ angle, further has diffraction peaks at one or more of the following: 11.61±0.2°, 20.02±0.2°, 22.51±0.2°, 22.90±0.2°, 23.25±0.2°, 23.49±0.2°, 24.46±0.2°, 25.11±0.2°, 26.94±0.2°, 28.37±0.2°, 28.97±0.2°, and 29.32±0.2°;
(7) said crystalline form A of the compound of formula IV, its X-ray powder diffraction pattern using Cu-Kα radiation, expressed in terms of 2θ angle, further has diffraction peaks at one or more of the following: 11.45±0.2°, 17.44±0.2°, 21.45±0.2°, 21.78±0.2°, 24.82±0.2°, 25.95±0.2°, and 26.68±0.2°;
for example, said crystalline form A of the compound of formula IV, its X-ray powder diffraction pattern using Cu-Kα radiation, expressed in terms of 2θ angle, has diffraction peaks at 5.14±0.2°, 9.01±0.2°, 15.03±0.2°, 11.45±0.2°, 17.44±0.2°, 21.45±0.2°, 21.78±0.2°, 24.82±0.2°, and 25.95±0.2°;
preferably, said crystalline form A of the compound of formula IV, its X-ray powder diffraction pattern using Cu-Kα radiation, expressed in terms of 2θ angle, further has diffraction peaks at one or more of the following: 6.11±0.2°, 6.60±0.2°, 7.35±0.2°, 8.63±0.2°, 14.62±0.2°, 16.45±0.2°, 20.40±0.2°, 23.81±0.2°, 25.47±0.2°, 27.96±0.2°, and 28.43±0.2°;
(8) said crystalline form B of the compound of formula IV, its X-ray powder diffraction pattern using Cu-Kα radiation, expressed in terms of 2θ angle, further has diffraction peaks at one or more of the following: 13.86±0.2°, 14.11±0.2°, 14.71±0.2°, 15.44±0.2°, 16.07±0.2°, 23.18±0.2°, 24.18±0.2°, and 25.17±0.2°;
for example, said crystalline form B of the compound of formula IV, its X-ray powder diffraction pattern using Cu-Kα radiation, expressed in terms of 2θ angle, has diffraction peaks at 7.11±0.2°, 7.57±0.2°, 7.72±0.2°, 8.10±0.2°, 13.86±0.2°, 14.11±0.2°, 14.71±0.2°, 23.18±0.2°, 24.18±0.2°, and 25.17±0.2°;
preferably, said crystalline form B of the compound of formula IV, its X-ray powder diffraction pattern using Cu-Kα radiation, expressed in terms of 2θ angle, further has diffraction peaks at one or more of the following: 10.27±0.2°, 12.55±0.2°, 12.67±0.2°, 13.19±0.2°, 16.73±0.2°, 17.92±0.2°, 18.38±0.2°, 18.58±0.2°, 20.18±0.2°, 21.2±0.2°, 22.2±0.2°, 25.58±0.2°, and 27.82±0.2°;
(9) said crystalline form A of the compound of formula V, its X-ray powder diffraction pattern using Cu-Kα radiation, expressed in terms of 2θ angle, further has diffraction peaks at one or more of the following: 7.97±0.2°, 8.96±0.2°, 17.93±0.2°, 18.39±0.2°, 21.99±0.2°, and 27.73±0.2°;
for example, said crystalline form A of the compound of formula V, its X-ray powder diffraction pattern using Cu-Kα radiation, expressed in terms of 2θ angle, has diffraction peaks at 5.15±0.2°, 8.34±0.2°, 15.51±0.2°, 15.88±0.2°, 8.96±0.2°, 17.93±0.2°, 18.39±0.2°, 21.99±0.2°, and 27.73±0.2°;
preferably, said crystalline form A of the compound of formula V, its X-ray powder diffraction pattern using Cu-Kα radiation, expressed in terms of 2θ angle, further has diffraction peaks at one or more of the following: 5.98±0.2°, 8.96±0.2°, 10.49±0.2°, 10.80±0.2°, 11.34±0.2°, 11.66±0.2°, 11.99±0.2°, 12.73±0.2°, 13.15±0.2°, 18.68±0.2°, 19.73±0.2°, 20.14±0.2°, 22.76±0.2°, and 23.24±0.2°;
(10) said crystalline form B of the compound of formula V, its X-ray powder diffraction pattern using Cu-Kα radiation, expressed in terms of 2θ angle, further has diffraction peaks at one or more of the following: 8.32±0.2°, 10.26±0.2°, 13.93±0.2°, 14.67±0.2°, 20.35±0.2°, 20.49±0.2°, 21.15±0.2°, and 21.65±0.2°;
for example, said crystalline form B of the compound of formula V, its X-ray powder diffraction pattern using Cu-Kα radiation, expressed in terms of 2θ angle, has diffraction peaks at 6.83±0.2°, 12.89±0.2°, 14.44±0.2°, 15.91±0.2°, 25.08±0.2°, 25.50±0.2°, 8.32±0.2°, 20.35±0.2°, and 20.49±0.2°;
preferably, said crystalline form B of the compound of formula V, its X-ray powder diffraction pattern using Cu-Kα radiation, expressed in terms of 2θ angle, further has diffraction peaks at one or more of the following positions: 8.52±0.2°, 9.17±0.2°, 13.46±0.2°, 16.25±0.2°, and 17.94±0.2°;
(11) said crystalline form C of the compound of formula V, its X-ray powder diffraction pattern using Cu-Kα radiation, expressed in terms of 2θ angle, further has diffraction peaks at one or more of the following positions: 15.68±0.2°, 15.87±0.2°, 18.01±0.2°, 22.55±0.2°, and 25.67±0.2°;
for example, in said crystalline form C of the compound of formula V, the relative intensity of the diffraction peak at 11.51±0.2° in its X-ray powder diffraction pattern using Cu-Kα radiation, expressed in terms of 2θ angle, is 80%-100%, preferably the diffraction peak at 11.51±0.2° is the strongest peak;
for example, said crystalline form C of the compound of formula V, its X-ray powder diffraction pattern using Cu-Kα radiation, expressed in terms of 2θ angle, has diffraction peaks at 5.19±0.2°, 8.39±0.2°, 10.47±0.2°, 11.51±0.2°, 11.67±0.2°, 15.68±0.2°, 15.87±0.2°, 18.01±0.2°, and 22.55±0.2°;
preferably, said crystalline form C of the compound of formula V, its X-ray powder diffraction pattern using Cu-Kα radiation, expressed in terms of 2θ angle, further has diffraction peaks at one or more of the following positions: 11.67±0.2° , 12.73±0.2°, 12.89±0.2°, 14.59±0.2°, 15.05±0.2°, 18.76±0.2°, 19.81±0.2°, 20.48±0.2°, 20.84±0.2°, 21.68±0.2°, 22.10±0.2°, 24.80±0.2°, and 25.27±0.2°;
(12) said crystalline form A of the compound of formula VI, its X-ray powder diffraction pattern using Cu-Kα radiation, expressed in terms of 2θ angle, further has diffraction peaks at one or more of the following positions: 6.96±0.2°, 8.60±0.2°, 14.84±0.2°, 15.39±0.2°, 16.63±0.2°, and 17.08±0.2°;
for example, said crystalline form A of the compound of formula VI, its X-ray powder diffraction pattern using Cu-Kα radiation, expressed in terms of 2θ angle, has diffraction peaks at 10.58±0.2°, 12.16±0.2°, 13.98±0.2°, 6.96±0.2°, 8.60±0.2°, 14.84±0.2°, 15.39±0.2°, 16.63±0.2°, and 17.08±0.2°;
preferably, said crystalline form A of the compound of formula VI, its X-ray powder diffraction pattern using Cu-Kα radiation, expressed in terms of 2θ angle, further has diffraction peaks at one or more of the following positions: 6.73±0.2°, 9.38±0.2°, 10.28±0.2°, 13.45±0.2°, 19.28±0.2°, and 18.67±0.2°;
(13) said crystalline form A of the compound of formula VII, its X-ray powder diffraction pattern using Cu-Kα radiation, expressed in terms of 2θ angle, further has diffraction peaks at one or more of the following positions: 5.13±0.2°, 6.73±0.2°, 8.61±0.2°, 17.74±0.2°, 24.43±0.2°, and 25.32±0.2°;
for example, said crystalline form A of the compound of formula VII, its X-ray powder diffraction pattern using Cu-Kα radiation, expressed in terms of 2θ angle, has diffraction peaks at 9.00±0.2°, 17.44±0.2°, 21.74±0.2°, 5.13±0.2°, 6.73±0.2°, 8.61±0.2°, 17.74±0.2°, 24.43±0.2°, and 25.32±0.2°;
preferably, said crystalline form A of the compound of formula VII, its X-ray powder diffraction pattern using Cu-Kα radiation, expressed in terms of 2θ angle, further has diffraction peaks at one or more of the following positions: 15.55±0.2°, 10.05±0.2°, 11.57±0.2°, 22.37±0.2°, and 26.36±0.2°;
(14) said crystalline form A of the compound of formula VIII, its X-ray powder diffraction pattern using Cu-Kα radiation, expressed in terms of 2θ angle, further has diffraction peaks at one or more of the following positions: 9.06±0.2°, 9.72±0.2°, 11.10±0.2°, 15.91±0.2°, 16.55±0.2°, 18.00±0.2°, 18.27±0.2°, and 19.16±0.2°;
for example, said crystalline form A of the compound of formula VIII, its X-ray powder diffraction pattern using Cu-Kα radiation, expressed in terms of 2θ angle, has diffraction peaks at 7.66±0.2°, 10.09±0.2°, 11.92±0.2°, 13.91±0.2°, 9.06±0.2°, 9.72±0.2°, 11.10±0.2°, 15.91±0.2°, and 16.55±0.2°;
preferably, said crystalline form A of the compound of formula VIII, its X-ray powder diffraction pattern using Cu-Kα radiation, expressed in terms of 2θ angle, further has diffraction peaks at one or more of the following positions: 20.35±0.2°, 20.89±0.2°, 21.10±0.2°, 21.68±0.2°, 22.27±0.2°, 22.49±0.2°, 22.97±0.2°, 23.50±0.2°, 24.84±0.2°, 25.52±0.2°, and 29.36±0.2°;
(15) said crystalline form A of the compound of formula IX, its X-ray powder diffraction pattern using Cu-Kα radiation, expressed in terms of 2θ angle, further has diffraction peaks at one or more of the following positions: 10.57±0.2°, 12.34±0.2°, 12.77±0.2°, 14.06±0.2°, 14.40±0.2°, 14.97±0.2°, 19.88±0.2°, 22.64±0.2°, 23.18±0.2°, and 24.22±0.2°;
for example, said crystalline form A of the compound of formula IX, its X-ray powder diffraction pattern using Cu-Kα radiation, expressed in terms of 2θ angle, has diffraction peaks at 5.96±0.2°, 16.71±0.2°, 17.79±0.2°, 20.33±0.2°, 24.66±0.2°, 10.57±0.2°, 12.34±0.2°, 12.77±0.2°, 14.06±0.2°, 14.40±0.2°, and 14.97±0.2°;
preferably, said crystalline form A of the compound of formula IX, its X-ray powder diffraction pattern using Cu-Kα radiation, expressed in terms of 2θ angle, further has diffraction peaks at one or more of the following positions: 9.93±0.2°, 10.13±0.2°, 11.03±0.2°, 11.88±0.2°, 17.56±0.2°, 18.41±0.2°, 18.70±0.2°, 20.68±0.2°, 22.11±0.2°, 25.43±0.2°, 25.98±0.2°, 27.35±0.2°, 27.52±0.2°, 28.08±0.2°, and 29.17±0.2°;
(16) said crystalline form B of the compound of formula IX, its X-ray powder diffraction pattern using Cu-Kα radiation, expressed in terms of 2θ angle, further has diffraction peaks at one or more of the following positions: 8.20±0.2°, 10.13±0.2°, 11.12±0.2°, 13.18±0.2°, 16.13±0.2°, 18.67±0.2°, 19.47±0.2°, and 19.85±0.2°;
for example, said crystalline form B of the compound of formula IX, its X-ray powder diffraction pattern using Cu-Kα radiation, expressed in terms of 2θ angle, has diffraction peaks at 5.34±0.2°, 9.34±0.2°, 9.92±0.2°, 12.84±0.2°, 16.61±0.2°, 8.20±0.2°, 10.13±0.2°, 13.18±0.2°, and 16.13±0.2°;
preferably, said crystalline form B of the compound of formula IX, its X-ray powder diffraction pattern using Cu-Kα radiation, expressed in terms of 2θ angle, further has diffraction peaks at one or more of the following positions: 11.47±0.2°, 16.36±0.2°, 18.60±0.2°, and 21.53±0.2°;
(17) said crystalline form A of the compound of formula X, its X-ray powder diffraction pattern using Cu-Kα radiation, expressed in terms of 2θ angle, further has diffraction peaks at one or more of the following positions: 11.43±0.2°, 13.53±0.2°, 17.24±0.2°, 18.49±0.2°, 18.77±0.2°, 21.08±0.2°, 24.57±0.2°, and 28.44±0.2°;
for example, said crystalline form A of the compound of formula X, its X-ray powder diffraction pattern using Cu-Kα radiation, expressed in terms of 2θ angle, has diffraction peaks at 7.46±0.2°, 12.01±0.2°, 21.70±0.2°, 11.43±0.2°, 13.53±0.2°, 17.24±0.2°, 18.49±0.2°, 18.77±0.2°, and 21.08±0.2°;
preferably, said crystalline form A of the compound of formula X, its X-ray powder diffraction pattern using Cu-Kα radiation, expressed in terms of 2θ angle, further has diffraction peaks at one or more of the following positions: 5.71±0.2°, 13.93±0.2°, 14.91±0.2°, 15.34±0.2°, 22.12±0.2°, 22.80±0.2°, 22.95±0.2°, 24.99±0.2°, and 26.08±0.2°;
(18) said crystalline form B of the compound of formula X, its X-ray powder diffraction pattern using Cu-Kα radiation, expressed in terms of 2θ angle, further has diffraction peaks at one or more of the following positions: 11.98±0.2°, 18.4±0.2°, 21.27±0.2°, 23.27±0.2°, 27.26±0.2°, and 27.77±0.2°;
for example, said crystalline form B of the compound of formula X, its X-ray powder diffraction pattern using Cu-Kα radiation, expressed in terms of 2θ angle, has diffraction peaks at 8.96±0.2°, 10.81±0.2°, 17.92±0.2°, 22.07±0.2°, 11.98±0.2°, 18.4±0.2°, 21.27±0.2°, and 23.27±0.2°;
preferably, said crystalline form B of the compound of formula X, its X-ray powder diffraction pattern using Cu-Kα radiation, expressed in terms of 2θ angle, further has diffraction peaks at one or more of the following positions: 5.95±0.2°, 14.09±0.2°, 15.80±0.2°, 16.97±0.2°, 20.03±0.2°, 22.42±0.2°, and 23.79±0.2°;
(19) said crystalline form C of the compound of formula X, its X-ray powder diffraction pattern using Cu-Kα radiation, expressed in terms of 2θ angle, further has diffraction peaks at one or more of the following positions: 6.36±0.2°, 12.82±0.2°, 17.07±0.2°, 17.48±0.2°, 18.61±0.2°, 20.24±0.2°, and 20.94±0.2°;
for example, said crystalline form C of the compound of formula X, its X-ray powder diffraction pattern using Cu-Kα radiation, expressed in terms of 2θ angle, has a relative intensity of 50%-100% for the diffraction peak at 22.61±0.2°, preferably the diffraction peak at 22.61±0.2° is the strongest peak;
for example, said crystalline form C of the compound of formula X, its X-ray powder diffraction pattern using Cu-Kα radiation, expressed in terms of 2θ angle, has diffraction peaks at 11.30±0.2°, 22.61±0.2°, 24.69±0.2°, 26.16±0.2°, 28.57±0.2°, 6.36±0.2°, 18.61±0.2°, 12.82±0.2°, and 17.07±0.2°;
preferably, said crystalline form C of the compound of formula X, its X-ray powder diffraction pattern using Cu-Kα radiation, expressed in terms of 2θ angle, further has diffraction peaks at one or more of the following positions: 12.11±0.2° and 15.12±0.2°;
(20) said crystalline form D of the compound of formula X, its X-ray powder diffraction pattern using Cu-Kα radiation, expressed in terms of 2θ angle, further has diffraction peaks at one or more of the following positions: 15.77±0.2°, 16.88±0.2°, 19.82±0.2°, 21.76±0.2°, 23.62±0.2°, 24.95±0.2°, and 27.32±0.2°;
for example, said crystalline form D of the compound of formula X, its X-ray powder diffraction pattern using Cu-Kα radiation, expressed in terms of 2θ angle, has diffraction peaks at 10.68±0.2°, 21.44±0.2°, 15.77±0.2°, 16.88±0.2°, 19.82±0.2°, 21.76±0.2°, 23.62±0.2°, and 24.95±0.2°;
preferably, said crystalline form D of the compound of formula X, its X-ray powder diffraction pattern using Cu-Kα radiation, expressed in terms of 2θ angle, further has diffraction peaks at one or more of the following positions: 7.27±0.2°, 7.87±0.2°, 13.59±0.2°, 14.54±0.2°, 16.25±0.2°, 17.29±0.2°, 17.66±0.2°, 19.63±0.2°, 20.27±0.2°, 22.62±0.2°, 23.09±0.2°, 24.33±0.2°, 24.64±0.2°, 26.37±0.2°, and 28.88±0.2°;
(21) said crystalline form A of the compound of formula XI, its X-ray powder diffraction pattern using Cu-Kα radiation, expressed in terms of 2θ angle, further has diffraction peaks at one or more of the following positions: 10.67±0.2°, 18.82±0.2°, 19.49±0.2°, and 23.58±0.2°;
for example, said crystalline form A of the compound of formula XI, its X-ray powder diffraction pattern using Cu-Kα radiation, expressed in terms of 2θ angle, has diffraction peaks at 12.78±0.2°, 14.09±0.2°, 14.98±0.2°, 10.67±0.2°, 18.82±0.2°, 19.49±0.2°, 23.58±0.2°, and 21.80±0.2°;
preferably, said crystalline form A of the compound of formula XI, its X-ray powder diffraction pattern using Cu-Kα radiation, expressed in terms of 2θ angle, further has diffraction peaks at one or more of the following positions: 6.9±0.2°, 9.39±0.2°, 17.22±0.2°, 19.35±0.2°, 21.80±0.2°, 22.45±0.2°, 24.15±0.2°, and 27.926±0.2°;
(22) said crystalline form B of the compound of formula XI, its X-ray powder diffraction pattern using Cu-Kα radiation, expressed in terms of 2θ angle, further has diffraction peaks at one or more of the following: 9.31±0.2°, 12.92±0.2°, 13.62±0.2°, 24.17±0.2°, and 27.37±0.2°;
for example, said crystalline form B of the compound of formula XI, its X-ray powder diffraction pattern using Cu-Kα radiation, expressed in terms of 2θ angle, has diffraction peaks at 8.22±0.2°, 19.38±0.2°, 20.38±0.2°, 23.43±0.2°, 24.74±0.2°, 9.31±0.2°, 12.92±0.2°, and 24.17±0.2°;
preferably, said crystalline form B of the compound of formula XI, its X-ray powder diffraction pattern using Cu-Kα radiation, expressed in terms of 2θ angle, further has diffraction peaks at one or more of the following: 15.13±0.2°, 16.29±0.2°, 17.63±0.2°, 17.77±0.2°, 18.12±0.2°, 18.63±0.2°, 21.04±0.2°, 21.28±0.2°, and 26.46±0.2°;
(23) said crystalline form C of the compound of formula IX, its X-ray powder diffraction pattern using Cu-Kα radiation, expressed in terms of 2θ angle, further has diffraction peaks at one or more of the following: 9.21±0.2°, 16.11±0.2°, 16.57±0.2°, 19.39±0.2°, and 20.01±0.2°; for example, said crystalline form C of the compound of formula IX, its X-ray powder diffraction pattern using Cu-Kα radiation, expressed in terms of 2θ angle, has a diffraction peak at 19.82±0.2° with a relative intensity of 60%-100%, preferably a relative intensity of 70%-80%, for example 71.2%;
for example, said crystalline form C of the compound of formula IX, its X-ray powder diffraction pattern using Cu-Kα radiation, expressed in terms of 2θ angle, has diffraction peaks at 5.23±0.2°, 19.82±0.2°, 9.21±0.2°, 16.11±0.2°, 16.57±0.2°, 19.39±0.2°, 9.80±0.2°, 11.12±0.2°, and 20.01±0.2°;
preferably, said crystalline form C of the compound of formula IX, its X-ray powder diffraction pattern using Cu-Kα radiation, expressed in terms of 2θ angle, further has diffraction peaks at one or more of the following: 8.07±0.2°, 8.26±0.2°, 9.80±0.2°, 11.12±0.2°, 12.72±0.2°, 15.24±0.2°, 15.48±0.2°, 18.20±0.2°, 18.71±0.2°, 21.52±0.2°, 23.51±0.2°, and 23.83±0.2°;
(24) said crystalline form D of the compound of formula IX, its X-ray powder diffraction pattern using Cu-Kα radiation, expressed in terms of 2θ angle, further has diffraction peaks at one or more of the following: 8.52±0.2°, 12.18±0.2°, 13.12±0.2°, 16.60±0.2°, 20.07±0.2°, and 24.00±0.2°;
for example, said crystalline form D of the compound of formula IX, its X-ray powder diffraction pattern using Cu-Kα radiation, expressed in terms of 2θ angle, has a diffraction peak at 6.13±0.2° with a relative intensity of 80%-100%, preferably the diffraction peak at 6.13±0.2° is the strongest peak;
for example, said crystalline form D of the compound of formula IX, its X-ray powder diffraction pattern using Cu-Kα radiation, expressed in terms of 20 angle, has diffraction peaks at 6.13±0.2°, 10.87±0.2°, 18.38±0.2°, 8.52±0.2°, 12.18±0.2°, 13.12±0.2°, 16.60±0.2°, 20.07±0.2°, and 24.00±0.2°;
preferably, said crystalline form D of the compound of formula IX, its X-ray powder diffraction pattern using Cu-Kα radiation, expressed in terms of 2θ angle, further has diffraction peaks at one or more of the following: 14.25±0.2°, 14.42±0.2°, 17.00±0.2°, 19.09±0.2°, 19.34±0.2°, 21.12±0.2°, 21.28±0.2°, 21.59±0.2°, 21.72±0.2°, 23.14±0.2°, and 24.27±0.2°;
(25) said crystalline form E of the compound of formula IX, its X-ray powder diffraction pattern using Cu-Kα radiation, expressed in terms of 2θ angle, further has diffraction peaks at one or more of the following: 13.85±0.2°, 14.30±0.2°, 17.74±0.2°, 19.04±0.2°, 21.93±0.2°, 22.25±0.2°, 23.08±0.2°, and 23.43±0.2°;
for example, said crystalline form E of the compound of formula IX, its X-ray powder diffraction pattern using Cu-Kα radiation, expressed in terms of 20 angle, has diffraction peaks at 5.66±0.2°, 11.50±0.2°, 17.97±0.2°, 19.83±0.2°, 23.95±0.2°, 13.85±0.2°, 14.30±0.2°, 17.74±0.2°, 19.04±0.2°, and 21.93±0.2°;
preferably, said crystalline form E of the compound of formula IX, its X-ray powder diffraction pattern using Cu-Kα radiation, expressed in terms of 2θ angle, further has diffraction peaks at one or more of the following: 8.45±0.2°, 9.02±0.2°, 9.84±0.2°, 11.19±0.2°, 12.70±0.2°, 15.59±0.2°, 16.56±0.2°, 16.93±0.2°, 18.75±0.2°, 20.55±0.2°, 25.05±0.2°, and 26.98±0.2°;
(26) said crystalline form F of the compound of formula IX, its X-ray powder diffraction pattern using Cu-Kα radiation, expressed in terms of 2θ angle, further has diffraction peaks at one or more of the following: 10.34±0.2°, 15.73±0.2°, 16.34±0.2°, 20.42±0.2°, 20.72±0.2°, 24.34±0.2°, and 25.74±0.2°;
for example, said crystalline form F of the compound of formula IX, its X-ray powder diffraction pattern using Cu-Kα radiation, expressed in terms of 2θ angle, has diffraction peaks at 6.15±0.2°, 16.55±0.2°, 17.30±0.2°, 21.48±0.2°, 10.34±0.2°, 15.73±0.2°, 16.34±0.2°, 20.42±0.2°, and 20.72±0.2°;
preferably, said crystalline form F of the compound of formula IX, its X-ray powder diffraction pattern using Cu-Kα radiation, expressed in terms of 2θ angle, further has diffraction peaks at one or more of the following: 6.99±0.2°, 11.85±0.2°, 12.27±0.2°, 12.57±0.2°, 13.97±0.2°, 18.42±0.2°, 20.98±0.2°, 21.22±0.2°, 21.83±0.2°, 21.97±0.2°, 26.02±0.2°, and 26.83±0.2°;
(27) crystalline form G of the compound of formula IX, its X-ray powder diffraction pattern using Cu-Kα radiation, expressed in terms of 2θ angle, further has diffraction peaks at one or more of the following: 23.83±0.2°, 25.37±0.2°, 25.63±0.2°, and 27.60±0.2°;
for example, crystalline form G of the compound of formula IX, its X-ray powder diffraction pattern using Cu-Kα radiation, expressed in terms of 2θ angle, has diffraction peaks at 7.65±0.2°, 13.76±0.2°, 20.35±0.2°, 21.01±0.2°, 21.57±0.2°, 23.83±0.2°, 25.37±0.2°, 25.63±0.2°, and 27.60±0.2°;
preferably, crystalline form G of the compound of formula IX, its X-ray powder diffraction pattern using Cu-Kα radiation, expressed in terms of 2θ angle, further has diffraction peaks at one or more of the following: 11.87±0.2°, 12.60±0.2°, 12.76±0.2°, 16.07±0.2°, 20.05±0.2°, 22.75±0.2°, and 23.83±0.2°;
(28) said crystalline form H of the compound of formula IX, its X-ray powder diffraction pattern using Cu-Kα radiation, expressed in terms of 2θ angle, further has diffraction peaks at one or more of the following: 10.39±0.2°, 11.07±0.2°, 11.60±0.2°, 12.56±0.2°, 17.07±0.2°, 19.09±0.2°, 21.12±0.2°, 21.46±0.2°, 25.23±0.2°, and 27.27±0.2°;
for example, said crystalline form H of the compound of formula IX, its X-ray powder diffraction pattern using Cu-Kα radiation, expressed in terms of 2θ angle, has diffraction peaks at 8.27±0.2°, 9.70±0.2°, 16.55±0.2°, 19.78±0.2°, 11.07±0.2°, 12.56±0.2°, 17.07±0.2°, 19.09±0.2°, and 21.46±0.2°;
preferably, said crystalline form H of the compound of formula IX, its X-ray powder diffraction pattern using Cu-Kα radiation, expressed in terms of 2θ angle, further has diffraction peaks at one or more of the following: 5.21±0.2°, 11.60±0.2°, 15.29±0.2°, 18.79±0.2°, and 23.36±0.2°;
(29) said crystalline form I of the compound of formula IX, its X-ray powder diffraction pattern using Cu-Kα radiation, expressed in terms of 2θ angle, further has diffraction peaks at one or more of the following: 14.20±0.2°, 16.67±0.2°, 18.00±0.2°, 18.74±0.2°, 20.07±0.2°, and 24.06±0.2°;
for example, said crystalline form I of the compound of formula IX, its X-ray powder diffraction pattern using Cu-Kα radiation, expressed in terms of 2θ angle, has a relative intensity of 80%-100% for the diffraction peak at 6.01±0.2°, preferably the diffraction peak at 6.01±0.2° is the strongest peak;
for example, said crystalline form I of the compound of formula IX, its X-ray powder diffraction pattern using Cu-Kα radiation, expressed in terms of 20 angle, has diffraction peaks at 6.01±0.2°, 10.63±0.2°, 12.79±0.2°, 14.20±0.2°, 16.67±0.2°, 18.00±0.2°, 20.07±0.2°, and 24.06±0.2°;
preferably, said crystalline form I of the compound of formula IX, its X-ray powder diffraction pattern using Cu-Kα radiation, expressed in terms of 2θ angle, further has diffraction peaks at one or more of the following: 11.99±0.2°, 17.84±0.2°, and 23.22±0.2°;
(30) said crystalline form J of the compound of formula IX, its X-ray powder diffraction pattern using Cu-Kα radiation, expressed in terms of 2θ angle, further has diffraction peaks at one or more of the following: 15.30±0.2°, 16.41±0.2°, 18.79±0.2°, 19.07±0.2°, 19.66±0.2°, and 21.25±0.2°.

5. The crystalline form C of the compound of formula I or said nitrogen-containing fused-ring compound according to claim 3, **characterized in that** it satisfies one or more of the following conditions:
(1) said crystalline form A of the compound of formula II, its X-ray powder diffraction pattern using Cu-Kα radiation, expressed in terms of 2θ angle, has diffraction peaks as shown in table 1;
**table 1**
| **peak** | **diffraction Angle (°, 2θ)** | **relative Intensity** | **peak** | **diffraction angle (°, 2θ)** | **relative intensity** |
|---|---|---|---|---|---|
| 1 | 5.98 | 18.40% | 27 | 24.6 | 13.90% |
| 2 | 9.00 | 68.00% | 28 | 25.35 | 3.60% |
| 3 | 10.5 | 6.10% | 29 | 25.62 | 8.20% |
| 4 | 10.84 | 62.30% | 30 | 26.41 | 1.60% |
| 5 | 11.94 | 28.40% | 31 | 27.17 | 6.30% |
| 6 | 13.46 | 4.10% | 32 | 27.72 | 63.30% |
| 7 | 14.16 | 8.50% | 33 | 28.50 | 7.20% |
| 8 | 14.72 | 8.70% | 34 | 28.96 | 12.50% |
| 9 | 15.31 | 1.40% | 35 | 29.18 | 6.80% |
| 10 | 15.46 | 1.60% | 36 | 29.68 | 15.80% |
| 11 | 15.79 | 1.20% | 37 | 30.29 | 9.40% |
| 12 | 17.15 | 2.20% | 38 | 31.08 | 5.90% |
| 13 | 17.91 | 100.00% | 39 | 31.99 | 7.20% |
| 14 | 18.36 | 40.90% | 40 | 32.22 | 7.00% |
| 15 | 19.02 | 2.50% | 41 | 32.57 | 6.60% |
| 16 | 19.99 | 12.90% | 42 | 32.73 | 5.30% |
| 17 | 20.65 | 8.90% | 43 | 33.61 | 1.40% |
| 18 | 21.30 | 40.50% | 44 | 34.17 | 3.90% |
| 19 | 21.71 | 18.00% | 45 | 34.63 | 0.70% |
| 20 | 22.05 | 77.00% | 46 | 35.46 | 0.50% |
| 21 | 22.45 | 23.80% | 47 | 35.82 | 1.70% |
| 22 | 22.63 | 14.80% | 48 | 36.41 | 3.40% |
| 23 | 23.29 | 23.90% | 49 | 37.53 | 4.00% |
| 24 | 23.68 | 4.60% | 50 | 37.96 | 3.90% |
| 25 | 23.96 | 8.40% | 51 | 38.63 | 7.00% |
| 26 | 24.14 | 7.70% | 52 | 39.67 | 1.50% |
preferably, said crystalline form A of the compound of formula II, its X-ray powder diffraction pattern using Cu-Kα radiation is substantially as shown in figure 1;
(2) said crystalline form A of the compound of formula II, its differential scanning calorimetry curve has endothermic peaks at peak temperatures of 98.33±3°C and 206.94±3°C;
preferably, the differential scanning calorimetry curve of said crystalline form A of the compound of formula II is substantially as shown in figure 2;
(3) said crystalline form A of the compound of formula II, its thermogravimetric analysis curve shows a weight loss of about 0.48% in the temperature range from 34.7±3°C to 70.0±3°C, a weight loss of about 2.11 % in the temperature range from 70.0±3°C to 95.0±3°C, a weight loss of about 1.38% in the temperature range from 95.0±3°C to 140.0±3°C, and a weight loss of about 0.34% in the temperature range from 140.0±3°C to 220.0±3°C;
preferably, the thermogravimetric analysis curve of said crystalline form A of the compound of formula II is substantially as shown in figure 3;
(4) in said crystalline form A of the compound of formula II, x' is water, and q is 1;
(5) said crystalline form B of the compound of formula II, its X-ray powder diffraction pattern using Cu-Kα radiation, expressed in terms of 2θ angle, has diffraction peaks as shown in table 2;
**table 2**
| **peak** | **diffraction angle (°, 2θ)** | **relative intensity** | **peak** | **diffraction angle (°, 2θ)** | **relative intensity** |
|---|---|---|---|---|---|
| 1 | 8.10 | 23.20% | 25 | 23.32 | 4.00% |
| 2 | 8.37 | 37.80% | 26 | 23.92 | 18.60% |
| 3 | 10.22 | 9.10% | 27 | 24.20 | 13.60% |
| 4 | 11.46 | 3.80% | 28 | 24.4 | 65.40% |
| 5 | 11.83 | 2.40% | 29 | 24.83 | 100.00% |
| 6 | 13.33 | 26.90% | 30 | 25.42 | 16.90% |
| 7 | 13.94 | 41.50% | 31 | 25.70 | 2.90% |
| 8 | 14.26 | 10.40% | 32 | 26.24 | 2.80% |
| 9 | 14.79 | 17.30% | 33 | 26.82 | 4.20% |
| 10 | 15.67 | 42.90% | 34 | 27.43 | 7.20% |
| 11 | 16.61 | 5.90% | 35 | 27.60 | 9.30% |
| 12 | 17.63 | 17.40% | 36 | 28.04 | 10.20% |
| 13 | 17.91 | 6.40% | 37 | 28.67 | 14.60% |
| 14 | 18.70 | 5.20% | 38 | 29.01 | 7.00% |
| 15 | 19.13 | 9.10% | 39 | 30.1 | 11.20% |
| 16 | 19.62 | 5.40% | 40 | 31.02 | 10.90% |
| 17 | 20.03 | 9.80% | 41 | 32.10 | 6.70% |
| 18 | 20.72 | 53.70% | 42 | 32.72 | 5.60% |
| 19 | 21.15 | 30.20% | 43 | 33.49 | 2.70% |
| 20 | 21.65 | 2.20% | 44 | 33.70 | 2.60% |
| 21 | 21.84 | 6.00% | 45 | 34.38 | 7.30% |
| 22 | 22.14 | 54.20% | 46 | 34.82 | 10.80% |
| 23 | 22.36 | 4.40% | 47 | 36.33 | 1.60% |
| 24 | 23.02 | 3.30% | 48 | 38.57 | 6.30% |
preferably, said crystalline form B of the compound of formula II, its X-ray powder diffraction pattern using Cu-Kα radiation is substantially as shown in figure 4;
(6) in said crystalline form B of the compound of formula II, x' is acetone, and q is 0.56, 0.5, or 0.6, for example, q is 0.56;
(7) said crystalline form C of the compound of formula I, its X-ray powder diffraction pattern using Cu-Kα radiation, expressed in terms of 2θ angle, has diffraction peaks as shown in table 3;
**table 3**
| **peak** | **diffraction angle (°, 2θ)** | **relative intensity** | **peak** | **diffraction angle (°, 2θ)** | **relative intensity** |
|---|---|---|---|---|---|
| 1 | 9.02 | 16.10% | 26 | 28.10 | 2.40% |
| 2 | 10.21 | 11.40% | 27 | 28.60 | 2.30% |
| 3 | 11.25 | 3.90% | 28 | 29.04 | 4.90% |
| 4 | 11.51 | 100.00% | 29 | 29.45 | 24.40% |
| 5 | 12.88 | 28.70% | 30 | 29.49 | 14.70% |
| 6 | 13.61 | 6.60% | 31 | 30.14 | 13.50% |
| 7 | 14.58 | 14.20% | 32 | 30.35 | 12.90% |
| 8 | 15.05 | 22.80% | 33 | 30.68 | 2.00% |
| 9 | 16.16 | 5.70% | 34 | 31.26 | 1.80% |
| 10 | 16.96 | 7.10% | 35 | 31.72 | 1.70% |
| 11 | 17.22 | 5.40% | 36 | 32.56 | 1.90% |
| 12 | 18.01 | 59.10% | 37 | 33.13 | 1.90% |
| 13 | 18.91 | 12.10% | 38 | 34.12 | 4.40% |
| 14 | 19.94 | 1.60% | 39 | 34.87 | 2.40% |
| 15 | 20.48 | 26.60% | 40 | 34.97 | 8.80% |
| 16 | 20.85 | 42.00% | 41 | 35.48 | 5.60% |
| 17 | 21.68 | 32.30% | 42 | 36.05 | 1.90% |
| 18 | 22.56 | 98.50% | 43 | 36.42 | 2.30% |
| 19 | 23.11 | 29.20% | 44 | 36.89 | 1.80% |
| 20 | 24.79 | 31.90% | 45 | 37.06 | 1.90% |
| 21 | 25.68 | 84.80% | 46 | 38.06 | 2.70% |
| 22 | 26.21 | 5.00% | 47 | 38.40 | 3.10% |
| 23 | 26.94 | 15.00% | 48 | 39.22 | 2.80% |
| 24 | 27.26 | 11.20% | 49 | 39.73 | 2.00% |
| 25 | 27.44 | 35.40% | | | |
preferably, said crystalline form C of the compound of formula I, its X-ray powder diffraction pattern using Cu-Kα radiation is substantially as shown in figure 5;
(8) said crystalline form C of the compound of formula I, its differential scanning calorimetry curve has an endothermic peak at a peak temperature of 238.92±3°C;
preferably, the differential scanning calorimetry curve of said crystalline form C of the compound of formula I is substantially as shown in figure 6;
(9) said crystalline form C of the compound of formula I, its thermogravimetric analysis curve shows a weight loss of about 0.81% in the temperature range from 35.56±3°C to 230.00±3°C;
preferably, the thermogravimetric analysis curve of said crystalline form C of the compound of formula I is substantially as shown in figure 7;
(10) said crystalline form C of the compound of formula I is solvent-free;
(11) said crystalline form A of the compound of formula III, its X-ray powder diffraction pattern using Cu-Kα radiation, expressed in terms of 2θ angle, has diffraction peaks as shown in table 4;
**table 4**
| **peak** | **diffraction angle (°, 2θ)** | **relative intensity** | **peak** | **diffraction angle (°, 2θ)** | **relative intensity** |
|---|---|---|---|---|---|
| 1 | 4.61 | 100.00% | 18 | 19.13 | 6.10% |
| 2 | 5.02 | 34.20% | 19 | 19.30 | 8.20% |
| 3 | 6.57 | 21.10% | 20 | 19.96 | 2.40% |
| 4 | 6.89 | 14.10% | 21 | 20.81 | 6.40% |
| 5 | 9.19 | 70.70% | 22 | 21.68 | 8.00% |
| 6 | 9.97 | 16.20% | 23 | 23.09 | 5.90% |
| 7 | 10.48 | 8.90% | 24 | 24.03 | 11.90% |
| 8 | 10.64 | 8.30% | 25 | 24.50 | 5.70% |
| 9 | 11.12 | 2.40% | 26 | 25.14 | 4.20% |
| 10 | 12.19 | 2.00% | 27 | 25.56 | 7.00% |
| 11 | 13.76 | 62.30% | 28 | 26.13 | 14.40% |
| 12 | 14.69 | 9.60% | 29 | 27.54 | 1.40% |
| 13 | 14.90 | 14.80% | 30 | 28.17 | 7.20% |
| 14 | 15.95 | 1.80% | 31 | 31.80 | 3.10% |
| 15 | 17.09 | 1.20% | 32 | 34.16 | 1.90% |
| 16 | 18.10 | 4.20% | 33 | 35.40 | 1.80% |
| 17 | 18.34 | 9.10% | 34 | 36.04 | 2.20% |
preferably, said crystalline form A of the compound of formula III, its X-ray powder diffraction pattern using Cu-Kα radiation is substantially as shown in figure 8;
(12) in said crystalline form A of the compound of formula III, w is 1, 1.1, or 1.06, for example 1.06;
(13) in said crystalline form A of the compound of formula III, e is 0 or 1.5;
(14) said crystalline form B of the compound of formula III, its X-ray powder diffraction pattern using Cu-Kα radiation, expressed in terms of 2θ angle, has diffraction peaks as shown in table 5;
**table 5**
| **peak** | **diffraction angle (°, 2θ)** | **relative intensity** | **peak** | **diffraction angle (°, 2θ)** | **relative intensity** |
|---|---|---|---|---|---|
| 1 | 6.47 | 32.50% | 34 | 26.96 | 13.30% |
| 2 | 7.19 | 18.80% | 35 | 27.15 | 14.10% |
| 3 | 9.11 | 13.90% | 36 | 27.47 | 4.90% |
| 4 | 11.63 | 19.40% | 37 | 27.96 | 40.00% |
| 5 | 12.94 | 7.20% | 38 | 28.62 | 44.60% |
| 6 | 13.41 | 8.00% | 39 | 29.00 | 7.10% |
| 7 | 14.45 | 71.00% | 40 | 29.16 | 8.10% |
| 8 | 15.41 | 9.70% | 41 | 29.39 | 4.40% |
| 9 | 15.75 | 10.40% | 42 | 30.04 | 15.60% |
| 10 | 16.23 | 15.50% | 43 | 30.37 | 29.10% |
| 11 | 16.43 | 10.70% | 44 | 30.63 | 6.10% |
| 12 | 16.72 | 13.20% | 45 | 31.00 | 16.20% |
| 13 | 17.37 | 49.40% | 46 | 31.42 | 5.90% |
| 14 | 18.21 | 51.20% | 47 | 31.87 | 4.20% |
| 15 | 18.82 | 3.70% | 48 | 32.20 | 25.10% |
| 16 | 19.44 | 47.20% | 49 | 32.64 | 11.10% |
| 17 | 20.17 | 51.30% | 50 | 32.92 | 10.10% |
| 18 | 20.50 | 23.70% | 51 | 33.64 | 1.30% |
| 19 | 20.63 | 32.20% | 52 | 34.05 | 13.20% |
| 20 | 20.82 | 100.00% | 53 | 34.44 | 2.00% |
| 21 | 21.66 | 4.60% | 54 | 35.03 | 2.90% |
| 22 | 22.22 | 2.30% | 55 | 35.66 | 5.10% |
| 23 | 22.41 | 31.50% | 56 | 36.07 | 4.60% |
| 24 | 23.01 | 12.10% | 57 | 36.29 | 6.50% |
| 25 | 23.45 | 2.80% | 58 | 36.49 | 5.50% |
| 26 | 23.63 | 6.90% | 59 | 37.03 | 6.20% |
| 27 | 23.81 | 2.50% | 60 | 37.34 | 2.70% |
| 28 | 24.58 | 83.80% | 61 | 38.15 | 3.90% |
| 29 | 25.33 | 9.00% | 62 | 38.45 | 5.20% |
| 30 | 25.84 | 20.50% | 63 | 39.01 | 2.50% |
| 31 | 26.01 | 39.10% | 64 | 39.26 | 5.20% |
| 32 | 26.17 | 27.40% | 65 | 39.81 | 5.90% |
| 33 | 26.47 | 47.20% | | | |
preferably, said crystalline form B of the compound of formula III, its X-ray powder diffraction pattern using Cu-Kα radiation is substantially as shown in figure 9;
(15) said crystalline form B of the compound of formula III, its differential scanning calorimetry curve has an endothermic peak at a peak temperature of 186.88±3°C;
preferably, the differential scanning calorimetry curve of said crystalline form B of the compound of formula III is substantially as shown in figure 10;
(16) said crystalline form B of the compound of formula III, its thermogravimetric analysis curve shows a weight loss of about 1.42% in the temperature range from 24.07±3°C to 120.0±3°C, and a weight loss of about 9.84% in the temperature range from 120.00±3°C to 205.00±3°C;
preferably, the thermogravimetric analysis curve of said crystalline form B of the compound of formula III is substantially as shown in figure 11;
(17) in said crystalline form B of the compound of formula III, w is 1.76, 1.7, or 1.8, for example 1.76;
(18) in said crystalline form B of the compound of formula III, e is 0;
(19) said crystalline form C of the compound of formula III, its X-ray powder diffraction pattern using Cu-Kα radiation, expressed in terms of 2θ angle, has diffraction peaks as shown in table 6;
**table 6**
| **peak** | **diffraction angle (°, 2θ)** | **relative intensity** | **peak** | **diffraction angle (°, 2θ)** | **relative intensity** |
|---|---|---|---|---|---|
| 1 | 9.84 | 22.30% | 21 | 26.94 | 17.80% |
| 2 | 10.51 | 14.40% | 22 | 27.48 | 51.00% |
| 3 | 11.61 | 12.00% | 23 | 28.37 | 25.40% |
| 4 | 12.19 | 61.90% | 24 | 28.97 | 25.90% |
| 5 | 13.39 | 33.30% | 25 | 29.32 | 26.90% |
| 6 | 14.40 | 24.90% | 26 | 29.95 | 6.50% |
| 7 | 17.35 | 10.30% | 27 | 30.42 | 15.70% |
| 8 | 18.16 | 86.70% | 28 | 31.25 | 11.50% |
| 9 | 18.29 | 78.90% | 29 | 31.60 | 5.70% |
| 10 | 19.39 | 53.40% | 30 | 32.24 | 11.00% |
| 11 | 20.02 | 15.90% | 31 | 32.62 | 9.60% |
| 12 | 20.81 | 37.40% | 32 | 33.45 | 3.30% |
| 13 | 21.75 | 64.40% | 33 | 34.04 | 6.10% |
| 14 | 22.51 | 33.80% | 34 | 34.68 | 7.70% |
| 15 | 22.90 | 13.30% | 35 | 35.30 | 10.10% |
| 16 | 23.25 | 34.10% | 36 | 36.16 | 13.90% |
| 17 | 23.49 | 29.40% | 37 | 36.57 | 4.00% |
| 18 | 24.46 | 12.20% | 38 | 37.90 | 6.30% |
| 19 | 25.11 | 16.90% | 39 | 38.47 | 7.40% |
| 20 | 26.41 | 100.00% | | | |
preferably, said crystalline form C of the compound of formula III, its X-ray powder diffraction pattern using Cu-Kα radiation is substantially as shown in figure 12;
(20) in said crystalline form C of the compound of formula III, w is 1.7, 1.8, or 1.6, for example 1.7;
(21) said crystalline form A of the compound of formula IV, its X-ray powder diffraction pattern using Cu-Kα radiation, expressed in terms of 20 angle, has diffraction peaks as shown in table 7;
**table 7**
| **peak** | **diffraction angle (°, 2θ)** | **relative intensity** | **peak** | **diffraction angle (°, 2θ)** | **relative intensity** |
|---|---|---|---|---|---|
| 1 | 5.14 | 60.20% | 12 | 20.40 | 33.50% |
| 2 | 6.11 | 16.10% | 13 | 21.45 | 33.20% |
| 3 | 6.60 | 14.80% | 14 | 21.78 | 58.50% |
| 4 | 7.35 | 20.40% | 15 | 23.81 | 40.20% |
| 5 | 8.63 | 23.70% | 16 | 24.82 | 67.70% |
| 6 | 9.01 | 100.00% | 17 | 25.47 | 51.50% |
| 7 | 11.45 | 39.40% | 18 | 25.95 | 49.20% |
| 8 | 14.62 | 18.30% | 19 | 26.68 | 54.90% |
| 9 | 15.03 | 35.20% | 20 | 27.96 | 6.00% |
| 10 | 16.45 | 17.10% | 21 | 28.43 | 29.90% |
| 11 | 17.44 | 33.50% | | | |
; preferably, said crystalline form A of the compound of formula IV, its X-ray powder diffraction pattern using Cu-Kα radiation is substantially as shown in figure 13;
(22) in said crystalline form A of the compound of formula IV, r is 1, 0.97, or 0.9, for example 0.97;
(23) in said crystalline form A of the compound of formula IV, t is 0;
(24) said crystalline form B of the compound of formula IV, its X-ray powder diffraction pattern using Cu-Kα radiation, expressed in terms of 2θ angle, has diffraction peaks as shown in table 8;
**table 8**
| **peak** | **diffraction angle (°, 2θ)** | **relative intensity** | **peak** | **diffraction angle (°, 2θ)** | **relative intensity** |
|---|---|---|---|---|---|
| 1 | 6.87 | 9.90% | 35 | 21.52 | 8.90% |
| 2 | 7.11 | 22.80% | 36 | 21.98 | 11.20% |
| 3 | 7.57 | 24.00% | 37 | 22.20 | 20.90% |
| 4 | 7.72 | 24.70% | 38 | 22.51 | 14.50% |
| 5 | 8.10 | 26.40% | 39 | 22.86 | 8.00% |
| 6 | 9.96 | 5.10% | 40 | 23.18 | 67.20% |
| 7 | 10.27 | 16.20% | 41 | 23.62 | 11.60% |
| 8 | 11.20 | 2.00% | 42 | 24.18 | 94.90% |
| 9 | 11.55 | 2.30% | 43 | 24.74 | 12.60% |
| 10 | 11.86 | 2.80% | 44 | 25.17 | 100.00% |
| 11 | 12.55 | 13.30% | 45 | 25.58 | 31.60% |
| 12 | 12.67 | 14.40% | 46 | 26.00 | 14.30% |
| 13 | 13.19 | 12.00% | 47 | 26.39 | 4.40% |
| 14 | 13.49 | 8.90% | 48 | 26.80 | 13.60% |
| 15 | 13.86 | 27.20% | 49 | 27.16 | 18.40% |
| 16 | 14.11 | 55.00% | 50 | 27.40 | 14.20% |
| 17 | 14.71 | 63.20% | 51 | 27.81 | 41.70% |
| 18 | 15.44 | 21.70% | 52 | 28.09 | 14.80% |
| 19 | 16.07 | 43.00% | 53 | 28.58 | 5.40% |
| 20 | 16.45 | 5.20% | 54 | 29.49 | 14.30% |
| 21 | 16.73 | 18.90% | 55 | 29.89 | 16.50% |
| 22 | 17.01 | 4.60% | 56 | 30.12 | 13.30% |
| 23 | 17.55 | 4.20% | 57 | 31.45 | 8.00% |
| 24 | 17.92 | 19.00% | 58 | 32.04 | 14.30% |
| 25 | 18.38 | 46.30% | 59 | 32.83 | 4.20% |
| 26 | 18.58 | 44.20% | 60 | 33.66 | 2.50% |
| 27 | 19.10 | 8.20% | 61 | 34.52 | 4.60% |
| 28 | 19.46 | 15.30% | 62 | 35.43 | 2.70% |
| 29 | 19.80 | 12.50% | 63 | 35.85 | 2.10% |
| 30 | 20.18 | 28.90% | 64 | 36.35 | 3.90% |
| 31 | 20.39 | 11.90% | 65 | 36.70 | 7.70% |
| 32 | 20.59 | 15.90% | 66 | 38.31 | 6.40% |
| 33 | 20.82 | 11.70% | 67 | 38.66 | 3.80% |
| 34 | 21.21 | 27.10% | 68 | 39.47 | 4.30% |
preferably, said crystalline form B of the compound of formula IV, its X-ray powder diffraction pattern using Cu-Kα radiation is substantially as shown in figure 14;
(25) said crystalline form B of the compound of formula IV, its differential scanning calorimetry curve has endothermic peaks at peak temperatures of 34.85±3°C, 90.79±3°C, and 204.38±3°C;
preferably, the differential scanning calorimetry curve of said crystalline form B of the compound of formula IV is substantially as shown in figure 15;
(26) said crystalline form B of the compound of formula IV, its thermogravimetric analysis curve shows a weight loss of about 2.06% in the temperature range from 33.44±3°C to 50.0±3°C, a weight loss of about 0.56% in the temperature range from 50.0±3°C to 100.0±3°C, a weight loss of about 0.39% in the temperature range from 100.00±3°C to 180.00±3°C, and a weight loss of about 1% in the temperature range from 180.00±3°C to 230.00±3°C;
Preferably, the thermogravimetric analysis curve of said crystalline form B of the compound of formula IV is substantially as shown in figure 16;
(27) in said crystalline form B of the compound of formula IV, r is 1, 0.9, or 0.96, for example 0.96;
(28) in said crystalline form B of the compound of formula IV, t is 0 or 1.7;
preferably, in said crystalline form B of the compound of formula IV, r is 1 and t is 0; or, r is 0.96 and t is 0;
(29) said crystalline form A of the compound of formula V, its X-ray powder diffraction pattern using Cu-Kα radiation, expressed in terms of 2θ angle, has diffraction peaks as shown in table 9;
**table 9**
| **peak** | **diffraction angle (°, 2θ)** | **relative intensity** | **peak** | **diffraction angle (°, 2θ)** | **relative intensity** |
|---|---|---|---|---|---|
| 1 | 5.15 | 24.70% | 35 | 23.24 | 33.70% |
| 2 | 5.98 | 8.10% | 36 | 23.63 | 23.60% |
| 3 | 7.97 | 16.00% | 37 | 23.98 | 58.60% |
| 4 | 8.34 | 44.50% | 38 | 24.1 | 43.00% |
| 5 | 8.96 | 21.40% | 39 | 24.47 | 7.40% |
| 6 | 10.49 | 33.70% | 40 | 24.88 | 14.30% |
| 7 | 10.80 | 26.70% | 41 | 25.13 | 39.10% |
| 8 | 11.34 | 32.40% | 42 | 25.54 | 5.60% |
| 9 | 11.66 | 24.10% | 43 | 25.83 | 6.90% |
| 10 | 11.99 | 21.90% | 44 | 26.18 | 13.30% |
| 11 | 12.73 | 15.30% | 45 | 26.44 | 10.80% |
| 12 | 13.15 | 7.00% | 46 | 26.83 | 11.90% |
| 13 | 13.46 | -0.10% | 47 | 27.28 | 20.30% |
| 14 | 14.07 | 2.80% | 48 | 27.73 | 100.00% |
| 15 | 14.61 | 1.40% | 49 | 28.44 | 9.60% |
| 16 | 14.84 | 5.00% | 50 | 28.63 | 11.10% |
| 17 | 15.51 | 38.40% | 51 | 28.94 | 22.30% |
| 18 | 15.88 | 44.10% | 52 | 29.17 | 18.40% |
| 19 | 16.68 | 6.50% | 53 | 29.59 | 5.80% |
| 20 | 17.26 | 4.00% | 54 | 30.38 | 7.70% |
| 21 | 17.93 | 75.20% | 55 | 31.27 | 5.70% |
| 22 | 18.39 | 84.80% | 56 | 31.76 | 8.30% |
| 23 | 18.68 | 37.50% | 57 | 32.11 | 11.50% |
| 24 | 19.06 | 11.40% | 58 | 32.72 | 5.20% |
| 25 | 19.73 | 38.50% | 59 | 32.97 | 4.70% |
| 26 | 20.14 | 31.90% | 60 | 33.52 | 8.90% |
| 27 | 20.52 | 12.40% | 61 | 33.93 | 6.50% |
| 28 | 20.76 | 14.20% | 62 | 34.60 | 3.10% |
| 29 | 21.05 | 29.80% | 63 | 35.44 | 7.30% |
| 30 | 21.28 | 26.00% | 64 | 36.13 | 2.10% |
| 31 | 21.64 | 22.90% | 65 | 37.14 | 4.10% |
| 32 | 21.99 | 88.50% | 66 | 37.70 | 6.90% |
| 33 | 22.51 | 13.30% | 67 | 38.63 | 6.10% |
| 34 | 22.76 | 42.90% | 68 | 39.60 | 2.50% |
preferably, said crystalline form A of the compound of formula V, its X-ray powder diffraction pattern using Cu-Kα radiation is substantially as shown in figure 17;
(30) said crystalline form A of the compound of formula V, its differential scanning calorimetry curve has endothermic peaks at peak temperatures of 88.96±3°C and 192.67±3°C;
preferably, the differential scanning calorimetry curve of said crystalline form A of the compound of formula V is substantially as shown in figure 18;
(31) said crystalline form A of the compound of formula V, its thermogravimetric analysis curve shows a weight loss of about 0.92% in the temperature range from 32.34±3°C to 70.0±3°C, and a weight loss of about 2.1% in the temperature range from 70.0±3°C to 180.0±3°C;
preferably, the thermogravimetric analysis curve of said crystalline form A of the compound of formula V is substantially as shown in figure 19;
(32) in said crystalline form A of the compound of formula V, y is 1.1, 1.2, or 1.14, for example 1.14;
preferably, in said crystalline form A of the compound of formula V, i is 0, y is 1.1, u is 0.9; or, i is 0, y is 1.14, u is 0.91;
(33) said crystalline form B of the compound of formula V, its X-ray powder diffraction pattern using Cu-Kα radiation, expressed in terms of 2θ angle, has diffraction peaks as shown in table 10;
**table 10**
| **peak** | **diffraction angle (°, 2θ)** | **relative intensity** | **peak** | **diffraction angle (°, 2θ)** | **relative intensity** |
|---|---|---|---|---|---|
| 1 | 3.41 | 5.60% | 25 | 21.65 | 22.50% |
| 2 | 6.83 | 38.40% | 26 | 22.05 | 20.10% |
| 3 | 7.27 | 6.10% | 27 | 22.31 | 15.20% |
| 4 | 8.32 | 22.40% | 28 | 22.83 | 13.30% |
| 5 | 8.52 | 17.60% | 29 | 23.01 | 16.40% |
| 6 | 9.17 | 12.30% | 30 | 23.56 | 24.00% |
| 7 | 10.26 | 21.70% | 31 | 23.98 | 25.70% |
| 8 | 11.72 | 8.60% | 32 | 24.68 | 19.70% |
| 9 | 12.89 | 100.00% | 33 | 25.08 | 49.20% |
| 10 | 13.46 | 16.40% | 34 | 25.50 | 59.20% |
| 11 | 13.76 | 7.80% | 35 | 25.92 | 9.70% |
| 12 | 13.93 | 20.10% | 36 | 26.58 | 2.90% |
| 13 | 14.44 | 51.20% | 37 | 27.08 | 9.50% |
| 14 | 14.67 | 21.10% | 38 | 27.63 | 8.30% |
| 15 | 15.91 | 47.80% | 39 | 28.15 | 24.60% |
| 16 | 16.25 | 23.60% | 40 | 29.06 | 17.10% |
| 17 | 16.61 | 10.10% | 41 | 31.52 | 8.10% |
| 18 | 17.10 | 7.60% | 42 | 31.96 | -3.30% |
| 19 | 17.94 | 14.80% | 43 | 32.73 | 11.80% |
| 20 | 19.20 | 3.90% | 44 | 34.25 | 9.60% |
| 21 | 20.00 | 8.00% | 45 | 35.91 | 7.00% |
| 22 | 20.35 | 50.60% | 46 | 37.15 | 2.40% |
| 23 | 20.49 | 48.30% | 47 | 38.73 | 12.90% |
| 24 | 21.15 | 19.40% | | | |
preferably, said crystalline form B of the compound of formula V, its X-ray powder diffraction pattern using Cu-Kα radiation is substantially as shown in figure 20;
(34) in said crystalline form B of the compound of formula V, y is 1.1, 1, or 1.09, for example 1.09;
(35) in said crystalline form B of the compound of formula V, u and i are 0;
(36) said crystalline form C of the compound of formula V, its X-ray powder diffraction pattern using Cu-Kα radiation, expressed in terms of 2θ angle, has diffraction peaks as shown in table 11;
**table 11**
| **peak** | **diffraction angle (°, 2θ)** | **relative intensity** | **peak** | **diffraction angle (°, 2θ)** | **relative intensity** |
|---|---|---|---|---|---|
| 1 | 5.19 | 49.10% | 41 | 24.56 | 2.70% |
| 2 | 7.95 | 4.30% | 42 | 24.80 | 30.20% |
| 3 | 8.39 | 51.00% | 43 | 25.27 | 28.10% |
| 4 | 9.03 | 9.70% | 44 | 25.67 | 60.90% |
| 5 | 9.44 | 0.90% | 45 | 26.00 | 4.60% |
| 6 | 10.22 | 8.60% | 46 | 26.19 | 18.70% |
| 7 | 10.47 | 48.10% | 47 | 26.42 | 11.90% |
| 8 | 11.19 | 16.20% | 48 | 26.94 | 12.40% |
| 9 | 11.51 | 100.00% | 49 | 27.19 | 13.70% |
| 10 | 11.67 | 23.90% | 50 | 27.47 | 39.90% |
| 11 | 12.73 | 21.10% | 51 | 28.11 | 7.00% |
| 12 | 12.89 | 17.90% | 52 | 28.43 | 2.50% |
| 13 | 13.14 | 5.10% | 53 | 28.73 | 8.90% |
| 14 | 13.62 | 3.90% | 54 | 29.08 | 13.50% |
| 15 | 14.59 | 12.80% | 55 | 29.41 | 33.20% |
| 16 | 15.05 | 13.40% | 56 | 30.12 | 10.90% |
| 17 | 15.68 | 33.50% | 57 | 30.33 | 9.90% |
| 18 | 15.87 | 26.00% | 58 | 30.53 | 3.90% |
| 19 | 16.16 | 5.20% | 59 | 31.260 | 2.00% |
| 20 | 16.79 | 9.60% | 60 | 31.67 | 9.10% |
| 21 | 16.94 | 7.20% | 61 | 32.16 | 2.40% |
| 22 | 17.24 | 3.60% | 62 | 32.54 | 3.70% |
| 23 | 17.57 | 3.80% | 63 | 32.70 | 2.80% |
| 24 | 17.90 | 25.20% | 64 | 33.02 | 3.50% |
| 25 | 18.01 | 56.50% | 65 | 33.44 | 7.20% |
| 26 | 18.39 | 10.90% | 66 | 33.93 | 6.00% |
| 27 | 18.76 | 34.70% | 67 | 34.27 | 3.70% |
| 28 | 19.09 | 7.00% | 68 | 34.75 | 3.40% |
| 29 | 19.81 | 32.70% | 69 | 34.98 | 9.70% |
| 30 | 20.48 | 32.90% | 70 | 35.47 | 8.40% |
| 31 | 20.84 | 28.10% | 71 | 35.73 | 3.10% |
| 32 | 21.68 | 29.00% | 72 | 36.39 | 4.20% |
| 33 | 22.10 | 32.30% | 73 | 36.89 | 1.20% |
| 34 | 22.55 | 79.40% | 74 | 37.39 | 6.80% |
| 35 | 23.10 | 34.60% | 75 | 37.96 | 4.20% |
| 36 | 23.23 | 25.20% | 76 | 38.42 | 3.70% |
| 37 | 23.49 | 9.60% | 77 | 38.71 | 4.50% |
| 38 | 23.70 | 12.70% | 78 | 39.16 | 6.40% |
| 39 | 23.94 | 27.10% | 79 | 39.66 | 3.30% |
| 40 | 24.15 | 30.10% | | | |
preferably, said crystalline form C of the compound of formula V, its X-ray powder diffraction pattern using Cu-Kα radiation is substantially as shown in figure 21;
(37) said crystalline form C of the compound of formula V, its differential scanning calorimetry curve has endothermic peaks at peak temperatures of 188.32±3°C and 221.66±3°C;
preferably, the differential scanning calorimetry curve of said crystalline form C of the compound of formula V is substantially as shown in figure 22;
(38) said crystalline form C of the compound of formula V, its thermogravimetric analysis curve shows a weight loss of about 0.71% in the temperature range from 29.55±3°C to 180.0±3°C, and a weight loss of about 0.85% in the temperature range from 180.0±3°C to 220.0±3°C;
preferably, the thermogravimetric analysis curve of said crystalline form C of the compound of formula V is substantially as shown in figure 23;
(39) in said crystalline form C of the compound of formula V, y is 1.1, 1, or 1.05, for example 1.05;
preferably, in said crystalline form C of the compound of formula V, u and i are 0, y is 1;or, u is 0, i is 0.1, y is 1.05;
(40) said crystalline form A of the compound of formula VI, its X-ray powder diffraction pattern using Cu-Kα radiation, expressed in terms of 2θ angle, has diffraction peaks as shown in table 12;
**table 12**
| **peak** | **diffraction angle (°, 2θ)** | **relative intensity** | **peak** | **diffraction angle (°, 2θ)** | **relative intensity** |
|---|---|---|---|---|---|
| 1 | 5.53 | 16.80% | 24 | 19.86 | 7.80% |
| 2 | 6.73 | 20.90% | 25 | 20.60 | 15.60% |
| 3 | 6.96 | 47.50% | 26 | 21.27 | 14.80% |
| 4 | 7.74 | 9.70% | 27 | 21.76 | 11.20% |
| 5 | 8.60 | 42.10% | 28 | 22.35 | 13.80% |
| 6 | 9.38 | 14.10% | 29 | 22.55 | 32.30% |
| 7 | 9.91 | 9.40% | 30 | 22.76 | 21.80% |
| 8 | 10.28 | 21.00% | 31 | 23.38 | 39.60% |
| 9 | 10.58 | 90.90% | 32 | 23.95 | 11.20% |
| 10 | 12.16 | 53.50% | 33 | 24.52 | 9.20% |
| 11 | 13.45 | 21.50% | 34 | 25.15 | 25.40% |
| 12 | 13.74 | 12.60% | 35 | 25.71 | 40.00% |
| 13 | 13.98 | 100.00% | 36 | 27.37 | 13.20% |
| 14 | 14.84 | 42.10% | 37 | 27.68 | 12.40% |
| 15 | 15.39 | 43.10% | 38 | 28.84 | 16.60% |
| 16 | 15.73 | 7.20% | 39 | 29.91 | 23.30% |
| 17 | 16.63 | 35.60% | 40 | 31.77 | 11.50% |
| 18 | 17.08 | 52.80% | 41 | 32.66 | 10.20% |
| 19 | 17.37 | 5.40% | 42 | 34.77 | 16.80% |
| 20 | 18.67 | 21.60% | 43 | 35.23 | 6.90% |
| 21 | 18.97 | 20.90% | 44 | 37.12 | 3.80% |
| 22 | 19.16 | 26.60% | 45 | 38.12 | 8.40% |
| 23 | 19.28 | 34.30% | | | |
preferably, said crystalline form A of the compound of formula VI, its X-ray powder diffraction pattern using Cu-Kα radiation is substantially as shown in figure 24;
(41) in said crystalline form A of the compound of formula VI, o is 1, 1.1, or 1.01, for example 1.01;
(42) said crystalline form A of the compound of formula VII, its X-ray powder diffraction pattern using Cu-Kα radiation, expressed in terms of 2θ angle, has diffraction peaks as shown in table 13;
**table 13**
| **peak** | **diffraction angle (°, 2θ)** | **relative intensity** | **peak** | **diffraction angle (°, 2θ)** | **relative intensity** |
|---|---|---|---|---|---|
| 1 | 5.13 | 16.30% | 16 | 21.16 | 9.00% |
| 2 | 6.73 | 13.80% | 17 | 21.74 | 49.70% |
| 3 | 8.61 | 32.10% | 18 | 22.37 | 12.10% |
| 4 | 9.00 | 100.00% | 19 | 23.18 | 6.10% |
| 5 | 10.05 | 9.80% | 20 | 23.71 | 7.80% |
| 6 | 10.34 | 6.50% | 21 | 24.43 | 18.70% |
| 7 | 11.17 | 4.40% | 22 | 25.32 | 31.50% |
| 8 | 11.57 | 9.50% | 23 | 26.36 | 12.50% |
| 9 | 13.93 | 5.60% | 24 | 27.29 | 7.90% |
| 10 | 15.06 | 7.50% | 25 | 27.93 | 11.90% |
| 11 | 15.55 | 13.90% | 26 | 28.33 | 13.10% |
| 12 | 17.44 | 58.10% | 27 | 29.14 | 10.00% |
| 13 | 17.74 | 18.10% | 28 | 29.86 | 8.70% |
| 14 | 18.62 | 8.90% | 29 | 31.07 | 1.70% |
| 15 | 20.08 | 3.30% | | | |
preferably, said crystalline form A of the compound of formula VII, its X-ray powder diffraction pattern using Cu-Kα radiation is substantially as shown in figure 25;
(43) in said crystalline form A of the compound of formula VII, p is 1:1, 1:1.1, or 1:1.03, for example 1:1.03;
(44) said crystalline form A of the compound of formula VIII, its X-ray powder diffraction pattern using Cu-Kα radiation, expressed in terms of 2θ angle, has diffraction peaks as shown in table 14;
**table 14**
| **peak** | **diffraction angle (°, 2θ)** | **relative intensity** | **peak** | **diffraction angle (°, 2θ)** | **relative intensity** |
|---|---|---|---|---|---|
| 1 | 6.48 | 14.30% | 22 | 20.35 | 19.20% |
| 2 | 7.66 | 100.00% | 23 | 20.89 | 20.10% |
| 3 | 9.06 | 21.40% | 24 | 21.10 | 9.60% |
| 4 | 9.72 | 20.80% | 25 | 21.68 | 31.10% |
| 5 | 10.09 | 45.10% | 26 | 22.27 | 24.30% |
| 6 | 11.10 | 18.60% | 27 | 22.49 | 19.60% |
| 7 | 11.92 | 44.30% | 28 | 22.97 | 28.30% |
| 8 | 12.36 | 10.80% | 29 | 23.50 | 22.00% |
| 9 | 12.98 | 9.10% | 30 | 24.84 | 32.30% |
| 10 | 13.91 | 79.00% | 31 | 25.52 | 42.40% |
| 11 | 14.42 | 10.20% | 32 | 25.97 | 18.20% |
| 12 | 15.14 | 6.30% | 33 | 26.70 | 10.20% |
| 13 | 15.91 | 19.50% | 34 | 27.34 | 8.50% |
| 14 | 16.55 | 20.20% | 35 | 28.19 | 8.50% |
| 15 | 17.04 | 9.40% | 36 | 29.36 | 20.20% |
| 16 | 17.60 | 12.70% | 37 | 30.28 | 10.90% |
| 17 | 18.00 | 18.50% | 38 | 30.81 | 10.10% |
| 18 | 18.27 | 41.70% | 39 | 31.77 | 5.80% |
| 19 | 18.51 | 58.50% | 40 | 32.97 | 9.50% |
| 20 | 19.16 | 50.10% | 41 | 34.37 | 11.60% |
| 21 | 19.72 | 11.20% | | | |
preferably, said crystalline form A of the compound of formula VIII, its X-ray powder diffraction pattern using Cu-Kα radiation is substantially as shown in figure 26;
(45) in said crystalline form A of the compound of formula VIII, a is 0, 0.1, or 0.07, for example 0.07;
(46) said crystalline form A of the compound of formula IX, its X-ray powder diffraction pattern using Cu-Kα radiation, expressed in terms of 2θ angle, has diffraction peaks as shown in table 15;
**table 15**
| **peak** | **diffraction angle (°, 2θ)** | **relative intensity** | **peak** | **diffraction angle (°, 2θ)** | **relative intensity** |
|---|---|---|---|---|---|
| 1 | 5.96 | 57.90% | 39 | 25.98 | 27.10% |
| 2 | 8.20 | 4.10% | 40 | 26.26 | 6.70% |
| 3 | 8.37 | 5.70% | 41 | 26.66 | 6.70% |
| 4 | 9.93 | 10.20% | 42 | 27.35 | 21.60% |
| 5 | 10.13 | 8.40% | 43 | 27.52 | 30.80% |
| 6 | 10.57 | 36.50% | 44 | 27.79 | 7.70% |
| 7 | 11.03 | 14.20% | 45 | 28.08 | 31.60% |
| 8 | 11.88 | 14.90% | 46 | 28.30 | 10.80% |
| 9 | 12.34 | 33.30% | 47 | 28.97 | 15.00% |
| 10 | 12.77 | 24.90% | 48 | 29.17 | 39.40% |
| 11 | 13.42 | 5.20% | 49 | 29.35 | 12.90% |
| 12 | 14.06 | 32.00% | 50 | 29.92 | 7.40% |
| 13 | 14.40 | 28.30% | 51 | 30.36 | 40.50% |
| 14 | 14.97 | 29.20% | 52 | 31.21 | 6.60% |
| 15 | 15.68 | 2.80% | 53 | 31.65 | 5.50% |
| 16 | 15.85 | 1.80% | 54 | 31.83 | 16.00% |
| 17 | 16.71 | 100.00% | 55 | 31.96 | 37.80% |
| 18 | 17.56 | 29.70% | 56 | 32.62 | 2.80% |
| 19 | 17.79 | 73.50% | 57 | 32.95 | 11.80% |
| 20 | 18.41 | 31.00% | 58 | 33.39 | 6.40% |
| 21 | 18.70 | 15.60% | 59 | 33.72 | 3.40% |
| 22 | 19.19 | 5.90% | 60 | 34.17 | 10.50% |
| 23 | 19.67 | 13.00% | 61 | 34.59 | 8.40% |
| 24 | 19.88 | 43.60% | 62 | 35.19 | 3.00% |
| 25 | 20.33 | 57.50% | 63 | 35.19 | 3.00% |
| 26 | 20.68 | 15.20% | 64 | 35.48 | 3.80% |
| 27 | 21.20 | 29.10% | 65 | 35.91 | 5.40% |
| 28 | 21.49 | 2.70% | 66 | 36.12 | 4.50% |
| 29 | 22.11 | 32.50% | 67 | 36.35 | 9.70% |
| 30 | 22.64 | 45.20% | 68 | 36.98 | 2.20% |
| 31 | 23.18 | 32.40% | 69 | 37.41 | 6.20% |
| 32 | 23.86 | 2.20% | 70 | 37.62 | 10.10% |
| 33 | 24.22 | 46.00% | 71 | 37.83 | 4.30% |
| 34 | 24.66 | 72.50% | 72 | 38.32 | 3.70% |
| 35 | 25.15 | 6.60% | 73 | 38.88 | 7.90% |
| 36 | 25.43 | 32.70% | 74 | 39.02 | 5.20% |
| 37 | 25.65 | 8.80% | 75 | 39.54 | 5.00% |
| 38 | 25.84 | 13.40% | | | |
preferably, said crystalline form A of the compound of formula IX, its X-ray powder diffraction pattern using Cu-Kα radiation is substantially as shown in figure 27;
(47) said crystalline form A of the compound of formula IX, its differential scanning calorimetry curve has endothermic peaks at peak temperatures of 95.83±3°C and 194.7±3°C;
preferably, the differential scanning calorimetry curve of said crystalline form A of the compound of formula IX is substantially as shown in figure 28;
(48) said crystalline form A of the compound of formula IX, its thermogravimetric analysis curve shows a weight loss of about 2.03% in the temperature range from 30.52±3°C to 60.0±3°C, a weight loss of about 5.13% in the temperature range from 60.0±3°C to 100.0±3°C, and a weight loss of about 0.5% in the temperature range from 100.0±3°C to 190.0±3°C;
preferably, the thermogravimetric analysis curve of said crystalline form A of the compound of formula IX is substantially as shown in figure 29;
(49) in said crystalline form A of the compound of formula IX, g is water, d is 1, and h is 3;
(50) said crystalline form B of the compound of formula IX, its X-ray powder diffraction pattern using Cu-Kα radiation, expressed in terms of 2θ angle, has diffraction peaks as shown in table 16;
**table 16**
| **peak** | **diffraction angle (°, 2θ)** | **relative intensity** | **peak** | **diffraction angle (°, 2θ)** | **relative intensity** |
|---|---|---|---|---|---|
| 1 | 5.34 | 100.00% | 21 | 19.47 | 31.00% |
| 2 | 8.20 | 23.30% | 22 | 19.85 | 52.90% |
| 3 | 9.08 | 13.30% | 23 | 21.53 | 18.40% |
| 4 | 9.34 | 63.50% | 24 | 22.14 | 7.00% |
| 5 | 9.92 | 50.50% | 25 | 22.88 | 22.70% |
| 6 | 10.13 | 32.20% | 26 | 24.41 | 31.40% |
| 7 | 11.12 | 16.10% | 27 | 25.43 | 23.50% |
| 8 | 11.47 | 17.90% | 28 | 25.75 | 23.10% |
| 9 | 12.19 | 13.10% | 29 | 26.48 | 23.50% |
| 10 | 12.84 | 54.40% | 30 | 26.83 | 12.40% |
| 11 | 13.18 | 34.90% | 31 | 27.34 | 8.80% |
| 12 | 14.89 | 8.90% | 32 | 27.95 | 19.80% |
| 13 | 15.26 | 13.80% | 33 | 28.26 | 9.20% |
| 14 | 16.13 | 63.90% | 34 | 28.67 | 10.00% |
| 15 | 16.36 | 46.10% | 35 | 29.12 | 9.00% |
| 16 | 16.61 | 73.50% | 36 | 29.96 | 22.10% |
| 17 | 16.89 | 13.20% | 37 | 30.84 | 8.80% |
| 18 | 17.31 | 9.50% | 38 | 32.47 | 14.10% |
| 19 | 18.60 | 37.50% | 39 | 35.25 | 5.30% |
| 20 | 18.67 | 60.70% | | | |
preferably, said crystalline form B of the compound of formula IX, its X-ray powder diffraction pattern using Cu-Kα radiation is substantially as shown in figure 30;
(51) in said crystalline form B of the compound of formula IX, d is 1;
(52) in said crystalline form B of the compound of formula IX, h is 0;
(53) said crystalline form A of the compound of formula X, its X-ray powder diffraction pattern using Cu-Kα radiation, expressed in terms of 2θ angle, has diffraction peaks as shown in table 17;
**table 17**
| **peak** | **diffraction angle (°, 2θ)** | **relative intensity** | **peak** | **diffraction angle (°, 2θ)** | **relative intensity** |
|---|---|---|---|---|---|
| 1 | 5.71 | 7.20% | 25 | 23.85 | 3.90% |
| 2 | 7.46 | 81.90% | 26 | 24.23 | 3.00% |
| 3 | 8.67 | 2.90% | 27 | 24.57 | 20.40% |
| 4 | 11.43 | 25.50% | 28 | 24.99 | 9.80% |
| 5 | 12.01 | 100.00% | 29 | 25.44 | 3.30% |
| 6 | 12.35 | 2.10% | 30 | 26.08 | 8.90% |
| 7 | 13.53 | 27.00% | 31 | 26.59 | 10.50% |
| 8 | 13.70 | 9.00% | 32 | 26.90 | 6.70% |
| 9 | 13.93 | 7.70% | 33 | 28.05 | 5.80% |
| 10 | 14.75 | 5.80% | 34 | 28.44 | 20.50% |
| 11 | 14.91 | 13.20% | 35 | 28.69 | 8.10% |
| 12 | 15.32 | 9.10% | 36 | 28.98 | 6.30% |
| 13 | 17.24 | 28.30% | 37 | 29.33 | 4.20% |
| 14 | 17.71 | 1.60% | 38 | 30.17 | 3.30% |
| 15 | 18.49 | 25.50% | 39 | 30.87 | 6.10% |
| 16 | 18.77 | 21.20% | 40 | 31.21 | 5.40% |
| 17 | 19.02 | 4.70% | 41 | 32.38 | 2.70% |
| 18 | 21.08 | 24.30% | 42 | 33.52 | 2.80% |
| 19 | 21.70 | 49.30% | 43 | 34.64 | 1.80% |
| 20 | 22.12 | 11.90% | 44 | 36.84 | 2.90% |
| 21 | 22.48 | 2.60% | 45 | 37.81 | 2.60% |
| 22 | 22.80 | 10.50% | 46 | 38.41 | 1.90% |
| 23 | 22.95 | 17.80% | 47 | 38.84 | 5.70% |
| 24 | 23.35 | 2.00% | 48 | 39.54 | 2.50% |
preferably, the X-ray powder diffraction pattern of said crystalline form A of the compound of formula X, using Cu-Kα radiation, is substantially as shown in figure 31;
(54) in said crystalline form A of the compound of formula X, k is 0, and j is 1, 1.1, or 1.02, for example 1.02;
(55) said crystalline form B of the compound of formula X, its X-ray powder diffraction pattern using Cu-Kα radiation, expressed in terms of 2θ angle, has diffraction peaks as shown in Table 18;
**table 18**
| **peak** | **diffraction angle (°, 2θ)** | **relative intensity** | **peak** | **diffraction angle (°, 2θ)** | **relative intensity** |
|---|---|---|---|---|---|
| 1 | 5.95 | 20.80% | 18 | 23.79 | 58.00% |
| 2 | 8.96 | 81.20% | 19 | 24.14 | 23.80% |
| 3 | 10.81 | 91.00% | 20 | 24.55 | 42.60% |
| 4 | 11.98 | 46.80% | 21 | 25.57 | 28.00% |
| 5 | 14.09 | 23.00% | 22 | 26.29 | 12.50% |
| 6 | 14.70 | 14.40% | 23 | 26.99 | 3.00% |
| 7 | 15.80 | 48.20% | 24 | 27.26 | 74.60% |
| 8 | 16.97 | 29.20% | 25 | 27.77 | 70.10% |
| 9 | 17.92 | 100.00% | 26 | 29.66 | 34.10% |
| 10 | 18.4 | 64.10% | 27 | 30.28 | 15.00% |
| 11 | 20.03 | 30.00% | 28 | 31.01 | 15.20% |
| 12 | 20.73 | 23.10% | 29 | 32.21 | 15.40% |
| 13 | 21.27 | 45.00% | 30 | 32.53 | 26.80% |
| 14 | 22.07 | 86.10% | 31 | 36.29 | 25.50% |
| 15 | 22.42 | 36.20% | 32 | 37.50 | 16.40% |
| 16 | 22.76 | 32.30% | 33 | 38.64 | 20.10% |
| 17 | 23.27 | 51.70% | 34 | 39.29 | 20.30% |
preferably, the X-ray powder diffraction pattern of said crystalline form B of the compound of formula X, using Cu-Kα radiation, is substantially as shown in figure 32;
(56) in said crystalline form B of the compound of formula X, k is 0, and j is 0.9, 1, or 0.93, for example 0.93;
(57) said crystalline form C of the compound of formula X, its X-ray powder diffraction pattern using Cu-Kα radiation, expressed in terms of 2θ angle, has diffraction peaks as shown in table 19;
**table 19**
| **peak** | **diffraction angle (°, 2θ)** | **relative intensity** | **peak** | **diffraction angle (°, 2θ)** | **relative intensity** |
|---|---|---|---|---|---|
| 1 | 4.65 | -0.90% | 11 | 18.61 | 39.10% |
| 2 | 6.36 | 51.00% | 12 | 20.24 | 26.60% |
| 3 | 9.42 | 16.00% | 13 | 20.94 | 35.50% |
| 4 | 11.30 | 98.80% | 14 | 22.61 | 100.00% |
| 5 | 12.11 | 26.70% | 15 | 24.69 | 71.00% |
| 6 | 12.82 | 55.10% | 16 | 26.16 | 52.30% |
| 7 | 13.65 | 15.50% | 17 | 28.57 | 38.80% |
| 8 | 15.12 | 30.30% | 18 | 31.64 | 9.30% |
| 9 | 17.07 | 40.10% | 19 | 32.75 | 4.40% |
| 10 | 17.48 | 37.10% | | | |
; preferably, the X-ray powder diffraction pattern of said crystalline form C of the compound of formula X, using Cu-Kα radiation, is substantially as shown in figure 33;
(58) in said crystalline form C of the compound of formula X, j is 1;
Preferably, in said crystalline form C of the compound of formula X, k is 0 and j is 1; or, z is acetonitrile and k is 0.07;
(59) said crystalline form D of the compound of formula X, its X-ray powder diffraction pattern using Cu-Kα radiation, expressed in terms of 2θ angle, has diffraction peaks as shown in table 20;
**table 20**
| **peak** | **diffraction angle (°, 2θ)** | **relative intensity** | **peak** | **diffraction angle (°, 2θ)** | **relative intensity** |
|---|---|---|---|---|---|
| 1 | 7.27 | 11.50% | 32 | 24.95 | 30.20% |
| 2 | 7.87 | 7.90% | 33 | 25.38 | 1.20% |
| 3 | 9.25 | 4.50% | 34 | 25.67 | 1.60% |
| 4 | 9.57 | 2.80% | 35 | 26.37 | 2.40% |
| 5 | 10.14 | 1.70% | 36 | 26.69 | 9.50% |
| 6 | 10.68 | 100.00% | 37 | 27.32 | 24.90% |
| 7 | 11.49 | 3.20% | 38 | 28.12 | 6.50% |
| 8 | 12.03 | 3.10% | 39 | 28.42 | 2.00% |
| 9 | 13.33 | 1.60% | 40 | 28.88 | 20.30% |
| 10 | 13.59 | 8.50% | 41 | 29.29 | 1.90% |
| 11 | 14.54 | 6.30% | 42 | 29.54 | 2.90% |
| 12 | 15.55 | 8.20% | 43 | 29.95 | 7.20% |
| 13 | 15.77 | 24.60% | 44 | 30.34 | 3.30% |
| 14 | 16.25 | 1.30% | 45 | 30.63 | 7.00% |
| 15 | 16.5 | 7.40% | 46 | 31.16 | 5.70% |
| 16 | 16.88 | 20.10% | 47 | 31.82 | 8.00% |
| 17 | 17.29 | 4.30% | 48 | 31.99 | 9.20% |
| 18 | 17.66 | 10.70% | 49 | 32.37 | 12.40% |
| 19 | 18.59 | 1.60% | 50 | 32.70 | 5.40% |
| 20 | 19.63 | 7.10% | 51 | 33.04 | 3.30% |
| 21 | 19.82 | 26.00% | 52 | 33.33 | 2.40% |
| 22 | 20.27 | 11.50% | 53 | 34.29 | 4.70% |
| 23 | 21.44 | 73.20% | 54 | 34.76 | 2.60% |
| 24 | 21.76 | 21.90% | 55 | 35.07 | 1.60% |
| 25 | 22.62 | 10.80% | 56 | 36.50 | 2.00% |
| 26 | 23.09 | 9.80% | 57 | 37.34 | 2.30% |
| 27 | 23.37 | 8.90% | 58 | 37.59 | 2.80% |
| 28 | 23.62 | 32.00% | 59 | 37.90 | 8.20% |
| 29 | 24.00 | 6.80% | 60 | 38.56 | 1.20% |
| 30 | 24.33 | 11.80% | 61 | 38.82 | 4.10% |
| 31 | 24.64 | 11.70% | | | |
preferably, the X-ray powder diffraction pattern of said crystalline form D of the compound of formula X, using Cu-Kα radiation, is substantially as shown in figure 34;
(60) said crystalline form D of the compound of formula X, its differential scanning calorimetry curve has endothermic peaks at peak temperatures of 62.55±3°C, 86.97±3°C, and 267.15±3°C;
preferably, the differential scanning calorimetry curve of said crystalline form D of the compound of formula X is substantially as shown in figure 35;
(61) said crystalline form D of the compound of formula X, its thermogravimetric analysis curve shows a weight loss of about 4.49% in the temperature range from 32.27±3°C to 60.0±3°C, a weight loss of about 2.41% in the temperature range from 60.0±3°C to 120.0±3°C, and a weight loss of about 0.6% in the temperature range from 120.0±3°C to 240.0±3°C;
preferably, the thermogravimetric analysis curve of said crystalline form D of the compound of formula X is substantially as shown in figure 36;
(62) in said crystalline form D of the compound of formula X, j is 1 or 1.02, for example 1.02;
(63) in said crystalline form D of the compound of formula X, k is 5, 4.8, or 4, for example 4.8;
preferably, in said crystalline form D of the compound of formula X, z is water, j is 1, and k is 5;or, j is 1.02 and k is 4.8;
(64) said crystalline form A of the compound of formula XI, its X-ray powder diffraction pattern using Cu-Kα radiation and expressed in terms of 2θ angle has diffraction peaks as shown in table 21;
**table 21**
| **peak** | **diffraction angle (°, 2θ)** | **relative intensity** | **peak** | **diffraction angle (°, 2θ)** | **relative intensity** |
|---|---|---|---|---|---|
| 1 | 4.69 | 1.80% | 19 | 23.03 | 1.40% |
| 2 | 5.58 | 2.20% | 20 | 23.58 | 45.80% |
| 3 | 6.9 | 4.30% | 21 | 24.15 | 6.00% |
| 4 | 9.39 | 5.20% | 22 | 26.48 | 1.90% |
| 5 | 10.67 | 37.80% | 23 | 27.17 | 1.00% |
| 6 | 11.15 | 3.10% | 24 | 27.93 | 13.80% |
| 7 | 12.78 | 95.20% | 25 | 28.85 | 2.40% |
| 8 | 14.09 | 100.00% | 26 | 30.21 | 5.60% |
| 9 | 14.98 | 76.20% | 27 | 31.25 | 2.40% |
| 10 | 15.52 | 2.40% | 28 | 32.47 | 3.00% |
| 11 | 15.88 | 0.90% | 29 | 33.25 | 4.70% |
| 12 | 17.22 | 9.30% | 30 | 35.12 | 4.00% |
| 13 | 18.82 | 27.70% | 31 | 36.16 | 2.70% |
| 14 | 19.35 | 9.80% | 32 | 36.70 | 5.60% |
| 15 | 19.49 | 16.80% | 33 | 37.18 | 2.20% |
| 16 | 20.82 | 1.70% | 34 | 38.16 | 4.50% |
| 17 | 21.80 | 15.90% | 35 | 39.03 | 3.10% |
| 18 | 22.45 | 7.40% | | | |
preferably, said crystalline form A of the compound of formula XI, its X-ray powder diffraction pattern using Cu-Kα radiation is substantially as shown in figure 37;
(65) in said crystalline form A of the compound of formula XI, m is 0, and n is 0.6, 0.7, or 0.62, for example 0.62;
(66) said crystalline form B of the compound of formula XI, its X-ray powder diffraction pattern using Cu-Kα radiation and expressed in terms of 2θ angle has diffraction peaks as shown in table 22;
**table 22**
| **peak** | **diffraction angle (°, 2θ)** | **relative intensity** | **peak** | **diffraction angle (°, 2θ)** | **relative intensity** |
|---|---|---|---|---|---|
| 1 | 5.54 | 3.50% | 23 | 22.74 | 6.00% |
| 2 | 6.92 | 5.20% | 24 | 23.05 | 11.60% |
| 3 | 8.22 | 77.00% | 25 | 23.43 | 82.60% |
| 4 | 9.31 | 44.20% | 26 | 24.17 | 43.60% |
| 5 | 9.60 | 13.00% | 27 | 24.77 | 68.40% |
| 6 | 10.63 | 8.80% | 28 | 25.91 | 2.60% |
| 7 | 11.56 | 11.30% | 29 | 26.46 | 14.90% |
| 8 | 12.92 | 46.00% | 30 | 26.86 | 11.40% |
| 9 | 13.32 | 31.20% | 31 | 27.37 | 27.40% |
| 10 | 13.62 | 7.90% | 32 | 29.06 | 11.70% |
| 11 | 13.83 | 15.60% | 33 | 30.04 | 13.70% |
| 12 | 15.13 | 18.50% | 34 | 30.27 | 20.30% |
| 13 | 16.29 | 18.70% | 35 | 30.85 | 20.40% |
| 14 | 17.02 | 8.70% | 36 | 31.40 | 18.40% |
| 15 | 17.63 | 16.60% | 37 | 32.21 | 12.00% |
| 16 | 17.77 | 23.40% | 38 | 33.10 | 10.30% |
| 17 | 18.12 | 33.20% | 39 | 34.32 | 15.80% |
| 18 | 18.63 | 31.00% | 40 | 34.88 | 11.10% |
| 19 | 19.38 | 90.40% | 41 | 36.29 | 2.20% |
| 20 | 20.38 | 100.00% | 42 | 37.62 | 5.40% |
| 21 | 21.04 | 32.30% | 43 | 38.73 | 9.20% |
| 22 | 21.28 | 30.90% | 44 | 39.87 | 5.30% |
preferably, said crystalline form B of the compound of formula XI, its X-ray powder diffraction pattern using Cu-Kα radiation is substantially as shown in figure 38;
(67) in said crystalline form B of the compound of formula XI, n is 1.1, 1.2, or 1.13, for example 1.13;
preferably, in said crystalline form B of the compound of formula XI, n is 1.1 and m is 1.1;or, n is 1.13 and m is 1.07;
(68) said crystalline form C of the compound of formula IX, its X-ray powder diffraction pattern using Cu-Kα radiation and expressed in terms of 2θ angle has diffraction peaks as shown in table 23;
**table 23**
| **peak** | **diffraction angle (°, 2θ)** | **relative intensity** | **peak** | **diffraction angle (°, 2θ)** | **relative intensity** |
|---|---|---|---|---|---|
| 1 | 5.23 | 100.00% | 24 | 19.39 | 32.40% |
| 2 | 5.78 | 5.00% | 25 | 19.82 | 71.20% |
| 3 | 8.07 | 10.80% | 26 | 20.01 | 44.60% |
| 4 | 8.26 | 15.40% | 27 | 20.87 | 9.40% |
| 5 | 9.21 | 38.30% | 28 | 21.52 | 12.70% |
| 6 | 9.80 | 15.20% | 29 | 21.89 | 7.90% |
| 7 | 10.14 | 8.20% | 30 | 22.27 | 15.40% |
| 8 | 10.44 | 6.60% | 31 | 22.76 | 6.20% |
| 9 | 10.92 | 8.20% | 32 | 23.51 | 21.30% |
| 10 | 11.12 | 14.80% | 33 | 23.83 | 12.00% |
| 11 | 11.56 | 4.40% | 34 | 24.28 | 6.90% |
| 12 | 12.72 | 24.10% | 35 | 24.53 | 8.90% |
| 13 | 13.08 | 6.90% | 36 | 25.26 | 12.70% |
| 14 | 14.60 | 4.30% | 37 | 25.50 | 12.60% |
| 15 | 15.24 | 17.70% | 38 | 26.44 | 22.20% |
| 16 | 15.48 | 14.20% | 39 | 26.72 | 8.70% |
| 17 | 16.11 | 35.50% | 40 | 27.34 | 10.20% |
| 18 | 16.57 | 40.00% | 41 | 28.32 | 11.00% |
| 19 | 16.98 | 17.70% | 42 | 29.73 | 9.60% |
| 20 | 17.36 | 6.40% | 43 | 30.06 | 5.40% |
| 21 | 17.89 | 11.00% | 44 | 31.49 | 5.00% |
| 22 | 18.20 | 19.60% | 45 | 32.60 | 5.40% |
| 23 | 18.71 | 15.10% | | | |
preferably, said crystalline form C of the compound of formula IX, its X-ray powder diffraction pattern using Cu-Kα radiation is substantially as shown in figure 39;
(69) in said crystalline form C of the compound of formula IX, d is 1, 1.1, or 1.02, for example 1.02;
preferably, in said crystalline form C of the compound of formula IX, g is tetrahydrofuran, d is 1, and h is 0.2; or, g is tetrahydrofuran, d is 1.02, and h is 0.16; or, d is 1 and h is 0;
(70) said crystalline form D of the compound of formula IX, its X-ray powder diffraction pattern using Cu-Kα radiation and expressed in terms of 2θ angle has diffraction peaks as shown in table 24;
**table 24**
| **peak** | **diffraction angle (°, 2θ)** | **relative intensity** | **peak** | **diffraction angle (°, 2θ)** | **relative intensity** |
|---|---|---|---|---|---|
| 1 | 6.13 | 100.00% | 27 | 23.14 | 7.90% |
| 2 | 8.52 | 16.50% | 28 | 24.00 | 19.80% |
| 3 | 10.19 | 4.60% | 29 | 24.27 | 8.20% |
| 4 | 10.87 | 27.30% | 30 | 25.28 | 0.90% |
| 5 | 12.18 | 22.40% | 31 | 25.55 | 1.10% |
| 6 | 12.59 | 3.10% | 32 | 25.79 | 2.10% |
| 7 | 13.12 | 19.70% | 33 | 26.01 | 1.20% |
| 8 | 13.40 | 1.40% | 34 | 26.47 | 3.20% |
| 9 | 14.25 | 14.00% | 35 | 26.93 | 3.20% |
| 10 | 14.42 | 10.90% | 36 | 27.44 | 2.30% |
| 11 | 15.42 | 10.40% | 37 | 27.87 | 2.30% |
| 12 | 16.60 | 24.70% | 38 | 28.33 | 12.30% |
| 13 | 17.00 | 5.80% | 39 | 28.82 | 5.10% |
| 14 | 17.57 | 1.60% | 40 | 29.35 | 3.00% |
| 15 | 17.84 | 3.60% | 41 | 29.78 | 2.70% |
| 16 | 18.38 | 33.50% | 42 | 30.73 | 1.80% |
| 17 | 19.09 | 11.90% | 43 | 31.20 | 2.10% |
| 18 | 19.34 | 13.10% | 44 | 31.96 | 1.30% |
| 19 | 19.64 | 3.30% | 45 | 32.52 | 2.10% |
| 20 | 20.07 | 20.90% | 46 | 34.49 | 0.80% |
| 21 | 20.39 | 3.10% | 47 | 35.57 | 0.20% |
| 22 | 21.12 | 8.40% | 48 | 36.00 | 1.70% |
| 23 | 21.28 | 8.20% | 49 | 37.01 | 0.90% |
| 24 | 21.59 | 10.60% | 50 | 37.65 | 1.40% |
| 25 | 21.72 | 8.50% | 51 | 38.27 | 2.00% |
| 26 | 22.10 | 2.30% | 52 | 39.07 | 1.30% |
preferably, said crystalline form D of the compound of formula IX, its X-ray powder diffraction pattern using Cu-Kα radiation is substantially as shown in figure 40;
(71) in said crystalline form D of the compound of formula IX, d is 1;
Preferably, in said crystalline form D of the compound of formula IX, g is water, d is 1, and h is 0, 1, or 3;
(72) said crystalline form E of the compound of formula IX, its X-ray powder diffraction pattern using Cu-Kα radiation and expressed in terms of 2θ angle has diffraction peaks as shown in table 25;
**table 25**
| **peak** | **diffraction angle (°, 2θ)** | **relative intensity** | **peak** | **diffraction angle (°, 2θ)** | **relative intensity** |
|---|---|---|---|---|---|
| 1 | 5.11 | 1.20% | 34 | 23.95 | 89.20% |
| 2 | 5.66 | 78.90% | 35 | 24.46 | 8.70% |
| 3 | 8.11 | 4.90% | 36 | 25.05 | 20.10% |
| 4 | 8.45 | 14.30% | 37 | 25.49 | 9.20% |
| 5 | 9.02 | 22.30% | 38 | 25.85 | 14.10% |
| 6 | 9.84 | 12.30% | 39 | 26.14 | 11.30% |
| 7 | 11.19 | 25.00% | 40 | 26.58 | 76.50% |
| 8 | 11.50 | 65.80% | 41 | 26.98 | 25.20% |
| 9 | 12.29 | 7.80% | 42 | 27.86 | 35.60% |
| 10 | 12.70 | 13.30% | 43 | 28.05 | 27.00% |
| 11 | 13.85 | 36.70% | 44 | 28.34 | 4.30% |
| 12 | 14.30 | 41.20% | 45 | 28.86 | 29.50% |
| 13 | 15.36 | 5.00% | 46 | 29.05 | 5.60% |
| 14 | 15.59 | 14.80% | 47 | 29.72 | 13.70% |
| 15 | 16.20 | 8.60% | 48 | 30.36 | 17.10% |
| 16 | 16.56 | 23.50% | 49 | 30.58 | 32.90% |
| 17 | 16.93 | 6.90% | 50 | 30.95 | 6.40% |
| 18 | 17.18 | 18.90% | 51 | 31.54 | 6.50% |
| 19 | 17.74 | 68.20% | 52 | 31.74 | 5.10% |
| 20 | 17.97 | 92.90% | 53 | 32.45 | 6.10% |
| 21 | 18.75 | 22.60% | 54 | 32.80 | 2.10% |
| 22 | 19.04 | 64.50% | 55 | 33.23 | 5.10% |
| 23 | 19.34 | 17.70% | 56 | 33.69 | 4.10% |
| 24 | 19.52 | 18.90% | 57 | 34.65 | 1.80% |
| 25 | 19.83 | 100.00% | 58 | 35.36 | 3.40% |
| 26 | 20.55 | 12.80% | 59 | 36.12 | 3.30% |
| 27 | 21.07 | 3.60% | 60 | 36.55 | 8.60% |
| 28 | 21.37 | 5.60% | 61 | 37.16 | 3.80% |
| 29 | 21.93 | 47.00% | 62 | 37.52 | 7.80% |
| 30 | 22.25 | 58.60% | 63 | 38.01 | 11.40% |
| 31 | 22.47 | 15.70% | 64 | 38.72 | 6.20% |
| 32 | 23.08 | 40.90% | 65 | 39.56 | 2.40% |
| 33 | 23.43 | 53.10% | | | |
preferably, said crystalline form E of the compound of formula IX, its X-ray powder diffraction pattern using Cu-Kα radiation is substantially as shown in figure 41;
(73) said crystalline form E of the compound of formula IX, its differential scanning calorimetry curve has endothermic peaks at peak temperatures of 102.94±3°C and 183.71±3°C; preferably, the differential scanning calorimetry curve of said crystalline form E of the compound of formula IX is substantially as shown in figure 42;
(74) said crystalline form E of the compound of formula IX, its thermogravimetric analysis curve shows a weight loss of about 3.11% in the temperature range from 33.06±3°C to 55.0±3°C, a weight loss of about 3.82% in the temperature range from 55±3°C to 120.0±3°C, and a weight loss of about 1.21% in the temperature range from 120.0±3°C to 190.0±3°C;
preferably, the thermogravimetric analysis curve of said crystalline form E of the compound of formula IX is substantially as shown in figure 43;
(75) in said crystalline form E of the compound of formula IX, d is 1, 1.1, or 1.02, for example 1.02;
preferably, in said crystalline form E of the compound of formula IX, d is 1, g is isopropanol, and h is 1;or, d is 1.02, g is isopropanol, and h is 0.98;
(76) said crystalline form F of the compound of formula IX, its X-ray powder diffraction pattern using Cu-Kα radiation, expressed in terms of 2θ angle, has diffraction peaks as shown in table 26;
**table 26**
| **peak** | **diffraction angle (°, 2θ)** | **relative intensity** | **peak** | **diffraction angle (°, 2θ)** | **relative intensity** |
|---|---|---|---|---|---|
| 1 | 6.15 | 55.40% | 39 | 23.46 | 3.70% |
| 2 | 6.99 | 9.10% | 40 | 24.03 | 2.60% |
| 3 | 9.91 | 0.40% | 41 | 24.34 | 60.90% |
| 4 | 10.34 | 26.50% | 42 | 24.64 | 3.60% |
| 5 | 10.75 | 0.80% | 43 | 25.25 | 10.30% |
| 6 | 11.52 | 4.60% | 44 | 25.74 | 45.90% |
| 7 | 11.85 | 12.00% | 45 | 26.02 | 20.90% |
| 8 | 12.27 | 8.20% | 46 | 26.65 | 6.20% |
| 9 | 12.57 | 8.70% | 47 | 26.83 | 26.20% |
| 10 | 12.73 | 1.30% | 48 | 27.27 | 6.30% |
| 11 | 13.35 | 5.50% | 49 | 27.87 | 7.20% |
| 12 | 13.97 | 15.30% | 50 | 28.13 | 9.90% |
| 13 | 14.14 | 2.80% | 51 | 28.46 | 13.30% |
| 14 | 15.52 | 4.30% | 52 | 28.74 | 1.30% |
| 15 | 15.73 | 24.80% | 53 | 29.71 | 7.40% |
| 16 | 16.34 | 26.30% | 54 | 29.99 | 2.10% |
| 17 | 16.55 | 67.90% | 55 | 30.19 | 1.50% |
| 18 | 16.73 | 5.50% | 56 | 30.52 | 1.60% |
| 19 | 17.08 | 5.00% | 57 | 30.72 | 4.30% |
| 20 | 17.30 | 86.30% | 58 | 31.28 | 18.20% |
| 21 | 17.73 | 0.90% | 59 | 31.73 | 4.60% |
| 22 | 18.07 | 1.30% | 60 | 32.33 | 1.90% |
| 23 | 18.42 | 17.40% | 61 | 32.70 | 4.00% |
| 24 | 18.92 | 7.80% | 62 | 32.98 | 4.30% |
| 25 | 19.37 | 0.70% | 63 | 33.24 | 8.80% |
| 26 | 19.82 | 0.50% | 64 | 33.38 | 9.50% |
| 27 | 20.42 | 41.00% | 65 | 33.68 | 7.50% |
| 28 | 20.72 | 39.90% | 66 | 34.42 | 8.60% |
| 29 | 20.98 | 23.30% | 67 | 35.05 | 7.20% |
| 30 | 21.22 | 26.20% | 68 | 35.67 | 6.40% |
| 31 | 21.48 | 100.00% | 69 | 36.24 | 1.10% |
| 32 | 21.83 | 19.90% | 70 | 36.57 | 1.80% |
| 33 | 21.97 | 18.40% | 71 | 36.88 | 1.10% |
| 34 | 22.24 | 8.20% | 72 | 37.36 | 6.30% |
| 35 | 22.43 | 6.30% | 73 | 38.14 | 3.10% |
| 36 | 22.69 | 1.80% | 74 | 38.48 | 6.40% |
| 37 | 22.97 | 8.10% | 75 | 39.12 | 1.70% |
| 38 | 23.27 | 4.20% | | | |
preferably, said crystalline form F of the compound of formula IX, its X-ray powder diffraction pattern using Cu-Kα radiation is substantially as shown in figure 44;
(77) said crystalline form F of the compound of formula IX, its differential scanning calorimetry curve has an endothermic peak at a peak temperature of 266.84±3°C;
preferably, the differential scanning calorimetry curve of said crystalline form F of the compound of formula IX is substantially as shown in figure 45;
(78) said crystalline form F of the compound of formula IX, its thermogravimetric analysis curve shows a weight loss of about 2.2% in the temperature range from 34.19±3°C to 260.0±3°C;
preferably, the thermogravimetric analysis curve of said crystalline form F of the compound of formula IX is substantially as shown in figure 46;
(79) in said crystalline form F of the compound of formula IX, d is 1, 1.1, or 1.02, for example 1.02;
preferably, in said crystalline form F of the compound of formula IX, d is 1, and h is 0;
(80) said crystalline form G of the compound of formula IX, its X-ray powder diffraction pattern using Cu-Kα radiation, expressed in terms of 2θ angle, has diffraction peaks as shown in table 27;
**table 27**
| **peak** | **diffraction angle (°, 2θ)** | **relative intensity** | **peak** | **diffraction angle (°, 2θ)** | **relative intensity** |
|---|---|---|---|---|---|
| 1 | 7.65 | 25.60% | 26 | 23.10 | 7.00% |
| 2 | 9.66 | 3.90% | 27 | 23.51 | 6.80% |
| 3 | 10.73 | 1.20% | 28 | 23.83 | 23.40% |
| 4 | 11.10 | 1.80% | 29 | 24.47 | 3.10% |
| 5 | 11.87 | 7.90% | 30 | 25.37 | 100.00% |
| 6 | 12.60 | 8.50% | 31 | 25.63 | 35.80% |
| 7 | 12.76 | 8.00% | 32 | 26.65 | 12.20% |
| 8 | 13.76 | 28.70% | 33 | 27.17 | 8.70% |
| 9 | 14.11 | 5.20% | 34 | 27.60 | 51.60% |
| 10 | 16.07 | 13.10% | 35 | 28.36 | 8.50% |
| 11 | 17.04 | 3.70% | 36 | 29.17 | 15.80% |
| 12 | 17.43 | 5.10% | 37 | 29.89 | 2.90% |
| 13 | 17.64 | 11.10% | 38 | 31.46 | 4.60% |
| 14 | 17.82 | 8.60% | 39 | 31.79 | 9.50% |
| 15 | 18.41 | 8.90% | 40 | 32.38 | 2.80% |
| 16 | 18.75 | 6.10% | 41 | 33.26 | 6.70% |
| 17 | 18.97 | 9.60% | 42 | 33.57 | 4.70% |
| 18 | 19.41 | 2.20% | 43 | 33.86 | 3.80% |
| 19 | 20.05 | 12.10% | 44 | 34.25 | 6.50% |
| 20 | 20.35 | 40.00% | 45 | 34.78 | 2.50% |
| 21 | 21.01 | 49.00% | 46 | 36.21 | 2.60% |
| 22 | 21.31 | 10.50% | 47 | 37.19 | 1.70% |
| 23 | 21.57 | 63.70% | 48 | 38.07 | 4.70% |
| 24 | 22.23 | 2.10% | 49 | 38.41 | 3.40% |
| 25 | 22.75 | 15.20% | 50 | 38.74 | 1.90% |
preferably, said crystalline form G of the compound of formula IX, its X-ray powder diffraction pattern using Cu-Kα radiation is substantially as shown in figure 47;
(81) in said crystalline form G of the compound of formula IX, d is 1, 1.1, or 1.2, for example 1.1;
preferably, in said crystalline form G of the compound of formula IX, d is 1, g is 1,4-dioxane, and h is 3, or, d is 1.1, g is 1,4-dioxane, and h is 2.96;
(82) said crystalline form H of the compound of formula IX, its X-ray powder diffraction pattern using Cu-Kα radiation, expressed in terms of 2θ angle, has diffraction peaks as shown in table 28;
**table 28**
| **peak** | **diffraction angle (°, 2θ)** | **relative intensity** | **peak** | **diffraction angle (°, 2θ)** | **relative intensity** |
|---|---|---|---|---|---|
| 1 | 5.21 | 14.10% | 24 | 22.44 | 11.80% |
| 2 | 8.27 | 27.00% | 25 | 23.36 | 16.20% |
| 3 | 9.70 | 29.10% | 26 | 24.46 | 4.60% |
| 4 | 10.39 | 9.80% | 27 | 24.92 | 4.80% |
| 5 | 11.07 | 17.20% | 28 | 25.23 | 30.70% |
| 6 | 11.60 | 11.30% | 29 | 26.14 | 26.00% |
| 7 | 12.56 | 18.00% | 30 | 26.33 | 19.10% |
| 8 | 13.05 | 4.30% | 31 | 27.27 | 16.00% |
| 9 | 14.96 | 4.00% | 32 | 27.95 | 16.80% |
| 10 | 15.29 | 10.80% | 33 | 28.31 | 7.50% |
| 11 | 16.55 | 43.40% | 34 | 29.56 | 23.20% |
| 12 | 17.07 | 23.20% | 35 | 30.05 | 10.70% |
| 13 | 17.46 | 2.80% | 36 | 30.38 | 10.10% |
| 14 | 18.08 | 2.20% | 37 | 30.87 | 9.50% |
| 15 | 18.49 | 11.50% | 38 | 31.27 | 4.00% |
| 16 | 18.79 | 19.70% | 39 | 32.21 | 12.70% |
| 17 | 19.09 | 29.50% | 40 | 33.40 | 1.80% |
| 18 | 19.78 | 100.00% | 41 | 35.02 | 2.10% |
| 19 | 20.81 | 9.10% | 42 | 36.28 | 3.00% |
| 20 | 21.12 | 15.80% | 43 | 37.31 | 2.10% |
| 21 | 21.46 | 23.00% | 44 | 38.27 | 3.70% |
| 22 | 21.93 | 3.50% | 45 | 38.92 | 2.80% |
| 23 | 22.24 | 7.90% | | | |
preferably, said crystalline form H of the compound of formula IX, its X-ray powder diffraction pattern using Cu-Kα radiation is substantially as shown in figure 48;
(83) said crystalline form H of the compound of formula IX, its differential scanning calorimetry curve has endothermic peaks at peak temperatures of 45.53±3°C, 155.23±3°C, and 191.45±3°C;
preferably, the differential scanning calorimetry curve of said crystalline form H of the compound of formula IX is substantially as shown in figure 49;
(84) said crystalline form H of the compound of formula IX, its thermogravimetric analysis curve shows a weight loss of about 1.74% in the temperature range from 32.99±3°C to 100.0±3°C, and a weight loss of about 5.38% in the temperature range from 100±3°C to 180.0±3°C;
preferably, the thermogravimetric analysis curve of said crystalline form H of the compound of formula IX is substantially as shown in figure 50;
(85) said crystalline form H of the compound of formula IX, wherein g is methyl tert-butyl ether, and h is 0.5, 0.4, or 0.45, for example 0.45;
(86) said crystalline form H of the compound of formula IX, wherein d is 1;
preferably, in said crystalline form H of the compound of formula IX, g is methyl tert-butyl ether, h is 0.5, and d is 1;
(87) said crystalline form I of the compound of formula IX, its X-ray powder diffraction pattern using Cu-Kα radiation, expressed in terms of 2θ angle, has diffraction peaks as shown in table 29;
**table 29**
| **peak** | **diffraction angle (°, 2θ)** | **relative intensity** | **peak** | **diffraction angle (°, 2θ)** | **relative intensity** |
|---|---|---|---|---|---|
| 1 | 6.01 | 100.00% | 21 | 22.21 | 2.80% |
| 2 | 8.34 | 2.70% | 22 | 22.46 | 7.20% |
| 3 | 10.02 | 4.60% | 23 | 22.68 | 3.60% |
| 4 | 10.63 | 16.60% | 24 | 23.22 | 14.60% |
| 5 | 11.08 | 3.70% | 25 | 24.06 | 10.00% |
| 6 | 11.99 | 7.00% | 26 | 24.73 | 2.20% |
| 7 | 12.4 | 5.80% | 27 | 25.35 | 5.30% |
| 8 | 12.79 | 18.40% | 28 | 25.66 | 3.90% |
| 9 | 14.20 | 14.50% | 29 | 26.08 | 4.80% |
| 10 | 15.04 | 6.30% | 30 | 26.37 | 3.40% |
| 11 | 16.67 | 26.20% | 31 | 27.68 | 5.90% |
| 12 | 17.84 | 17.20% | 32 | 28.08 | 6.60% |
| 13 | 18.00 | 19.30% | 33 | 28.81 | 2.00% |
| 14 | 18.47 | 3.40% | 34 | 29.18 | 4.90% |
| 15 | 18.74 | 8.60% | 35 | 30.92 | 2.90% |
| 16 | 19.54 | 1.30% | 36 | 31.84 | 1.40% |
| 17 | 20.07 | 11.90% | 37 | 32.99 | 1.00% |
| 18 | 20.42 | 1.30% | 38 | 33.72 | 1.50% |
| 19 | 20.90 | 3.50% | 39 | 37.94 | 2.80% |
| 20 | 21.20 | 4.40% | 40 | 39.25 | 1.80% |
preferably, said crystalline form I of the compound of formula IX, its X-ray powder diffraction pattern using Cu-Kα radiation is substantially as shown in figure 51;
(88) said crystalline form I of the compound of formula IX, wherein d is 1;
(89) in said crystalline form I of the compound of formula IX, h is 0;
preferably, in said crystalline form I of the compound of formula IX, d is 1 and h is 0;
(90) said crystalline form J of the compound of formula IX, its X-ray powder diffraction pattern using Cu-Kα radiation, expressed in terms of 2θ angle, has diffraction peaks as shown in table 30;
**table 30**
| **peak** | **diffraction angle (°, 2θ)** | **relative intensity** | **peak** | **diffraction angle (°, 2θ)** | **relative intensity** |
|---|---|---|---|---|---|
| 1 | 5.27 | 57.30% | 13 | 19.07 | 35.50% |
| 2 | 8.18 | 50.00% | 14 | 19.66 | 35.70% |
| 3 | 9.72 | 40.00% | 15 | 20.21 | 62.00% |
| 4 | 10.54 | 39.80% | 16 | 21.25 | 27.60% |
| 5 | 11.06 | 100.00% | 17 | 22.33 | 2.00% |
| 6 | 11.68 | 12.90% | 18 | 23.07 | 20.40% |
| 7 | 12.44 | 84.30% | 19 | 25.08 | 0.50% |
| 8 | 12.88 | 36.10% | 20 | 26.48 | 35.00% |
| 9 | 15.30 | 32.70% | 21 | 27.52 | 7.20% |
| 10 | 16.41 | 32.30% | 22 | 28.16 | 20.90% |
| 11 | 17.77 | 8.00% | 23 | 29.27 | 20.40% |
| 12 | 18.79 | 95.30% | | | |
preferably, said crystalline form J of the compound of formula IX, its X-ray powder diffraction pattern using Cu-Kα radiation is substantially as shown in figure 52;
(91) said crystalline form J of the compound of formula IX, wherein d is 1;
Preferably, in said crystalline form J of the compound of formula IX, d is 1 and h is 0.

6. The nitrogen-containing fused-ring compound according to claim 1, **characterized in that** said compound of formula IX is a single crystal of the compound of formula IX, wherein g is water, h is 3, and d is 1;
it has the following unit cell parameters: triclinic crystal system, space group P-1;a = 10.6990(12) Å, α = 89.299(7)°, b = 10.7640(13) Å, β = 86.519(6)°, c = 14.9358(17) Å, γ = 84.364(6)°, unit cell volume = 1708.6(3) Å³, number of asymmetric units per unit cell Z = 2, crystal density is 1.46 mg/m³.

7. A method for preparing the crystalline form C of the compound of formula I according to any one of claims 3-6 or said nitrogen-containing fused-ring compound, **characterized in that** it is any one of the following schemes:
scheme 1:
scheme 1 comprises the following steps: cooling a mixture of a compound and a solvent to induce crystallization, and drying to obtain a crystalline form;
wherein said compound is said compound of formula II, the solvent is a mixed solvent of acetone and water, to obtain crystalline form B of the compound of formula II;
wherein said compound is said compound of formula II, the solvent is ethyl acetate or acetonitrile, to obtain crystalline form C of the compound of formula I;
wherein said compound is said compound of formula III, the solvent is ethyl acetate, to obtain crystalline form A or crystalline form B of the compound of formula III;
wherein said compound is said compound of formula III, the solvent is acetonitrile, to obtain crystalline form C of the compound of formula III;
wherein said compound is said compound of formula IV, the solvent is ethyl acetate, to obtain crystalline form A of the compound of formula IV;
wherein said compound is said compound of formula IV, the solvent is acetonitrile, to obtain crystalline form B of the compound of formula IV;
wherein said compound is said compound of formula V, the solvent is ethyl acetate, to obtain crystalline form A of the compound of formula V;
wherein said compound is said compound of formula V, the solvent is acetonitrile, to obtain crystalline form B of the compound of formula V;
wherein said compound is said compound of formula V, the solvent is acetonitrile, and crystalline form B of the compound of formula V is added to said mixture, to obtain crystalline form C of the compound of formula V;
wherein said compound is said compound of formula VI, the solvent is acetonitrile, to obtain crystalline form A of the compound of formula VI;
wherein said compound is said compound of formula VII, the solvent is acetonitrile, to obtain crystalline form A of the compound of formula VII;
wherein said compound is said compound of formula VIII, the solution is ethyl acetate, to obtain crystalline form A of the compound of formula VIII;
wherein said compound is said compound of formula IX, the solution is a mixed solution of acetone and water, to obtain crystalline form A of the compound of formula IX;
wherein said compound is said compound of formula IX, the solution is acetonitrile, to obtain crystalline form B of the compound of formula IX;
wherein said compound is said compound of formula X, the solvent is a mixed solution of acetone and water, to obtain crystalline form A of the compound of formula X;
wherein said compound is said compound of formula X, the solvent is ethyl acetate, to obtain crystalline form B of the compound of formula X;
wherein said compound is said compound of formula X, the solvent is acetonitrile, to obtain crystalline form C of the compound of formula X;
wherein said compound is said compound of formula X, the solvent is a mixed solution of acetone and water, to obtain crystalline form D of the compound of formula X;
wherein said compound is said compound of formula XI, the solvent is ethyl acetate, to obtain crystalline form A of the compound of formula XI;
wherein said compound is said compound of formula XI, the solvent is acetonitrile, to obtain crystalline form B of the compound of formula XI;
scheme 2,
scheme 2 comprises the following steps: subjecting a mixture of a compound and a solvent to crystallization and drying to obtain a crystalline form;
wherein said compound is crystalline form A of said compound of formula IX, the solution is tetrahydrofuran, to obtain crystalline form C of the compound of formula IX;
wherein said compound is crystalline form A of said compound of formula IX, the solution is isopropanol, to obtain crystalline form E of the compound of formula IX;
wherein said compound is crystalline form A of said compound of formula IX, the solution is acetonitrile and n-heptane, to obtain crystalline form F of the compound of formula IX;
wherein said compound is crystalline form A of said compound of formula IX, the solution is 1,4-dioxane, to obtain crystalline form G of the compound of formula IX;
wherein said compound is crystalline form A of said compound of formula IX, the solution is a mixed solution of acetonitrile and methyl tert-butyl ether, to obtain crystalline form H of the compound of formula IX;
scheme 3:
scheme 3 comprises the following steps: under nitrogen protection, heating said crystalline form A of the compound of formula IX at 55°C to obtain crystalline form D of the compound of formula IX;
or, under nitrogen protection, heating said crystalline form A of the compound of formula IX at 25°C to obtain crystalline form I of the compound of formula IX;
or, under nitrogen protection, heating said crystalline form H of the compound of formula IX at 170°C to obtain crystalline form J of the compound of formula IX;
scheme 4:
scheme 4 comprises the following steps:
step (1): mixing a mixture of the compound of formula I, acetone, and water with crystalline form A of the compound of formula IX;
step (2): adding a mixture of toluene-4-sulfonic acid, acetone and water, and crystallizing to obtain a single crystal of the compound of formula IX.

8. The preparation method according to claim 7, **characterized in that** it satisfies one or more of the following conditions:
(1) said cooling crystallization is cooling from 50°C to 25°C;
(2) said mixed solution of acetone and water is a mixed solution of acetone and water in a volume ratio of 19: 1;
(3) said drying is centrifugation and/or vacuum drying;
(4) in step (1), the mass ratio of said compound of formula I to acetone is (0.5-2):(8-12), for example 1.63:10.3;
(5) in step (1), the mass ratio of said acetone to water is (9-11):(0.5-1.5), for example 10.3:1.3;
(6) in step (1), the mass ratio of said crystalline form A of the compound of formula IX to said compound of formula I is 1:100 to 1:10, for example 8:163;
(7) in step (2), the mass ratio of said toluene-4-sulfonic acid, said acetone and said water is (1-2.5):(5-10):(0.5-1.5), for example 0.58:2.57:0.32;
(8) in step (2), said toluene-4-sulfonic acid is toluene-4-sulfonic acid monohydrate;
(9) in step (2), said mixture is added in two portions, preferably the first addition is performed at 45-55°C, and the mass ratio of said toluene-4-sulfonic acid to said compound of formula I is 1:(2-4), for example 0.58:1.63;the second addition is performed at 20-30°C, and the mass ratio of said toluene-4-sulfonic acid to said compound of formula I is 1:(4-8), for example 0.29:1.63.

9. A pharmaceutical composition comprising the compound of formula II, the compound of formula III, the compound of formula IV, the compound of formula V, the compound of formula VI, the compound of formula VII, the compound of formula VIII, the compound of formula IX, the compound of formula X, or the compound of formula XI according to any one of claims 1-6, or crystalline form C of the compound of formula I according to any one of claims 3-5, and a pharmaceutically acceptable excipient.

10. Use of the pharmaceutical composition according to claim 9, or the compound of formula II, the compound of formula III, the compound of formula IV, the compound of formula V, the compound of formula VI, the compound of formula VII, the compound of formula VIII, the compound of formula IX, the compound of formula X, or the compound of formula XI according to any one of claims 1-6, or crystalline form C of the compound of formula I according to any one of claims 3-5, in the manufacture of a medicament, wherein said medicament is for treating lung cancer, breast cancer, HR-deficient ovarian cancer, gastric cancer, prostate cancer, pancreatic cancer, or colon cancer.
